(19) Europäisches Patentamt
European Patent Office
Office européen des brevets

(11) EP 3 977 982 A1

(12) EUROPEAN PATENT APPLICATION

(43) Date of publication:
**06.04.2022 Bulletin 2022/14**

(21) Application number: **21191386.8**

(22) Date of filing: **15.05.2012**

(51) International Patent Classification (IPC):
***A61K 9/00*** *(2006.01)* ***A61K 9/06*** *(2006.01)*
***A61K 31/568*** *(2006.01)* ***A61K 47/34*** *(2017.01)*
***A61K 47/44*** *(2017.01)* ***A61P 5/26*** *(2006.01)*

(52) Cooperative Patent Classification (CPC):
**A61K 47/34; A61K 9/0043; A61K 31/568; A61K 47/44; A61P 5/24; A61P 5/26; A61P 15/00; A61P 15/02; A61P 15/08; A61P 15/10; A61P 25/24;** A61M 31/00

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.05.2011 US 201161486324 P**
**16.05.2011 US 201161486634 P**

(62) Document number(s) of the earlier application(s) in accordance with Art. 76 EPC:
**12738595.3 / 2 709 588**

(71) Applicant: **Acerus Biopharma Inc.**
**Mississauga, ON L5L 1J9 (CA)**

(72) Inventors:
- **KREPPNER, Wayne**
  **Georgetown, L7G 5H1 (CA)**
- **FOGARTY, Siobhan**
  **Blackrock (IE)**
- **OBEREGGER, Werner**
  **Waterdown, L0R 2H4 (CA)**
- **MAES, Paul, José, Pierre, Marie**
  **Visé (BE)**

(74) Representative: **Roberts, Michael Austin**
**Reddie & Grose LLP**
**The White Chapel Building**
**10 Whitechapel High Street**
**London E1 8QS (GB)**

Remarks:
•This application was filed on 13-08-2021 as a divisional application to the application mentioned under INID code 62.
•Claims filed after the date of filing of the application (Rule 68(4) EPC).

# (54) CONTROLLED RELEASE NASAL TESTOSTERONE GELS, METHODS AND PRE-FILLED MULTI-DOSE APPLICATOR SYSTEMS FOR PERNASAL ADMINISTRATION

(57) The present invention relates to intranasal testosterone gels for the controlled release of testosterone into the systemic circulation of males and females for providing constant effective testosterone blood levels, without inducing undesired testosterone spike in blood levels, following pernasal administration. The intranasal testosterone gels of the present invention are safe, convenient to use, well tolerated, stable and easily and reproducibly manufactured on scale up. Moreover, because supra- normal and sub-normal testosterone blood levels are believed to be essentially kept to a minimum or avoided and the testosterone serum levels are believed to remain essentially constant during dose life, i.e., the intranasal testosterone gels of the present invention mimic or restore testosterone blood levels to normal physiologic daily rhythmic testosterone levels, the novel intranasal testosterone gels of the present invention are uniquely suited for testosterone replacement or supplemental therapy and effective for treating males diagnosed with, for example, male testosterone deficiency, such as, low sexual libido, low sexual drive, low sexual activity, low fertility, low spermatogenesis, aspermatogenesis, depression and/or hypogonadism, and females who are diagnosed with, for example, female sexual dysfunction, such as, low sexual libido, low sexual drive, low sexual activity, low amygdala reactivity, low sexual stimulation, hypoactive sexual desire disease ("HSDD"), female sexual arousal disorder and/or anorgasmia. The present invention also relates to methods and pre- filled multi-dose airless applicator systems for pernasal administration of the nasal testosterone gels of the present invention.

EP 3 977 982 A1

## Description

This is a divisional application of European patent application no. 12738595.3.

## Field of the Invention

The present invention relates to intranasal testosterone gels for the controlled release of testosterone into the systemic circulation of males and females after pernasal application for providing constant effective testosterone blood levels, without causing unwanted testosterone blood level spikes, over dose life, which are suitable for use in testosterone replacement or supplemental therapy and effective to treat males and females in need of testosterone replacement or testosterone supplemental therapy to treat, for example, male testosterone deficiency or female sexual dysfunction. The present invention also relates to methods and pre-filled multi-dose applicator systems for pernasal administration of the nasal testosterone gels.

## Background

Nasal drug delivery offers many advantages that include rapid adsorption due to abundant capillary vessels, fast onset of action, avoidance of hepatic first-pass metabolism, utility for chronic medication and ease of administration.

It is known that, in contrast to large and/or ionized molecules, lipophilic pharmaceutical compounds having a sufficiently low molecular weight in general are readily adsorbed by the mucous membrane of the nose. For such drugs it is possible to obtain pharmacokinetic profiles similar to those obtained after intravenous injection.

However, maintaining constant in vivo therapeutic drug concentrations for an extended period of time has been problematic because of the rapid mucociliary clearance of the therapeutic agent from the site of deposition resulting in a short span of time available for absorption and of the presence of enzymes that may cause degradation in the nasal cavity.

Effort has been made to overcome these limitations including the use of bioadhesive systems that increase residence time in the nasal cavity, the use of enhancers to improve permeability of the nasal membrane or the use of stabilizers that prevent degradation of drugs.

For example, in GB 1987000012176, the use of bioadhesive microspheres has been proposed by Illum and, in PCT/GB98/01147, the use of in-situ gelling pectin formulations by West Pharmaceuticals.

Investigations on the nasal absorption of sexual steroids, rather small and lipophilic compounds, have shown that they are readily absorbed by the mucous membrane of the nose and are found very quickly in serum. Due to this fact, to the short half-life of the compounds and to limited possibilities for formulating nasal application forms with sustained release, sexual steroid use in clinical practice has been limited up to now, because hormone replacement therapy, in general, is a long-term application.

Several formulations were proposed for these drugs. Thus, in the case of testosterone, which is nearly water-insoluble and somewhat better in vegetable oil, Hussain et al., "Testosterone 17β-N,N-dimethylglyc-inate hydrochloride: A prodrug with a potential for nasal delivery of testosterone", J. Pharmaceut. Sci. 91(3): 785-789 (2002), suggested that testosterone might be an ideal candidate for nasal administration, if its solubility in water could be increased. Hussain et al therefore proposed the use of a water-soluble pro-drug, testosterone 17β-N,N-dimethylglycinate, and found serum levels equal to intravenous administration with peak plasma concentrations within 12 min (25 mg dose) and 20 min (50 mg dose), respectively, and elimination half-lives of about 55 min. It should be mentioned that this speed is not necessary/desirable because sex hormone replacement or supplemental therapy is not an emergency therapy requiring peak plasma concentations immediately following adminsitration.

Ko et al., "Emulsion formulations of testosterone for nasal administration", J. Microencaps., 15(2): 197-205 (1998), proposed the use of charged testosterone submicron O/W emulsion formulations (water/Tween80, soybean oil/Span80) based on the hypothesis that increased absorption is possible upon solubilization of the drug and/or prolongation of the formulation residence time in the nose. Ko et al. found a higher relative bioavailability of the positively (55%) and negatively (51%) charged emulsion compared to the neutral one (37%). $T_{max}$ was observed in every case at about 20 min after administration. It is difficult to assess these results because Ko et al. did not take blood samples before application and thus it is not possible to evaluate the differences in the decrease of serum levels, although from a graph it seems that, after intravenous application (hydroalcoholic solution), the level shows the longest elimination half time. In practice, however, such an emulsion is not suitable because the amount of surfactant needed to achieve the droplet size (430 nm) is not acceptable for nasal application.

The solubility of progesterone in water and oil is somewhat comparable to that of testosterone, but investigators have had different approaches.

For example, Cicinelli et al., "Progesterone administration by nasal spray", Fertil Steril 56(1): 139-141 (1991), "Nasally-administered progesterone: comparison of ointment and spray formulations", Maturitas 13(4): 313-317 (1991),

"Progesterone administration by nasal sprays in menopausal women: comparison between two different spray formulations", Gynecol Endocrinol 6(4): 247-251 (1992), "Effects of the repetitive administration of progesterone by nasal spray in postmenopausal women", Fertil Steril, 60(6): 1020-1024 (1993) and "Nasal spray administration of unmodified progesterone: evaluation of progesterone serum levels with three different radioimmunoassay techniques", Maturitas 19(1): 43-52 (1994), shows that when progesterone is dissolved in almond oil (20 mg/ml) and administered by nasal spray, this leads to higher progesterone bioavailability than that provided by progesterone dissolved in dimethicone or a PEG-based ointment. After nasal application of progesterone in almond oil $C_{max}$ levels were observed after 30 to 60 minutes, decreasing significantly 6 to 8 hours after single administration.

Steege et al. "Bioavailability of nasally administered progesterone", Fertil Steril, 46(4): 727-729 (1986), shows that when progesterone is dissolved in polyethylene glycol (200 mg/ml), a $T_{max}$ at 30 min is achieved and that the duration of serum level is at least 8 hours but with high variations.

When progesterone is formulated in ethanol/propylene glycol/water, however, the $T_{max}$ is only 5.5 min. See Kumar et al., "Pharmacokinetics of progesterone after its administration to ovariectomized rhesus monkeys by injection, infusion, or nasal spraying", Proc. Natl. Acad. Sci. U.S.A., 79: 4185-9 (1982).

Provasi et al., "Nasal delivery progesterone powder formulations comparison with oral administration", Boll. Chim. Farm. 132(10): 402-404 (1993), investigated powder mixtures (co-ground and co-lyophilized progesterone/cyclodextrin) containing progesterone and shows that when these powder mixtures are administered nasally, a progesterone $T_{max}$ of within 2-5 min and a serum level decrease of within about 20 min are achieved.

These results are quite similar to that found for testosterone (see above) and for an already marketed aqueous nasal spray containing estradiol, formulated in cyclodextrin (Aerodiol®). Maximum plasma levels are reached within about 10-30 minutes decreasing to about 10% of the peak value after 2 hours. Again, this speed is not necessary for sex hormone replacement therapy and not desirable in view of the short elimination half-life of hormones.

Apart from the "liberation/adsorption" problem referenced above, in connection with sexual hormones and bioavailability, and the nearly exclusively crucial liver metabolism and short half-life problems, there is also the problem of high testosterone protein-binding in the circulating plasma of men and women. Approximately 40% of circulating plasma testosterone, e.g., binds to sex hormone binding globulin (SHBG)-in men about 2% testosterone and in women up to 3% testosterone remains unbound (free)-and the remainder binds to albumin and other proteins. The fraction bound to albumin dissociates easily and is presumed to be biologically active, whereas the SHBG fraction is not. The amount of SHBG in plasma, however, determines the distribution of testosterone in free and bound forms, where free testosterone concentrations determine (limit) the drug's half-life.

Notwithstanding the above, there still are needs for testosterone formulation systems for controlling the release of testosterone into the systemic circulation of men and women that (a) are therapeutically effective when administered intranasally to male and female patients, (b) provide constant effective testosterone blood levels, without unwanted testosterone blood level spike, over dose life, and (c) are safe, convenient to use, well tolerated, stable and easily and reproducibly manufactured on scale up.

## Summary of the Invention

The present invention overcomes the above-mentioned disadvantages and drawbacks associated with current testosterone replacement or supplemental therapy and is directed to novel sustained or controlled release testosterone gels, for pernasal administration, which uniquely provide constant effective testosterone blood levels, without causing undesired testosterone blood level spikes, over dose life when the novel intranasal testosterone gels are administered pernasal to males or females. In addition, the novel intranasal testosterone gels of the present invention are safe, convenient to use, well tolerated, stable and easily and reproducibly manufactured on scale up. Moreover, because supra-normal and sub-normal testosterone levels are believed to be essentially kept to a minimum or avoided and the testosterone serum levels are believed to remain essentially constant during their dose life, i.e., the intranasal testosterone gels of the present invention mimic or restore testosterone blood levels to normal physiologic daily rhythmic testosterone levels when the novel intranasal testosterone gels of the present invention are administered pernasally, the novel intranasal testosterone gels of the present invention are uniquely suited for testosterone replacement or supplemental therapy and are effective to treat males and females in need of testosterone replacement or testosterone supplemental therapy to treat, for example, male testosterone deficiency or female sexual dysfunction.

The present invention is also directed to novel methods for pernasal administration of the nasal testosterone gels. Generally speaking, the novel methods of the present invention involve depositing the intranasal testosterone gels topically into the nasal cavity of each nostril to deliver a therapeutically effective amount of testosterone over dose life for providing constant effective testosterone blood levels for use in testosterone replacement or supplemental therapy and for effectively treating males and females in need of testosterone replacement or testosterone supplemental therapy to treat, for example, male testosterone deficiency or female sexual dysfunction.

In accordance with the novel methods of the present invention, the intranasal testosterone gels are topically

deposited on the outer external walls (opposite the nasal septum) inside the naval cavity of each nostril, preferably at about the middle to about the upper section of the outer external wall (opposite the nasal septum) just under the cartilage section of the outer external wall inside the naval cavity of each nostril. Once gel deposition is complete within each nostril of the nose, the outer nose is then preferably gently and carefully squeezed and/or rubbed by the patient, so that the deposited gel remains in contact with the mucosal membranes within the nasal cavity for sustained or controlled release of the testosterone over dose life. In accordance with the present invention, typical testosterone gel dosage amounts deposited pernasal application ranges from about 140 microliters to about 180 microliters.

While the intranasal testosterone gels of the present invention are preferred pharmaceutical preparations when practicing the novel methods of the pesent invention, it should be understood that the novel topical intranasal gel formulations and methods of the present invention also contemplate the pernasal administration of any suitable testosterone formulations or any suitable active ingredient, either alone or in combination with testosterone or other active ingredients, such as neurosteroids or sexual hormones (e.g., androgens and progestins, like testosterone, estradiol, estrogen, oestrone, progesterone, etc.), neurotransmitters, (e.g., acetylcholine, epinephrine, norepinephrine, dopamine, serotonin, melatonin, histamine, glutamate, gamma aminobutyric acid, aspartate, glycine, adenosine, ATP, GTP, oxytocin, vasopressin, endorphin, nitric oxide, pregnenolone, etc.), prostaglandin, benzodiazepines like diazepam, midazolam, lorazepam, etc., and PDEF inhibitors like sildenafil, tadalafil, vardenafil, etc., in any suitable pharmaceutical preparation, such as a liquid, cream, ointment, salve or gel. Examples of additional topical formulations for practice in accordance with the novel methods of the present invention include the topical pernasal formulations dislcosed in, for example, U.S. Patent Nos. 5,578,588, 5,756,071 and 5,756,071 and U.S. Patent Publication Nos. 2005/0100564, 2007/0149454 and 2009/0227550, all of which are incorporated herein by reference in their entirties.

The present invention is also directed to novel pre-filled, multi-dose applicator systems for pernasal administration to strategically and uniquely deposit the nasal testosterone gels at the preferred locations within the nasal cavity for practicing the novel methods and teachings of the present invention. Generally, speaking the applicator systems of the present invention are, e.g., airless fluid, dip-tube fluid dispensing systems or pumps or any other system suitable for practicing the methods of the present invention. The applicator systems or pumps include, for example, a chamber, pre-filled with multiple doses of an intranasal testosterone gel of the present invention, that is closed by an actuator nozzle. The actuator nozzle may comprise an outlet channel and tip, wherein the actuator nozzle is shaped to conform to the interior surface of a user's nostril for (a) consistent delivery of uniform dose amounts of an intranasal testosterone gel of the present invention during pernasal application within the nasal cavity, and (b) deposition at the instructed location within each nostril of a patient as contemplated by the novel methods and teachings of the present invention. Examples of pre-filled, multi-dose applicator systems include the COMOD system available from Ursatec, Verpackung-GmbH, Schillerstr. 4, 66606 St. Wendel, Germany or the Albion or Digital airless applicator systems available from Airlessystems, RD 149 27380 Charleval, France or 250 North Route 303 Congers, NY 10950, as shown in Figs. 1-4

The salient elements of the novel intranasal testosterone gels according to the present invention comprise (a) testosterone in an effective amount, e.g., an amount of between about 0.5% and about 10% or higher, by weight; (b) at least one lipophilic or partly lipophilic carrier; (c) a super solvent or a mixture of super solvents for increasing the solubility of the testosterone, (d) a gel-forming or viscosity regulating agent for controlling the release of the testosterone from the gels following intranasal administration, and, optionally, (e) a surface active agent or a mixture of surface active agents, i.e., surfactant(s), having surface tension decreasing activity.

In accordance with the present invention, the testosterone drug can be in, for instance, crystalline, amorphous, micronized, non-micronized, powder, small particle or large particle form when formulating the intranasal testosterone gels of the present invention. An Exemplary range of testosterone particle sizes include from about 0.5 microns to about 200 microns. Preferably, the testosterone particle size is in a range of from about 5 microns to about 100 microns, and the testosterone is in crystalline or amorphous and non-micronized or micronized form. Preferably, the testosterone is in crystalline or amorphous micronized form.

In one embodiment in accordance with the present invention, the lipophilic carrier is an oil, preferably, a liquid oil. The oil can be natural, synthetic, semi-synthetic, vegetal or mineral, mostly hydrophobic. Preferably, the oils are any acceptable vegetable oil, such as, castor oil, almond oil, linseed oil, canola oil, coconut oil, corn oil, cottonseed oil, palm oil, peanutoil, poppy seed oil and soybean oil. Also contemplated by the present invention, the oils can be a mineral oil (light mineral or paraffin), synthetic or refined isopropyl myristate, isopropyl palmitate, capryl caprylate, methyl stearate, medium chain triglycerides, propylene glycol dicaprylocaprate, cetostearyl alcohol, stearyl alcohol and mixtures thereof.

More preferably, the oil is any acceptable vegetable oil.

Most preferably, the oil is castor oil, such as Crystal O® or Crystal LC USP.

In accordance with the present invention, the carrier is present in the intranasal testosterone gels in an amount of between about 30% and about 98% by weight, preferably, between about 42% and about 96% by weight, more preferably, between about 67% and about 95% by weight, even more preferably, between about 82% and about 95% by weight, and most preferably between about 87% and about 94.5% by weight of the testosterone gel.

The intranasal testosterone gels of the present invention are uniquely formulated with at least one super solvent

for enhancing testosterone solubility. In accordance with the present invention, the super solvents are generally characterized as non aqueous solvents that are miscible with the carrier or oil and are present in the intranasal testosterone gels in amounts suitable to form a gel during gel formulation or gel manufacture and in advance of pernasal application. In accordance with the present invention, intranasal gels of the present invention are not emulsified *in situ* following application of the gels into the nasal cavity. Typically, the super solvents are present in the intranasal testosterone gels in amounts ranging from about 1% to about 50% by weight. Also, the super solvents as contemplated by the present invention can be characterized as (1) enhancing testosterone solubility in the intranasal testosterone gels, (2) being acceptable to the nasal mucosal within the nasal cavities and (3) having no surfactant activity. Examples of super solvents in accordance with the present invention include dimethyl isosorbide, pharma grade, such as Super Refined® Arlasolve™-DMI, diethylene glycol monoethyl ether, such as Transcutol-P®, glycerin, propylene glycol, 1-methyl 2-pyrrolidone, glycerol and satisfactory mixtures thereof.

Preferably, the super solvent comprises dimethyl isosorbide (Super Refined® Arlasolve™-DMI.

While the super solvents of the present invention may be generally present within the intranasal testosterone gels in amounts ranging from about 1% to about 50% by weight, the preferable amounts range from about 1% to about 25% by weight, more preferably, from about 5% to about 20% by weight, and more preferably, from about 5% to about 15%. Most preferably, the super solvents of the present invention are present in the intranasal testosterone gels in an amount at about 15% by weight.

In addition, the intranasal testosterone gels of the present invention include a gel-forming or viscosity regulating agent to (1) form a gel, (2) enhance gel viscosity, and (3) control the release of the testosterone from an intranasal testosterone gel following pernasal administration, as contemplated herein by the present invention, i.e., to provide a gel with suitable viscosity and having a slow constant rate of release of the testosterone from the intranasal testosterone gel following pernasal administration, so that a constant effective testosterone blood level or profile, without testosterone spike, is achieved and maintained over dose life in a male or female patient in need of testosterone replacement therapy to treat, e.g., male testosterone deficiency or female sexual dysfunction, respectively.

Preferably, a viscosity regulating agent of the present invention comprises a thickener or gelling agent and examples include cellulose and cellulose derivatives, e.g., hydroxypropyl cellulose and hydroxyethyl cellulose, polysaccharides, carbomers, acrylic polymers, such as Carbopol®, polyvinyl alcohol and other vinylic polymers, povidone, colloidal silicon dioxide, such as Aerosil® 200 or Cab-O-Sil®, lipophilic silicon dioxide, such as Aerosil® R972, cetyl alcohols, stearic acid, glyceryl behenate, wax, beeswax, petrolatum, lipophilic gum, triglycerides, lanolin, inulin and suitable mixtures thereof.

More preferably, the gel-forming or viscosity increasing agent is colloidal silicon dioxide, and even more preferably, $SiO_2$ and polyvinyl alcohol.

In accordance with the present invention, the get-forming or viscosity regulating agent is present within the intranasal testosterone gels in amounts generally ranging from about 0.5% to about 10% by weight, preferably, about 0.5% to about 5% by weight, more preferably, about 1% to about 4% by weight, and most preferably, at about 3% by weight.

The intranasal testosterone gels of the present invention have in general, a viscosity in the range of between about 3,000 cps and about 27,000 cps. It should nevertheless be understood by those versed in this art that, while the above-mentioned viscosity range is believed to be a preferred viscosity range, any suitable viscosities or viscosity ranges that do not defeat the objectives of the present invention are contemplated.

The intranasal testosterone gels of the present invention may optionally, but not necessarily, be formulated with at least one surfactant, such as lecithin, fatty acid esters of polyvalent alcohols, fatty acid esters of sorbitanes, fatty acid esters of polyoxyethylensorbitans, fatty acid esters of polyoxyethylene, fatty acid esters of sucrose, fatty acid esters of polyglycerol, sorbitol, glycerine, polyethylene glycol, macrogol glycerol fatty acid ester and satisfactory mixtures thereof. Examples include oleoyl macrogolglyceride and suitable mixtures of oleoyl macrogolglycerides.

Other examples of surfactants suitable for use in accordance with the present invention include those illustrated in U.S. Patent Nos. 5,578,588, 5,756,071, and 5,576,071 and in U.S. Patent Publication Nos. 2005/0100564, 2007/0149454 and 2009/0227550, all of which are incorporated herein by reference in their entireties.

The amount of testosterone in an intranasal testosterone gel of the present invention that will be therapeutically effective in a specific situation will depend upon such things as the dosing regimen, the application site, the particular gel formulation, dose longevity and the condition being treated. As such, it is generally not practical to identify specific administration amounts herein; however, it is believed that those skilled in the art will be able to determine appropriate therapeutically effective amounts based on the guidance provided herein, information available in the art pertaining to testosterone replacement or supplemental therapy, and routine testing.

The term "a therapeutically effective amount" means an amount of testosterone sufficient to induce a therapeutic or prophylactic effect (1) for use in testosterone replacement or supplemental therapy, and/or (2) to treat (a) males diagnosed with male testosterone deficiency, namely, low sexual libido, drive or sexual activity, low fertility, low spermatogenesis, aspermatogenesis, depression and/or hypogonadism in males, and (b) female sexual dysfunction ("FSD"), namely, low sexual libido, drive or sexual activity, low amygdala reactivity, low sexual stimulation, female sexual arousal

disorder, hypoactive sexual desire disorder ("HSDD") and/or female orgasmic disorder ("anorgasmia") in females.

In general, the amount of testosterone present in an intranasal gel formulation of the present invention will be an amount effective to treat a targeted condition, to prevent recurrence of the condition, or to promote sexual stimulation and/or reproduction in males or amygdala reactivity or sexual stimulation in females, as indicated above and herein throughout. In certain embodiments, the amount or concentration of testosterone is at least about 0.5% by weight, such as, for example, at least about 1%, at least about 2%, at least about 3%, at least about 4%, at least about 5%, at least about 6%, at least about 7%, at least about 8%, at least about 9%, and at least about 10% by weight based on the total weight of the intranasal testosterone gel formulation. In other embodiments, the amount of testosterone is at most about 10% by weight, such as, for example, at most about 9%, at most about 8%, at most about 7%, at most about 6%, at most about 5%, at most about 4%, at most about 3%, at most about 2%, at most about 1%, and at most about 0.5%, including any and all increments there between including about 0.25% increments, more or less, by weight based on the total weight of the intranasal testosterone gel formulation. In certain embodiments, the amount or concentration of testosterone is at least about 0.1% by weight, such as, for example, at least about 0.125%, at least about 0.15%, at least about 0.175%, at least about 0.2%, at least about 0.225%, at least about 0.25%, at least about 0.275%, at least about 0.3%, at least about 0.325%, at least about 0.35%, at least about 0.375%, at least about 0.4%, at least about 0.425%, at least about 0.45%, at least about 0.475%, at least about 0.5%, at least about 0.525%, at least about 0.55%, at least about 0.575%, at least about 0.6%, at least about 0.625%, at least about 0.65%, at least about 0.675%, at least about 0.7%, at least about 0.725%, at least about 0.75%, at least about 0.775%, at least about 0.8%, at least about 0.825%, at least about 0.85%, at least about 0.875%, at least about 0.9%, at least about 0.925%, at least about 0.95%, at least about 0.975%, etc. in about 0.25%, more or less, increments up to about 10% by weight based on the total weight of the intranasal testosterone gels of the present invention.

Also as contemplated by the present invention, the testosterone may be present in each pernasal dosage of the intranasal testosterone gels of the present invention in amounts ranging from about 0.5 to about 10% by weight, preferably from about 1% to about 9% by weight, more preferably, for male treatment, from about 7% to about 9%, and more preferably, from about 7.5% to about 8.5% by weight, and most preferably, at about 8% by weight, and for female treatment, from about 0.1% to about 2% by weight, from about 0.5% to 1% by weight, from about 1% to about 2% by weight,, from about 2% to about 3% by weight, from about 3% to about 4% by weight, from about 4% to about 5% by weight, etc., wherein each pernasal dosage is in the general size range of between about 140 microliters and 180 microliters, preferably between about 140 microliters and 160 microliters, and more preferably between about 140 microliters and about 150 microliters pernasal dosage.

For treatment of male testosterone deficiency, such as low sexual libido or drive, low sexual activity, low spermatogenesis, aspermatogenesis, depression and/or hypogonadism, an effective amount of testosterone drug is preferably present within the intranasal testosterone gels of the present invention in amounts generally ranging from at least about 0.05 mg to about 0.13 mg or more per microliter dose, e.g., about 140 microliters to about 180 microliters, administered in each nostril (pernasal) for a total intranasal testosterone dose of at least about 20 mg to about 36 mg pernasal application. Thus, and by way of example, to achieve delivery of 20 mg of testosterone as a total dose, each 140 microliter dose should contain about 0.07 mg of testosterone, whereas a 180 microliter dose should have about 0.55 mg of testosterone. If total dose of about 28 mg testosterone is desired, each 140 microliter dose should contain about 0.1 mg of testosterone, whereas a 180 microliter dose should have about 0.78 mg of testosterone. If, however, about 36 mg testosterone is desired as a total dose, each 180 microliter dose should contain about 0.1 mg of testosterone, whereas each 140 microliter dose should contain about 0.13 mg of testosterone. It is currently believed that an 8% intranasal testosterone gel in a pernasal dosage amount of about 140 microliters (about 11.2 mg of testosterone per 140 microliters) is preferred for delivering a total combined testosterone dose of about 22.4 mg per application (about 11.2 mg of testosterone pernasal application), or for delivering a total combined testosterone daily dose of about 22.4 mg and about 44.8 mg when administered once or twice per day pernasal, respectively, to treat a male testosterone deficiency to restore testosterone to normal testosterone blood levels observed in healthy young males, i.e., from about 200 nanograms/dl to about 1500 nanograms/dl of testosterone.

In acordance with the present invention, examples of rates of diffusion of the testosterone in the intranasal gels of the present invention through a Franz cell membrane, as contemplated by the present invention, range from between about 28 and 100 slope/mgT%, and preferably about 30 and 95 slope/mgT%. For those intranasal gels formulated with between about 4.0% and 4.5% testosterone, the preferred rates of diffusion of testosterone are between about 28 and 35 slope/mgT%. See, for example, Examples 9 and 10.

For treatment of female sexual dysfunction, such as low sexual libido, low sexual drive, low sexual activity, low amygdala reactivity, anorgasmia and/or HSDD, it is currently believed that the total testosterone dosage amount delivered each day to increase amygdala reactivity in middle age women, i.e., ages between about 40 and about 65 , or to restore testosterone to normal testosterone levels comparable to that of healthy young women, i.e., ages between about 30 and about 45 , e.g., from a low of about 30 nanograms/dl to a high of about 150 nanograms/dl, to treat FSD, may be, for example, in the general range of from about 100 micrograms to about 5000 micrograms or more. This can be

accomplished by delivering, for example, from or up to about 0.1 mg (about 0.050 mg per nostril), from or up to about 0.2 mg (about 0.1 mg per nostril), 300 micrograms (about 0.15 mg per nostril), from or up to about 0.4 mg, (about 0.2 mg per nostril), from or up to about 0.5 mg (about 0.25 mg per nostril), from or up to about 0.6 mg (about 0.3 mg per nostril), from or up to about 0.7 mg (about 0.35 mg per nostril), from or up to about 0.8 mg (about 0.4 mg per nostril), from or up to about 0.9 mg (about 0.45 mg per nostril), from or up to about 1 mg (about 0.5 mg per nostril), from or up to about 1.1 mg (about 0.55 mg per nostril), from or up to about 1.2 mg (about 0.6 mg per nostril), from or up to about 1.5 mg (about 0.75 mg per nostril), from or up to about 1.8 mg (about 0.9 mg per nostril), from or up to about 2 mg (about 1 mg per nostril), from or up to about 2.5 mg (about 1.25 mg per nostril), from or up to about 3 mg (about 1.5 mg per nostril), from or up to about 3.5 mg (about 1.75 mg per nostril), from or up to about 4 mg (about 2 mg per nostril), from or up to about 4.5 mg (about 2.25 mg per nostril), from or up to about 5 mg (about 2.5 mg per nostril), or even up to about 5.25 mg, 5.5 mg, 5.75 mg, 6 mg, 6.25 mg, 6.5 mg, 6.75 mg, 7 mg, 7.25 mg, 7.5 mg, or even higher testosterone amounts, per total daily dose administered, e.g., once or twice per day to treat female sexual dysfunction, or twice the above mg amounts as a total daily administered once per day to treat female sexual dysfunction.

While the present invention has identified what it believes to be preferred concentrations of intranasal testosterone gel formulations, numbers of applications per day, durations of therapy, pernasal methods and pre-filled, multi-dose applicator systems, it should be understood by those versed in this art that any effective concentration of testosterone in an intranasal gel formulation of the present invention that delivers an effective amount of testosterone and any numbers of applications per day, week, month or year, as described herein, that can effectively treat male testosterone deficiency or female sexual dysfunction, without causing unwanted testosterone spiking or treatment limiting reactions or related adverse events is contemplated by the present invention.

The above summary of the present invention is not intended to describe each disclosed embodiment or every implementation of the present invention. The description that further exemplifies illustrative embodiments. In several places throughout the application, guidance is provided through examples, which examples can be used in various combinations. In each instance, the examples serve only as representative groups and should not be interpreted as exclusive examples.

## Brief Description of the Drawings

The foregoing and other objects, advantages and features of the present invention, and the manner in which the same are accomplished, will become more readily apparent upon consideration of the following detailed description of the present invention taken in conjunction with the accompanying figures and examples, which illustrate embodiments, wherein:

Fig. 1 is a side view of a first embodiment of the invention;.

Fig. 2 is a cross-sectional side view of the distributor pump of the first embodiment of the invention;

Fig. 3 is a side view of a second embodiment of the invention;

Fig. 4 is a cross-sectional side view of the distributor pump of the second embodiment of the invention;

Fig. 5 is a side view of a second embodiment of the invention concerning an airless bottle assembly of the invention;

Fig. 6 is a side view of a second embodiment of the invention concerning digital actuator and rounded cap;

Fig. 7A depicts the right nostril of subject #1 after a single dose syringe administration;

Fig.7B depicts the left nostril of subject #1 after a multiple dose dispenser administration;

Fig. 8A depicts the right nostril of subject #2 after a single dose syringe administration;

Fig. 8B depicts the left nostril of subject #2 after a multiple dose dispenser administration;

Fig. 9A depicts the right nostril of subject #3 after a single dose syringe administration;

Fig. 9B depicts the left nostril of subject #3 after a multiple dose dispenser administration;

Figs. 10A and 10B illustrate use of a multiple dose dispenser in accordance with the present invention;

Fig. 11 illustrates a multiple dose dispenser in accordance with the present invention;

Fig. 12 depicts a Franz Cell apparatus position layouts for comparing testing in accordance with Example 5;

Fig. 13 depicts individual amount of testosterone released from the compositions in acoordance with Example 5;

Fig. 14 depicts individual testosterone concentration versus time (linear y-axis), that are grouped by subject in accordance with Example 6. Number. Black: baseline; blue: syringe; salmon: multiple dose dispenser. T=0 is at 21:00 clock-time (± 30 minutes), t=12 is at 9:00 (± 30 minutes) clock-time;

Fig. 15 depicts individual (blue) and median (black) testosterone concentration versus time (linear y-axis), that are grouped by treatment;

Fig. 16 depicts the probability density of the log ratio of testosterone levels that are reached with the multiple dose dispenser over levels tht are reached with the syringe;

Fig. 17 depicts solubility of testosterone in different vehicles at 32°C and at 50°C;

Fig. 18 depicts Ternary solvent mixture optimization: Contour plot shows that, in order to achieve more than 6% testosterone solubility, higher levels of DMI and Transcutol are required;

Fig. 19 depicts a flow diagram for manufacturing TBS-1.

Figs 20A and 20B depict a flow diagram of a manufacturing process of an antranasal testosterone gel of the present invention;

Fig. 21 depicts a mean concentration-time curves of testosterone (solid squares) and DHT (open squares) after single-dose administration of 3 different TBS-1 strengths (7.6 mg = squares; 15.2 mg = circles; 22.8 mg triangles). The lower limit of normal range for testosterone is indicated with the dashed line (based on morning serum samples);

Fig. 22 depicts testosterone diffusion rate of intranasal testosterone gel formulations of Example 6 using Franz cells method;

Fig. 23 depicts the pharmacokinetic profiles of 15 male subjects using the formulas of Example 6;

Fig. 24 depicts a comparison between TBS 1 A 8% (Part I);

Fig. 25 depicts a comparison between TBS 1 A 8% (Part I);

Fig. 26 depicts a comparison between 6 hours and 24 hours run (RD11101 and RD11102)

Fig. 27 depicts a comparison between TBS 1 A 4% (Part I);

Fig. 28 depicts a comparison between TBS 1 A 4% (Part II);

Fig. 29 depicts a comparison between TBS 1 A 4% (Part III);

Fig. 30 depicts a comparison slower diffusion;

Fig. 31 depicts a comparison between 6 hours and 24 hours run (RD11063 and RD11085); Fig. 32 depicts a comparison between 400mg and 1 gram of gel (RD11063);

Fig. 33 depicts the linear regression curve for peak response versus testosterone amount (μg/ml);

Fig. 34 depicts release rate for a TBS1A gel 4.0%;

Fig. 35 depicts release rate for a Nasobol Gel 4.0%;

Fig. 36 depicts a typical chromatogram of diluent;

Fig. 37 depicts a typical chromatogram of testosterone working standard solution;

Fig. 38 depicts a typical chromatogram of testosterone sample solution;

Fig. 39 depicts the 4.0% day 1 release rate for testosterone 4.0% gel.

Fig. 40 depicts the 4.0% day 2 release rate for testosterone 4.0% gel;

Fig. 41 depicts the 4.0% day 3 release rate for testosterone 2.0% gel;

Fig. 42 depicts the 4.0% day 3 release rate for testosterone 4.0% gel;

Fig. 43 depicts the 4.0% day 3 release rate for testosterone 8.0% gel;

Fig. 44 depicts the testosterone gel 2% day 4 release rate;

Fig. 45 depicts the testosterone gel 4% day 4 release rate;

Fig. 46 depicts the testosterone gel 8% day 4 release rate;

Fig. 47 depicts the 4.0% day 1 release rate for testosterone 4.5% gel;

Fig. 48 depicts the 4.5% day 2 release rate for testosterone 4.5% gel;

Fig. 49 depicts the 4.5% day 3 release rate for testosterone 2.25% gel;

Fig. 50 depicts the 4.5% day 3 release rate for testosterone 4.5% gel;

Fig. 51 depicts the 4.5% day 3 release rate for testosterone 9.0% gel;

Fig. 52 depicts the 4.5% day 4 release rate for testosterone 2.25% gel;

Fig. 53 depicts the 4.5% day 4 release rate for testosterone 4.5% gel; and

Fig. 54 depicts the 4.5% day 4 release rate for testosterone 9.0% gel.

## Detailed Description of the Invention

By way of illustrating and providing a more complete appreciation of the present invention and many of the attendant advantages thereof, the following detailed description and examples are given concerning the novel intranasal testosterone gels, application devices and methods of the present invention.

The following items characterize the present invention.

(1) An intranasal testosterone gel for topical administration into a nasal cavity to control release of testosterone over a period of at least about 6 hours following the nasal cavity administration to provide replacement testosterone in a male for treating male testosterone deficiency, without causing an unacceptable spike in testosterone blood level in the male over dose life, said intranasal testosterone gel comprising:

(a) testosterone in an amount effective to treat male testosterone deficiency;
(b) at least one lipophilic or partly lipophilic carrier;
(c) a super solvent for increasing testosterone solubility in said intranasal testosterone gel; and
(d) a viscosity regulating agent in an amount effective to control the release of said testosterone from said intranasal testosterone gel, wherein said intranasal testosterone gel does not need a surfactant.

(2) The intranasal testosterone gel of item 1, wherein said lipophilic carrier comprises an oil.
(3) The intranasal testosterone gel of item 2, wherein said oil is a liquid oil.

(4) The intranasal testosterone gel of item 3, wherein said oil is selected from a group of oils consisting of a natural oil, a synthetic oil, a semi-synthetic oil, a vegetal oil, a mineral oil and mixtures thereof.

(5) The intranasal testosterone gel of item 4, wherein said oil is selected from a group of oils consisting of castor oil, almond oil, linseed oil, canola oil, coconut oil, corn oil, cottonseed oil, palm oil, peanut oil, poppy seed oil, soybean oil and mixtures thereof.

(6) The intranasal testosterone gel of item 2, wherein said oil is a vegetable oil.

(7) The intranasal testosterone gel of item 2, wherein said oil is castor oil.

(8) The intranasal testosterone gel of item 2, wherein said oil is present in an amount of between about 30% and about 98% by weight of said intranasal testosterone gel.

(9) The intranasal testosterone gel of item 2, wherein said oil is present in an amount of between about 60% and about 98% by weight of said intranasal testosterone gel.

(10) The intranasal testosterone gel of item 2, wherein said oil is present in an amount of between about 75% and about 95% by weight of said intranasal testosterone gel.

(11) The intranasal testosterone gel of item 2, wherein said oil is present in an amount of between about 85% and 95% by weight of said intranasal testosterone gel.

(12) The intranasal testosterone gel of item 2, wherein said oil is present in an amount of about 90% by weight of said intranasal testosterone gel.

(13) The intranasal testosterone gel of item 1, wherein said lipophilic carrier is selected from a group of lipophilic carriers consisting of a mineral oil, a paraffin, an isopropyl a myristate, an isopropyl palmitate, a capryl caprylate, a methyl stearate, a medium chain triglyceride, a propylene glycol, a dicaprylocaprate, a cetostearyl alcohol, a stearyl alcohol and mixtures thereof.

(14) The intranasal testosterone gel of any one of items 1-13, wherein said super solvent is dimethyl isosorbide.

(15) The intranasal testosterone gel of any one of items 1-13, wherein said super solvent is diethylene glycol monoethyl ether.

(16) The intranasal testosterone gel of any one of items 1-13, wherein said super solvent is glycerol.

(17) The intranasal testosterone gel of any one of items 1-13, wherein said super solvent is polyethylene glycol.

(18) The intranasal testosterone gel of any one of items 1-13, wherein said super solvent is selected from a group consisting of dimethyl isosorbide, diethylene glycol monoethyl ether, glycerol, polyethylene glycol, 1 -methyl 2-pyrrolidone and mixtures thereof.

(19) The intranasal testosterone gel of any one of items 1-18, wherein said super solvent is in an amount of from about 1% to about 80% by weight of said intranasal testosterone gel.

(20) The intranasal testosterone gel of any one of items 1-18, wherein said super solvent is in an amount of from about 1% to about 50% by weight of said intranasal testosterone gel.

(21) The intranasal testosterone gel of any one of items 1-18, wherein said super solvent is in an amount of from about 10% to about 25% by weight of said intranasal testosterone gel.

(22) The intranasal testosterone gel of any one of items 1-18, wherein said super solvent is in an amount of about 15% by weight of said intranasal testosterone gel.

(23) The intranasal testosterone gel of any one of items 1-22, wherein said viscosity regulating agent comprises a thickener or gelling agent.

(24) The intranasal testosterone gel of item 23, wherein said thickener or gelling agent is selected from a group of thickeners or gelling agents consisting of a cellulose, a cellulose derivative, a hydroxypropyl cellulose, a hydroxyethyl cellulose, a lipophilic gum, an acrylic polymer, carbopol, a polysaccharide, a carbomer, polyvinyl alcohol, povidone, a silicon dioxide, a cetyl alcohol, a stearic acid, a wax, beeswax, a petrolatum, a triglyceride, lanolin, glyceryl behenate , inulin or a mixture thereof.

(25) The intranasal testosterone gel of item 23, wherein said thickener or gelling agent is a silicon dioxide selected from a group of silicon dioxides consisting of colloidal silicon dioxide, silicon dioxide or lipophilic silicon dioxide.

(26) The intranasal testosterone gel of any one of items 1-25, wherein said viscosity regulating agent is in an amount of from about 0.5% to about 10% by weight of said intranasal testosterone gel.

(27) The intranasal testosterone gel of any one of items 1-25, wherein said viscosity regulating agent is in an amount of from about 0.5% to 7.5% by weight of said intranasal testosterone gel.

(28) The intranasal testosterone gel of any one of items 1-25, wherein said viscosity regulating agent is in an amount of from about 1% to about 5% by weight of said intranasal testosterone gel.

(29) The intranasal testosterone gel of any one of items 1-25, wherein said viscosity regulating agent is in an amount of about 3% by weight of said intranasal testosterone gel.

(30) The intranasal testosterone gel of any one of items 1-29, wherein said testosterone is in an amount of from about 0.05% to about 10% by weight of said intranasal testosterone gel.

(31) The intranasal testosterone gel of any one of items 1-29, wherein said testosterone is in an amount of from about 4% to about 10% by weight of said intranasal testosterone gel.

(32) The intranasal testosterone gel of any one of items 1-29, wherein said testosterone is in an amount of from about 5% to about 9 % by weight of said intranasal testosterone gel.

(33) The intranasal testosterone gel of any one of items 1129, wherein said testosterone is in an amount of from about 6% to about 8% by weight of said intranasal testosterone gel.

(34) The intranasal testosterone gel of any one of items 1-29, wherein said testosterone is in an amount of about 7% by weight of said intranasal testosterone gel.

(35) The intranasal testosterone gel of any one of items 1-29, wherein said testosterone is in an amount of about 10% by weight of said intranasal testosterone gel.

(36) The intranasal testosterone gel of any one of items 1-29, wherein said testosterone is in an amount of about 9.5% by weight of said intranasal testosterone gel.

(37) The intranasal testosterone gel of any one of items 1-29, wherein said testosterone is in an amount of about 9% by weight of said intranasal testosterone gel.

(38) The intranasal testosterone gel of any one of items 1-29, wherein said testosterone is in an amount of about 8.5% by weight of said intranasal testosterone gel.

(39) The intranasal testosterone gel of any one of items 1-29, wherein said testosterone is in an amount of about 8% by weight of said intranasal testosterone gel.

(40) The intranasal testosterone gel of any one of items 1-29, wherein said testosterone is in an amount of about 7.5% by weight of said intranasal testosterone gel.

(41) The intranasal testosterone gel of any one of items 1-29, wherein said testosterone is in an amount of about 7% by weight of said intranasal testosterone gel. (42) The intranasal testosterone gel of any one of items 1-29, wherein said testosterone is in an amount of about 6.75% by weight of said intranasal testosterone gel.

(43) The intranasal testosterone gel of any one of items 1-29, wherein said testosterone is in an amount of about 6.5% by weight of said intranasal testosterone gel.

(44) The intranasal testosterone gel of any one of items 1-29, wherein said testosterone is in an amount of about 6% by weight of said intranasal testosterone gel.

(45) The intranasal testosterone gel of any one of items 1-29, wherein said testosterone is in an amount of about 5.5% by weight of said intranasal testosterone gel.

(46) The intranasal testosterone gel of any one of items 1-129, wherein said testosterone is in an amount of about 5% by weight of said intranasal testosterone gel.

(47) The intranasal testosterone gel of any one of items 1-29, wherein said testosterone is in an amount of from about 5 mg to about 10 mg.

(48) The intranasal testosterone gel of any one of items 1-29, wherein said testosterone is in an amount of from about 5.5 mg to about 9.5 mg.

(49) The intranasal testosterone gel of any one of items 1-29, wherein said testosterone is in an amount of from about 6 mg to about 9 mg.

(50) The intranasal testosterone gel of any one of items 1-29, wherein said testosterone is in an amount of from about 6.5 mg to about 8.5 mg.

(51) The intranasal testosterone gel of any one of items 1-29, wherein said testosterone is in an amount of about 10 mg.

(52) The intranasal testosterone gel of any one of items 1-29, wherein said testosterone is in an amount of about 9.5 mg.

(53) The intranasal testosterone gel of any one of items 1-29, wherein said testosterone is in an amount of about 9.25 mg.

(54) The intranasal testosterone gel of any one of items 1-29, wherein said testosterone is in an amount of about 9 mg.

(55) The intranasal testosterone gel of any one of items 1-29, wherein said testosterone is in an amount of about 8.75 mg.

(56) The intranasal testosterone gel of any one of items 1-29, wherein said testosterone is in an amount of about 8.5 mg.

(57) The intranasal testosterone gel of any one of items 1-129, wherein said testosterone is in an amount of about 8.25 mg.

(58) The intranasal testosterone gel of any one of items 1-29, wherein said testosterone is in an amount of about 8 mg.

(59) The intranasal testosterone gel of any one of items 1-29, wherein said testosterone is in an amount of about 7.75 mg.

(60) The intranasal testosterone gel of any one of items 1-29, wherein said testosterone is in an amount of about 7.5 mg.

(61) The intranasal testosterone gel of any one of items 1-29, wherein said testosterone is in an amount of about 7.25 mg.

(62) The intranasal testosterone gel of any one of items 1-29, wherein said testosterone is in an amount of about 7 mg.

(63) The intranasal testosterone gel of any one of items 1-29, wherein said testosterone is in an amount of about

6.7 mg.

(64) The intranasal testosterone gel of any one of items 1-29, wherein said testosterone is in an amount of about 6.5 mg.

(65) The intranasal testosterone gel of any one of items 1-29, wherein said testosterone is in an amount of about 6.25 mg.

(66) The intranasal testosterone gel of any one of items 1-29, wherein said testosterone is in an amount of about 6. mg.

(67) The intranasal testosterone gel of any one of items 1-29, wherein said testosterone is in an amount of about 5.75 mg.

(68) The intranasal testosterone gel of any one of items 1-29, wherein said testosterone is in an amount of about 5.5 mg.

(69) The intranasal testosterone gel of any one of items 1-29, wherein said testosterone is in an amount of about 5.25 mg.

(70) The intranasal testosterone gel of any one of items 1-29, wherein said testosterone is in an amount of about 5 mg.

(71) The intranasal testosterone gel of any one of items 1-70, wherein said intranasal testosterone gel further includes a surfactant.

(72) The intranasal testosterone gel of item 71, wherein said surfactant is selected from a group of surfactants consisting of lecithin, a fatty acid ester of a polyvalent alcohol, a fatty acid ester of sorbitane, a fatty acid ester of polyoxyethylensorbitans, a fatty acid ester of polyoxyethylene, a fatty acid ester of sucrose, a fatty acid ester of polyglycerol, sorbitol, glycerine, polyethylene glycol, a macrogol glycerol fatty acid ester or a mixture thereof.

(73) The intranasal testosterone gel of any one of items 1-71, wherein said surfactant is oleoyl macrogolglyceride or a mixture of oleoyl macrogolglycerides.

(74) The intranasal testosterone gel of item 73, wherein said oleoyl macrogolglyceride is Labrafil® M 1944 CS.

(75) The intranasal testosterone gel of any one of items 1-74, wherein said surfactant is in an amount of from about 0.05% to about 10% by weight of said intranasal testosterone gel.

(76) The intranasal testosterone gel of any one of items 1-74, wherein said surfactant is in an amount of from about 1% to about 4% by weight of said intranasal testosterone gel.

(77) The intranasal testosterone gel of any one of items 1-74, wherein said surfactant is in an amount of about 3% by weight of said intranasal testosterone gel.

(78) The intranasal testosterone gel of any one of items 1-77, wherein constant effective testosterone blood levels are generated in the male following intranasal administration of said intranasal testosterone gel.

(79) The intranasal testosterone gel of any one of items 1-77, wherein following topical nasal cavity administration of said intranasal testosterone gel, a testosterone blood level is achieved in the male in a range of from about 200 nanograms/dl to up to about 1500 nanograms/dl during dose life.

(80) The intranasal testosterone gel of any one of items 1-77, wherein following topical nasal cavity administration of said intranasal testosterone gel, a testosterone blood level is achieved in the male at a level of up to about 380 nanograms/dl during dose life.

(81) The intranasal testosterone gel of any one of items 1-77, wherein the male testosterone deficiency causes a decrease in sexual libido or sexual activity.

(82) The intranasal testosterone gel of any one of items 1-77, wherein the male testosterone deficiency causes male depression.

(83) The intranasal testosterone gel of any one of items 1-77, wherein the male testosterone deficiency is hypogonadism.

(84) The intranasal testosterone gel of any one of items 1-77, wherein the male testosterone deficiency causes a decrease in male fertility, low spermatogenesis or aspermatogenesis.

(85) A method of treating male testosterone deficiency with testosterone, said method comprising

applying topically said intranasal testosterone gel onto the nasal mucosal in the nasal cavity of each nostril at least once per day to achieve a constant effective testosterone blood level to treat the male testosterone deficiency.

(86) A method of treating male testosterone deficiency of item 85, wherein said intranasal testosterone gel is said topically applied at least twice a day onto the nasal mucosal in the nasal cavity of each nostril.

(87) A method of treating male testosterone deficiency of items 85 or 86, wherein said intranasal testosterone gel is said topically applied to the outer external wall inside the naval cavity.

(88) A method of treating male testosterone deficiency of item 87, wherein said intranasal testosterone gel is said topically applied at about the middle section to about the upper section of the outer external wall inside the naval cavity.

(89) A method of treating male testosterone deficiency of item 87, wherein said intranasal testosterone gel is said topically applied at about or under the cartilage section of the outer external wall inside the naval cavity.

(90) A method of treating male testosterone deficiency of any one of items 85-89, wherein said method includes the further step of:

messaging outer skin of nose to distribute said applied intranasal testosterone gel throughout the nasal cavity of

each nostril.

(91) A method of treating male testosterone deficiency of any one of items 85-90, wherein said topical application step is performed with a device pre-filled with said intranasal testosterone gel.

(92) A method of treating male testosterone deficiency of item 91, wherein the pre-filled device includes a dispensing element for topically applying said intranasal testosterone gel at about the middle section to about the upper section of the outer external wall inside the naval cavity.

(93) A method of treating male testosterone deficiency of item 91, wherein the pre-filled device includes a dispensing element for topically applying said intranasal testosterone gel at about or under the cartilage section of the outer external wall inside the naval cavity.

(94) A method of treating male testosterone deficiency of any one of items 85-93, wherein the male testosterone deficiency causes a decrease in sexual libido or sexual activity.

(95) A method of treating male testosterone deficiency of any one of items 85-93, wherein the male testosterone deficiency causes male depression.

(96) A method of treating male testosterone deficiency of any one of items 8593, wherein the male testosterone deficiency is hypogonadism.

(97) A method of treating female sexual dysfunction of any one of items 85-93, wherein the male testosterone deficiency causes a decrease in male fertility, low spermatogenesis or aspermatogenesis.

(98) A device pre-filled with said intranasal testosterone gel of any one of items 1- 97 for topically applying said intranasal testosterone gel, said device including a dispensing element for topically applying said intranasal testosterone gel at about the middle section to about the upper section of the outer external wall inside the naval cavity.

(99) A device pre-filled with said intranasal testosterone gel of any one of items 1- 98 for topically applying said intranasal testosterone gel, said device including a dispensing element for topically applying said intranasal testosterone gel at about or under the cartilage section of the outer external wall inside the naval cavity.

(100) An intranasal testosterone gel of any one of items 1-99, wherein said intranasal testosterone gel has a viscosity of from about 1,000 cps to about 27,000 cps.

(101) An intranasal testosterone gel of any one of items 1-100, wherein the control release of the testosterone is over a period of at least about 8 hours following the nasal cavity administration.

(102) An intranasal testosterone gel of any one of items 1-100, wherein the control release of the testosterone is over a period of at least about 10 hours following the pernasal cavity administration.

(103) An intranasal testosterone gel of any one of items 1-100, wherein the control release of the testosterone is over a period of at least about 12 hours following the nasal cavity administration.

(104) A method of treating male testosterone deficiency of any one of items 1-103, wherein said intranasal testosterone gel is said topically applied at least once a day onto the nasal mucosal in the nasal cavity of each nostril.

(105) A method of treating male testosterone deficiency of any one of items 1-104, wherein said intranasal testosterone gel is said topically applied at least twice a day onto the nasal mucosal in the nasal cavity of each nostril.

(106) An intranasal testosterone gel of any one of items 1-104, wherein the intranasal testosterone gel is a bioequivalent intranasal testosterone gel.

(107) An intranasal testosterone gel for topical administration into a nasal cavity to control release of testosterone over a period of at least about 6 hours following the nasal cavity administration to provide replacement or supplemental testosterone in a patient, without causing an unacceptable spike in testosterone blood level in the patient over dose life, said intranasal testosterone gel comprising:

(a) testosterone in an amount effective to treat female sexual dysfunction;
(b) at least one lipophilic or partly lipophilic carrier;
(c) a super solvent for increasing testosterone solubility in said intranasal testosterone gel; and
(d) a viscosity regulating agent in an amount effective to control the release of said testosterone from said intranasal testosterone gel, wherein said intranasal testosterone gel does not need a surfactant

(108) The intranasal testosterone gel of item 107, wherein said lipophilic carrier comprises an oil.

(109) The intranasal testosterone gel of item 108, wherein said oil is a liquid oil.

(110) The intranasal testosterone gel of item 107, wherein said oil is selected from a group of oils consisting of a natural oil, a synthetic oil, a semi- synthetic oil, a vegetal oil, a mineral oil and mixtures thereof.

(111) The intranasal testosterone gel of item 107, wherein said oil is selected from a group of oils consisting of castor oil, almond oil, linseed oil, canola oil, coconut oil, corn oil, cottonseed oil, palm oil, peanut oil, poppy seed oil, soybean oil and mixtures thereof.

(112) The intranasal testosterone gel of item 107, wherein said oil is a vegetable oil.

(113) The intranasal testosterone gel of item 107, wherein said oil is castor oil.

(114) The intranasal testosterone gel of item 107, wherein said oil is present in an amount of between about 30%

and about 98% by weight of said intranasal testosterone gel.

(115) The intranasal testosterone gel of item 107, wherein said oil is present in an amount of between about 60% and about 98% by weight of said intranasal testosterone gel.

(116) The intranasal testosterone gel of item 107, wherein said oil is present in an amount of between about 75% and about 95% by weight of said intranasal testosterone gel.

(117) The intranasal testosterone gel of item 107, wherein said oil is present in an amount of between about 85% and 95% by weight of said intranasal testosterone gel.

(118) The intranasal testosterone gel of item 107, wherein said oil is present in an amount of about 90% by weight of said intranasal testosterone gel.

(119) The intranasal testosterone gel of item 107, wherein said lipophilic carrier is selected from a group of lipophilic carriers consisting of a mineral oil, a paraffin, an isopropyl a myristate, an isopropyl palmitate, a capryl caprylate, a methyl stearate, a medium chain triglyceride, a propylene glycol, a dicaprylocaprate, a cetostearyl alcohol, a stearyl alcohol and mixtures thereof.

(120) The intranasal testosterone gel of any one of items 107-119, wherein said super solvent is dimethyl isosorbide.

(121) The intranasal testosterone gel of any one of items 105-119, wherein said super solvent is diethylene glycol monoethyl ether.

(122) The intranasal testosterone gel of any one of items 107-119, wherein said super solvent is glycerol.

(123) The intranasal testosterone gel of any one of items 107-119, wherein said super solvent is polyethylene glycol.

(124) The intranasal testosterone gel of any one of items 107-119, wherein said super solvent is selected from a group consisting of dimethyl isosorbide, diethylene glycol monoethyl ether, glycerol, polyethylene glycol, 1 -methyl 2-pyrrolidone and mixtures thereof.

(125) The intranasal testosterone gel of any one of items 107-124, wherein said super solvent is in an amount of from about 1% to about 50% by weight of said intranasal testosterone gel.

(126) The intranasal testosterone gel of any one of items 105-124, wherein said super solvent is in an amount of from about 1% to about 30% by weight of said intranasal testosterone gel.

(127) The intranasal testosterone gel of any one of items 107-124, wherein said super solvent is in an amount of from about 10% to about 25% by weight of said intranasal testosterone gel.

(128) The intranasal testosterone gel of any one of items 107-124, wherein said super solvent is in an amount of about 15% by weight of said intranasal testosterone gel.

(129) The intranasal testosterone gel of any one of items 107-128, wherein said viscosity regulating agent comprises a thickener or gelling agent.

(130) The intranasal testosterone gel of item 229, wherein said thickener or gelling agent is selected from a group of thickeners or gelling agents consisting of a cellulose, a cellulose derivative, a hydroxypropyl cellulose, a hydroxyethyl cellulose, a lipophilic gum, an acrylic polymer, carbopol, a polysaccharide, a carbomer, polyvinyl alcohol, povidone, a silicon dioxide, a cetyl alcohol, a stearic acid, a wax, beeswax, a petrolatum, a triglyceride, lanolin, glyceryl behenate, inulin or a mixture thereof. (131) The intranasal testosterone gel of item 129, wherein said thickener or gelling agent is a silicon dioxide selected from a group of silicon dioxides consisting of colloidal silicon dioxide, silicon dioxide or lipophilic silicon dioxide.

(132) The intranasal testosterone gel of any one of items 107-131, wherein said viscosity regulating agent is in an amount of from about 0.5% to about 10% by weight of said intranasal testosterone gel.

(133) The intranasal testosterone gel of any one of items 107-131, wherein said viscosity regulating agent is in an amount of from about 0.5% to 7.5% by weight of said intranasal testosterone gel.

(134) The intranasal testosterone gel of any one of items 107-131, wherein said viscosity regulating agent is in an amount of from about 5% to about 8% by weight of said intranasal testosterone gel.

(135) The intranasal testosterone gel of any one of items 107-131, wherein said viscosity regulating agent is in an amount of about 6.75% by weight of said intranasal testosterone gel.

(136) The intranasal testosterone gel of any one of items 107-131, wherein said viscosity regulating agent is in an amount of from about 1% to about 5% by weight of said intranasal testosterone gel.

(137) The intranasal testosterone gel of any one of items 107-131, wherein said viscosity regulating agent is in an amount of about 3% by weight of said intranasal testosterone gel.

(138) An intranasal testosterone gel of any one of items 107-131, wherein the intranasal testosterone gel is a bioequivalent intranasal testosterone gel.

(139) An intranasal testosterone gel of any one of items 1-138 that is pharmaceutically equivalent.

(140) An intranasal testosterone gel of any one of items 1-138 that is therapeutically equivalent.

(141) An applicator system for pernasal administration of a plurality of doses of a controlled release testosterone gel to deposit the controlled release testosterone gel on an interior surface of an external wall within a nasal cavity located about midway up the nasal cavity, said applicator system comprising:

(a) a chamber pre-filled with the plurality of doses of the testosterone gel;
(b) an actuator nozzle, said actuator nozzle comprising an outlet channel and a tip and having a shape that conforms to the interior surface within the nasal cavity; and
(c) wherein the controlled release testosterone gel comprises amongst other ingredients

(i) testosterone in an amount effective for use in treating testosterone replacement or supplemental therapy,
(ii) at least one lipophilic or partly lipophilic carrier,
(iii) a geling agent

(d) wherein, each said dose deposited within the nasal cavity on the interior surface of the external wall is an uniform dose amount having a dose life; so that, each deposited said dose on the interior surface provides sustained release of a therapeutically effective amount of testosterone from the controlled release testosterone gel over said dose life to achieve a constant testosterone blood level, without significant fluctuations in testosterone blood level, for effective use in treating testosterone replacement or supplemental therapy.

(142) An applicator system of item 141, wherein said applicator system is an airless fluid dispensing system.
(143) An applicator system of item 141, wherein said applicator system is a dip-tube fluid dispensing system.
(144) An applicator system of item 142 or 143, wherein said applicator system is a pump.
(145) An applicator system of item 141, wherein said applicator system is a COMOD system.
(146) An applicator system of item 141, wherein said applicator system is a digital airless applicator system.
(147) An applicator system of any one of items 141-146, wherein the testosterone replacement or supplemental therapy is female sexual dysfunction.
(148) An applicator system of any one of items 141-146, wherein the testosterone replacement or supplemental therapy is male testosterone deficiency.
(149) An applicator system of any one of items 141-146, wherein the dose amount is from about60 to about 180 microliters.
(150) An applicator system of any one of items 141-149, wherein the controlled release testosterone gel further includes a surfactant.
(151) A method of treating a patient in need of testosterone replacement or supplemental therapy, said method comprising:

(a) depositing a dose amount of a controlled release testosterone gel on an interior surface of an external wall within a nasal cavity located about midway up the nasal cavity of a nose having an outer skin surface, wherein the dose has a dose life and the controlled release testosterone gel comprises

(i) testosterone in an amount effective for use in treating testosterone replacement or supplemental therapy, and
(ii) a pharmaceutical vehicle; and,

(b) depressing the outer skin surface of the nose at a location at about opposite the interior surface on which the dose amount was deposited within the nasal cavity to distribute the dose amount within the nasal cavity, wherein, the deposited and distributed dose amount on the interior surface provides sustained release of a therapeutically effective amount of the testosterone from the deposited and distributed dose amount, so that, over the dose life, a constant effective testosterone blood level is achieved, without causing significant fluctuations in testosterone blood level, for effectively treating the patient in need of testosterone replacement or supplemental therapy.

(152) A method of item 151, wherein the dose amount is from about 130 to about 180 microliters.
(153) A method of items 151 or 152, wherein the testosterone replacement or supplemental therapy is female sexual dysfunction.
(154) A method of items 151 or 152, wherein the testosterone replacement or supplemental therapy is male testosterone deficiency.
(155) A method of any one of the items 150-154, wherein the controlled release testosterone gel further includes a surfactant.
(156) An intranasal testosterone gel for topical administration into a nasal cavity to control release of testosterone over a period of at least about 6 hours following the nasal cavity administration to provide replacement testosterone in a male for treating male testosterone deficiency, without causing an unacceptable spike in testosterone blood level in the female over dose life, said intranasal testosterone gel comprising:

(a) testosterone in an amount effective to treat female sexual dysfunction;
(b) at least one lipophilic or partly lipophilic carrier;
(c) a super solvent for increasing testosterone solubility in said intranasal testosterone gel; and
(d) a viscosity regulating agent in an amount effective to control the release of said testosterone from said intranasal testosterone gel, wherein said intranasal testosterone gel does not need a surfactant.

(157) A Testosterone nasal gel according to any of the items 1-156 showing a rate of release through a membrane durapore 0.45 μπ;ι, HVLP02500 of from about 90 micrograms to about 300 micrograms Testosterone /cm2*min".5 /4.0% active.

In general, the present invention relates to an intranasal testosterone gel pharmaceutical composition comprising testosterone and a pharmaceutically acceptable vehicle for testosterone, which vehicle comprises a super solvent or suitable mixtures of super solvents, a gel-forming or viscosity regulating agent to control the release of testosterone from the intranasal testosterone gels and, optionally, a surface active agent or a mixture of surface active agents, i.e., surfactant(s), having surface tension decreasing activity. More specifically, the present invention is drawn to intranasal testosterone gels for topical pernasal administration, e.g., onto the mucosal membranes inside the nasal cavity for each nostril, for the sustained or controlled release of testosterone into the systemic circulations of males and females for providing constant effective testosterone blood levels, without testosterone spike, over dose life, which are effective to effectively treat males and females in need of testosterone replacement or testosterone supplemental therapy who, for example, have been diagnosed with or suffer from either male testosterone deficiency or female sexual dysfunction, wherein the intranasal testosterone gels comprise: (a) a testosterone drug in an amount effective to achieve constant effective testosterone blood levels, for example, between about 0.5% and about 10% by weight; (b) at least one lipophilic or partly lipophilic carrier, such as a liquid oil, to solubilize the testosterone drug; (c) a super solvent or a mixture of super solvents for increasing or enhancing testosterone solubility, especially at higher testosterone drug concentrations, (d) a gel-forming or viscosity regulating agent for creating a sustained release profile for the testosterone; and, optionally, (e) a surface active agent or a mixture of surface active agents, i.e., surfactant(s), having surface tension decreasing activity. While the present invention may be embodied in many different forms, several specific embodiments are discussed herein with the understanding that the present disclosure is to be considered only as an exemplification of the principles of the present invention, and it is not intended to limit the present invention to the embodiments described or illustrated.

Unless otherwise indicated, all numbers expressing quantities, ratios, and numerical properties of ingredients, reaction conditions, and so forth used in the specification and claims are to be understood as being modified in all instances by the term "about", whether or not the term "about" is actually used in sentence construction.

All parts, percentages, ratios, etc. herein are by weight unless indicated otherwise.

As used herein, the singular forms "a" or "an" or "the" are used interchangeably and are intended to include the plural forms as well and fall within each meaning, unless expressly stated otherwise. Also as used herein, "at least one" is intended to mean "one or more" of the listed element(s). Singular word forms are intended to include plural word forms and are likewise used herein interchangeably where appropriate and fall within each meaning, unless expressly stated otherwise. Except where noted otherwise, capitalized and non-capitalized forms of all terms fall within each meaning.

The intranasal testosterone gels of the present invention are chemically and physically stable and can in the dosage form of, for example, a suspension or a solution of the pharmacologically active substance. Preferably, the intranasal testosterone gels are filled into a preservative-free, airless multi-dose device able to accurately deliver doses of the above testosterone gel, also at higher viscosities.

Once at the absorption site, it is believed that the testosterone will be efficiently trapped at the deposition site and be absorbed at a predictable rate across the mucous membrane of the patient, thereby limiting possible, deactivation by metabolizing enzymes and/or protein-binding and testosterone spike.

The intranasal testosterone gels of the present invention comprise (a) the hormone, testosterone, in an amount of from about 0.5% up to about 10% by weight; (b) at least one lipophilic or partly lipophilic carrier; (c) a super solvent or a mixture of super solvents for increasing the solubility of testosterone, (d) a gel-forming or viscosity regulating agent in order to create a sustained release effect regarding testosterone release from the intranasal testosterone gels following pernasal administration and, optionally, (e) a surface active agent or a mixture of surface active agents, i.e., surfactant(s), having surface tension decreasing activity.

The testosterone hormonal drug of this invention may be introduced into the intranasal testosterone gels in a processed form such as microspheres, liposomes, micronized, etc.

The term "lipophilic carrier" shall comprise, but not limited to, a vegetable oil such as castor oil, soybean oil, sesame oil or peanut oil, fatty acid ester such as ethyl-and oleyloleat, isopropylmyristate, medium chain triglycerides, glycerol esters of fatty acids, or polyethylene glycol, phospholipids, white soft paraffin, or hydrogenated castor oil.

Particularly preferred is castor oil, such as Crystal O® or Crystal LC USP.

The incorporation of the testosterone is also possible into an oil mixture and contemplated by the present invention.

The particular amount of oil that constitutes an effective amount is dependent on the particular viscosity regulating agent (see below) used in the testosterone gel. It is therefore not practical to enumerate specific amounts for use with specific formulations of the invention.

Generally, however, the lipophilic part can be present in a formulation in an amount between about 30% and about 98% by weight, preferably between about 42% and about 96% by weight, more preferably between about 67% and about 94% by weight, even more preferably between about 82% and about 95% by weight and most preferably between about 87% and about 94.5% by weight of the testosterone gel.

As discussed above, the intranasal testosterone gels of the present invention include at least one super solvent for enhancing testosterone solubility. The super solvents are generally characterized as non aqueous solvents that are miscible with the carrier or oil and are present in the intranasal testosterone gels in amounts suitable to form a gel during gel formulation or gel manufacture and in advance of pernasal application (the gels do not emulsifiy *in situ* following application into the nasal cavity). Thus, the super solvents as contemplated by the present invention are characterized as (1) enhancing testosterone solubility in the intranasal testosterone gels, (2) being acceptable to the nasal mucosal within the nasal cavities and (3) having no surfactant activity.

Examples of super solvents include dimethyl isosorbide, pharma grade, such as Super Refined Aralasolve®-DMI. diethylene glycol monoethyl ether, such as Transcutol-P®, glycerin, propylene glycol, 1-methyl 2-pyrrolidone, glycerol and satisfactory mixtures thereof. A preferred super solvent for use in accordance with the present invention is a dimethyl isosorbide, such as Super Refined® Arlasolve™-DMI.

While the super solvents of the present invention may be generally present within the intranasal testosterone gels in amounts ranging from about 1% to about 80% by weight, preferable ranges are from about 1% to about 70% by weight, from about 1% to about 60% by weight, from about 1% to about 50% by weight, from about 1% to about 40% by weight, from about 1% to about 30% by weight, from about 1% to about 20% by weight and from about 1% to about 10% by weight. A more preferable range is from about 1% to about 25% by weight, whereas an even more preferable range is from about 5% to about 20% by weight, and an even more preferable range is from about 5% to about 15%. One preferable concentration for a super solvent formulated in an intranasal testosterone gel of the present invention is about 15% by weight. See Fig.18.

The term "viscosity regulating agent" shall mean a thickener or gelling agent. Examples are, but not limited to, cellulose and cellulose derivatives thereof, such as hydroxypropyl cellulose and hydroxyethyl cellulose, polysaccharides, carbomers, acrylic polymers, such as Carbopol®, polyvinyl alcohol and other vinylic polymers, povidone, Co-Polyvidone (Kollidon VA64) colloidal silicon dioxide, such as Aerosil® 200 or Cab-O-Sil®, such as Cab-O-Sil® M-5P, lipophilic silicon dioxide, such as Aerosil® R972, cetyl alcohols, stearic acid, glyceryl behenate, wax, beeswax, petrolatum, triglycerides, lanolin and suitable mixtures thereof. It is believed, however, that colloidal silicon dioxide (such as Aerosil® 200, as available from Degussa), $SiO_2$ and polyvinyl alcohol are particularly useful.

The incorporation of the testosterone drug is also possible into a mixture of thickeners or gelling agents.

The particular amount of thickener/gelling agent that constitutes an effective amount is dependent on the particular oil or oil mixture (see above) used in the formulation. It is therefore not practical to enumerate specific amounts for use with specific formulations of the invention. Generally, however, the thickener/gelling agent(s) can be present in a formulation in an amount from about 0.5 to about 10% by weight, preferably about 0.5 to about 5% by weight, more preferably about 1 to about 3% by weight, and most preferably at about 3% by weight.

The Testosterone gel may further optionally, but not necessarily, include a surfactant such as, but not limited to, lecithin, fatty acid ester of polyvalent alcohols, of sorbitanes, of polyoxyethylensorbitans, of polyoxyethylene, of sucrose, of polyglycerol and/or at least one humectant such as sorbitol, glycerine, polyethylene glycol, or macrogol glycerol fatty acid ester. Particularly useful, however, are oleoyl macrogolglycerides (such as Labrafil® M 1944 CS, as available from Gattefossé (France)).

The incorporation of the testosterone drug is also possible into a surfactant mixture.

The particular amount of surfactant that constitutes an effective amount is dependent on the particular oil or oil mixture (see above) used in the testosterone gel. It is therefore not practical to enumerate specific amounts for use with specific formulations of the invention. Generally, however, the surfactant can be present in a formulation in an amount of from between about 0.5 to about 10% by weight, preferably about 0.5 to about 5% by weight, more preferably 1 to 4% by weight, and most preferably at about 3% by weight.

The intranasal testosterone gels of the present invention can be applied once a day ("QD"), twice a day ("BID"), three times a day ("TID"), four times-a-day ("QID") or as needed ("prn"). Regardless of the administration regimen, the intranasal testosterone gels are topically applied onto the nasal mucosal in the nasal cavity, preferably, of each nostril per application ("pernasal"). More specifically, the intranasal testosterone gels are topically applied to the outer external walls (opposite the nasal septum) inside the naval cavity of each nostril, preferably at about the middle to about the

upper section of the outer external wall (opposite the nasal septum) or at about under the cartilage section of the outer external wall (opposite the nasal septum) inside the naval cavity of each nostril. It is believed that, if the gels are applied to deep up into the nostrils, the gel dosages will unfortunately wash into the throat or, if the gels are applied to shallow down in the forefront or adjacent the external openings of the nostrils, the gel dosages will unfortunately flow out from inside the naval cavity possibly leading, in either situation, to ineffective dosing and poor compliance.

Once a selected dose of an intranasal testosterone gel of the present invention has been topically applied or deposited onto the appropriate designated region on the outer external walls (opposite the nasal septum) in the nasal cavity of each nostril of a male or female patient in need of testosterone replacement or supplemental therapy, it is preferable for the male or female patient to message the outer skin of his/her nose to distribute the applied or deposited intranasal testosterone gel dose evenly and throughout the nasal cavity of each nostril.

The intranasal testosterone gels, once formulated, are preferably filled into a preservative-free, airless nasal spray or dispensing multi-dose device, such as the COMOD system available from Ursatec, Verpackung-GmbH, Schillerstr. 4, 66606 St. Wendel, Germany, or the Albion or Digital airless applicator systems available from Airlessystems, RD 149 27380 Charleval, France or 250 North Route 303 Congers, NY 10950, which allow pernasal application without contamination from fingertips, as shown in Figs. 1-6. Preferably, the airless nasal pre-filled spray or applicator multi-dose device includes a dispensing element for topically applying the intranasal testosterone gel dose at about a location within each nostril as described herein above. The dispensing element by way of example is bent or curved-shape to strategically permit consistent topical applications of the intranasal testosterone gels in about the prescribed amounts and at about the preferred location within each nostril (pernasal) to maximize effectiveness in the treatment of female sexual dysfunction in females patients and testosterone deficiency in male patients with the intranasal testosterone gels of the present invention.

By "constant effective testosterone blood levels" is meant that after a single topical application or after daily dosing, whether using a QD, BID, TID, QID or prn dosing regimen, the serum level of testosterone in a male or female patient in need of testosterone replacement or supplemental therapy is higher than baseline, i.e., the testosterone serum level is either (a) restored to, (b) approaching or (c) is very similar to, resembles or closely mimics normal testosterone blood levels found in healthy young men such as, by way of example, a male testosterone blood level of between about 200 nanograms and about 1500 nanograms of testosterone per deciliter of blood, or more particularly a male testosterone blood level of between about 300 ng/dl and about 1200 ng/dl, or more particularly a male testosterone blood level of between about 350 ng/dl and about 800 ng/dl, or more particularly a male testosterone blood level of between about 350 ng/dl and about 600 ng/dl, or more particularly a male testosterone blood level of between about 380 ng/dl and about 450 ng/dl, or more particularly a male testosterone blood level of at about 380 ng/dl, and in healthy young women, such as, by way of example, a female testosterone blood level of between about 30 and about 150 nanograms of testosterone per deciliter of blood, or more particularly a female testosterone blood level of between about 35 ng/dl and 95 ng/dl, or more particularly a female testosterone blood level of between about 40 ng/dl and 70 ng/dl, or more particularly a female testosterone blood level of at between about 40 ng/dl and 50 ng/dl, or more particularly a female testosterone blood level of at about 40 ng/dl, and such testosterone blood levels are generally constantly maintained over dose life, e.g., for about a 6 hour dose life, more preferably for about an 8 hour dose life and more preferably for at least about a 10 hour dose life, and even more preferably for at least about a 12 hour dose life, and/or throughout duration of testosterone replacement or supplemental therapy, whether administered as a single dose or multiple dosages, or administered once-a-day, twice-a-day, three times-a-day, four times-a-day, administered as prn, or administered in accordance with any suitable treatment regimen that does not defeat the objectives of the present invention.

Because testosterone is nearly insoluble in water, liberation from the formulation is the speed-limiting step for adsorption. It has been surprisingly found that the incorporation of testosterone in an oily formulation containing a suitable surfactant according to the invention leads to physiologic serum levels and to a steady, sustained action of testosterone over time.

It is believed that the release of the hormone is sustained due to its solubility in the oily carrier and to the viscosity of the intranasal testosterone gel formulation remaining on the mucous membrane for a prolonged duration of time.

It also is believed that, upon contact of an intranasal testosterone gel of the present invention with the humidity of the mucous membrane, the testosterone's release is controlled or slowed by properties containing the testosterone. Thus, by adding a gel-forming or viscosity regulating agent to the intranasal testosterone gels of the present invention to create a desired gel viscosity, the dissolution pattern of the testosterone from the intranasal testosterone gels becomes more favorable and effective because there is no testosterone spike variability in dissolution ensuring constant effective testosterone blood levels or constant testosterone dose bioequivalence over dose life.

The intranasal testosterone gels of the present invention can be manufactured as follows. Add a lipophilic carrier, e.g., castor oil, to a homogenizer under vacuum and nitrogen The testosterone can then be slowly added to the lipophilic carrier and homogenized until mixing is complete to form an intermediate product. Once the intermediate homogenate is allowed to cool to about room temperature, the super solvent and optionally, a surfactant, can then be

added to the intermediate product to form a basic mixture. Once the basic mixture is cooled, the gel-forming or viscosity regulating agent can then be added to the cooled basic mixture and mixed under vacuum to achieve a final product with a desired gel viscosity.

More specifically, the starting materials should be are kept in quarantine and sterile. After each manufacturing step (e.g., dissolution, homogenization), the resulting product should be stored in quarantine until next production step. Samples for quality control may be taken at different stages of the manufacturing process. The batch of a finished product should likewise be stored in quarantine until use.

The protocol for the total manufacturing process of a respective batch and the batch number are should be established and recorded. All equipment, containers and samples for in-process controls should be labeled using this number.

The necessary amount of the testosterone for the bulk mixture is calculated on the basis of content determination of a respective batch. This determination is part of the quarantine procedure for the starting material. The amount is calculated in such a way that the necessary content of 100% testosterone will be reached in the bulk mixture.

The calculation and the corresponding weighing procedure should be documented in the production, protocol.

The respective substances are weighed using an electronic balance with registration of weight, and sieved.

The manufacture of an intranasal testosterone gel is performed by thickening of an oil mixture and packaging by blow-fill-seal technology.

A lipophilic carrier, e.g., castor oil, and a super solvent, e.g., DMI, (Step 1) are introduced into a mixing vessel (e.g., a FrymaKoruma Vacuum-Mixing, Dispersing and Homogenising machine type Dinex 700). The carrier and super solvent mixture are covered by nitrogen (Step 2) to exclude oxygen and preheated, for example to from about 40°C to about 50°C.

Sieved (mesh size is about 2 mm) testosterone (Step 3) is added to the carrier and super solvent mixture and processed to give the Intermediate Product as follows.

Control of temperature of the carrier and super solvent mixture is necessary in the following steps to prevent a significant increase in temperature due to the shear intensity applied to dissolve testosterone.

| Step No. | Position Name |
|---|---|
| 1. | Carrier, such as castor oil, and Super Solvent such as DMI |
| 2. | Nitrogen |
| 3. | Testosterone |
| 4. | Intermediate Product |

Homogenization of the Intermediate Product is done by controlling the temperature of the mixture not to exceed about 50°C. Three (3) such cycles are believed to be necessary until the testosterone is completely dissolved. Each cycle has the following course: Homogenization in dispersing mode for a specified mix cycle.

Optionally, a surfactant, such as an oleoyl macrogol-glycerides, (Step 5) is added to the Intermediate Product to give the basic mixture.

| Step No. | Position Name |
|---|---|
| 4. | Intermediate Product |
| 5. | Oleoyl macrogol-glycerides |
| 6. | Basic Mixture |

After the 3$^{rd}$ homogenization cycle, the Basic Mixture (Step 6) should be checked for content of testosterone taking care that the dissolution of testosterone is complete. The content can be examined by UV method and the Basic Mixture should have a testosterone amount in mg equivalent to the selected testosterone concentration.

After the last homogenization step, the Basic Mixture should be cooled until a temperature of about 40°C (±2°) or less is reached. Thereafter, the gel-forming agent, such as colloidal silicon dioxide, (Step 7) can be added to the Basic Mixture.

| Position No. | Position Name |
|---|---|
| 6. | Basic Mixture |
| 7. | Colloidal Silicon Dioxide |

(continued)

| Position No. | Position Name |
|---|---|
| 8. | Final Mixture |

The introduction of colloidal silicon dioxide is performed and then the mixer is adjusted to the conditions: Homogenization takes place in dispersing mode under vacuum with agitation.

The Final Mixture can be checked visually for homogeneity and, after release, when subjected to deaeration under vacuum and agitation.

The resulting homogeneous intranasal testosterone gel is then ready for discharge into stainless steel holding tanks. Before closing the container, the Final Bulk mixture can be coated or covered with nitrogen of about 0.5 to about 1.5 bar.

As described above, to administer the intranasal testosterone gels of the present invention, it is preferable to use multi-dose devices that allow delivery of precise dosage amounts to the external wall in each nostril of the middle-upper nasal cavity (under cartilage) for depositing the dosage thereon. Once the testosterone gel has been administered onto the external wall of the nasal cavity of a nostril, the outer nose should be gently messaged with fingers to evenly distribute the intranasal testosterone gel throughout the nasal cavity without or minimal dosage loss into the throat or outside the nose. Examples of multi-dose devices for pernasal deposition at the preferred location within the nose in accordance with the present invention include the COMOD system available from Ursatec, Verpackung-GmbH, Schillerstr. 4, 66606 St. Wendel, Germany or the Albion or Digital airless applicator systems available from Airlessystems, RD 149 27380 Charleval, France or 250 North Route 303 Congers, NY 10950, as shown in Figs. 1-4.

A nasal multi-dose dispenser device according to embodiments of the present invention, such as the Albion or Digital airless applicator systems available from Airlessystems, is comprised of a fluid container and a distributor pump for delivery of multiple doses of a gel or other topical formulation. In one embodiment of the present invention, the nasal multi-dose dispenser device is adapted for an airless fluid dispensing system. In another embodiment of the present invention, the nasal multi-dose dispenser device is adapted for a dip tube fluid dispensing system.

An example of an airless system that is contemplated by the present invention is one that will deliver a liquid, including gel, without the need for a pressured gas or air pump to be in contact with the liquid (or gel). In general, an airless system of the present invention comprises a flexible pouch containing the liquid, a solid cylindrical container a moving piston, an aspirating pump, a dosing valve and a delivery nozzle, as depicted, for example, in Figs. 1-4.

In accordance with the present invention, the multi-dose dispenser 100 of Fig. 1 is provided with a fluid container 120, a distributor pump 140 and a cap 102.

The fluid container 120 comprises a container body 122, a base 124 and a neck 126. The distributor pump 140 is fastened to the neck by a sleeve 128. The top end of the container body 122 is closed by the distributor pump 140. The sleeve 128 tightly pinches a neck gasket 150 against the top end of the container body 122. The container body 122 forms a vacuum and houses the fluid to be dispensed.

The distributor pump 140 is closed by its actuator nozzle 130, which retains the stem 144 at the stem head. The actuator nozzle 130 comprises an outlet channel 132 and tip 134.

The actuator nozzle 130 is shaped to conform with the interior surface of a user's nostril. The actuator nozzle 130 is moveable between a downward open position and upward closed position. The user removes the cap 102 and inserts the actuator nozzle 130 in the user's nostril. When the user pushes the actuator nozzle 130 downwards to the open position, fluid in the dosing chamber 180 is withdrawn by the distributor pump 140 and exits at the tip 134 via the outlet channel 132 of the actuator nozzle 130.

Fig. 2 shows a cross-sectional view of the distributor pump 140.

The distributor pump has a body 142 provided with a bottom intake having an inlet valve 160 with a ball 162 as its valve member. The ball 162 is held in place by a cage 164 and by a return spring 170.

At its bottom end, the stem 144 carries a spring cap 172. A piston 174 is located above the spring cap 172. The stem 144 passes through an axial orifice of the piston base 176.

The side walls of the piston 174 seals against the distributor pump body 142 via lips. The sleeve 128 tightly pinches a stem gasket 152 against the stem collar 146, distributor pump body 142 and top of the piston 174.

A precompression spring 178 placed between the piston base 176 and the stem collar 146. The precompression spring 178 biases the actuator nozzle 130 via the stem 144 to the closed position.

The return spring 170, which returns the piston 174 back upwards, is compressed between two opposed seats on the cage 164 and the spring cap 172.

The distributor pump 140 has a dosing chamber 180 formed between the cage 164 and piston 174. When the user pushes the actuator nozzle downwards to the open position, fluid in the dosing chamber is withdrawn by the distributor pump 140 and dispensed from the tip of the actuator nozzle 130.

When the user releases the actuator nozzle 130 upwards to the closed position, a fluid in the container body 122 is withdrawn into the dosing chamber 180 by the distributor pump 140. Thus, a dose of fluid is ready for the next actuation of the actuator nozzle by the user.

In another embodiment of the present invention, the dispenser 200 of Fig. 3 is provided with a fluid container 220, a distributor pump 240 and a cap 202.

The fluid container 220 comprises a container body 222, a base 224 and a neck 226. The distributor pump 240 is fastened to the neck by a sleeve 228. The top end of the container body 222 is closed by the distributor pump 240. The sleeve 228 tightly pinches a neck gasket 250 against the top end of the container body 222. The container body 222 houses the fluid to be dispensed.

The distributor pump 240 is closed by its actuator nozzle 230, which retains the stem 244 at the stem head. The actuator nozzle 230 comprises an outlet channel 232 and tip 234. The actuator nozzle 230 is shaped to conform with the interior surface of a user's nostril. The actuator nozzle 230 is moveable between a downward open position and upward closed position. The user removes the cap 202 and inserts the actuator nozzle 230 in the user's nostril. When the user pushes the actuator nozzle 230 downwards to the open position, fluid in the dosing chamber 280 is withdrawn by the distributor pump 240 and exits at the tip 234 via the outlet channel 232 of the actuator nozzle 230.

Fig. 4 shows a cross-sectional view of the distributor pump 240.

The distributor pump has a body 242 provided with a bottom intake having an inlet valve 260 with a ball 262 as its valve member. The ball 262 is held in place by a cage 264 and by a return spring 270. Optionally, a dip tube 290 can extend downward from the inlet valve 260 and is immersed in the liquid contained in the container body.

At its bottom end, the stem 244 carries a spring cap 272. A piston 274 is located above the spring cap 272. The stem 244 passes through an axial orifice of the piston base 276.

The side walls of the piston 274 seals against the distributor pump body 242 via lips. The sleeve 228 tightly pinches a stem gasket 252 against the stem collar 246, distributor pump body 242 and top of the piston 274.

A precompression spring 278 placed between the piston base 276 and the stem collar 246. The precompression spring 278 biases the actuator nozzle 230 via the stem 244 to the closed position.

The return spring 270, which returns the piston 274 back upwards, is compressed between two opposed seats on the cage 264 and the spring cap 272.

The distributor pump 240 has a dosing chamber 280 formed between the cage 264 and piston 274. When the user pushes the actuator nozzle downwards to the open position, air enters the dosing chamber 280, which forces the fluid in the dosing chamber to be withdrawn by the distributor pump 240 and dispensed from the tip of the actuator nozzle 230.

When the user releases the actuator nozzle 230 upwards to the closed position, the air contained in the dosing chamber 280 forces the fluid in the container body 222 to be withdrawn into the dosing chamber 280. Thus, a dose of fluid is ready for the next actuation of the actuator nozzle by the user.

The amount of fluid withdrawn by the distributor pump into the dosing chamber may be a fixed volume. The distributor pumps may be of a variety of sizes to accommodate a range of delivery volumes. For example, a distributor pump may have a delivery volume of 140 μl.

The dispensers of the present invention may dispense topical intranasal gel or other topical intranasal formulations, preferably pernasally, which contain alternative or additional active ingredients, such as neurosteroids or sexual hormones (e.g., androgens and progestins, like testosterone, estradiol, estrogen, oestrone, progesterone, etc.), neurotransmitters, (e.g., acetylcholine, epinephrine, norepinephrine, dopamine, serotonin, melatonin, histamine, glutamate, gamma aminobutyric acid, aspartate, glycine, adenosine, ATP, GTP, oxytocin, vasopressin, endorphin, nitric oxide, pregnenolone, etc.), prostaglandin, benzodiazepines like diazepam, midazolam, lorazepam, etc., and PDEF inhibitors like sildenafil, tadalafil, vardenafil, etc., in the form of a liquid, cream, ointment, salve or gel. The dispensers may be suitable for cosmetic, dermatological or pharmaceutical applications. Examples of topical intranasal formulations for topical pernasal application, which can be dispensed in accordance with the present invention include the pernasal testosterone gels of the present invention or other intranasal topical gels wherein the testosterone is replaced or combined with a another active ingredient in effective amounts, such as those active ingredients discussed herein above. In addition, other testosterone formulations suitable and contemplated for dispensing from the dispensers and/or in accordance with the methods of the present invention include the formulations dislcosed in, for example, U.S. Patent Nos. 5,578,588, 5,756,071 and 5,756,071 and U.S. Patent Publication Nos. 2005/0100564, 2007/0149454 and 2009/0227550, all of which are incorporated herein by reference in their entirties.

Examples of various embodiments of the present invention will now be further illustrated with reference to the following examples. Thus, the following examples are provided to illustrate the invention, but are not intended to be limiting thereof. Parts and percentages are by weight unless otherwise specified.

## Example 1

Examples of Testosterone Gel Formulations

Examples of testosterone gels of the present invention are illustrated in Tables 1-3 below.

**Table 1**

| Material | Gel 1 | Gel 2 | Gel 3 | Gel 4 | Gel 5 |
|---|---|---|---|---|---|
| Testosterone* | 3.5 % | 4.0% | 4.5% | 5.5% | 2.5% |
| Castor Oil | 94.5 % | 90% | 88% | 82.5% | 91.5% |
| Super Refined® Arlasolve™ DMI | ----- | 4% | 5.5% | 10% | 4% |
| $SiO_2$ | 2.0% | 2.0% | 2.0% | 2.0% | 2.0% |
| Total % | 100% | 100% | 100% | 100% | 100% |
| *micronized is used | | | | | |

**Table 2**

| Materials | Gel 6 | Gel 7 | Gel 8 | Gel 9 |
|---|---|---|---|---|
| | | | | |
| Testosterone* | 5.5 % | 6.0 % | 6.50 % | 7.0% |
| Transcutol P® | 5.0% | 5.0% | 10.0 % | 10 0 % |
| Povidone K17 | 4 | 4 | 4 | 4 |
| $SiO_2$ | 3.0% | 3.0% | 3.0% | 3.0% |
| Castor Oil | 80.5 % | 80.0 % | 745 % | 74.0 % |
| Total % | 100% | 100% | 100% | 100% |
| *Micronized is prefarably used | | | | |

**Table 3**

| Materials | Gel 10 | Gel 11 | Gel 12 | Gel 13 |
|---|---|---|---|---|
| | | | | |
| Testosterone* | 6.0 % | 6.5.0 % | 9.0% | 10.0 % |
| Super Refined® Arlasolve™ DMI | 10.0 % | 10.0% | 25.0% | 50.0 % |
| Kollidon VA64 | 4 | 4 | 4 | 4 |
| $SiO_2$ | 3.0% | 3.5% | 3.0% | 3.0% |
| Castor Oil | 73.0 % | 76,0 % | 63.0 % | 37.0 % |
| Total | 100 | 100 | 100 | 100 |
| *Micronized is preferably used | | | | |

Intranasal testosterone gel formulations 14-21 are further examples of gel formulations contemplated by the present invention (Per Hundred parts). Testosterone in micronized form is preferred.

**Gel 14**

| | |
|---|---|
| Castor Oil | 83 |
| DMI (dimethyl isosorbide) | 10 |
| Testosterone | 4 |
| Aerosil® 200 | 3 |

**Gel 15**

| | |
|---|---|
| Castor Oil | 79 |
| DMI | 12 |
| Testosterone | 5 |
| Cab-O-sil® M5P | 4 |

**Gel 16**

| | |
|---|---|
| Mid chain triglycerides (Labrafac®) | 91.5 |
| DMI | 5.5 |
| Testosterone | 0.5 |
| Cab-O-sil® | 2.25 |
| PVA | 0.25 |

**Gel 17**

| | |
|---|---|
| Labrafac® WL 1349 | 63 |
| DMI | 20 |
| Transcutol® (Diethylene glycol monoethyl ether) | 5 |
| Testosterone | 7 |
| Kollidon | 2 |
| HPC | 0.1 |
| Aerosil® 200 | 2,9 |

**Gel 18**

| | |
|---|---|
| Labrafac® PG (P-glycol dicaproylate) | 40 |
| DMI | 25 |
| Propylene Glycol | 10 |
| Transcutol® | 10 |
| Testosterone | 8 |
| Povidone K30 | 2 |
| HPC | 0.2 |
| Aerosil® R972 | 4.8 |

**Gel 19**

| | |
|---|---|
| Isopropyl Myristate | 18 |
| Almond Oil | 50 |
| 2-Pyrrolidone | 10 |
| Transcutol® | 10 |
| Testosterone | 6.5 |
| Carbopol® 934 | 0.5 |
| Glyceryl Behenate | 5.0 |

**Gel 20**

| | |
|---|---|
| Mid chain triglycerides (Labrafac®) | 4 |
| *Labrafil® M1944CS* | 55 |
| DMI | 20 |
| Transcutol® | 10 |
| Testosterone | 8 |
| Cab-O-Sil® | 2.75 |
| HPC | 0.25 |

**Gel 21**

| | |
|---|---|
| Mid chain triglycerides (Labrafac®) | 55 |
| *Caprylocaproyl macrogolglycerides* | 4 |
| DMI | 20 |
| Transcutol® | 12 |
| Testosterone | 6 |
| Cab-O-Sil® | 2.75 |
| HPC-L | 0.25 |
| "Surface tension agent" in italic | |

**Gel 22**

| | |
|---|---|
| Castor Oil | 58 |
| DMI | 20 |
| Transcutol P | 5 |
| Kollidon 17 PF | 5 |
| Si O2 | 2 |
| Testosterone | 8 |

**Gel 23**

| | |
|---|---|
| Castor Oil | 62 |
| DMI | 20 |
| Transcutol P | 5 |
| Kollidon 17 PF | 2 |
| HPC GF | 1 |
| Si O2 | 2 |
| Testostérone | 8 |

**Gel 24**

| | |
|---|---|
| Castor Oil | 62 |
| DMI | 20 |
| Transcutol P | 5 |
| Kollidon VA 64 | 2 |
| HPC GF | 1 |
| Si O2 | 2 |
| Testostérone | 8 |

**Gel 25**

| | |
|---|---|
| Castor Oil | 62.5 |
| DMI | 20 |
| Transcutol P | 5 |
| Kollidon 17 PF | 2 |
| HPC HF | 0.5 |
| Si O2 | 2 |
| Testostérone | 8 |

**Gel 26**

| | |
|---|---|
| Castor Oil | 62.5 |
| DMI | 20 |
| Transcutol P | 5 |
| Kollidon VA 64 | 2 |
| HPC HF | 0.5 |
| Si O2 | 2 |
| Testosterone | 8 |

**Gel 27**

| | |
|---|---|
| Castor Oil | 58 |
| DMI | 20 |
| Transcutol P | 5 |
| Kollidon 17 PF | 5 |
| Si O2 | 2 |
| Testosterone | 8 |

**Gel 28**

| | |
|---|---|
| Castor Oil | 74 |
| DMI | 15 |
| Transcutol P | 2.5 |
| Kollidon 17 PF | 2 |
| HPC XHF | 0.5 |
| Si O2 | 2 |
| Testosterone | 4 |

| Material | TBS1A 4 % | Quantity per 1 kg batch | TBS1A 4 % alternate | Quantity per 1 kg batch | TBS1A 8 % | Quantity per 1 kg batch |
|---|---|---|---|---|---|---|
| Super refined Arlasolve | 25.0 | 250 g | 15.0 | 150 g | 25.0 | 250 g |
| Transcutol P | 10.0 | 100 g | 5.0 | 50 g | 10.0 | 100 g |
| Plasdone K17 | 3.0 | 30 g | 3.0 | 30 g | 3.0 | 30 g |
| Plasdone S 630 | 2.0 | 20 g | 2.0 | 20 g | 2.0 | 20 g |
| Klucel HF | 0.5 | 5 g | 0.5 | 5 g | 0.5 | 5 g |

(continued)

| Material | TBS1A 4 % | Quantity per 1 kg batch | TBS1A 4 % alternate | Quantity per 1 kg batch | TBS1A 8 % | Quantity per 1 kg batch |
|---|---|---|---|---|---|---|
| Testosterone micronized | 4.0 | 40 g | 4.0 | 40 g | 8.0 | 80 g |
| Castor Oil | 50.5 | 505 g | 65.5 | 655 g | 46.5 | 465 g |
| Cab-o-Sil M5P | 5.0 | 50 g | 5.0 | 50 g | 5.0 | 50 g |
| | 100.0 | 1000 g | 100.0 | 1000 g | 100 | 1000 g |

Potential batch size 1.0 - 2.0 kg. Critical volume available is Super Refined Arlasolve. May limit batch size to 1 kg (or 1.5 kg) to assure sufficient material on hand in case of having to repeat an IMP batch.

**Excipients and role**

Castor Oil - main solvent

Povidone - Solvating polymer (Kollidon 17PF or Plasdone K 17)

Copolyvidone - solvating polymer (Kollidon VA 64 or Plasdone S630)

Dimethyl Isosorbide - super solvent (Arlasolve)

Diethylene Glycol Monoethyl Ether - super solvent (Transcutol)

Hydroxypropyl cellulose (HPC) - viscosifying agent/ SR agent (Klucel XHF or G250)

Silicon Dioxide - viscosifying agent (Aerosil)

## Example 2

An open label, balanced, randomized, crossover, two-group, two-treatment (Dose Level 1 and 2), two-period, pharmacokinetic study of two dose levels of intranasal testosterone gel formulation, i.e. Compleo™ of Trimel Biopharma, Inc., Canada, in healthy, adult, male human subjects

Test product: Testosterone gel for pernasal administration.

Profile Level 1:

Nasobol® syringes pre-filled with 4.5% testosterone gel to deliver 6.75mg of testosterone per each nostril (manufactured by Trimel Biopharma, Inc. Canada). The Nasobol® formulation is as follows:

4.5% Testosterone
4% Labrafil® M1944
3% Aerosil® ($SiO_2$)
88.5% Castor Oil.

Profile Level 2:

Compleo™ syringes pre-filled with 6.5% testosterone gel to deliver 9.75 mg of testosterone per each nostril (manufactured for Trimel Biopharma, Inc. Canada), based on a pre-filled weight of 150 mg of Compleo™ gel. The Compleo™ gel formulation is as follows:

| | |
|---|---|
| Castor Oil | 65.5 |
| DMI | 20.0 |
| Transcutol® (Diethylene glycol monoethyl ether) | 5.0 |
| Testosterone | 6.5 |
| HPC | 0.1 |
| Aerosil® 200 | 2.9 |

## Example 3

*Contains Nonbinding*

*Recommendations*

**Guidance on**

**Testosterone**

This guidance represents the Food and Drug Administration's (FDA's) current thinking on this topic. It does not create or confer any rights for or on any person and does not operate to bind FDA or the public. You can use an alternative approach if the approach satisfies the requirements of the applicable statutes and regulations. If you want to discuss an alternative approach, contact the Office of Generic Drugs.

| | |
|---|---|
| **Active ingredient:** | Testosterone |
| **Form/Route:** | Extended Release Tablets/Buccal |
| **Recommended studies:** | 2 Studies |

Type of study: Fasting
Design: Single-dose, two-way crossover *in-vivo* Strength: 30 mg
Subjects: Testosterone-deficient (hypogonadal) males Additional Comments:

- Subjects should not currently be receiving any treatment for their hypogonadism.
- The inclusion criterion for testosterone-deficient (hypogonadal) males is serum testosterone levels below 2.5 ng/ml.
- At least three predose levels will serve as baseline.
- A 'fed' BE study is not recommended because the product is a buccal adhesive, not to

be ingested. This obviates the need for oral dose dumping assessment due to food.
Type of study: *In vitro* adhesion comparative performance testing study
Design: A tensiometry study is recommended to compare the peak detachment force for test and reference products.[1] Water is recommended between the buccal tablets and the base plate of the tensiometer. The loading weight and length of time the loading weight is applied to press the buccal tablet into contact with the base plate should be specified. Following removal of the weight, the rate at which the buccal tablet is pulled away from the base plate should be specified. The peak detachment force should be measured as the force required to detach the buccal tablet from the base plate. The comparative adhesion test should be conducted using 12 individual units of the test and reference products.

Prior to conducting studies for submission to the ANDA, the firm should determine appropriate loading weight, length of time the loading weight is applied to press the buccal tablet into contact with the base plate of the tensiometer, and the rate at which the buccal tablet is pulled away from the base plate.[2] These studies should be conducted to assure the appropriateness of the test conditions to the test and reference products. See, for example, HE Junginger et al: Mucoadhesive hydrogels in drug delivery. Encyclopedia Pharm Technol (2002); and SJ Jackson, AC Perkins: In vitro

assessment of the mucoadhesion of cholestyramine to porcine and human gastric mucosa. Eur J Pharm Biopharm. 52:121-127 (2001).

**Analytes to measure (in appropriate biological fluid):** Total testosterone in plasma. **Bioequivalence based on (90% CI):** Baseline-adjusted testosterone
**Waiver request of in-vivo testing:** Not Applicable

Dissolution test method and sampling times:
Please note that a **Dissolution Methods Database** is available to the public at the OGD website at http://www.fda.gov/cder/ogd/index.htm. Please find the dissolution information for this product at this website. Please conduct comparative dissolution testing on 12 dosage units each of all strengths of the test and reference products. Specifications will be determined upon review of the application.

## Example 4

Testosterone is indicated as a hormone replacement therapy for males having conditions associated with a deficiency or absence of endogenous testosterone. Lack of testosterone may cause sexual dysfunction, muscle loss, increase in fat, infertility, decreased beard and body hair and other conditions.
Cornpleo™ is a semi-solid Castor oil-based bioadhesive gel formulation containing the hormone Testosterone. Compleo™ is being assessed as a treatment for Hypogonadism in males (both primary and secondary) and is administered to the nasal cavity. In previous clinical studies testing the efficacy of Compleo™, a number of different dispensers have been used to administer the gel to the nasal cavity, including single dose blow fill seal dispensers, and single dose syringes. Recently, a multiple dose dispenser with a tip for nasal deposition has been designed to deliver Compleo™ to the nasal mucosa. The key components of the multiple dose dispenser include a barrel, piston, base, pump and actuator. The dispenser utilises atmospheric pressure and is designed to deliver the required dose. A valve is opened in the pump mechanism when the digital actuator is pressed. This allows atmospheric pressure to act on the piston via the base of the barrel, forcing it upwards. Consequently, the gel is forced through the tip to the correct location in the nasal cavity.
This study was designed to compare the placement properties for the multiple dose dispenser and the single dose syringe. The study was conducted to examine the placement of the gel from each dispenser, the ease of use for each device and the size of the gel droplet.
Three healthy subjects were included in the study (two male and one female), with each subject testing both the single dose syringe and the multiple dose dispenser filled with placebo gel. Placement location and droplet size were observed and recorded by the principle investigator. A photograph was taken following each administration for comparison purposes. After administration, the subjects are asked for feedback on ease of use for each dispenser.
The primary objective of this study was to compare the placement of the gel following intranasal administration from the two different dispensers through visual observation by the principle investigator.
This is an open label study in healthy subjects using placebo gel (125ul) that is administered intra-nasally using two different dispensers, a single dose syringe and a multiple dose dispenser.
Subjects are required to visit the study site on one (1) occasion. The study drug is administered according to the following schedule:

| Subject Number | Administration 1 | Administration 2 |
|---|---|---|
| 1 | Single Dose Syringe/Right nostril | Multiple Dose Dispenser/Left nostril |
| 2 | Multiple Dose Dispenser/Left nostril | Single Dose Syringe/Right nostril |
| 3 | Single Dose Syringe/Right nostril | Multiple Dose Dispenser/Left nostril |

Following gel administration in each nostril, a photograph is taken of the nasal cavity using the KarlStorz 0° rigid endoscope and the Storz AIDA image capture platform. A visual observation is made and recorded by the principle investigator on the location of the gel deposit and the size of the gel droplet. Each subject is then asked about the ease of use of the dispenser.
Three healthy subjects , one female (subject 1) and two male (subjects 2 and 3) participated in the study.
The description of the gel placement from principle investigator and the ease of use assessment by the subject is summarized in Table 1.
All of the subjects were able to administer the investigational product effectively using both the multiple dose dispenser and the single dose syringe. The placement of the gel inside the nasal cavity was the same following administration via the multiple dose dispense and the single dose syringe. Similarly the size of the gel deposit was consistent from each of the dispensers regardless of the nostril into which the gel was applied. These observations are supported by the photographs following each administration as provided in Figs. 7-9.

Two of the subjects, #1 and #3, comment that the multiple dose dispenser is more comfortable than the single dose dispenser with respect to ease of use.

**Table 1: Description Data following gel administration by the two dispensers**

| Subject Number | Dispenser | Nostril | Location of gel deposition | Size of Gel deposit | Ease of use of dispenser | Comments |
|---|---|---|---|---|---|---|
| 1 | Single Dose | Right | Lateral nasal wall near valve | Adequate | No issues observed | Less comfortable |
| | Multiple Dose | Left | Lateral nasal wall near valve | Adequate | No issues | Very comfortable |
| 2 | Single Dose | Right | Lateral nasal wall near valve | Adequate | No issues | |
| | Multiple Dose | Left | Lateral nasal wall near valve | Adequate | No issues | |
| 3 | Single Dose | Right | Lateral nasal wall near valve | Adequate | No issues observed | Less comfortable |
| | Multiple Dose | Left | Lateral nasal wall near valve | Adequate | No issues observed | Very comfortable |

The purpose of this study is to compare the gel deposition between the two different dispensers. Proper placement of the gel in the nasal cavity and ease of use for the patient are key considerations for any proposed dispenser. The syringe is used to administer Compleo in previous clinical trials while the multiple dose dispenser is proposed as the dispenser of choice for future clinical studies.

The results of the study demonstrate that both the multiple dose dispenser and the single dose syringe are capable of depositing the gel in the correct location in the nasal cavity and provide a gel deposit that is similar in size. The observations of gel placement and deposition are confirmed by photography.

The subjects report differences in the ease of use of the dispensers. Two of the three subjects respond that the multiple dose dispenser is more comfortable than the single dose syringe with respect to the insertion in the nasal cavity and deposition of the gel.

This study demonstrates that the gel deposition by the multiple dose dispenser is equivalent to the single dose syringe and is capable of depositing gel in the correct location of the nasal cavity. With the success of the multiple dose dispenser in properly placing the gel into the nasal cavity, the administration instructions from this study are used to create dosing instructions for patients. A copy of this dosing pamphlet can be found in Figs. 10-11.

## Example 5

In Vitro Release Rate (Ivrt) Comparison Testing

IVRT experimental approach is used for comparison of products in semi-solid dosage form through evaluation of the drug release. In order to have fair comparison, products to be compared should be of comparable age and their release rates should be determined on the same day, under the same conditions. To ensure an unbiased comparison, sample position within the bank of Franz cells are randomized. The test (T) product and reference (R) product in each run is randomized or pre-assigned in a mixed arrangement.

| Méthod Parameter Main | Alternate parameters |
|---|---|
| *Franz Cells*<br>- membrane : durapore 0.45 μm, HVLP02500<br>- ring diameter 15 mm<br>- surface : 1.767 mm" | *Franz Cells*<br>- membrane : durapore 0.45 μm, HVLP02500<br>- diamèter 15 mm<br>- surface : 1.767 mm" |

(continued)

| Méthod Parameter Main | Alternate parameters |
|---|---|
| - **thickness: 3.2 mm**<br>- Gel **Volume: 565.44 mm"**<br>- **receiving media volume: 12 ml Ethanol**<br>**Water 50/50**<br>600 rpm | - **thickness** : **1.63 mm**<br>- gel **Volume: 288.02 mm"**<br>- **Volume media recptor: 7.5 ml**<br><br>ETOH/water 50/50<br>600 rpm |
| *Assay*<br>**UPLC**<br>Concentrations from 3 μg/ml to 200 μg/ml | *Assay*<br>**HPLC**<br>Concentrations 5 μg/ml to 100 μg/ml |

The slope comparison test recommended by the FDA is performed and provides the evidence of the reproducibility of the IVRT method.

The two different formulations of the testosterone gel products, Table 1, are applied on 12 cells of the modified Franz-Cell apparatus system: 6 cells for reference product (R) and 6 cells for test product (T), as depicted in Fig. 12. The two gel products, Testosterone Nasabol Gel 4%, lot# E10-007, and TBS1A Testosterone Nasal Gel 4%, lot# IMP 11002, are described in Example 6 and designated as 4% TSA-1A and TBS1.

Table I

| Material | TBS1 | TBS-1A 4 % (A) |
|---|---|---|
| Dimethyl isosorbide | 0 | 25.0 |
| Diethyleneglycol ethyl ether | 0 | 10.0 |
| Povidone | 0 | 3.0 |
| Copovidone | 0 | 2.0 |
| Hydroxypropyl cellulose | 0 | 0.5 |
| Testosterone micronized | 4.0 | 4.0 |
| Castor oil | 88.0 | 50.5 |
| Labrafil M1944CS | 4.0 | 0 |
| Colloidal silicon dioxide | 4.0 | 5.0 |
| Water | 0 | 0 |
| Total | 100.0 | 100.0 |

Samples are collected at 1, 2, 3, 4, 5 and 6 hours and are tested.

*Franz Cell Apparatus Position Layouts for Comparison Testing*

The Release Rates (slope) from the six cells of T- product and from the other six cells of the

R-product are obtained. A 90% Confidence Interval (CI) for the ratio (T/R) of median release rates is computed.

A table with six rows and seven columns is generated and reference slopes (RS) are listed across the first row and test slopes (TS) are listed down the first column of Table 2. Individual T/R ratios (30) between each test slope and each reference slope are computed and the corresponding values are entered in the table.

Table 2

| Calculation of T/R Ratios | | | | | | |
|---|---|---|---|---|---|---|
| **Slope** | **RS1** | **RS2** | **RS3** | **RS4** | **RS5** | **RS6** |
| **TS1** | TS 1/RS 1 | TS 1/RS2 | TS 1/RS3 | TS 1/RS4 | TS 1/RS5 | TS 1/RS6 |

(continued)

| Calculation of T/R Ratios | | | | | | |
|---|---|---|---|---|---|---|
| **Slope** | **RS1** | **RS2** | **RS3** | **RS4** | **RS5** | **RS6** |
| **TS2** | TS2/RS 1 | TS2/RS2 | TS2/RS3 | TS2/RS4 | TS2/RS5 | TS2/RS6 |
| **TS3** | TS3/RS 1 | TS3/RS2 | TS3/RS3 | TS3/RS4 | TS3/RS5 | TS3/RS6 |
| **TS4** | TS4/RS I | TS4/RS2 | TS4/RS3 | TS4/RS4 | TS4/RS5 | TS4/RS6 |
| **TS5** | TS5/RS 1 | TS5/RS2 | TS5/RS3 | TS5/RS4 | TS5/RS5 | TS5/RS6 |
| **TS6** | TS6/RS 1 | TS6/RS2 | TS6/RS3 | TS6/RS4 | TS6/RS5 | TS6/RS6 |

These 30 T/R ratios are ranked from lowest to highest. The **sixth** and **twenty-fifth** ordered ratios represent low and upper limits of the 90% CI for the ratios of median release rates.

*Standard criteria:* Test and reference product are considered to be the same if the 90% CI falls within the limits of **75% -133.3%.**

Two batches of Testosterone Nasabol Gel 4%, lot# E10-007, and TBS IA Testosterone Nasal Gel 4%, lot# IMP 11002, are tested and evaluated for sameness.

A statistical comparison is carried out by taking the ratio of release rates from 6 cells of the reference lot # E10-007 (R) against 5 cells of the test batch lot# IMP 11002 (T).

During the *in vitro* drug releases test, the reference batch and the test batch are applied in a randomized manner on the cells on Apparatus A and B of the modified Franz Cell System.

Release Rate (slope) from five cells of the test product (T) and six cells of the reference product (R) are compared. A 90% Confidence Interval (CI) for the ratio (T/R) of median release rates is computed.

The 90% Confidence Interval is represented by the sixth and twenty-fifth Release Rate ratios
when ranked from lowest to highest. These ratios correspond to 160.77% and 202.90% respectively and do not meet the limits for sameness (CI 75% - 133.33%).

Therefore, the two batches of Testosterone Nasabol Gel 4%, lot# E10-007 and TBS1A Testosterone Nasal Gel 4%, lot# IMP 11002 are not considered the same.

Two gel products, Testosterone Nasabol Gel 4%, lot# E10-007, and TBS1A Testosterone Nasal Gel 4%, lot# IMP 11002, are tested and evaluated for sameness. The Mean Release Rate (slope) for the Test lot# IMP 11002 is about 1.8 times higher than for the Reference lot# E10-007. The two tested products are found to be not the same.

The *In Vitro* Release Rate (IVRT) testing results and raw data are in Tables 3-8 below and Fig. 13.

Table 3

| Franz Cell | | | | | | | | | |
|---|---|---|---|---|---|---|---|---|---|
| **Calibration Block Report Linear Regression Curve** | | | | **Weighted** | **System Precision Report** | | | | |
| **Peak Name: Testosterone** | | | | | **Sys. Suit. Std 6** | | | | |
| **Lot#IMP11001** | | | | | | | | | |
| **Level** | **Weighting** | **Amount** | **Response** | **Response Factors** | | **Inj.** | **Response** | | |
| 1 | 1.00000 | 1.019406 | 16408.514988 | 16096 | | 1 | 824164 | | |
| 2 | 0.16000 | 6.371288 | 103313.035806 | 16215 | | 2 | 826891 | | |
| 3 | 0.04000 | 25.485150 | 413283.640897 | 16217 | | 3 | 831692 | | |
| 4 | 0.02000 | 50.970300 | 831225.896397 | 16308 | | 4 | 829717 | | |
| 5 | 0.01000 | 101.940900 | 1663494.184060 | 16318 | | 5 | 830837 | | |
| 6 | 0.00500 | 203.881200 | 3324002.333557 | 16304 | | 6 | 829685 | | |
| | | | | | **Average** | | 828831 | | |
| | | | | | **STDEV:** | | 2802 | | |
| | | | | | **%RSD** | | 0.3 | | *<2.0% recommended* |
| | | | | | **Accuracy (Recovery)** | | **Report** | | |
| | | | | | | | 1 | 2 | 3 |
| | | | | | Check Std 1 | | 50124 | 49790 | 50068 |
| | | | | | Check Std 2 | | 831913 | 827185 | 828024 |
| | | | | | Check Std 3 | | 3317936 | 327208 | 3313901 |
| | | | | | | | 1 | 2 | 3 |
| Response Factors Average | | 16132 | | | Check Std. µg/mL | | | | |
| Response Factors %RSD: | | 0.5% < 10% acceptable | | | 3.0717 (%) | | 100.7 | 100.0 | 100.6 |
| | | | | | Check Std. µg/mL | | | | |
| Intercept / Lowest Std(%) | | 1.9 < 20% acceptable | | | 51.1944 (%) | | 99.7 | 99.1 | 99.2 |
| | | | | | Check Std. µg/mL | | | | |
| Intercept (1/X): | | -307.4012 | | | 204.776 (%) | | 99.4 | 98.0 | 99.3 |
| Slope (1/X): | | 16306.099286 | | | | | | | |
| Y= 16305.1 1/X + | | -307.4 | | | 90% - 110% lowest standard | | | | |
| RSQ (1/X): | | 0.999999 > 0.9 acceptable | | | 98% - 102% higher standards | | | | |

Table 4

**4% Gel Release Rate Comparison**

Testosterone TBS1A Testosterone Nasal Gel 4%

Test Lot# IMP11002

**Concentration of Active (µg/mL) versus Time**

| | Released (µg/mL) Calculation by Linear Regression Curve | | | | | | | |
|---|---|---|---|---|---|---|---|---|
| | Cell A#1 | Cell A#3 Amount | | | | | Mean 1-5 | %RSD 1-5 |
| Time | | | | | | | | |
| 60.00 | 118.922 | 115.401 | 122.547 | 123.279 | 118.672 | 114.557 | 118.896 | 3.0 |
| 120.0 | 195.377 | 182.991 | 201.133 | 205.222 | 191.880 | * | 195.321 | 4.4 |
| 180.00 | 280.637 | 246.686 | 274.656 | 282.957 | 264.605 | 261.063 | 268.434 | 5.1 |
| 240.00 | 344.420 | 291.933 | 329.143 | 346.540 | 317.162 | 324.788 | 325.664 | 6.2 |
| 300.00 | 401.961 | 330.531 | 378.137 | 403.828 | 369.302 | 388.545 | 378.717 | 7.2 |
| 360.00 | 462.471 | 379.994 | 428.269 | 462.433 | 417.526 | 445.583 | 432.713 | 7.3 |
| **Actual Amount of Active Released (µg/cm²) versus Time$^{0.5}$** | | | | | | | | |
| | Amount Released (µg/cm²) | | | | | | | |
| | Cell A#1 | Cell A#3 | Cell A#5 | Cell B#2 | Cell B#4 | Cell B#6 | Mean 1-5 | %RSD 1-5 |
| **Time$^{0.5}$** | | | | | | | | |
| 7.75 | 807.574 | 783.664 | 832.191 | 837.161 | 805.876 | 777.932 | 807.400 | 3.0 |
| 10.95 | 1360.413 | 1275.306 | 1400.526 | 1428.501 | 1336.594 | #N/A | 1360.268 | 4.4 |
| 13.42 | 1994.677 | 1759.622 | 1956.716 | 2014.450 | 1884.747 | #N/A | 1922.042 | 5.4 |
| 15.49 | 2507.220 | 2136.684 | 2404.439 | 2526.291 | 2316.520 | #N/A | 2378.231 | 6.7 |
| 17.32 | 2995.422 | 2481.397 | 2830.278 | 3013.375 | 2760.332 | #N/A | 2816.161 | 7.7 |
| 18.97 | 3520.067 | 2910.814 | 3277.707 | 3256.612 | 3192.305 | #N/A | 3285.301 | 7.8 |
| Slope | 242.85 | 187.78 | 217.83 | 239.55 | 213.29 | | 220.26 | 10.1 |
| $R^2$ | 0.9912 | 0.9947 | 0.9958 | 0.9927 | 0.9942 | | 0.9937 | 0.2 |

* Via Cell B#6 at 2hr missing injection, release rate comparison will be calculated by 5 x 6 = 30 individual T/R ratios.

Table 5

**4% Gel Release RateComparison**

Testosterone Nasobol Gel 4% Gel

Test Lot# E10-007

**Concentration of Active (µg/mL) versus Time**

| | Amount Released (µg/mL) Calculation by Linear Regression Curve | | | | | | | |
|---|---|---|---|---|---|---|---|---|
| | Cell A#1 | Cell A#3 | Cell A#5 | Cell B#2 | Cell B#4 | Cell B#6 | Mean 1-6 | %RSD 1-6 |
| **Time** | | | | | | | | |
| 60.00 | 96.792 | 104.726 | 101.499 | 98.958 | 98.994 | 101.074 | 199.341 | 2.7 |
| 120.00 | 143.746 | 153.402 | 151.896 | 148.611 | 146.111 | 152.369 | 149.356 | 2.6 |
| 180.00 | 181.187 | 191.204 | 190.149 | 185.651 | 182.536 | 188.818 | 186.591 | 2.2 |
| 240.00 | 206.803 | 219.307 | 216.557 | 214.670 | 212.670 | 218.650 | 214.672 | 2.2 |
| 300.00 | 234.373 | 243.717 | 243.634 | 239.656 | 238.437 | 241.174 | 240.165 | 1.5 |
| 360.00 | 253.244 | 262.615 | 261.716 | 259.500 | 255.210 | 263.639 | 259.321 | 1.6 |
| **Actual Amount of Active Released (µg/cm²) versus Time$^{0.5}$** | | | | | | | | |
| | Amount Released (µg/cm²) | | | | | | | |
| | Cell A#1 | Cell A#3 | Cell A#5 | Cell B#2 | Cell B#4 | Cell B#6 | Mean 1-6 | %RSD 1-6 |
| **Time$^{0.5}$** | | | | | | | | |
| 7.75 | 657.294 | 711.172 | 689.258 | 672.003 | 672.247 | 686.372 | 681.391 | 2.7 |
| 10.95 | 1003.536 | 1071.352 | 1060.212 | 1037.186 | 1020.219 | 1063.304 | 1042.635 | 2.6 |

(continued)

| | Amount Released (μg/cm²) | | | | | | | |
|---|---|---|---|---|---|---|---|---|
| | Cell A#1 | Cell A#3 | Cell A#5 | Cell B#2 | Cell B#4 | Cell B#6 | Mean 1-6 | %RSD 1-6 |
| **Time$^{0.5}$** | | | | | | | | |
| 13.42 | 1298.462 | 1371.463 | 1362.959 | 1330.766 | 1308.915 | 1353.934 | 1337.750 | 2.2 |
| 15.49 | 1523.682 | 1616.405 | 1596.093 | 1576.120 | 1565.197 | 1609.943 | 1581.240 | 2.2 |
| 17.32 | 1769.419 | 1844.221 | 1841.242 | 1810.596 | 1800.351 | 1824.766 | 1815.099 | 1.5 |
| 18.97 | 1963.883 | 2041.513 | 2032.969 | 2013.163 | 1981.718 | 2045.561 | 2013.135 | 1.7 |
| Slope | 116.80 | 119.04 | 120.10 | 119.69 | 118.02 | 120.59 | 119.04 | 1.2 |
| $R^2$ | 0.9990 | 0.9997 | 0.9996 | 0.9996 | 0.9992 | 0.9997 | 0.9995 | 0.0 |

Table 6

| | | | | Comparison Study Franz Cell Release Rate Comparison | | | |
|---|---|---|---|---|---|---|---|
| R - Reference Lot# E10-007 | | | | Testosterone Nasobol Gel 4% Gel | | | |
| T -Test Lot- IMP 11002 | | | | TSB1A Testosterone Nasal Gel 4% | | | |
| | | | | **R** | | | |
| | | **116.80** | **119.04** | **120.10** | **119.69** | **118.02** | **120.59** |
| | **242.85** | 2.0792 | 2.0401 | 2.0221 | 2.0290 | 2.0577 | 2.0138 |
| | **187.78** | 1.6077 | 1.5775 | 1.5635 | 1.5689 | 1.5911 | 1.5572 |
| **T** | **217.83** | 1.8650 | 1.8299 | 1.8137 | 1.8200 | 1.8457 | 1.8064 |
| | **239.55** | 2.0509 | 2.0123 | 1.9946 | 2.0014 | 2.0297 | 1.9865 |
| | **213.29** | 1.8261 | 1.7918 | 1.7759 | 1.7820 | 1.8072 | 1.7687 |

Note: Test Lot Vial B#6 at 2 hour was missing injection. Comparison was calculated by 5x6=30 individual T/R ratios, and the limits of 90% would be sixth and twenty-fifth order individual T/R ratios.

Table 7

| | |
|---|---|
| **Sixth Ordered Ratio** | **160.77%** |
| **Twenty-fifth Ordered Ratio:** | **202.90%** |

**Test and reference products are considered to be the "same" of the 90% CI falls within the limits of 75% - 133.33%.**

Table 8

| Amount of Active Released (μg/cm²) | | |
|---|---|---|
| Time$^{0.5}$ | Lot# IMP11002 | Lot#E10-007 |
| 7.75 | 807.400 | 681.391 |
| 10.95 | 1360.268 | 1042.635 |
| 13.42 | 1922.042 | 1337.75 |
| 15.49 | 2378.231 | 1581.24 |
| 17.30 | 2816.161 | 18.15.099 |
| 18.97 | 3285.301 | 2013.135 |

Table 9

In Vitro release Rate Testing

Products: TBSIA Testosterone Nasal Gel 4% and Testosterone

Nasobol Gel 4%

Objective: Release the rate comparison between the two testosterone gel

Products

| Side | Sample Information | Release Rate Results |
|---|---|---|
| Reference Batch | Testosterone Nasobol Gel 4%<br><br>The reference Lot# E10-007<br><br>Expiry date: N/A<br>Diteba Sample ID: CSB-SPL-00200<br><br>Number of Cells: 6<br>Position of Cells:<br>System (1) A#2, #4, #6; System (2) B#1, #3,#5 | Average Slope: 119.87 μg/cm$^2$ . min$^{-0.5}$<br><br>RSD of Slopes: 1.8%<br>$R^2$ of lowest Linearity: 0.9995 |
| Test Batch | TBS1A Testosterone Nasal Gel 4%<br><br>The test batch (Lot# IMP 11001) Expiry date: N/A<br><br>Number of Cells: 6<br>Position of Cells:<br>System (1) A#1, #3, #5; System (2) B#2, #4,#6 | Average Slope: 300.02 μg/cm$^2$ . min$^{-0.5}$<br><br>RSD of Slopes: 9.3%<br>$R^2$ of lowest linearity: 0.9935 |

| Release Rate Comparison | Comparison Results<br>Comparison Limits: 75.00% to 133.33% |
|---|---|
| Stage One | 8th ordered ratio: 228.50%<br><br>29th ordered ratio: 264.03% |
| Stage Two | 110th ordered ratio: N/A<br><br>215th ordered ratio: N/A |

**Example 6**

A phase-1 open label, balanced, randomized, crossover, two groups, two-treatments, two period, pilot study in health male subjects to determine the feasibility of a multiple dose dispenser for testosterone intranasal gel as measured by pharmacokinetics.

Testosterone replacement therapy aims to correct testosterone deficiency in hypogonadal men. Trimel BioPharm has developed an intranasal testosterone gel (TBS-1) as alternative to the currently available testosterone administration forms. To date, a syringe was used to deliver TBS-1 in clinical studies. Trimel identified a multiple dose dispenser intended for commercial use. The purpose of the study was to demonstrate the relative performance of the multiple dose dispenser in comparison to the syringe used previously in clinical trials.

This was an open label, balanced, randomized, crossover, two-group, two-treatment, two-period, pharmacokinetic study of TBS-1 testosterone nasal gel in healthy, male subjects aged 18 to 28. Treatment consisted of 4.5% TBS-1 testosterone gel as a single dose of 5.5 mg of testosterone per nostril, delivered using either a syringe or the multiple dose dispenser, for a total dose of 11.0 mg given at 21:00 hours. Prior to first administration, subjects were admitted to the unit for blood sampling in order to determine a baseline testosterone profile. Wash-out between drug administrations was at least 48 hours.

All subjects completed the study successfully and treatment was well tolerated.

The total exposure to testosterone as estimated by the mean area under the serum concentration-time curve ($AUC_{0-12}$ in ng.hr/dL), is higher after TBS-1 administration using the dispenser or syringe than endogenous levels alone (7484 and 7266, respectively, versus 4911ng*h/dL. Mean $C_{max}$ is higher after administration with the dispenser than after administration using a syringe (1028 versus 778.8 ng/dL, respectively). $T_{max}$ occurs earlier following administration using the dispenser compared to the syringe (2.75 versus 5.6 hours, respectively. Thus, testosterone absorption seems to be faster with the multiple dose dispenser than with a syringe, but the total absorbed amount is similar. Also, in previous studies the syringe Tmax obtained in patient was closer to 1.0 or 2.0 hours.

When plotting probability density of the log ratio of testosterone levels reached with the multiple dose dispenser over levels reached with the syringe as shown in Figure 3, no significant difference was demonstrated for either $AUC_{0-12}$ or $C_{max}$ within the lower and upper limit of the 95% confidence intervals. There is a trend toward a difference for $C_{max}$. However, this data does not confirm bioequivalence at a confidence interval level of 90% for either $AUC_{0-12}$ or $C_{max}$. If the trends found here are confirmed in a larger data set, the routes of administration would be almost equivalent for $AUC_{0-12}$, but t for $C_{max}$ further investigation may be required as the Cmax/tmax profile obtained in volunteers does not seem to match the one obtained in patients.

## Testosterone as a Treatment for Hypogonadism

Endogenous androgens are responsible for the normal growth and development of the male sex organs as well as promoting secondary sex characteristics including the growth and maturation of the prostate, seminal vesicles, penis, and scrotum; the development of male hair distribution, such as beard, pubic, chest, and axillary hair, laryngeal enlargements, vocal cord thickening, alterations in body musculature, and fat distribution.

Hypogonadism in men is characterized by a reduced concentration of serum testosterone resulting in signs and symptoms that may include decreased libido, erectile dysfunction, decreased volume of ejaculate, loss of body and facial hair, decreased bone density, decreased lean body mass, increased body fat, fatigue, weakness and anaemia.

The causes of hypogonadism can be primary or secondary in nature. In primary hypogonadism (congenital or acquired) testicular failure can be caused by cryptorchidism, bilateral torsion, orchitis, vanishing testis syndrome, orchidectomy, Klinefelter's syndrome, chemotherapy, or toxic damage from alcohol or heavy metals. These men usually have low serum testosterone levels and serum gonadotropin levels (FSH, LH) above the normal range.

In secondary hypogonadism (Hypogonadotropic Hypogonadism (congenital or acquired)) the defects reside outside the testes, and are usually at the level of the hypothalamus or the pituitary gland. Secondary hypogonadism can be caused by Idiopathic Gonadotropin or LHRH deficiency, or pituitary hypothalamic injury from tumors, trauma, or radiation. These men have low serum testosterone levels but have serum gonadotropin levels in the normal or low ranges.

Testosterone hormone therapy is indicated as a hormone replacement therapy in males for conditions associated with a deficiency or absence of endogenous testosterone. The currently available options for administration of testosterone are oral, buccal, injectable, and transdermal.

Trimel BioPharma has developed an intranasal testosterone gel (TBS-1) as a hormone replacement therapy for the treatment of male hypogonadism. The nasal mucosa offers an alternative route of administration that is not subjected to first pass metabolism, has high permeability, with rapid absorption into the systemic circulation. The advantages of the testosterone intranasal gel when compared to other formulations include ease of administration and no transference of testosterone to other family members.

## Investigational Medicinal Product

The investigational medicinal product in this trial was TBS-1, an intranasal testosterone dosage form. A description of its physical, chemical and pharmaceutical properties can be found in the Investigator's Brochure.

## Summary of Non-clinical and Clinical Studies

### *Summary of Non-clinical Studies*

An overview of the pharmacology, toxicology and preclinical pharmacokinetics of different testosterone preparations and administration routes is provided in the Investigator's Brochure Product-specific repeat dose toxicity and tolerance studies have been performed in *ex vivo* models and in different animal species.

### *Summary of Previous TBS-1 Clinical Studies*

To date, Trimel has completed four Phase II clinical trials in hypogonadal men. The most recently conducted study, TBS-1-2010-01, is described below and the other studies are summarized in the Investigator's Brochure.

The objective of study TBS-1-2010-01 is to examine the efficacy and tolerability of 4.0% and 4.5% TBS-1 testosterone gel in hypogonadal men. In this study, TBS-1 is administered using a syringe, not the commercial multiple dose dispenser. The doses and dosing regimens that were used in study TBS-1-2010-01 are described in Table 1 below.

The results from all treatment groups met the FDA criteria for efficacy; defined as that at least 75% of subjects should achieve an average total T concentration ($C_{avg}$) in the normal range, a 24 hour $C_{avg}$ value ≥ 300ng/dL and ≤ 1050 ng/dL.

**Table 1:** Summary of previous TBS-1 studies

| **Dosing regimen** | **Total daily dose** | **$C_{avg}$ (% of subjects with $C_{avg}$ within the reference range)** |
|---|---|---|
| 13.5 mg of TBS-1 (4.5%) BID | 27 mg/day | 419 ng/dL (100%) |
| 10.0 mg of TBS-1 (4.0%) TID | 30 mg/day | 413 ng/dL (87%) |
| 11.25 mg of TBS-1 (4.5%) TID | 33.75 mg/day | 396 ng/dL (85%) |

## Summary of Benefits and Risks to Subjects

### *Benefits*

Testosterone replacement therapy for hypogonadal men should correct the clinical abnormalities of testosterone deficiency. Since this was a Phase I study enrolling normal healthy men between the ages of 18 - 45, for a short period of time, it was not anticipated that these volunteers would directly benefit by taking part in this study. Volunteers were financially compensated for their participation.

### *Risks*

The risk to the subject by participating in this study was considered to be minimal. Testosterone replacement therapy is indicated for the treatment of hypogonadism and TBS-1 has been administered to over 100 men with minimal side effects.

As TBS-1 is an investigational drug that is in clinical development, the complete side effect profile was not fully known. Epistaxis, nasal congestion, nasal discomfort, nasal dryness and nasal inflammation have been reported following use of TBS-1. Side effects from approved (prolonged) testosterone replacement therapy include elevated liver enzymes (alanine aminotransferase, aspartate aminotransferase), increased blood creatine phosphokinase, increase in prostatic specific antigen, decreased diastolic blood pressure, increased blood pressure, gynecomastia, headache, increased hematocrit/hemoglobin levels, hot flushes, insomnia, increased lacrimation, mood swings, smell disorder, spontaneous penile erection, and taste disorder.

The main benefit of the intranasal drug delivery route is that with this method many of the different disadvantages observed with other products would not be expected. This would include skin-to-skin transfer, stickiness, unpleasant smell (gels), skin irritation (patches), elevated DHT (patches and oral), injection pain and high T and DHT peaks (intramuscular injection), food interaction (oral).

## Trial Rationale

Trimel identified a multiple dose dispenser that was intended as the commercial dispenser to be used in this clinical trial program. To date, a syringe has been used to deliver TBS-1 in the previous clinical trials. The purpose of this study was to demonstrate the comparability of the pharmacokinetic results obtained with a multiple dose dispenser or a syringe.

## References

1. Nasobol® Investigator Brochure Release Date 19th August 2010, Edition No: 5.
2. http://www.androgel.com/pdf/500122-00127_Rev_1E_Sep_2009_FPI_with_MedGuide.pdf (Last accessed on 6th September, 2010).
3. http://www.mattern-pharmaceuticals.com/downloads.Nasobol.pdf (Last accessed on 6th September, 2010).
4. http://www.medicines.org.uk/EMC/medicine/22159/SPC/Testim+Gel/ (Last accessed on 6th September, 2010).

**STUDY OBJECTIVES**

The primary study objective is to compare a pharmacokinetic profile of testosterone after administration of TBS-1 using two different dispensers in healthy male subjects.

The secondary objective is to assess the safety of TBS-1.

**Investigational Plan**

**Overall Study Design and Plan**

This is an open label, balanced, randomized, crossover, two-group, two-treatment, two-period, pharmacokinetic study of testosterone nasal gel formulation in healthy, adult, male human subjects. The study event schedule is summarized in Section ?????in Table 2.

Healthy male volunteers, aged 18 to 45 years (inclusive) were screened for this study. The goal was to randomize 12 male subjects for the study.

There was a washout period of 6 days between each drag administration.

**Discussion of Study Design**

As this is a relatively small Phase I PK study with the intent to compare a pharmacokinetic profile of testosterone after administration of TBS-1 from two different dispensers in healthy male subjects, a true sample size calculation is not performed. Based on typical early-stage, pharmacokinetic studies, groups of 6 subjects per cohort are sufficient for an acceptable description of the pharmacokinetic parameters after single dose administration.

**Selection of Study Population**

*Inclusion Criteria*

The following eligibility assessments have to be met for subjects to be enrolled into the study:

1. Healthy male human subjects within the age range of 18 to 45 years inclusive
2. Willingness to provide written informed consent to participate in the study
3. Body-mass index of ≤ 35 kg/$m^2$
4. Absence of significant disease or clinically significant abnormal laboratory values on laboratory evaluations, medical history or physical examination during screening
5. Normal otorhinolaryngological examination
6. Non-smokers for at least six months
7. Comprehension of the nature and purpose of the study and compliance with the requirement of the protocol

*Exclusion Criteria*

A subject is not eligible for inclusion in this study if any of the following criteria applied:

1. Personal / family history of allergy or hypersensitivity to testosterone or related drugs
2. Past history of anaphylaxis or angioedema
3. Any major illness in the past three months or any clinically significant ongoing chronic medical illness e.g. congestive heart failure, hepatitis, pancreatitis etc.
4. Presence of any clinically significant abnormal values during screening e.g. significant abnormality of Liver Function Test (LFT), Renal (kidney) Function Test (RFT), etc.
5. Hemoglobin < 13g/dl and Hematocrit > 52% during screening
6. Any cardiac, renal or liver impairment, any other organ or system impairment
7. History of seizure or clinically significant psychiatric disorders
8. Presence of disease markers for HIV 1 and/or 2, Hepatitis B and/or C virus
9. History of nasal surgery, specifically turbinoplasty, septoplasty, rhinoplasty, ("nose job"), or sinus surgery
10. Subject with prior nasal fractures
11. Subject with active allergies, such as rhinitis, rhinorrhea, or nasal congestion
12. Subject with mucosal inflammatory disorders, specifically pemphigus, or Sjogren’s syndrome
13. Subject with sinus disease, specifically acute sinusitis, chronic sinusitis, or allergic fungal sinusitis

14. History of nasal disorders (e.g. polyposis, recurrent epistaxis (> 1 nose bleed per month), abuse of nasal decongestants) or sleep apnea
15. Subject using any form of intranasal medication delivery, specifically nasal corticosteroids and oxymetazoline containing nasal sprays (e.g. Dristan 12-Hour Nasal Spray)
16. History of asthma and/ or on-going asthma treatment
17. Regular drinkers of more than three (3) units of alcohol daily (1 unit = 300 ml beer, 1 glass wine, 1 measure spirit), or consumption of alcohol within 48 hours prior to dosing and during the study.
18. Volunteer demonstrating a positive test for alcohol consumption (using breath alcohol analyzer) at the time of check-in during the admission periods.
19. History of, or current evidence of, abuse of alcohol or any drug substance, licit or illicit
20. Volunteers demonstrating a positive test for drugs of abuse in urine (Opiates, Benzodiazepines, Amphetamines, THC and cocaine) at the time of check-in during admission periods
21. Inaccessibility of veins in left and right arm
22. Receipt of any prescription drug therapy within four weeks of the first admission period.
23. Difficulty in abstaining from OTC medication (except occasional paracetamol/aspirin) for the duration of the study
24. Volunteers demonstrating serum PSA ≥ 4ng/ml
25. Participation in any other research study during the conduct of this study or 30 days prior to the initiation of this study.
26. Blood donation (usually 550 ml) at any time during this study, or within the 12 week period before the start of this study.

*Removal of Patients from Therapy or Assessment*

All 12 subjects who enroll, complete the study successfully, and no subjects are replaced.

**Treatments**

*Treatments Administered*

For the drug administration, subjects are instructed on how TBS-1 is applied intranasally with the pre-filled syringes or the multiple dose dispensers. Self-administration of TBS-1 is monitored by the study personnel. Each subject is instructed not to sniff or blow his nose for the first hour after administration.

**Table 2:** Treatment schedule

| GROUP | Subject number | BASELINE Day 1/2<br>Time 21:00-09:00 | PERIOD I Day 2/3<br>Time 21:00-09:00 | PERIOD II Day 4/5<br>Time 21:00-09:00 |
|---|---|---|---|---|
| A | 1-6 | 12 hour baseline T profile | TREATMENT 1 | TREATMENT 2 |
| B | 7-12 | 12 hour baseline T profile | TREATMENT 2 | TREATMENT 1 |

Treatment 1 consists of TBS-1 syringes that are pre-filled with 4.5% testosterone gel to deliver a single dose of 5.5 mg of testosterone per nostril, for a total dose of 11.0 mg that is administered at 21:00 hours (± 30 minutes) on Day 2 of Period I for Group A and Day 4 of Period II for Group B.
Treatment 2 consists of a TBS-1 multiple dose dispensers that are pre-filled with 4.5% testosterone gel to deliver a single dose of 5.5 mg of testosterone per nostril, for a total dose of 11.0 mg that is administered at 21:00 hours (± 30 minutes) on Day 2 of Period I for Group B and Day 4 of Period II for Group A.

*Identity of Investigational Product(s)*

The investigational product in this trial is TBS-1, an intranasal testosterone dosage form.
Study medication consists of TBS-1 gel and is packed either in a single use syringe that is designed to expel 125 μl of gel, with two syringes packaged per foil pouch, or in a multiple dose dispenser that is designed to expel 125 μl of gel/actuation.
Study medication is dispensed by the study pharmacist who prepares the individual study kits which contained two syringes in a pouch or the multiple dose dispenser.

*Method of Assigning Patients to Treatment Groups*

Treatment assignment is determined according to the randomization schedule at the end of Visit 1. Subjects who met the entry criteria are assigned randomly on a 1:1 basis to one of the two treatment groups (Group A or Group B). The randomization is balanced and the code is kept under controlled access. The personnel that are involved in dispensing of study drug is accountable for ensuring compliance to the randomization schedule.

*Selection and Timing of Dose*

As healthy males have endogenous testosterone levels that fluctuate with a circadian rhythm which peaks in the early morning, it is decided to dose the study medication at night.

*Blinding*

This is an open-label study for both the subjects and the investigator, as the physical differences in the intranasal dosing dispensers prevent blinding.

*Prior and Concomitant Therapy*

None of the subjects use prescription medication immediately prior to, during or the 2 weeks after the study. One subject receives a single dose of paracetamol (2 tablets of 500mg) just before discharge on the morning after the baseline visit (before administration of any study medication). There are no other reports of medication use.

*Treatment Compliance*

All subjects receive both doses of study medication according to the instructions and are monitored by study personnel for one-hour post-dosing to assure conformity to the TBS-1 instructions. All subjects remain in the clinic during the 12-hour PK sampling time period; during which they are monitored closely.

*Screening*

The screening visit (visit 1) takes place at a maximum of 21 days before the first study day. After giving informed consent, the suitability of the subject for study participation is assessed at screening which consists of the following items:

- Medical history
- Physical examination and Vital Signs.
- A fasting blood sample is taken to determine the following: Complete Blood Count, Chemistry profile; testing for HBV, HCV, HIV and PSA.
- Urinalysis, urine drug screen, and Breath Alcohol Testing.
- An otorhinolaryngological nasal endoscopic examination is performed by an ENT specialist.

Subjects meeting all of the inclusion and no exclusion criteria are enrolled into the study and are randomized into one of two treatment groups (1 or 2).

*Study Days*

Subjects are admitted to the clinical research centre at 19:30 hours on Day 1 (Visit 2, baseline), 2 (Visit 3, Period 1) and 4 (Visit 4, Period 2). After check-in tests for drug-abuse and alcohol consumption are performed. Vital signs are recorded and subjects are questioned about changes in their health.

During Visit 2, a 12 hour baseline testosterone profile is measured. Blood for the 12 hour baseline testosterone profile is drawn according to the following schedule: first sample at 20:45 hours and then at 0.33, 0.66, 1.00, 1.50, 2.00, 3.00, 4.00, 5.00, 6.00, 8.00, 10.00, and 12.00 hours relative to 21:00 time point (a total of 13 samples). On Day 2 vital signs are measured and safety parameters (symptoms, AEs) recorded before check-out.

Dosing is performed on the evenings of Day 2 and 4, at 21:00 hr. Before dosing an ENT examination is performed and a pre-dose, baseline serum testosterone blood sample is drawn. After dosing, a 12 hour testosterone PK profile is measured. The blood samples are drawn according to the following schedule after the 21:00 hour dosing: 0.33, 0.66, 1.00, 1.50, 2.00, 3.00, 4.00, 5.00, 6.00, 8.00, 10.00, and 12.00 hr time points (a total of 13 samples per period).

On Day 3 and 5 vital signs are measured, ENT examination are performed and safety parameters are recorded

(symptoms, AEs) after the last PK sampling and before check-out. On Day 5 a final examination is performed, consisting of a general physical examination and clinical laboratory investigation (Complete Blood Count, Chemistry profile and Urinalysis).

*Pharmacokinetic Sampling*

Blood samples for analysis of testosterone levels are collected in 4ml standard clotting tubes using an intravenous cannula. Tubes are left to clot for 30-45 minutes. Samples are centrifuged within one hour at 2000g for 10 minutes at 4°C, The serum is then transferred directly to two aliquots of 1 ml each and frozen at -40°C.

*Safety*

Blood samples for hematology are collected in 4ml EDTA tubes and sent to the hematology laboratory of the Leiden University Medical Center (LUMC) for routine analysis. Blood samples for blood chemistry are collected in 4 ml Heparin tubes and sent to the clinical chemistry laboratory for routine analysis.

*Drug Concentration Measurements*

Frozen serum samples for PK analysis are stored in the freezer at -40°C and are shipped on dry ice to the laboratory, at the end of the study. Samples are analyzed using a validated LC-MS method for the determination of testosterone levels. It is not possible to discriminate endogenous and exogenous testosterone from each other using this method.

*Quality Assurance*

The study is conducted in compliance with the pertaining CHDR Standard Operating Procedures and CHDR's QA procedures.

*Calculation of Pharmacokinetic Parameters*

A validated LC-MS/MS method is employed to determine serum testosterone. All samples from study participant completing both the periods are analyzed.
Incurred sample reanalysis is performed:

- $C_{min}$, $C_{max}$, and $t_{max}$ actual measured values. Values are determined relative to the testosterone administration time in treated subjects.
- Area under the concentration curve (AUC) is estimated for the 0 to 12 hour time interval using the trapezoidal rule.
- Significance is evaluated using the t-test. Additional exploratory analyses of PK parameters could be performed as necessary.

The relative pharmacokinetic profile of the pre-filled syringe and the multiple dose dispenser is determined using the $AUC_{0-12h}$ and $Cmax_{0-12h}$ corrected for the endogenous serum testosterone concentration. For bioequivalence, the relative mean of the dispenser to the pre-filled syringe using log transformed data for $AUC_{0-12h}$ and $Cmax_{0-12h}$ is corrected for the endogenous serum testosterone concentration, is determined to be between 80% to 125%.

*Analysis of Safety Parameters*

The Day 5 close-out findings is compared to the screening results and clinically significant changes were to be identified in the following:

1. Vital Signs and Adverse Events: Blood Pressure, Body Temperature, Respiratory Rate, Heart Rate.
2. Otorhinolaryngological examination with the nasal tolerance data presented in summary tables.
3. Complete Blood Count: white blood count, hemoglobin and hematocrit.
4. Clinical chemistry profile: sodium, potassium, chloride, glucose, urea, creatinine, calcium, phosphate, uric acid, total bilirubin, albumin, AST, ALT, ALP, GGT, CK and cholesterol.
5. Urinalysis.

*Determination of Sample Size*

As this is a relatively small Phase I PK study with the intent to compare a pharmacokinetic profile of testosterone after administration of TBS-1 from two different dispensers in healthy male subjects, a true sample size calculation is not performed.

*Subjects*

26 Subjects are enlisted

- 2 subjects are not screened due to planning problems
- 1 subject is not screened because he does not have a general practitioner

23 Subjects are screened

- 3 screening failures due to ENT abnormalities
- 1 screening failure due to positive hepatitis B test
- 1 screening failure due to positive hepatitis C test

18 Subjects passed screening

- 12 subjects are randomized and completed the study
- 1 subject is cancelled before the baseline visit due to concurrent illness
- 5 subjects are reserves, but not needed

No subjects discontinue after randomization.

*efficacy evaluation*

Data collected is used in the analysis. This yields three PK curves of 12 hours each, one without treatment (baseline), and one each after administration of TBS-1 using the multiple dose dispenser or syringe.

*Demographic Characteristics*

Subject demographics are summarized in Table 4 below.

**Table 4: Subject demographics**

| Variable | N | MEAN | STD | MIN | MAX |
|---|---|---|---|---|---|
| **Age (yrs)** | 12 | 23.4 | 3.0 | 18 | 28 |
| **BMI (kg/$m^2$)** | 12 | 23.55 | 2.45 | 20.9 | 28.4 |
| **Height (cm)** | 12 | 184.43 | 8.46 | 173.5 | 197.0 |
| **Weight (kg)** | 12 | 80.08 | 9.76 | 63.2 | 98.2 |

*Measurements of Treatment Compliance*

The nasal gel is self-administered by subjects. All administrations are successful.

*Efficacy Results and Tabulations of Individual Patient Data*

Fig. 14 shows the individual serum testosterone levels per occasion (baseline without medication, TBS-1 using the multiple dose dispenser and TBS-1 using syringes), where T=0 occurred at 21:00 hours clock time. Fig. 14 shows the individual and median testosterone concentration versus time grouped by treatment.

All subjects have testosterone levels within the normal range (24 hour $C_{mean} \geq 300$ ng/dL and $\leq 1050$ ng/dL). The baseline curves clearly show the slow circadian fluctuations in testosterone levels that are expected in a young,

healthy population with the highest levels in the early morning.

Although dose and volume of TBS-1 that is administered is exactly the same for both forms of administration, the graphs in Figs. 14 arid 15 suggest that there are differences in pharmacokinetic profile.

*Pharmacokinetic Parameters*

The following primary pharmacokinetic parameters, per occasion, are calculated:

- $AUC_{0-12}$: Area under the serum concentration-time curve (ng.hr/dL) for each occasion from 21:00 to 9:00 hrs, is calculated using the linear trapezoidal method.
- $C_{mean}$: Mean concentration (ng/dL) during each occasion from 21:00 to 9:00 hrs, is calculated as AUC-0-12/12.
- $C_{max}$: Maximum is observed concentration (ng/dL) during each occasion.
- $C_{min}$: Minimum is observed concentration (ng/dL) during each occasion.
- $t_{max}$: Time (hr) at which $C_{max}$ is observed.

Tables 5 to 7 below summarize the primary pharmacokinetic parameters for endogenous testosterone during the baseline visit when no treatment is administered, for TBS-1 when administered using the multiple dose dispenser, and for TBS-1 when administered using a syringe.

*Testosterone, baseline, no treatment*

**Table 5: Testosterone, no treatment**

| Parameter | Mean | SD | Median | Min | Max | N |
|---|---|---|---|---|---|---|
| **$AUC_{0-12}$** | 4911 | 1156 | 4726 | 3337 | 7164 | 12 |
| **$t_{max}$** | 8.833 | 3.486 | 10.0 | 2.0 | 12 | 12 |
| **$C_{max}$** | 514.2 | 117.5 | 480.0 | 384.0 | 746 | 12 |
| **$C_{min}$** | 298.6 | 89.01 | 308.0 | 134.0 | 453 | 12 |
| **$C_{mean}$** | 409.0 | 96,4 | 392.8 | 278.1 | 597 | 12 |
| **$AUC_{0-12}$ in ng*hr/dL; $t_{max}$ in hours; $C_{max}$ $C_{min}$ and $C_{mean}$ in ng/dL** | | | | | | |

*Testosterone, TBS-1 multiple dose dispenser*

**Table 6 : Testosterone, TBS-1 multiple dose dispenser**

| Parameter | Mean | SD | Median | Min | Max | N |
|---|---|---|---|---|---|---|
| **$AUC_{0-12}$** | 7484 | 1798 | 7347 | 4847 | 11350 | 12 |
| **$t_{max}$** | 2.751 | 3.961 | 1.25 | 0.3333 | 12 | 12 |
| **$C_{max}$** | 1028 | 283.1 | 970.5 | 645 | 1440 | 12 |
| **$C_{min}$** | 337.9 | 119.7 | 328.5 | 145 | 565 | 12 |
| **$C_{mean}$** | 623.6 | 149.9 | 612.3 | 403.9 | 945.7 | 12 |
| $AUC_{0-12}$ **in ng*hr/dL;** $t_{max}$ **in hours;** $C_{max}$, $C_{min}$ **and** $C_{mean}$ **in ng/dL** | | | | | | |

*Testosterone, TBS-1 syringe*

**Table 7 : Testosterone, TBS-1 syringe**

| Parameter | Mean | SD | Median | Min | Max | N |
|---|---|---|---|---|---|---|
| **$AUC_{0-12}$** | 7266 | 1360 | 7237 | 5186 | 9371 | 12 |
| **$t_{max}$** | 5.612 | 4.736 | 5.0 | 0.667 | 12 | 12 |
| **$C_{max}$** | 778.8 | 144.1 | 754.5 | 543 | 1100 | 12 |
| **$C_{min}$** | 355.9 | 66.96 | 337.0 | 291 | 498 | 12 |
| **$C_{mean}$** | 605.4 | 113.2 | 603.1 | 432.2 | 780.9 | 12 |

$AUC_{0-12}$ **in ng*hr/dL;** $t_{max}$ **in hours;** $C_{max}$, $C_{min}$ **and** $C_{mean}$ **in ng/dL**

The listing of individual primary pharmacokinetic parameters is included in Table 7A.

Table 7A

Efficacy Data *Individual PK Parameters*

Individual PK parameters 0-12 hrs for each occasion

| **Subject** | **Occasion** | | **Treatment** | **AUC_0-12t_max** | **C_max** | | **C_mean** | **C_min** | | | |
|---|---|---|---|---|---|---|---|---|---|---|---|
| | 1 | 1 | | No Treatment | 5722 | | 10.0000 | | 600 | 476. 9 | 321 |
| | 1 | 2 | | TBS-1 mdd | 9394 | | 12.0000 | | 1070 | 782.9 | 340 |
| | 1 | 3 | | TBS-1 syringe | 7802 | | 12.0000 | | 840 | 650.1 | 400 |
| | 2 | 1 | | No Treatment | 3731 | | 10.0000 | | 388 | 310.9 | 242 |
| | 2 | 2 | | TBS-1 syringe | 7367 | | 1.5000 | | 779 | 613.9 | 333 |
| | 2 | 3 | | TBS-1 mdd | 7592 | | 0.3333 | | 1420 | 632.7 | 386 |
| | 3 | 1 | | No Treatment | 4771 | | 3.0000 | | 498 | 395.4 | 332 |
| | 3 | 2 | | TBS-1 mdd | 6056 | | 0.6667 | | 645 | 504.7 | 395 |
| | 3 | 3 | | TBS-1 syringe | 7107 | | 5.0000 | | 691 | 592.3 | 312 |
| | 4 | 1 | | No Treatment | 7164 | | 2.0000 | | 746 | 597.0 | 453 |
| | 4 | 2 | | TBS-1 syringe | 8639 | | 6.0000 | | 837 | 720.0 | 498 |
| | 4 | 3 | | TBS-1 mdd | 8370 | | 0.3333 | | 1440 | 697.5 | 500 |
| | 5 | 1 | | No Treatment. | 3337 | | 10.0000 | | 384 | 278.1 | 134 |
| 5 | 2 | | | | | TBS-1 mdd | 4847 | 0.3500 | 1280 | 403.9 | 145 |
| 5 | 3 | | | | | TBS-1 syringe | 5439 | 1.0000 | 725 | 453.3 | 292 |
| 6 | 1 | | | | | No Treatment | 3673 | 10. 0000 | 422 | 305.2 | 166 |
| 6 | 2 | | | | | TBS-1 syringe | 5186 | 10.0200 | 543 | 432.2 | 304 |
| 6 | 3 | | | | | TBS-1 mdd | 5851 | 1.0000 | 715 | 487.6 | 325 |
| 7 | 1 | | | | | No Treatment | 4681 | 12.0000 | 456 | 390.1 | 324 |
| 7 | 2 | | | | | TBS-1 syringe | 6250 | 12.0000 | 661 | 520.8 | 291 |
| 7 | 3 | | | | | TBS-1 mdd | 6503 | 1.5000 | 881 | 541.2 | 159 |
| 8 | 1 | | | | | No Treatment | 4632 | 12.0000 | 473 | 386.0 | 295 |
| 8 | 2 | | | | | TBS-1 mdd | 7102 | 1.5000 | 813 | 591.9 | 332 |
| 8 | _3 | | | | | TBS-1 syringe | 8529 | 0.6667 | 1100 | 710.7 | 343 |
| 9 | 1 | | | | | No Treatment | 4222 | 12.0000 | 481 | 351.8 | 287 |
| 9 | 2 | | | | | TBS-1 mdd | 11350 | 3.0000 | 1350 | 945.7 | 276 |
| 9 | 3 | | | | | TBS-1 syringe | 6992 | 12.0000 | 7 30 | 582. 7 | 341 |
| 10 | 1 | | | | | No Treatment | 6503 | 10.0000 | 718 | 541.9 | 397 |
| 10 | 2 | | | | | TBS-1 syringe | 9371 | 5.0000 | 874 | 780.9 | 445 |
| 10 | 3 | | | | | TBS-1 mdd | 8747 | 10.0000 | 820 | 728.9 | 565 |
| 11 | 1 | | | | | No Treatment | 5541 | 5.0000 | 525 | 461.7 | 353 |

(continued)

Efficacy Data *Individual PK Parameters*

Individual PK parameters 0-12 hrs for each occasion

| **Subject** | **Occasion** | **Treatment** | **AUC_0-12t_max** | **C_max** | | **C_mean** | **C_min** | | | |
|---|---|---|---|---|---|---|---|---|---|---|
| 11 | 2 | | | | TBS-1 mdd | 7823 | 2.0000 | 848 | 651.9 | 315 |
| 11 | 3 | | | | TBS-1 syringe | 8550 | 1.5000 | 898 | 710.6 | 408 |
| 12 | 1 | | | | No Treatment. | 4950 | 10.0000 | 479 | 412.5 | 279 |
| 12 | 2 | | | | TBS-1 syringe | 5962 | 0.6667 | 668 | 496..8 | 304 |
| 12 | 3 | | | | TBS-1 **mdd** | 6171 | 0.3333 | 1060 | 514.2 | 317 |

mdd - multiple dose dispenser

Total testosterone exposure is estimated by the mean area under the serum concentration-time curve ($AUC_{0-12}$ in ng·hr/dL) is higher after TBS-1 administration using the dispenser or syringe than endogenous levels alone (7484 and 7266, respectively, versus 4911ng*h/dL). Between the methods of administration, the difference in mean $AUC_{0-12}$ is small. The significance of this difference is explored below.

Unexpectedly, mean $C_{max}$ is higher after administration with the dispenser than when with a syringe (1028 versus 778.8 ng/dL, respectively). $T_{max}$ occurs sooner after administration using the dispenser than after the syringe (2.75 versus 5.6 hours, respectively). Thus, after administration using the multiple dose dispenser serum testosterone seems to be absorbed faster than with a syringe. The significance of these differences is explored below.

Two subjects reach $t_{max}$ of testosterone only 10 and 12 hours after administration with the dispenser. In three subjects, $t_{max}$ is 10 and 12 hours after administration with the syringe, and $t_{max}$ is 5 and 6 hours in two others. Most likely, the endogenous testosterone peak fluctuation exceeded levels that is caused by exogenous testosterone administration. Thus, the calculated mean $t_{max}$ may be faster when testosterone is dosed high enough that the peak caused by exogenous administration exceeds the endogenous peak.

*Derived Pharmacokinetic Parameters*

The following derived pharmacokinetic parameters, combining results from occasions, are calculated:

- $AUC_{0-12_drug}$: difference between $AUC_{0-12}$ after treatment (syringe or dispenser) and no treatment (baseline occasion)
- $C_{max_drug}$: difference between $C_{max}$ after treatment (syringe or dispenser) and the observed concentration at $t_{max}$ in absence of treatment (baseline occasion)
- Ratio $AUC_{0-12_drug}$: % ratio between $AUC_{0-12_drug}$ using dispenser and syringe
- Ratio $C_{max_drug}$: % ratio between $C_{max_drug}$ using dispenser and syringe
- Mean and uncertainty (95%, 90% and 80% confidence interval) of the log of Ratio $AUC_{0-12_drug}$
- Mean and uncertainty (95%, 90% and 80% confidence interval) of the log of Ratio $C_{max_drug}$

*Testosterone Level Using TBS-1, Baseline Subtracted*

Tables 8 and 9 below show the AUC and $C_{max}$ for the different TBS-1 delivery methods after subtracting baseline levels of testosterone.

**Table 8:** Testosterone level using TBS-1 multiple dose dispenser, baseline subtracted

| Parameter | Mean | SD | Median | Min | Max | N |
|---|---|---|---|---|---|---|
| **$AUC_{0-12_drug}$** | 2573.0 | 1679.0 | 2211 | 1207 | 7126 | 12 |
| **$C_{max_drug}$** | 630.8 | 314.7 | 534 | 102 | 1111 | 12 |

**Table 9:** Testosterone level TBS-1 syringe, baseline subtracted

| Parameter | Mean | SD | Median | Min | Max | N |
|---|---|---|---|---|---|---|
| **$AUC_{0-12_drug}$** | 2356.0 | 900.9 | 2219 | 1012 | 3897 | 12 |
| **$C_{max_drug}$** | 379.9 | 177.1 | 357 | 121 | 782 | 12 |

***Testosterone Level TBS-1 Dispenser over Syringe Ratio***

Table 10 below shows the ratio of serum testosterone levels that are reached with the dispenser or syringe, after subtracting baseline testosterone levels. There is clearly a difference in $C_{max}$ between the administration forms (mean ratio dispenser over syringe $C_{max}$ 2.057), but the AUCs are comparable (mean ratio dispenser over syringe AUC 1.12).

**Table 10: Testosterone, ratio of TBS-1 multiple dose dispenser over syringe**

| Parameter | Mean | SD | Median | Min | Max | N |
|---|---|---|---|---|---|---|
| **Ratio $AUC_{0-12_drug}$** | 1.122 | 0.580 | 0.940 | 0.550 | 2.572 | 12 |
| **Ratio $C_{max_drug}$** | 2.057 | 1.339 | 1.983 | 0.227 | 4.455 | 12 |

(continued)

| Parameter | Mean | SD | Median | Min | Max | N |
|---|---|---|---|---|---|---|
| **logRatio $AUC_{0-12_drug}$** | 0.014 | 0.453 | -0.071 | -0.598 | 0.945 | 12 |
| **logRatio $C_{max_drug}$** | 0.455 | 0.860 | 0.684 | -1.484 | 1.494 | 12 |

Table 11 below shows the log of the ratio of serum testosterone levels that are reached when administering using the multiple dose dispenser over syringe, after subtracting baseline testosterone levels, with 95%, 90% and 80% confidence intervals.

When plotting probability density of the log ratio of testosterone levels that are reached with the multiple dose dispenser over levels that are reached with the syringe as shown in Figure 16, no significant difference is demonstrated for either $AUC_{0-12}$ or $C_{max}$ within 95% confidence intervals. There is a trend toward a difference for $C_{max}$. However, this data does not confirm bioequivalence at a confidence interval level of 90% for either $AUC_{0-12}$ or $C_{max}$, as the study is not powered for 2-one-sided tests.

**Table 11: Testosterone TBS-1 log ratios with different confidence intervals**

| Parameter | Mean | CI (%) | LLCI | ULCI |
|---|---|---|---|---|
| **logRatio $AUC_{0-12_drug}$** | 0.01398 | 95 | -0.27400 | 0.3019 |
| | | 90 | -0.2209574 | 0.24892 |
| | | 80 | -0.16438 | 0.19234 |
| **logRatio $C_{max_drug}$** | 0.45520 | 95 | -0.09145 | 1.0020 |
| | | 90 | 0.00917 | 0.90127 |
| | | 80 | 0.11658 | 0.79386 |
| **CI = confidence interval; log(0.8) = -0.22314; log(1.25) = 0.22314** | | | | |

*Handling of Dropouts or Missing Data*

No subjects drop out of the study. Blinded data review did not lead to removal of any data points.

*Extent of Exposure*

The pharmacokinetic results show that exposure to testosterone is only higher than the upper level of the normal range very briefly shortly after TBS-1 administration.

*Adverse Events (AEs)*

Treatment is well tolerated. There are 12 adverse event reports in total. Three events had their onset before the first administration of study medication and are therefore unrelated. Four reports of mild complaints such as sore throat are considered unlikely to be caused by study medication when considering the nature of the complaints and the time lapse after administration. One subject reschedules one occasion because of gastro-intestinal complaints that are unlikely to be related to study medication, onset of symptoms is days after study drug administration. Symptoms resolve without treatment.

Reports of bad smell and taste are the only complaints that are considered clearly related to administration of medication. These complaints are mild in intensity and could be considered a product characteristic rather than a medical condition. Bad smell and taste complaints do not lead to discontinuation of the study medication and diminishes with repeated dosing.

*Display of Adverse Events*

A listing of adverse events is included in Table 12.

Table 12

**Listing of Adverse Events**

| Treatment Treatment Chronicity | Subject Duration | Visit | Start Severity | SAE | Diagnosis related | action | symptoms |
|---|---|---|---|---|---|---|---|
| TBS-1 mdd | 2 | 3 | 06APRIL | 08:30 | OROPHARYNGEAL PAIN | None | Irritated |
| single occasion | | 0D01H20M | mild | | No unlikely | | throat. |
| | 3 | 2 | 30MAR11 | 12:00 | HEADACHE | None | Headache |
| single occasion | 0009H00M | | mild | no | unrelated | | |
| | | | 30MAR11 | 21:04 | APPLICATION SITE ODOUR | None | Smells nasty. |
| single occasion | 0D02H55M | | mild | no | definitely | | bad taste |
| | 5 | 2 | 30MAR11 | 20:40 | APPLICATION SITE ODOUR | None | it smells |
| nasty, single occasion | | | 0D0H30M | Mild | no definitely | | |
| | | | 30 MAR11 | 21:15 | DYSGEUSIA | None | bad taste. |
| single occasion | 0D00H45M | | mild | no | definitely | | |
| | 8 | 2 | 13APR11 | 20:45 | CATHETER SITE RASH | removed plastic | red rash in |
| single occasion | | 1D18H15M | | mild no | unrelated | | |
| | | | | | | Tape patch | left armpit where cannula is placed. |
| TBS-1 syringe | 1 | 3 | 06APR11 | 8:30 | OROPHARYNGEAL PAIN | None | Sore throat. |
| single occasion | 0D00H40M | | mild | no | unlikely | | |
| | 2 | 2 | 31 MAR11 | 13:00 | AGITATION | None | Feeling |
| single occasion | 0D00H20M | | mild | no | unlikely | | agitated |
| | 4 | 2 | 30MAR11 | 20:45 | APPLICATION SITE ODOUR | None | It smells |
| nasty. single | occasion | 0D00H27M | mild | no definitely | | | |
| | 6 | 2 | 30MAR11 | 20:33 | APPLICATION SITE ODOUR | None | it smells |
| nasty. single occasion | | 0D00H27M | | mild | No definitely | | |
| | 10 | 2 | 18APR11 | 23:00 | DIARRHOEA | None | Nausea, |
| single occasion | 1D21H00M | | mild | No | unlikely | | |
| | | | | | | | diarrhoea. |
| No Treatment | 11 | 1 | 13APR11 | 9:19 | HEADACHE | Paracetamol, | Headache |

(continued)

**Listing of Adverse Events**

| | | | | | |
|---|---|---|---|---|---|
| single occasion | 0D06H41M | mild | No | unrelated | sleep. |
| Note: mdd=multiple dose dispenser M=Missing U=Unknown | | | | | |

*Analysis of Adverse Events*

All adverse events are considered mild and are transient. Nasal tolerance is good. Initial complaints of bad smell or taste did not lead to discontinuation of the study.

*Deaths, Other Serious Adverse Events, and Other Significant Adverse Events*

There are no deaths, serious adverse events or other significant adverse events.

*Evaluation of Each Laboratory Parameter*

There are no abnormal hematology, blood chemistry or urine laboratory findings that are considered clinically significant in the opinion of the investigator.

*Vital Signs, Physical Findings and Other Observations Related to Safety*

There are no abnormal findings in vital signs, on physical examinations or other observations that are considered clinically significant in the opinion of the investigator.

*Safety Conclusions*

Treatment is well tolerated, nasal tolerance is good. All adverse events are considered mild and are transient. Initial complaints of bad smell or taste did not lead to study discontinuation.

*Discussion and Overall Conclusions*

This study compares the pharmacokinetic profile of TBS-1 testosterone nasal gel administered using a multiple dose dispenser to the profile of TBS-1 delivery using a syringe. In order to avoid carry-over effects that are caused by repeated dosing, the order of administration is randomized. bPrior to first administration, subjects are admitted to the unit for blood sampling in order to determine a baseline testosterone profile.

All 12 subjects, age range 18 to 28 years, complete the study successfully. Although not assessed at screening, all subjects have baseline testosterone levels within the normal range. Treatment is well tolerated and all reported adverse events are transient and considered mild. Complaints of bad smell and taste are reported, although this did not lead to discontinuation and decreased with repeated dosing.

As expected, the total exposure to testosterone (as estimated by the mean area under the serum concentration-time curve ($AUC_{0-12}$)) after TBS-1 administration using the dispenser or syringe exceed endogenous levels. The difference in mean $AUC_{0-12}$ between the two modes of administration is small.

Unexpectedly, mean $C_{max}$ is considerably higher after administration with the dispenser than when administering using a syringe. $T_{max}$ is also earlier after administration using the dispenser than after the using the syringe. Thus, testosterone absorption seems to be faster with the multiple dose dispenser than with a syringe, but the total absorbed amount is similar.

Two subjects reach $t_{max}$ of testosterone only 10 and 12 hours after administration with the dispenser. In three subjects, $t_{max}$ is 10 and 12 hours after the syringe, and $t_{max}$ is 5 and 6 hours in two others. Most likely, the endogenous testosterone peak fluctuation exceed levels that are caused by exogenous testosterone administration. Thus, the calculated mean $t_{max}$ may be faster when testosterone is dosed high enough that the peak caused by exogenous administration exceeds the endogenous peak.

When plotting probability density of the log ratio of testosterone levels that are reached with the multiple dose dispenser over levels that are reached with the syringe, no significant difference is demonstrated for either $AUC_{0-12}$ or $C_{max}$ within 95% confidence intervals. There is a trend toward a difference for $C_{max}$. However, this data does not confirm bioequivalence at a confidence interval level of 90% for either $AUC_{0-12}$ or $C_{max}$. This finding may be due to the fact that the ideal positioning of the delivering tip is easier to find with the multiple dose device than the syinge.

Also, in accordance with this Example 6, see Figs. 22 and 23.

The following formaltions are in Table 13 used in Examples 5-7 and in Figs. 22 and 23.

Table 13

| Material | TBS1 | TBS1V (vs. H20) | TBS-1A 4 % (A) | TBS-1A 4 % alternate (B) | TBS-1A 8 % |
|---|---|---|---|---|---|
| Dimethyl isosorbide | 0 | 0 | 25.0 | 15.0 | 25.0 |

(continued)

| Material | TBS1 | TBS1V (vs. H20) | TBS-1A 4 % (A) | TBS-1A 4 % alternate (B) | TBS-1A 8 % |
|---|---|---|---|---|---|
| Diethyleneglycol ethyl ether | 0 | 0 | 10.0 | 5.0 | 10.0 |
| Povidone | 0 | 0 | 3.0 | 3.0 | 3.0 |
| Copovidone | 0 | 0 | 2.0 | 2.0 | 2.0 |
| Hydroxypropyl cellulose | 0 | 0 | 0.5 | 0.5 | 0.5 |
| Testosterone micronized | 4.0 | 4.0 | 4.0 | 4.0 | 8.0 |
| Castor oil | 88.0 | 87.95 | 50.5 | 65.5 | 46.5 |
| Labrafil M1944CS | 4.0 | 4.0 | 0 | 0 | 0 |
| Colloidal silicon dioxide | 4.0 | 4.0 | 5.0 | 5.0 | 5.0 |
| Water | 0 | 0.05 | 0 | 0 | 0 |
| Total | 100.0 | 100.0 | 100.0 | 100.0 | 100.0 |

## Example 7

Intranasal testosterone gels, TBS-1 and TBS-1A, have been developed as a hormone replacement therapy for the treatment of hypogonadism. It is believed that TBS-1/TBS-1A gel will offer significant safety and efficacy over existing therapies for the treatment of hypogonadism.

TBS-1/TBS-1A are an innovative galenic formulation of testosterone for nasal administration. The advantages of TBS-1/TBS-1A nasal gel include a reduced amount of active ingredient in comparison to other testosterone replacement therapies and lack of transference to other family members.

The testosterone intranasal gels are referred to as Nasobol or TBS-1 (for the treatment of hypogonadism in males).

The investigational drugs, TBS-1 and TBS-1A, are an intranasal formulation of testosterone.

The chemical composition of testosterone is $C_{19}H_{28}O_2$ with a molecular weight of 288.42. Testosterone belongs to the pharmacological class of androgens.

Structural formula

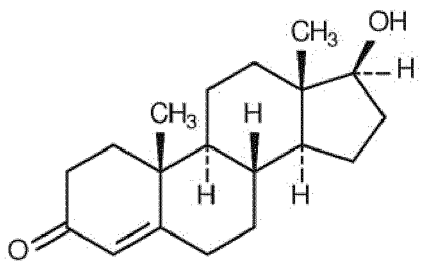

Table 1: Summary of the Active IMP, TBS-1

| | |
|---|---|
| (Brand) Name of Drug Product | TBS-1 |
| Common Name of Drug Product | Testosterone intranasal gel |
| Common Name of Drug Substance (Medicinal Ingredient) | Testosterone |
| Dosage Form | Gel |
| Strength | 4.0% |

(continued)

| | |
|---|---|
| (Brand) Name of Drug Product | TBS-1 |
| Route of Administration | Intranasal |
| Proposed Indication | Hypogonadism |

Table 2: Summary of the Active IMP, TBS-1A

| | |
|---|---|
| (Brand) Name of Drug Product | TBS-1 A |
| Common Name of Drug Product | Testosterone intranasal gel |
| Common Name of Drug Substance (Medicinal Ingredient) | Testosterone |
| Dosage Form | Gel |
| Strength | 4.0%, 8.0% |
| Route of Administration | Intranasal |
| Proposed Indication | Hypogonadism |

The specifications for testosterone drug substance are presented in Table 2.1.S.4.1-1.

Table 2.1.S.4.1-1: Testosterone Specifications

| **Test Parameter** | **Method/Reference** | **Acceptance Criteria** |
|---|---|---|
| Description | Visual (USP) | White or slightly creamy white crystals or crystalline powder |
| IR Absorbance Spectrum | IR spectroscopy (USP) | Matches reference spectrum |
| UV Absorbance Spectrum | UV/VIS spectroscopy (USP) | Matches reference spectrum |
| Melting Range Start | Class Ia (USP) | 153-157°C |
| Melting Range End | Class Ia (USP) | 153-157°C |
| Specific Optical Rotation | Polarmetry (USP) | 101-105° (calculated on the dried basis) |
| Loss on Drying | Gravimetry (USP) | ≤ 1 .0% |
| Related Substances | TLC (Ph. Eur.)<br><br>HPLC (Ph. Eur.) | Delta4-androstenediol ≤ 0.2%<br>Androstanolone ≤ 0.1%<br>Impurity A ≤ 0.1%<br>Impurity I ≤ 0.2%<br>Impurity C ≤ 0.5%<br>Impurity J ≤ 0.1%<br>any other ≤ 0.1%<br>Total impurities ≤ 0.6% |
| Volatile Substances | GC | Acetone ≤ 0.5%<br>2-propanol ≤ 0.5%<br>n-heptane ≤ 0.5% |
| Assay | TLC and UV (USP) | 97.0 - 103.0% (calculated on the dried basis) |

(continued)

| Test Parameter | Method/Reference | Acceptance Criteria |
|---|---|---|
| Particle Size | Laser diffraction spectroscopy 50%<br>Laser diffraction spectroscopy 90% | ≤ 50 μm<br>≤ 100μm |
| Microbial Limits | Ph. Eur. | Microbial count: colony forming aerobic bacterial and fungi ≤ 100/g<br>Enterobacteriaceae <10 CFU/g<br>P aeruginosa 0/g<br>S. aureus 0/g |

*Container Closure System* ***[testosterone USP***

Testosterone is dispensed into polyethylene bags; each polyethylene bag is then sealed and placed in a polyethylene-aluminum-laminated bag. The polyethylene-aluminum-laminated bag is placed in a plastic container which is shipped within a fiber drum that is closed with a tamperproof metallic seal.

*Stability [testosterone USP*

Testosterone USP has a retest period of five (5) years.

*MEDICINAL PRODUCT (TBS-1, Gel)*

TBS-1 gel is a viscous and thixotropic, oil-based formulation containing solubilized testosterone intended for intranasal application. The drug product is formulated with the compendial inactive ingredients: castor oil, oleoyl polyoxylglycerides, and colloidal silicon dioxide.

*Composition*

The compositions of the drug product to be administered in this clinical trial are provided in Tables 2.1.P.1-1.

Table 2.1.P.1-1: Components, Quantity, Quality Standards and Function, 4.0% TBS-1: Syringe

| Component | Amount (% w/w) | Quantity per Syringe (mg) | Quantity Delivered per Dose (mg) | Function | Quality Standard |
|---|---|---|---|---|---|
| Testosterone | 4.0% | 5.92 | 5.0 | Active ingredient | USP |
| Castor oil | 88.0% | 130.24 | 110 | Solvent | Ph. Eur./ USP |
| Oleoyl polyoxylglycerides | 4.0% | 5.92 | 5.0 | Wetting agent (hydrophilic oil) | Ph. Eur./NF |
| Colloidal silicon dioxide | 4.0% | 5.92 | 5.0 | Viscosity increasing agent | NF |

TBS-1 gel is supplied in unit-dose polypropylene syringes. Two syringes of each dosage are packaged in a protective aluminium foil pouch.

Testosterone used in TBS-1/TBS-1A gel appears as white or slightly creamy white crystals or crystalline powder. It is freely soluble in methanol and ethanol, soluble in acetone and isopropanol and insoluble in n-heptane. It can also be considered as insoluble in water ($S_{20°C}$=2.41 x $10^{-2}$ g/L ± 0.04 x $10^{-2}$ g/L); its n-Octanol/Water partition coefficient (log $P_{ow}$ determined by HPLC) is 2.84. The solubility of testosterone in oils is determined to be 0.8% in isopropylmyristate, 0.5% in peanut oil, 0.6% in soybean oil, 0.5% in corn oil, 0.7% in cottonseed oil and up to 4% in castor oil.

Because testosterone is fully dissolved within the formulation, physical characteristics of the drug substance do not influence the performance of the drug product TBS-1/TBS-1A gel. The manufacturability of TBS-1/TBS-1A gel however is influenced by the particle size of testosterone. When using a particle size of 50% ≤ 25 microns, 90% ≤ 50 microns the solubility of the drug substance in the matrix is especially favorable.

The molecular structure of testosterone contains no functional groups that can be protonated or deprotonated in the physiological pH-range. Therefore, testosterone is to be considered as a neutral molecule with no pKa value in the range 1-14. Because it is neutral, testosterone is compatible with excipients.

The excipients used in the formulation of the TBS-1/TBS-1A drug product are inactive ingredients used in semi-solid dosage forms. The ingredients are monographed in NF and/or USP/Ph. Eur. (except dimethyl isosorbide which is subject to a specific monograph) and are all listed in the "Inactive Ingredient" list for Approved Drug Products issued by the FDA.

*Castor oil Ph. Eur./USP*

The main excipient in TBS-1/TBS-1A gel is castor oil which amounts to approximately 50 to 65% of the formulation and serves as a solvent for testosterone. The characteristics of oil which can influence drug product performance are: the ability to solubilize drug substance, viscosity which influences the amount of gellant required, odor/taste which may impact patient compliance, and acid/hydroxyl/iodine/saponification value which impacts the potential for skin irritation. The solubility of testosterone is highest in castor oil compared to other solvents suitable for nasal application and castor oil is not irritating to mucous membrane.

For nasal delivery, small volumes are applied. It is expected that a testosterone semi-solid dosage form for nasal application would require a dose of 2.5 to 5 mg of the active per 100 μL per nostril. An aqueous solution of testosterone can contain only 0.002 mg per 100 μL while a castor oil solution, in contrast, can contain up to about 4.5mg per 100 μL.

*Oleoyl polyoxylglycerides NF*

Oleoyl polyoxylglycerides are also referred to as Labrafil M 1944 CS, apricot kernel oil PEG-6 esters, Peglicol-5-oleate, mixture of glycerides and polyethylene esters. The castor oil, which is used as a solvent for TBS-1 gel, is a fixed oil. Such oils have the advantage of being non-volatile or spreading (in contrast to essential oils or liquid paraffin), but have the disadvantage of being hydrophobic. The nasal mucosa contains 95-97% water. Without the oleoyl polyoxylglycerides, the castor oil containing the active ingredient would form a non-interactive layer on the mucous membrane. In order to achieve adequate contact between the castor oil layer and the mucous membrane, the hydrophilic oleoyl polyoxylglyceride is added to the formulation to form an emulsion between the castor oil and the mucosa fluid. Oleoyl polyoxylglycerides have a slight disadvantage of causing a minor decrease in the solubility of testosterone in castor oil.

Oleoyl polyoxylglycerides are used in semi-solids at concentrations ranging from about 3 to 20%, depending on the application. The amount of oleoyl polyoxylglycerides in TBS-1 gel is high enough to allow for a better contact of the carrier oil with the mucous membrane and low enough to have minimal impact on the amount of testosterone that can be incorporated into the carrier oil. A favorable concentration of oleoyl polyoxylglycerides in TBS-1 gel is found to be 4% of the formulation.

Oleoyl polyoxylglycerides show good mucosal tolerance and are used as an excipient in approved nasal and vaginal preparations. This oil is used in nasal sprays/drops together with other excipients such as olive oil, peanut oil, eucalyptol, niauli oil and in vaginal creams together with mineral oil, and tefose 63 (PEG-6-32 stearate and glycol stearate).

Diethyleneglycol ethyl ether (Transcutol P) Ph. Eur./NF

Testosterone solubility in the oil based TBS-1A gel is limited to about 4.5%. There is a need to increase the solubility of testosterone to higher levels in order to reduce the volume of gel delivered as the recommend volume of gel to the nasal cavity should not preferably exceed about 150 μL.

One possibility is the addition of surfactants to TBS-1A formulation, but the literature reports tolerance issues with high levels of surfactants on mucosae.

Diethyleneglycol ethyl ether alone can dissolve more than 10% testosterone, and is used with DMI to increase the overall solubility of testosterone in the gel. Diethyleneglycol ethyl ether is used in many dermal formulations approved in Europe, USA, Canada and several other countries.

*Dimethyl isosorbide (DMI)*

Dimethyl isosorbide (DMI), also referred to as super refined Arlasolve, is also a super solvent and is able to

dissolve more than 12% testosterone. Used in conjunction with the other excipients, DMI allows for testosterone levels in the gel up to at leat about 8.0%.

The safety profile for DMI is made public by, for example, the Australian Ministry of Health: http://www.nicnas.gov.au/search/cache.cgi?collection=nicnas-web&doc=http/www.nicnas.gov.au/publications/car/new/std/stdfullr/std1000fr/std105 2fr.pdf.pan.txt

*Povidone Ph. Eur./USP*

Povidone (Kollidon K or Plasdone K) is a vinylic polymer used for decades in various pharmaceutical dosage forms. Povidone functions as a binder and is also used as a film former in sprays and an inhibitor of crystal growth in saturated solutions. Povidone is only partly soluble in the TBS-1A mixture and its amount is limited.

*Copovidone Ph. Eur./USP*

Copovidone known as Kollidon VA 64 or Plasdone S 630 is a copolymer of vinyl acetate and pyrrolidone. Copovidone is similar in function to Povidone in that it is used as a binder in dozens of oral formulation but can also be found in topical formulation (Erythromycin dermal or clotrimazole vaginal) approved in Germany, and an anti-acne cream approved in UK).

Copovidone is somewhat more soluble than Povidone in the TBS-1A mixture, its activity on crystal growth is somewhat less than Povidone, but, unlike Povidone, Copovidone helps increase the viscosity of the gel.

*Colloidal silicon dioxide NF*

The oil in TBS-1/TBS-1A gel is thickened with a gel-forming agent, colloidal silicon dioxide. This compound is used commonly for thickening oleogels.

The intended dosage form for TBS-1/TBS-1A gel is a semi-solid, not a liquid. The formulation is thickened with colloidal silicon dioxide; instead of a solid fat as the viscosity obtained with the latter highly depend on of temperature, while the viscosity obtained with $SiO_2$ remains stable with temperature. In addition, colloidal silicon dioxide contributes to the thixotropic properties of the gel, simplifying drug delivery to the nostril.

Colloidal silicon dioxide is generally an inert material which is well tolerated as an excipient in mucosal applications such as suppositories. Colloidal silicon dioxide is typically used in these preparations at concentrations ranging from about 0.5 to 10%. The concentration of colloidal silicon dioxide in TBS-1/TBS-1A gel is high enough to achieve gel formation but at a level that has minimal impact on testosterone incorporation into the carrier oil.

Hydroxypropyl cellulose Ph. Eur./USP (Hyprolose)

Hydroxypropyl cellulose (Klucel or Nisso HPC) is a cellulose derivative used as a viscosity agent in pharmaceutical products. Hyprolose has been cited in literature as able to improve and prolong the absorption of hydrophilic drugs through the nasal route in animals (Dopamine).

Unlike it other cellulose derivatives, Hyprolose is also soluble in many organic solvent or semi-hydrophilic oils and therefore can be used as a secondary thickening agent in TBS-1A. Hyprolose is added to TBS-1A as DMI and Transcutol reduce the gelling efficiency of silicon dioxide as these amphiphilic solvents are believed to reduce the hydrogen bonds between the silica and the oil.

*Medicinal Product (TBS-1, Gel)*

The investigational drug products, TBS1 and TBS-1A gel, are formulations of testosterone in an intranasal gel proposed for a Phase I clinical trial to compare the delivery of 125 μl (5.0 mg) of a 4.0% w/w TBS-1gel to 125 μl (5.0 mg) of a 4.0% w/w TBS-1A gel and 62.5μl (5.0 mg) of a 8.0% w/w TBS-1A gel.

Challenges for nasal delivery include:

(1) requirements for larger particles than pulmonary administration (i.e., only particles > 10 μm are sufficiently heavy to avoid entering the respiratory tract);
(2) concentrations must be higher due to the smaller volumes that can be administered;
(3) rapid clearance of the therapeutic agent from the site of deposition results in a shorter time available for absorption;
(4) potential for local tissue irritation; and
(5) limited formulation manipulation possibilities to alter drug delivery profiles.

Reports in the literature summarize various strategies to overcome the limitations of nasal drug delivery, but in many cases it is not possible to adopt the strategies due to the physicochemical and/or pharmacokinetic properties of the molecules in question. Due to first-pass metabolism, biofeedback and inter-individual variability dosing of endocrine compounds can be somewhat difficult. Natural testosterone secretion in males is 5 to 10 mg per day. For the treatment of hypogonadism, the oral dose of testosterone is 300 to 400 mg per day, the transdermal dose of testosterone is 24.3 mg (by a patch) or 50 to 150 mg (by a gel), and the buccal dose is 60 mg.

For TBS-1/TBS-1A, the quantity of testosterone per dose that is much lower than that administered via currently approved testosterone-containing dosage forms is selected. The initial dose is selected by considering the doses used for other orally administered drugs that have also been formulated for nasal delivery. A 4% testosterone-containing formulation that delivers about 5.6 mg testosterone in 140 μL to each nostril is evaluated. This 4% formulation and a 4.5% testosterone-containing formulation are further used in a Phase II clinical study per the dosing regimen presented in Table 2.1.P.2.2.1-1.

Table 2.1.P.2.2.1-1: TBS-1 Formulations used in a Phase II Clinical Study

| Formulation | Dose | Times/day | Total Daily Dose |
|---|---|---|---|
| 4.0% TBS-1 | 10.0 mg | t.i.d. | 30.0 mg |
| 4.5% TBS-1 | 13.5 mg | b.i.d. | 27.0 mg |
| | 11.25 mg | t.i.d. | 33.75 mg |

The proposed clinical study will compare 125 μl (5.0 mg) of a 4.0% w/w TBS-1 gel to 125 μl (5.0 mg) of a 4.0% w/w TBS-1A gel and 62.5μl (5.0 mg) of a 8.0% w/w TBS-1A gel packaged in HDPE syringes sealed in an aluminium foil.

*Aqueous-Based Formulation (Formulation 1)*

The first approach to developing a nasal delivery formulation of testosterone is to create a purely aqueous-based product. The formulation of a microcrystalline suspension of testosterone initially developed (referred to as "Formulation 1") is listed in Table 2.1.P.2.2.1-2.

Table 2.1.P.2.2.1-2: Composition of Formulation 1

| Compound | Amount per 1 ml |
|---|---|
| Testosterone, micronized | 40.0 mg |
| Dextrose | 26.5 mg |
| Propylene glycol | 10.0 mg |
| Polysorbate 80 | 0.04 mg |
| Avicel RC 591 | 30.0 mg |
| 0.1-N Sodium hydroxide | q.s. about 2.6 mg |
| Purified water | 891 mg |
| * Avicel RC 591 (FMC) is a mixture of microcrystalline cellulose and carboxymethyl cellulose sodium | |

Formulation 1 is filled into a device that allowed preservative-free application of testosterone to the nose. Each actuation of the device delivered 5.6 mg of testosterone from the nasal actuator. Using Formulation 1, a proof-of-concept study is conducted in hypogonadal men; the study is an open label, multiple-dose, parallel, dose-ranging study in 5 subjects.

Overall, it is concluded that in order to maintain a normalized testosterone level over 24 hours, up to 6 nasal applications would be necessary using Formulation 1 which would not be acceptable. Therefore, it is decided to reformulate the product. The high membrane permeability and short elimination half-life of testosterone from plasma, posed unique challenges. To overcome the challenges, non aqueous based formulations are explored.

*Oil-Based Formulations (Formulations 2A-D) = TBS-1*

A purely oil-based formulation of testosterone is developed. The 5.6 mg per nostril dose evaluated with Formulation 1 in the proof-of-concept study results in a relatively high $C_{max}$ value. Therefore, to achieve a lower $C_{max}$ from the oily formulation, the quantity of testosterone is lowered for the oil-based formulations.

The formulation is filled into individual containers. The first trial laboratory scale batch (Batch No. 100304) is filled into glass vials. After production of the preliminary batch, non-clinical, stability and clinical batches are packaged in LDPE packaging using blow-fill-seal technology. The clinical product for this trial will be packaged into syringes with a syringe cap.

*Formulation 2A*

The quantity of testosterone in Formulation 2A is targeted at 3.5%. The exact formulation is listed in Table 2.1.P.2.2.1-3. Formulation 2A is used in one *in vitro* and two *in vivo* preliminary safety studies.

*Formulation 2B*

The quantity of testosterone in Formulation 2B is reduced from 3.5% to 3.2% along with an adjustment of the amount of castor oil. The exact formulation is listed in Table 2.1.P.2.2.1-3. Formulation 2B is used in the 3-month safety study in animals and in two clinical studies in Europe (i.e., a Phase I 24-hr kinetic and a Phase II dose ranging study).

*Formulation 2C*

The quantity of testosterone in Formulation 2C is increased from 3.5% to 4.0% along with an adjustment of the amount of castor oil. The exact formulation is listed in Table 2.1.P.2.2.1-3. Formulation 2C is used in a Phase II clinical study.

*Formulation 2D*

The quantity of testosterone in Formulation 2D is increased from 3.5% to 4.5% along with an adjustment of the amount of castor oil. The exact formulation is listed in Table 2.1.P.2.2.1-3. Formulation 2D is used in a Phase II clinical study.

Table 2.1.P.2.2.1-3: Oil-based Trial Formulations (TBS-1 Formulations)

| TBS-1 Batch No. | Formulation | Composition mg / g | | Use |
|---|---|---|---|---|
| 100304 | 2A | Testosterone<br>Oleoyl polyoxylglycerides<br>Colloidal silicon dioxide<br>Castor oil | 35.0<br>40.0<br>40.0<br>885.0 | -Safety study in eggs (HET-CAM test)<br>- Safety study in animals (4 hrs)<br>- Safety study in animals (14 days) |
| ED 187 | 2A | See above | | Stability |
| ED 188 | 2A | See above | | Stability |
| ED 189 | 2A | See above | | Stability |
| EI014 | 2B | Testosterone<br>Oleoyl polyoxylglycerides<br>Colloidal silicon dioxide<br>Castor oil | 32.0<br>40.0<br>40.0<br>888.0 | - Stability<br>- Safety study in animals (3 months)<br>-24-hr pK study in humans<br>- Dose-ranging study in humans |
| 9256 | 2B | See above | | Phase II study in humans |
| 0823 | 2C | Testosterone<br>Oleoyl polyoxylglycerides<br>Colloidal silicon dioxide<br>Castor oil | 40.0<br>40.0<br>40.0<br>880.0 | Phase II study in humans |

(continued)

| TBS-1 Batch No. | Formulation | Composition mg / g | | Use |
|---|---|---|---|---|
| 0743 | 2D | Testosterone<br>Oleoyl polyoxylglycerides<br>Colloidal silicon dioxide<br>Castor oil | 45.0<br>40.0<br>40.0<br>875.0 | - Stability<br>- Phase II study in humans |

*Oil-Based Formulation (Formulation 3) = TBS-1A*

Human *in vivo* data showing that absorption of testosterone following administration of TBS-1 in volumes greater than 150 μL become erratic as the recommended capacity of the nasal cavity is 150 μl. The 4.5% oil-based TBS-1 formulation is currently at its maximal solubility and cannot be used for dosages of over 5. 5mg/administration.

The TBS-1 formulation exhibits a rapid peak to trough profile. It is decided to reformulate the product with the addition of small amounts of polymer to possibly increase the elimination half-life of testosterone in vivo thus minimizing the peak to trough profile. The formulations, based on testosterone solubility and gel formation are listed in Table 2.1.P.2.2.1-4.

Table 2.1.P.2.2.1-4: Oil-based Trial Formulations

| Material | TBS-1A 4% | TBS-1A 4% alternate | TBS-1A 8 % |
|---|---|---|---|
| Dimethyl isosorbide | 25.0 | 15.0 | 25.0 |
| Diethyleneglycol ethyl ether | 10.0 | 5.0 | 10.0 |
| Povidone | 3.0 | 3.0 | 3.0 |
| Copovidone | 2.0 | 2.0 | 2.0 |
| Hydroxypropyl cellulose | 0.5 | 0.5 | 0.5 |
| Testosterone micronized | 4.0 | 4.0 | 8.0 |
| Castor oil | 50.5 | 65.5 | 46.5 |
| Colloidal silicon dioxide | 5.0 | 5.0 | 5.0 |
| Total | 100.0 | 100.0 | 100.0 |

*Overages [TBS-1, Gel]*

No overage is added to the formulation.

*Physicochemical and Biological Properties [TBS-1, Gel]*

A relevant parameter for the performance of the drug product is viscosity. The viscosity is important because it facilitates maintenance of the gel in the nasal cavity in contact with the nasal mucosa.

*Manufacturing Process Development (TBS-1, Gel)*

One preliminary batch (Batch No. 100304), four pilot scale batches (Batch No.ED 187, ED 188, ED 189 and ED 014) and three commercial scale (Batch 9256,0823 and 0743) batches of TBS-1 have been produced.

Overall, the manufacturing process is straight forward and is not complicated. The individual components are mixed and then filled into syringes for clinical materials for this clinical trial.

*Container Closure System (TBS-1, Gel)*

TBS-1/TBS-1A gel is supplied in unit-dose polypropylene syringes. Syringes have been used as the primary packaging of the clinical materials for TBS-1 clinical trial as they allow for ease of dosing, ability to generate multiple doses by varying the fill volume and consistency of dose delivered. The syringes body is moulded from polypropylene, the plunger is moulded from polyethylene and the cap is HDPE. These syringes are designed and manufactured to

deliver sterile and non-sterile solutions, liquids and gels at low volumes. For additional protection from the environment (i.e., exposure to dirt, light, humidity and oxygen), the syringes are packed in a foil-laminate overwrap pouch.

The syringes and caps are designed for use in a clinical setting and meet the requirements of the EU Medical Devices Directive 93/42/EEC of June 14, 1993 and as amended. As this container closure is only intended for use in this portion of the clinical program, no additional studies will be performed on the syringe and syringe components.

An extractable volume study was performed to determine the amount of gel that is retained in the syringe after dosing. Independent of the syringe fill quantity, 23 μg of gel is retained in the syringe.

*Microbial Attributes (TBS-1, Gel)*

According to the guidance on "Container Closure Systems for packaging Human Drugs and Biologies", III F.2. (May 1999) the product is classified as a Topical Drug Product and for safety reasons at batch release, the Microbial Limit Test according to USP <61> in connection with Ph. Eur. 5.1.4 / 2.6.12 for non-sterile dosage forms for nasal use is performed applying the following criteria:

| | |
|---|---|
| TAMC (total aerobic microbial count) | < $10^2$ CFU/g |
| TYMC (total yeast and mould count) | < $10^1$ CFU/g |
| P. aeruginosa | 0/g |
| S. aureus | 0/g |

*Compatibility (TBS-1, Gel)*

The drug is not administered with a diluent, another drug product or a dosage device and therefore compatibility studies were not performed.

*MANUFACTURE (TBS-1, GEL)*

Batch Formula (*TBS-1,* Gel)

One batch of the bulk finished product, 4.0% TBS-1, has been manufactured for the proposed clinical trial. The batch formula is presented in Table 2.1.P.3.2-1.

Table 2.1.P.3.2-1: Batch Formula 4.0% TBS-1 at the 250 kg Batch Size

| Components | Quantity per Batch (kg) |
|---|---|
| Testosterone | 10.00 |
| Oleoyl polyoxylglycerides | 10,00 |
| Colloidal silicon dioxide | 10.00 |
| Castor oil | 220.00 |

Description of Manufacturing Process and Process Controls (TBS-1, Gel)

The clinical trial material is manufactured according to the following process as depicted in Fig. 19.

*Overview of in-process controls during manufacture*

The in-process controls comprise the entire manufacturing process of the product, from the incoming inspection and release of drug substance and excipients to the packaging of the drug product.

*Description of Manufacturing Process*

*Mixing of the Ingredients - Bulk Gel*

The Pre-Mix is prepared by mixing, with a propeller mixer, the full amount of Testosterone with 25.0 kg of castor oil for 5 minutes.

Mixture I is prepared by adding the Pre-Mix to the remaining castor oil amount and mixing for 10 minutes to fully dissolve the Testosterone. The product temperature is maintined below 50°C for the entire mixing process.

The oleoyl polyxoylglycerides are pre-heated to 50°C and added to Mixture I. It is mixed for 10 minutes while maintaining product temperature below 50°C. This is identified as Mixture II.

Mixture III is prepared by adding the colloidal silicon dioxide to Mixture II and mixing for 10 minutes while maintaining product temperature below 50°C. A visual check is conducted after this step, to ensure that all of the Testosterone is dissolved and the gel is homogeneous. If the solution is clear and no undissolved Testosterone remains the cooling and discharge steps are initiated. In the event that undissolved Testosterone remains, the gel is mixed for an additional 10 minutes while maintaining product temperature below 50°C and the visual check is repeated.

At the completion of mixing the gel is stirred and cooled to a product temperature below 30°C. The product is then discharged into stainless steel drums and the bulk gel sample is taken for analytical analysis.

*Filling and Packaging - Clinical Supplies*

After release of the final gel mixture by the control laboratory, the filling and packaging process is carried out by filling a pre-determined volume into the syringe followed by the application of the syringe cap. Two syringes are packaged into a foil pouch.

The syringes are filled using a pipette with the gel taken from a sterile holding tank. The tip of the pipette is discarded after the syringe is filled and the syringe cap is applied. Each syringe is individually labelled.

Following the application of the label, two syringes are packaged in a pre-formed foil pouch and the pouch is sealed. Each pouch is labelled.

*Release*

The package product is stored in quarantine and samples are presented to the quality control laboratory to control the finished product.

The control of the finished product includes all parameters of the specification. All parameters have to conform to the release specification. After passing the quality control, the product TBS-1 gel is released.

*Specifications (TBS-1, Gel)*

All excipients in the TBS-1 gel are compendial excipients. All compendial excipients are tested according to the corresponding Ph. Eur/USP monograph.

Table 2.1.P.4.1-1: Excipients and Quality Standard

| Excipient | Quality Standard |
|---|---|
| Castor oil | USP/Ph. Eur. |
| Oleoyl polyoxylglycerides | Ph. Eur./NF |
| Colloidal silicon dioxide | NF |

Excipients of Human or Animal Origin (TBS-1, Gel)

None of the excipients in TBS-1 gel is of human or animal origin.

*Specification(s) (TBS-1, Gel)*

The TBS-1 bulk gel is tested to the following specifications for batch release.

Table 2.1.P.5.1-1: Specifications for TBS-1 Bulk Gel

| Test Parameter | Method/Reference | Acceptance Criteria |
|---|---|---|
| Appearance | Visual | Slightly yellowish gel |
| Identification A | STM.TBS1.001 | Retention time and spectrum corresponds to standard |

(continued)

| Test Parameter | Method/Reference | Acceptance Criteria | |
|---|---|---|---|
| Identification B | STM.TBS1.001 | UV spectrum matches reference spectrum | |
| Assay | STM.TBS1.001 | 95.0-105.0% | |
| Related | STM.TBS1.002 | TBS-1 RC4 | ≤ 0.2% |
| Compounds | | TBS-1 RC5<br>Each individual unknown impurity<br>Total impurities | ≤ 0.5%<br>≤ 0.2%<br>≤ 1.0% |
| Viscosity | Rotational viscosimeter Ph. Eur. 2.2.10/ USP<911> | Report results | |
| TBS-1 RC4 - 17β - hydroxyandrosta-4,6-dien-3-one (Delta-6-testosterone); EP Impurity I<br>TBS-1 RC5 - 17α-hydroxyandrost-4-en-3-one (Epitestosterone); EP Impurity C<br>TBS-1 gel is packaged in unit dose syringes or the multiple dose dispensers and is tested to the following specifications for batch release. | | | |

Table 2.1.P.5.1-2: Specifications for TBS-1 Gel Packaged in Unit Dose Syringes

| Test Parameter | Method/Reference | Acceptance Criteria | |
|---|---|---|---|
| Appearance | Visually | White barrel and cap filled with slightly yellow gel | |
| Identification A | STM.TBS-1.001/PV0591 | Retention time and spectrum corresponds to standard | |
| Identification B | STM.TBS-1.001/PV0591 | UV spectrum matches reference spectrum | |
| Assay | STM.TBS-1.001/PV0591 | 95.0-105.0% | |
| Related Compounds | STM.TBS-1.002/PV0591 | Impurity I/TBS-1 RC4<br>Impurity C/TBS-1 RC5<br>Each individual unknown impurity<br>Total impurities | ≤ 0.2%<br>≤ 0.5%<br>≤ 0.2%<br>≤ 1.0% |
| Uniformity of Dosage | Ph Eur. 2.9.40 | Complies with Ph. Eur. | |
| Microbial Limits | Ph. Eur. 2.6.12 and 2.6.13 | Total aerobic microbial count <<br>Total combined yeasts/mould count<br>P. aeruginosa<br>S. aureus | $10^2$ cfu/g<br>< 10 cf<br>0/g<br>0/g |
| TBS-1RC4 - 17β - hydroxyandrosta-4,6-dien-3-one (Delta-6-testosterone); EP Impurity I<br>TBS-1 RC5 - 17α-hydroxyandrost-4-en-3-one (Epitestosterone); EP Impurity C | | | |

*Analytical Procedures (TBS-1, Gel)*

Two independent procedures are used for the identification of testosterone in the drug product, an UV and an HPLC method.

*UV-Method*

The identification by UV is determined in the Assay method using a HPLC equipped with a Diode Array Detector (DAD).

The requirements are met if the uv spectra of the sample solution corresponds to that of the standard solution.

*Related Compounds -HPLC Determination*

The related compounds Impurity C/epitestosterone and Impurity I/Δ-6-testosterone in the finished product are analysed by HPLC, as well as the unknown impurities.

Summary of Chromatographic Conditions for Related Compounds Method

| | | | |
|---|---|---|---|
| Mobile Phase | Mobile Phase A: | Acetonitrile: Water; 10:90 | |
| | Mobile Phase B: | Acetonitrile: Water; 90:10 | |
| | Filter and de-aerate each mobile phase<br>Gradient Program | | |
| | Time | Mobile Phase A | Mobile Phase B |
| | 0 | 70 | 30 |
| | 13 | 70 | 30 |
| | 21 | 0 | 100 |
| | 24.99 | 0 | 100 |
| | 25 | 70 | 30 |
| | 30 | 70 | 30 |
| Column | Type: Hypersil BDS C18<br>Dimensions: 250 cm x 4.0 mm<br>Particle Size: 5 μm | | |
| Flow rate | 1.0 mL/min | | |
| Column Temp. | 40°C | | |
| Detector | 240nm & 290nm | | |
| Injection Volume | 15 μL | | |
| Run Time | 30 minutes | | |
| Retention Time (RT) | Related Compound | Typical RT | |
| | 1. Impurity I | 11.3 min. | |
| | 2. Testosterone | 14.1 min | |
| | 3. Impurity C | 18.3 min. | |
| Standard and Sample Concentrations | Standard contains 2.5 μg/mL of testosterone and 0.50 μg/mL of Impurity I.<br>Sample contains of 1.0 mg/mL of testosterone. | | |

*Summary of the Assay*

Summary of Chromatographic Conditions for Related Compounds Method

| | | |
|---|---|---|
| Mobile Phase | Mobile Phase : | Methanol : Water; 60:40 |
| | | |
| Column | Type: Thermo ODS Hypersil<br>Dimensions: 100 cm x 4.6 mm<br>Particle Size: 5 μm | |
| Flow rate | 1.0 mL/min | |
| Column Temp. | 40°C | |
| Detector | 245nm | |

(continued)

| | |
|---|---|
| Mobile Phase | Mobile Phase : Methanol : Water; 60:40 |
| | |
| Injection Volume | 4 μL |
| Run Time | 14 minutes |
| Retention Time (RT) | Testosterone Typical RT 5.0 min. |
| Standard and Sample Concentrations | Standard contains 300 μg/mL of testosterone<br>Sample contains of 300 μg/mL of testosterone. |

The measurement of the viscosity of TBS-1/TBS-1A is performed using a rotational viscosimeter

The results are the mean of all sample viscosities.

*Delivered Dose Uniformity (Ph Eur. 2.9.40)*

This method describes the procedure for determining the delivered dose uniformity of the finished product. Delivered dose uniformity is performed per Ph. Eur. 2.9.40.

*Microbial Limits (USP <61> and Ph. Eur. 2.6.12 and 2.6.13)*

Microbial Limits testing is performed per USP <61> and Ph. Eur 2.6.12 and 2.6.13

**Batch analysis (TBS-1, Gel)**

One preliminary batch (Batch No. 1000304), four pilot scale batches (Batch No. ED 187, ED 188, ED 189 and ED 014 and three commercial scale (Batch 9256, 0823 and 0743) batches of TBS-1 have been produced. A description of the TBS-1 batches is presented in Table 2.1.P.5.4-1.

Table 2.1.P.5.4-1: Description of TBS-1 Batches

| Formulation | 2A | 2A | 2A | 2A | 2B |
|---|---|---|---|---|---|
| Batch No. | 100304 | ED 187 | ED 188 | ED 189 | EI 014 |
| Batch Size | 500 g | 45 kg | 45 kg | 68 kg | 72 kg |
| Date of Manufacture | March 2004 | May 2004 | May 2004 | May 2004 | September 2004 |
| | | | | | |
| | | | | | |
| Equipment | Laboratory scale | Commercial process | Commercial process | Commercial process | Commercial process |
| Filling Quantity per Container | - | 350 mg | 350 mg | 350 mg | 230 mg |

Batch 0823, bulk 4.0% testosterone gel, was released and filled into the unit dose syringe (Batch 0942). Release data on the bulk gel is presented in Table 2.1.P.5.4-3 and on the finished products, Batches 0942, in Table 2.1.P.5.4-4

Table 2.1.P.5.4-3: Batch Analysis - Batch 0823 Bulk Gel

| Test Parameter | Acceptance Criteria | Results of Batch 0823 |
|---|---|---|
| Appearance | Clear, slightly yellowish gel | Complies |
| Identification A | Retention time and spectrum corresponds to standard | Complies to 5.0 min |
| Identification B | UV spectrum matches reference spectrum | Complies |

(continued)

| Test Parameter | Acceptance Criteria | Results of Batch 0823 |
|---|---|---|
| Assay | 95.0 -105% | 100% |
| Related Compounds | Imp I/TBS-1 RC4 ≤ 0.2%<br>Imp C/TBS-1 RC5 ≤ 0.5%<br>Each individual unknown impurity ≤ 0.2%<br>Total impurities ≤ 1.0% | 0.3%<br>< 0.5%<br>0.0%<br>0.3% |
| Viscosity | Report result | 5.086 mPas/30s |

Table 2.1.P.5.4-4: Batch Analysis - Batches 0942 Finished Product

| Test Parameter | Acceptance Criteria | Batch 0942 |
|---|---|---|
| Appearance | slightly yellow gel (syringe) | Complies |
| Identification A | Retention time and spectrum corresponds to standard | Complies 5.0 min |
| Identification B | UV spectrum matches reference spectrum | Complies |
| Assay | 95.0- 105.0% | 100% |
| Related Compounds | Imp I/TBS-1 RC4 ≤ 0.2%<br>Imp C/TBS-1 RC5 ≤ 0.5%<br>Each individual unknown impurity ≤ 0.2%<br>Total impurities ≤ 1.0% | 0.3%<br>< 0.05%<br>< 0.05%<br>0.3% |
| Uniformity of Dosage Units | Ph Eur. 2.9.40 | n.p. |
| Microbial Limits | TAMC < $10^2$ cfu/g<br>TYMC < 10 cfu/g<br>P. Aeruginosa 0/g<br>S. Aureus 0/g | Complies<br>Complies<br>Complies<br>Complies |
| n.p.: not performed<br>Note: Delivered dose uniformity is added as a test parameter after batch 0942 is release | | |

*Characterization of Impurities (TBS-1, Gel)*

Per the Testosterone CoA, there are five potential, identified impurities that might be present in testosterone drug substance for TBS-1: androstenedione (Ph. Eur. impurity A), androstenedione methyl enol ether (Ph. Eur. impurity J), delta-4-androstenediol (Ph. Eur. impurity D), delta-6-testosterone (Ph. Eur. impurity I) and epitestosterone (Ph. Eur. impurity C, main impurity).

It is believed that the impurities from the synthesis pathway of testosterone; their amount should not change during storage in the finished product.

During the initial product development, impurities A, D, I and C are assayed. Impurity A, androstenedione, and impurity D, delta-4-androstenediol, have been dropped from batch release testing as they are the starting material and a derivative of a starting material respectively and remain stable over a 30- month time period and following stress studies (photostability and temperature cycling). Impurity J, androstenedione methylenolether a derivative of the starting material androstenedione, is not tested for in the final drug product, rather, it is included with the "non-specified" impurities in the drug product.

Degradation products or impurities from the manufacturing process are specified as "unidentified impurities" and are limited to NMT 0.2% in the finished product.

*Appearance*

Acceptance criteria: Slightly yellow gel

*Identification*

Adequate identification of the active ingredient in the finished product is performed at release and shelf life by its HPLC retention time and at release by UV.

*Related Compounds*

| | | |
|---|---|---|
| Acceptance criteria: | Epitestosterone (Impurity C) | ≤ 0.5% |
| | Δ-6-testosterone (Impurity I) | ≤ 0.2% |
| | Single impurity | ≤ 0.2% |
| | Total impurities | ≤ 1.0% |

*Rationale:*

The maximum daily dose of testosterone is 33 mg.

*As per ICH Q-3B(R), the following thresholds are applicable:*

Reporting Threshold is 0.1%
Identification Threshold is 0.2%, which is lower than 2 mg daily intake calculated based on the maximum daily dose of 33 mg of testosterone.
Qualification Threshold is 0.5%, which is lower than 200 μg daily intake calculated based on the maximum daily dose of 33 mg of testosterone.
The limit for Impurity I (TBS-1 RC4) is 0.2% and is tighter than the ICH Q3B qualification threshold. The limit for Impurity C (TBS-1 RC5) is 0.5% which is lower than the 200 μg daily intake.

*Assay*

Acceptance criteria: 95.0 - 105.0%
The purpose of this assay is to establish the identity and to determine the testosterone content per gram based on the intended dose per application.
The range for the assay (± 5% of label claim) at release.

*Uniformity of Dose*

Acceptance criteria: as per Ph. Eur.2.9.40

*Microbial limits*

Acceptance criteria: as per Ph. Eur./USP
The microbiological testing and acceptance criteria was established for total yeasts and molds, total aerobic microbial count, *Staphylococcus aerus* and *Pseudonomas aeruginosa* based on ICH and Ph. Eur. recommendation 5.1.4 / 2.6.12., 2.6.13

*Reference Standards or Materials (TBS-1, Gel)*

For testing of the drug product the applicant in general uses/used USP or Ph. Eur. reference standards. In the case there is/was no official standard available the corresponding compound is/was provided by the manufacturer or by specialized laboratories.
Table 2.1.P.6.1 lists the reference standards used.

Table 2.1.P.6-1: Reference Standards

| Compound | Ph. Eur. Name | Batch No. | Supplier |
|---|---|---|---|
| Androstenedione | Impurity A | 116 H 0463 | Sigma-Aldrich Chemie GmbH, Schnelldorf, Germany |
| Epitestosterone | Impurity C | 100 H 4022 | Sigma-Aldrich Chemie |
| Δ-4-Androstenediol | Impurity D | 02-01834-580 | ChromaDex Inc., USA |
| Δ-6-Testosterone | Impurity I | C 241 | Steraloids Inc., USA |
| Testosterone USP | Testosterone | I | Promochem GmbH, Wesel, Germany |
| Testosterone USP | Testosterone | I 1 B 253 | |
| Testosterone USP | Testosterone | I 1 B 253 | |

*Container Closure System (TBS-1, Gel)*

The primary packaging for the clinical supplies will be unit-dose syringes.

*Unit dose syringes*

The unit dose syringes consist of a syringe closed with a syringe cap. The secondary packaging for these syringes is made up of an aluminium foil pouch appropriately labelled.

*Syringe and syringe cap*

The syringe consists of two components, the syringe body and the plunger. The body is moulded from polypropylene. The plunger is moulded from polyethylene.

The syringe cap is made from HDPE.

*Aluminum pouch*

For a further element of protection, two syringes are contained in secondary packaging consisting of an aluminium foil pouch. Two syringes are packaged in the aluminium foil pouch and each pouch is sealed.

The pouch consists of a flexible, 3-layered-foil-laminate of a) polyester 12 micron, b) aluminum 12 micron and c) a polyethylene 75 micron. It is manufactured by Floeter Flexibles GmbH, and supplied under the name "CLIMAPAC II 12-12-75".

*Stability (TBS-1, Gel)*

Stability studies on TBS-1 batches are performed.

Table 2.1.P.8.1-1: Stability Studies Conducted in Support of TBS-1

| Study Type | Container Closure System | Drug Product Batch No. | Storage Conditions | Stability Data available | Study End |
|---|---|---|---|---|---|
| ICH | White LDPE unit dose container; sterile air in pressure cushion; aluminum pouch secondary package (no nitrogen) | ED 187C<br>ED 188<br>ED 189 | 25°C/60% RH<br><br>40°C/75% RH | 12 months<br><br>6 months | Study completed |
| ICH | | EI014 | 25°C/60% RH | 36 months plus a 42 month analysis | Study completed |
| ICH Photostability | | ED 187B | 9 hours ≥ 200 Wh/$m^2$ (300-400 nm)<br>22 hours 1.2 Mill. Lxh. (400-800 nm) | Full exposure | Study completed |
| Thermal Cycling | | ED 188 | 12 hr -20°C cycle to 12 hr + 40°C | 4 weeks | Study completed |
| ICH | Syringe with Syringe Cap | Pilot Scale (non GMP)<br>4.0 mg<br>5.5 mg<br>7.0 mg | 25°C/60% RH<br><br>40°C/75% RH | 6 months | Study completed |
| ICH | Stainless Steel Drum under Nitrogen | 9256 | Ambient temperature | 6 months | Completed |
| ICH | Syringe with | Bulk 9256 | 25°C/60% | 6 | Completed |
| | Syringe Cap | 9445 - 4.0 mg<br>9246 - 5.5 mg<br>9247 - 7.0 mg | RH 40°C/75% RH | months | |
| ICH | Stainless Steel Drum under Nitrogen | 0743<br>0823 | 25°C/60% RH<br>40°C/75% RH | Initial | Ongoing |
| ICH | Syringe with Syringe Cap | 0943 | 25°C/60% RH<br>40°C/75% RH | initial | Ongoing |

Overall, stability data provided in this section are concluded to support a 24 month "use by" period for TBS-1 stored at controlled room temperature conditions [i.e., 25°C (77°F); excursions 15-30°C (59-86°F)]. The data also show that special storage conditions for the drug product are not required. The packaging configuration is adequate to protect the drug product from light and the drug product does not degrade or change physically following exposure to temperature cycling stress.

The clinical supplies are applied a 1 year re-test period, when stored at controlled room temperature conditions [i.e., 25°C (77°F); excursions 15-30°C (59-86°F)], to reflect the duration of the trial and the data available.

*Stability Data [TBS-1, Gel]*

In this section, stability data tables for a commercial size bulk Batch 9256, 0743 and 0823 and finish product lots 9445, 9446, 9447 and 0943 are provided.

A 6 month real time stability program is conducted on the commercial scale bulk (Batch 9256). A 36 month real time and a 6 month accelerated stability program is ongoing on three different doses of Batch 9256 packaged in 1ml syringes: Batch 9445 4.0 mg (3.2% gel), Batch 9446 5.5 mg (3.2% gel), Batch 9447 7.0 mg (3.2% gel).

A 6 month real time stability program is underway on the 4.5% gel and the 4.0% gel. A 36 month real time and a 6 month accelerated stability program is underway on Batch 0943 (bulk Batch 0743 filled in 1ml syringes).

Table 2.1.P.8.3-1: Stability Schedule for Commerical Scale Bulk TBS-1 Gel and Finished Product Filled in 1 ml Syringes

| **Storage Conditions (°C, % RH)** | **Product** | **Completed Test Int**ervals (Outstanding Test Intervals) |
|---|---|---|
| Ambient temperature | 9256 | **0m, 3m, 6m** |
| 25 ± 2°C, 60 ± 5 % | 9445 | **0m, 6m 12m** (24m, 36m) |
| 40 ± 2°C, 75 ± 5 % | 9445 | **0m, 3m, 6m** |
| 25 ± 2°C, 60 ± 5 % | 9446 | **0m, 6m, 9m** (18m, 30m. 36m) |
| 40 ± 2°C, 75 ± 5% | 9446 | **0m, 3m, 6m** |
| 25± 2°C, 60 ± 5% | 9447 | **0m, 6m, 12m** (24m, 36m) |
| 40 ± 2°C, 75 ± 5 % | 9447 | **0m, 3m, 6m** |
| 25± 2°C, 60 ± 5 % | 0943 | **0m, 3m** (9m, 18m, 30m, 36m) |
| 40 ± 2°C, 75 ± 5% | 0943 | **0m, 3m** (6m) |
| Ambient temperature | 0743 | **0m, 3m** (6m) |
| Ambient temperature | 0823 | **0m, 3m** (6m) |

Table 2.1.P.8.3-2: Stability Data TBS-1 Batch 9256 (3.2% Bulk Gel) Manufactured July 2009 Stored at Ambient Temperature

| **Test Parameter** | **Acceptance Criteria** | **Time 0** | **3 months** | **6 months** |
|---|---|---|---|---|
| Appearance | Slightly yellow gel | Complies | Complies | Complies |
| Colour of formulation | Colour ≤ 250 | 200 | 200 | 200 |
| Viscosity | 3,000 - 10,000 mPa x sec | 5504 | 5325 | 5198 |
| Density | 0.97 - 1.01 $g/cm^3$ | 0.99 | 0.99 | 0.99 |
| Iodine value | FIPO | 78.62 | 77.39 | 76.40 |
| Acid value | FIPO (mg KOH/g) | 1.98 | 2.00 | 2.16 |
| Peroxide value | FIPO (meq $O_2$/kg) | 3.56 | 3.16 | 2.63 |
| Identification | a. Retention time corresponds to RS<br>b. UV spectrum corresponds to RS | Complies<br>Complies | Complies<br>Complies | Complies<br>Complies |
| Impurities | Imp C ≤ 0.5 % | 0.166 % | 0.148 % | 0.189% |
| | Imp I ≤ 0.1 % | < 0.05 % | 0.05 % | <0.05 % |
| | Each individual unknown imp. ≤ 0.2 % | 0.064 % | 0.05 % | 0.075 % |
| | Total imp. ≤ 1.0 % | 0.230 % | 0.198 % | 0.264 % |
| | Imp. D ≤ 0.2 % | < 0.2 % | < 0.2 % | 0.2% |
| Assay | 95.0 - 105.0 % | 99.4 % | 98.3 % | 100.4 % |

(continued)

| Test Parameter | Acceptance Criteria | Time 0 | 3 months | 6 months |
|---|---|---|---|---|
| Microbial limits | TAMC < $10^2$ cfu/g | < 10 cfu/g | < 10 cfu/g | < 10 cfu/g |
| | TYMC < 10 cfu/g | < 10 cfu/g | < 10 cfu/g | < 10 cfu/g |
| | *S. aureus* 0/g | Not detected/g | Not detected/g | Not detected/g |
| | *P. aeruginosa* 0/g | Not detected/g | Not detected/g | Not detected/g |

Table 2.1.P.8.3-3: Stability Data 4.0 mg TBS-1 Batch 9445 (3.2 % gel) 1 ml Syringe (25 ± 2°C, 60 ± 5 % RH, horizontal)

| Test Parameter | Acceptance Criteria | Time 0 | 6 months | 12 months |
|---|---|---|---|---|
| Appearance | Slightly yellow gel | Complies | Complies | Complies |
| Colour of formulation | Colour ≤ 250 | 200 | 200 | 150 |
| Dissolution | ≥ 80% within 120 min | 87.8 % within 120 minutes | 93.1 % within 120 minutes | 79.6 % within 120 minutes |
| Impurities | Imp C ≤ 0.5 % | 0.127 % | 0.096% | 0.155% |
| | Imp I ≤ 0.1 % | < 0.05 % | < 0.05 % | < 0.05 % |
| | Each individual unknown imp. ≤ 0.2 % | < 0.05 % | < 0.05 % | RT 0.379: 0.108 %<br>RT 0.867: 0.062% |
| | Total imp. ≤ 1.0% | 0.127% | 0.096% | 0.325% |
| | Imp. D ≤ 0.2 % | < 0.2 % | < 0.2 % | < 0.2 % |
| Assay | 95.0 - 105.0 % | 99.3% | 99.6% | 96.7% |
| Microbial limits | TAMC < $10^2$cfu/g | < 10 cfu/g | - | - |
| | TYMC < 10 cfu/g | < 10 cfu/g | - | - |
| | *S. aureus* 0/g | Not detected/g | - | - |
| | *P. aeruginosa* 0/g | Not detected/g | - | - |

Table 2.1.P.8.3-4: Stability Data 4.0 mg TBS-1 Batch 9445 (3.2 % gel) 1 ml Syringe, (40 ± 2°C, 75 ± 5 % RH, horizontal)

| Test Parameter | Acceptance Criteria | Time 0 | 3 months | 6 months |
|---|---|---|---|---|
| Appearance | Slightly yellow gel | Complies | Complies | |
| Colour of formulation | Colour ≤ 250 | 200 | 200 | |
| Dissolution | ≥ 80% within 120 min | 87.8 % within 120 minutes | 87.3 % within 120 minutes | |
| Impurities | Imp C ≤ 0.5 % | 0.127 % | 0.128% | |
| | Imp I ≤ 0.1 % | < 0.05 % | < 0.05 % | |
| | Each individual unknown imp. ≤ 0.2 % | < 0.05 % | Rel RT 0.38: 0.177%<br>Rel RT 2.93: 0,066% | |
| | Total imp. ≤ 1.0 % | 0.127% | 0.371 % | |
| | Imp. D ≤ 0.2 % | < 0.2 % | < 0.2 % | |
| Assay | 95.0 - 105.0% | 99.3% | 99.3% | |

(continued)

| Test Parameter | Acceptance Criteria | Time 0 | 3 months | 6 months |
|---|---|---|---|---|
| Microbial limits | TAMC < $10^2$ cfu/g | < 10 cfu/g | < 10 cfu/g | |
| | TYMC < 10 cfu/g | < 10 cfu/g | < 10 cfu/g | |
| | *S. aureus* 0/g | Not detected/g | Not detected/g | |
| | *P. aeruginosa* 0/g | Not detected/g | Not detected/g | |

Table 2.1.P.8.3-5: Stability Data 5.5 mg TBS-1 Batch 9446 (3.2 % gel) 1 ml Syringe (25 ± 2°C, 60 ± 5 % RH, horizontal)

| Test Parameter | Acceptance Criteria | Time 0 | 3 months | 9 months | 12 months |
|---|---|---|---|---|---|
| Appearance | Slightly yellow gel | Complies | Complies | Complies | - |
| Colour of formulation | Colour ≤ 250 | 200 | 200 | 200 | |
| Dissolution | ≥ 80% within 120 min | 86.8 % within 120 minutes | 83.6 % within 120 minutes | 84.1 % within 120 minutes | - |
| Impurities | Imp C ≤ 0.5 % | 0.125 % | 0.126% | 0.105% | - |
| | Imp I ≤ 0.1 % | < 0.05 % | < 0.05 % | < 0.05 % | - |
| | Each individual unknown imp. ≤ 0.2 % | < 0.05 % | < 0.05 % | < 0.05 % | - |
| | Total imp. ≤ 1.0 % | 0.125% | 0.126% | 0.105% | - |
| | Imp. D ≤ 0.2 % | < 0.2 % | < 0.2 % | < 0.2 % | - |
| Assay | 95.0 - 105.0 % | 99.1% | 99.4% | 98.3% | - |
| Microbial limits | TAMC < $10^2$ cfu/g | < 10 cfu/g | < 10 cfu/g | - | - |
| | TYMC < 10 cfu/g | < 10 cfu/g | < 10 cfu/g | - | - |
| | *S. aureus* 0/g | Not detected/g | Not detected/g | - | - |
| | *P. aeruginosa* 0/g | Not detected/g | Not detected/g | - | - |

Table 2.1.P.8.3-6: Stability Data 5.5 mg TBS-1 Batch 9446 (3.2 % gel) 1 ml Syringe (40 ± 2°C, 75 ± 5 % RH, horizontal)

| Test Parameter | Acceptance Criteria | Time 0 | 3 months | 6 months | |
|---|---|---|---|---|---|
| Appearance | Slightly yellow gel | Complies | Complies | | |
| Colour of formulation | Colour ≤ 250 | 200 | 200 | | |
| Dissolution | ≥ 80% within 120 min | 86.8 % within 120 minutes | 86.8 % within 120 minutes | | |
| Impurities | Imp C ≤ 0.5 % | 0.125 % | 0.127% | | |
| | Imp I ≤ 0.1 % | < 0.05 % | < 0.05 % | | |
| | Each individual unknown imp. ≤ 0.2 % | < 0.05 % | Rel RT 0.38: 0.102%<br>Rel RT 3.01: 0.070 | | |
| | Total imp. ≤ 1.0 % | 0.25% | 0.299% | | |
| | Imp. D ≤ 0.2 % | ≤ 0.2 % | < 0.2 % | | |

(continued)

| Test Parameter | Acceptance Criteria | Time 0 | 3 months | 6 months | |
|---|---|---|---|---|---|
| Assay | 95.0 - 105.0 % | 99.1 % | 97.9 % | | |
| Microbial limits | TAMC < $10^2$ cfu/g | < 10 cfu/g | < 10 cfu/g | | |
| | TYMC < 10 cfu/g | < 10 cfu/g | < 10 cfu/g | | |
| | *S. aureus* 0/g | Not detected/g | Not detected/g | | |
| | *P.aeruginosa* 0/g | Not detected/g | Not detected/g | | |

Table 2.1.P.8.3-7: Stability Data 7.0 mg TBS-1 Batch 9447 (3.2 % gel) 1 ml Syringe (25 ± 2°C, 60 ± 5 % RH, horizontal)

| Test Parameter | Acceptance Criteria | Time 0 | 6 months | 12 months |
|---|---|---|---|---|
| Appearance | Slightly yellow gel | Complies | Complies | Complies |
| Colour of formulation | Colour ≤ 250 | 200 | 200 | 150 |
| Dissolution | ≥ 80% within 120 min | 83.5 % within 120 minutes | 90.9 % within 120 minutes | 80.0 % within 120 minutes |
| Impurities | Imp C ≤ 0.5 % | 0.132 % | 0.092% | 0.153% |
| | Imp I ≤ 0.1 % | < 0.05 % | < 0.05 % | < 0.05 % |
| | Each individual unknown imp, ≤ 0.2 % | < 0.05 % | < 0.05 % | RT 0.379: 0.083%<br>RT 0.867: 0.053% |
| | Total imp. ≤ 1.0 % | 0.132% | 0.092% | 0.289% |
| | Imp. D ≤ 0.2 % | < 0.2 % | < 0.2 % | < 0.2 % |
| Assay | 95.0 - 105.0 % | 98.7% | 99.3% | 96.9% |
| Microbial limits | TAMC < $10^2$ cfu/g | < 10 cfu/g | < 10 cfu/g | < 10 cfu/g |
| | TYMC < 10 cfu/g | < 10 cfu/g | < 10 cfu/g | < 10 cfu/g |
| | *S. aureus* 0/g | Not detected/g | Not detected/g | Not detected/g |
| | *P. aeruginosa* 0/g | Not detected/g | Not detected/g | Not detected/g |

Table 2.1.P.8.3-8: Stability Data 7.0 mg TBS-1 Batch 9447 (3.2 % gel) 1 ml Syringe (40 ± 2°C, 75 ± 5 % RH., horizontal)

| Test Parameter | Acceptance Criteria | Time 0 | 3 months | 6 months |
|---|---|---|---|---|
| Appearance | Slightly yellow gel | Complies | Complies | |
| Colour of formulation | Colour ≤ 250 | 200 | 200 | |
| Dissolution | ≥ 80% within 120 min | 83.5 % within 120 minutes | 85.4 % within 120 minutes | |

(continued)

| Test Parameter | Acceptance Criteria | Time 0 | 3 months | 6 months |
|---|---|---|---|---|
| Impurities | Imp C ≤ 0.5 % | 0.132% | 0132% | |
| | Imp I ≤ 0.1 % | < 0.05 % | < 0.05 % | |
| | Each individual unknown imp. ≤ 0.2 % | < 0.05 % | Rel RT 0.37: 0.074%<br>Rel RT 3.13: 0.069 | |
| | Total imp. ≤ 1.0 % | 0.132% | 0.275% | |
| | Imp. D ≤ 0.2 % | < 0.2 % | < 0.2 % | |
| Assay | 95.0 - 105.0 % | 98.7% | 99.1% | |
| Microbial limits | TAMC < $10^2$ cfu/g | < 10 cfu/g | < 10 cfu/g | |
| | TYMC < 10 cfu/g | < 10 cfu/g | < 10 cfu/g | |
| | *S. aureus* 0/g | Not detected/g | Not detected/g | |
| | *P. aeruginosa* 0/g | Not detected/g | Not detected/g | |

Table 2.1.P.8.3-9: Stability Data 5.6 mg TBS-1 Batch 0943 (4.5% gel) 1 ml Syringe (25 ± 2°C, 60 ± 5 % RH, horizontal)

| Test Parameter | Acceptance Criteria | Time 0 | 3 months | 6 months |
|---|---|---|---|---|
| Appearance | Slightly yellow gel | Complies | Complies | |
| Colour of formulation | Colour ≤ 250 | Complies | Complies | |
| Related Compounds | TBS-1 RC 5 (Imp C) ≤ 0.5 % | 0.34% | 0.33% | |
| | TBS-1 RC 4(Imp I) ≤ 0.1 % | < 0.05 % | < 0.05 % | |
| | Each individual unknown imp. ≤ 0.2 % | < 0.05 % | RRT 0.32: 0.06%<br>RRT 0.80: 0.05% | |
| | Total imp. ≤ 1.0 % | 0.3% | 0.4% | |
| Assay | 95.0 - 105.0 % | 101 % | 99% | |
| Microbial limits | TAMC < $10^2$ cfu/g | Complies | - | |
| | TYMC < $10^2$ cfu/g | Complies | - | |
| | *S. aureus* 0/g | Complies | - | |
| | *P. aeruginosa* 0/g | Complies | - | |

Table 2.1.P.8.3-10: Stability Data 5.6 mg TBS-1 Batch 0943 (4.5% gel) 1 ml Syringe (40 ± 2°C, 75 ± 5 % RH, horizontal)

| Test Parameter | Acceptance Criteria | Time 0 | 3 months 6 months | |
|---|---|---|---|---|
| Appearance | Slightly yellow gel | Complies | Complies | |
| Colour of formulation | Colour ≤ 250 | Complies | Complies | |
| Related Compounds | TBS-1 RC 5 (Imp C) ≤ 0.5 % | 0.3% | 0.3% | |
| | TBS-1 RC 4(Imp I) ≤ 0.1 % | < 0.05 % | < 0.05 % | |
| | Each individual unknown imp. ≤ 0.2 % | < 0.05 % | Rel RT0.32: 0.09 | |
| | Total imp. ≤ 1.0 % | 0.4% | 0.5% | |
| Assay | 95.0 - 105.0 % | 101% | 97% | |

(continued)

| Test Parameter | Acceptance Criteria | Time 0 | 3 months 6 months | |
|---|---|---|---|---|
| Microbial limits | TAMC < $10^2$ cfu/g | Complies | - | |
| | TYMC < 10 cfu/g | Complies | - | |
| | *S. aureus* 0/g | Complies | - | |
| | *P. aeruginosa* 0/g | Complies | - | |

Table 2.1.P.8.3-11: Stability Data TBS-1 Batch 0743 (4.5 % gel) Bulk Stored at Ambient Temperature

| Test Parameter | Acceptance Criteria | Time 0 | 3 months | 6 months |
|---|---|---|---|---|
| Appearance | Slightly yellow gel | Complies | Complies | |
| Colour of formulation | Colour ≤ 250 | Complies | Complies | |
| Related Compounds | TBS-1 RC 5 (Imp C) ≤ 0.5 % | 0.3% | 0.3% | |
| | TBS-1 RC 4(Imp I) ≤ 0.1 % | < 0.05 % | < 0.05 % | |
| | Each individual unknown imp. ≤ 0.2 % | < 0.05 % | Rel RT 0.32: 0.06% | |
| | Total imp. ≤ 1.0 % | 0.3% | 0.44% | |
| Assay | 95.0 - 105.0 % | 100% | 99% | |
| Microbial limits | TAMC < $10^2$ cfu/g | Complies | Complies | |
| | TYMC < 10 cfu/g | Complies | Complies | |
| | *S. aureus* 0/g | Complies | Complies | |
| | *P. aeruginosa* 0/g | Complies | Complies | |

Table 2.1.P.8.3-12: Stability Data TBS-1 Batch 0823 (4.0% gel) Bulk Stored at Ambient Temperature

| Test Parameter | Acceptance Criteria | Time 0 | 3 months | 6 months |
|---|---|---|---|---|
| Appearance | Slightly yellow gel | Complies | Complies | |
| Colour of formulation | Colour ≤ 250 | Complies | Compies | |
| Related Compounds | TBS-1 RC 5 (Imp C) ≤ 0.5% | 0.34% | 0.33% | |
| | TBS-1 RC 4(Imp I) ≤ 0.1 % | < 0.05 % | < 0.05 % | |
| | Each individual unknown imp. ≤ 0.2 % | < 0.05 % | Rel RT 0.32: 0.05% Rel RT0.80: 0.05% | |
| | Total imp. ≤ 1.0 % | 0.39% | 0.43% | |
| Assay | 95.0 - 105.0 % | 100% | 99% | |
| Microbial limits | TAMC < $10^2$ cfu/g | Complies | - | |
| | TYMC < 10 cfu/g | Complies | - | |
| | *S. aureus* 0/g | Complies | - | |
| | *P. aeruginosa* 0/g | Complies | - | |

*MEDICINAL PRODUCT (TBS-1A, Gel)*

TBS-1A gel is a viscous and thixotropic, oil-based formulation containing solubilized testosterone intended for

intranasal application. The drug product is formulated with castor oil, dimethyl isosorbide, diethyleneglycol ethyl ether, colloidal silicon dioxide, povidone, copovidone, hydroxypropyl cellulose.

Two different doses of TBS-1A gel will be administered in this clinical trial: 4% w/w and 8% w/w. An overage is added to each syring to account for the gel that is retained in the syringe after dosing. This overage remains constant at 23μl regardless of the volume of gel in the syringe.

Composition

The compositions of the drug product to be administered in this clinical trial are provided in Table 2.1.P.1-1 - 2.1.P.1.1-3.

Table 2.1.P.1-1: Components, Quantity, Quality Standards and Function, 4.0% TBS-1A

| Component | Amount (% w/w) | Quantity per Syringe (mg) | Quantity Delivered per Dose (mg) | Function | Quality Standard |
|---|---|---|---|---|---|
| Testosterone | 4.0 | 5.92 | 5.00 | Active ingredient | USP |
| Castor oil | 50.5 | 74.74 | 63.12 | Solvent | Ph. Eur/ USP |
| Diethyl isosorbide | 25.0 | 37.00 | 31.25 | Solvent | In-house testing |
| Diethylene glycol monoethyl ether | 10.0 | 14.80 | 12.50 | Solvent | Ph. Eur. |
| Colloidal silicon dioxide | 5.0 | 7.40 | 6.25 | Viscosity agent | NF |
| Povidone | 3.0 | 4.44 | 3.75 | Binding agent | Ph. Eur/ USP |
| Copovidone | 2.0 | 2.96 | 2.50 | Binding agent | Ph. Eur/ USP |
| Hydroxypropyl cellulose | 0.5 | 0.74 | 0.62 | Viscosity agent | Ph. Eur/ USP |

Table 2.1.P.1-2: Components, Quantity, Quality Standards and Function, 4.0% TBS-1A (Alternative)

| Component | Amount (% w/w) | Quantity per Syringe (mg) | Quantity Delivered per Dose (mg) | Function | Quality Standard |
|---|---|---|---|---|---|
| Testosterone | 4.0 | 5.92 | 5.00 | Active ingredient | USP |
| Castor oil | 65.5 | 96.94 | 81.87 | Solvent | Ph. Eur/ USP |
| Diethyl isosorbide | 15.0 | 22.2 | 18.75 | Solvent | In-house testing |
| Diethylene glycol monoethyl ether | 5.0 | 7.40 | 6.25 | Solvent | Ph. Eur. |
| Colloidal silicon dioxide | 5.0 | 7.40 | 6.25 | Viscosity agent | NF |
| Povidone | 3.0 | 4.44 | 3.75 | Binding agent | Ph. Eur/ USP |
| Copovidone | 2. 0 | 2.96 | 2.50 | Binding agent | Ph. Eur/ USP |
| Hydroxypropyl cellulose | 0.5 | 0.74 | 0.62 | Viscosity agent | Ph. Eur/ USP |

Table 2.1.P.1-3: Components, Quantity, Quality Standards and Function, 8.0% TBS-1A

| Component | Amount (% w/w) | Quantity per Syringe (mg) | Quantity Delivered per Dose (mg) | Function | Quality Standard |
|---|---|---|---|---|---|
| Testosterone | 8.0 | 11.84 | 10.00 | Active ingredient | USP |
| Castor oil | 46.5 | 68.82 | 58.12 | Solvent | Ph. Eur/ USP |
| Diethyl isosorbide | 25.0 | 37.00 | 31.25 | Solvent | In-house testing |
| Diethylene glycol monoethyl ether | 10.0 | 14.80 | 12.50 | Solvent | Ph. Eur. |
| Colloidal silicon dioxide | 5.0 | 7.40 | 6.25 | Viscosity agent | NF |
| Povidone | 3.0 | 4.44 | 3.75 | Binding agent | Ph. Eur/ USP |
| Copovidone | 2.0 | 2.96 | 2.50 | Binding agent | Ph. Eur/ USP |
| Hydroxypropyl cellulose | 0.5 | 0.74 | 0.62 | Viscosity agent | Ph. Eur/ USP |

Container

TBS-1A gel is supplied in unit-dose polypropylene syringes. Two syringes of each dosage are packaged in a protective aluminium foil pouch.

*Medicinal Product (TBS-1A, Gel)*

Manufacturing Process Development (TBS-1A, Gel)

One batch of 4% TBS-1A (Batch No. IMP 11001), 4% (alternative) TBS-1A (Batch No. IMP 11002) and 8% TBS-1A (Batch No. IMP 11003) have been manufactured.

Overall, the manufacturing process is straight forward and is not complicated. The individual components are mixed and then filled into syringes for clinical materials for this clinical trial.

*Batch Formula (TBS-1A, Gel)*

One batch of the bulk finished product, 4%, 4% (alternative) and 8% TBS-1A, is manufactured for the proposed clinical trial. The batch formula for is presented in Table 2.1.P.3.2-1.

Table 2.1.P.3.2-1: Batch Formula for 4% and 8% TBS-1A at the 1,500 g Batch Size and 4% TBS-1A (alternative) at the 1,000 g Batch Size

| | Quantity per Batch (g) | | |
|---|---|---|---|
| Components | 4% TBS-1A | 4% TBS-1A (alternative) | 8% TBS-1A |
| Testosterone | 60.0 | 40.0 | 120.0 |
| Castor oil | 757.5 | 655.0 | 697.5 |
| Diethyl isosorbide | 375.0 | 150,0 | 375.0 |
| Diethylene glycol monoethyl ether | 150.0 | 50.0 | 150.0 |
| Colloidal silicon dioxide | 75.0 | 50.0 | 75.0 |

(continued)

| | Quantity per Batch (g) | | |
|---|---|---|---|
| Components | 4% TBS-1A | 4% TBS-1A (alternative) | 8% TBS-1A |
| Povidone | 45.0 | 30.0 | 45.0 |
| Copovidone | 30.0 | 20.0 | 30.0 |
| Hydroxypropyl cellulose | 7.5 | 5.0 | 7.5 |
| Total | 1,500 g | 1,000 g | 1,500 g |

Description of Manufacturing Process and Process Controls (TBS-1A, Gel)

The clinical trial material is manufactured according to the following process as shown in Figs. 20A and 20B.

*Description of Manufacturing Process*

*Mixing of the Ingredients - Bulk Gel*

The Pre-Mix Stage 1 is prepared by mixing dimethyl isosorbide and diethylene glycol ethyl ether with a propeller mixer.

Pre-Mix Stage II is prepared by adding the povidone and copovidone to Pre-Mix I until fully dissolved. The product temperature is maintined below 50°C for the entire mixing process.

Pre-Mix Stage III is prepared by slowly adding hydroxypropyl cellulose to a cooled (30 - 35°C) Pre-Mix II. Mix until the solution is completely clear and maintain temperature between 40 - 50°C.

Once Pre-Mix Stage III is clear, adjust the settings on the propeller mixer and add the testosterone micronized powder. Mix until all the testosterone is dissolved and maintain the temperature at 40 - 50°C. This is identified as the Active Mixture.

Add the castor oil into a suitable size stainless steel vessel and heat to 40-50°C. Place the propeller mixer into the castor oil and slowly add the Active Mixture. Mix until a clear solution is formed. Cool the Active Mixture to 40°C and slowly add colloidal silicon dioxide. Mix until completely dissolved and the solution is free of entrapped air and cool the mixture to 30°C. The Bulk Gel is then discharged into stainless steel drums and the bulk gel sample is taken for analytical analysis.

*Filling and Packaging - Clinical Supplies*

After release of the Bulk Gel by the control laboratory, the filling and packaging process is carried out by filling a pre-determined volume into the syringe followed by the application of the syringe cap. Two syringes are packaged into a foil pouch.

The syringes are filled using a pipette with the gel taken from a sterile holding tank. The tip of the pipette is discarded after the syringe is filled and the syringe cap is applied. Each syringe is individually labelled.

Following the application of the label, two syringes are packaged in a pre-formed foil pouch and the pouch is sealed. Each pouch is labelled.

*CONTROL OF EXCIPIENTS (TBS-1A, GEL)*

All excipients in the TBS-1A gel are compendial excipients with the exception of dimethyl isosorbide, manufactured by Croda USA. All compendial excipients are tested according to the corresponding Ph. Eur./USP monograph.

Table 2.1.P.4.1-1: Exipients and Specifications

| Excipient | Specification |
|---|---|
| Testosterone | USP |
| Castor oil | Ph. Eur./USP |
| Dimethyl isosorbide | Tested to in house specifications |
| Diethyleneglycol monoethyl ether | Ph. Eur. |

(continued)

| Excipient | Specification |
|---|---|
| Colloidal silicon dioxide | NF |
| Povidone | Ph. Eur./USP |
| Copovidone | Ph. Eur./USP |
| Hydroxypropyl cellulose | Ph. Eur./USP |

Dimethyl isosorbide

Dimethyl isosorbide is common in other pharmaceuticals. Trimel BioPharma conducts the following release test on dimethyl isosorbide per the manufacture's, Croda USA, analytical methods. Data is compared to manufacturer's Certificate of Analysis.

Table 2.1.P.4.1-2: Specifications for Dimethyl Isosorbide

| Test Parameter | Method | Specification |
|---|---|---|
| Appearance | Visual | Clear liquid |
| Colour | APHA | NMT 60 |
| Refractive Index | USP<834>/EP 2.2.24 | Between 1.460 and 1.470 at 25° |
| pH | USP | Between 5.0 and 7.5 |
| Peroxide Value | USP<401>/EP 2.5.5 | NMT 5.0 |
| Water | USP<921> Method I/EP 2.5.12 | NMT 0.2% |
| Residue on Ingition/ Sulphated Ash | USP<281>/EP 2.4.14 | NMT 0.1% |
| Total Aldehydes | FU450900 | NMT 20 ppm |
| Residual Sodium | LU151600 | NMT 5 ppm |
| Residual Potassium | LU151600 | NMT 5 ppm |
| Heavy Metals | USP<231> Method I | NMT 10 ppm |
| Assay (Purity) | LU243000 | 98.0 - 100.0% |
| Formaldehyde | LU700220 | NMT 10 ppm |

None of the excipients in TBS-1 A gel is of human or animal origin.

*Batch Analysis (TBS-1, Gel)*

One batch of 4% TBS-1A (Batch No. IMP 11001), 4% TBS-1A (alternative) (Batch No. IMP 11002) and 8% TBS-1A (Batch No. IMP 11003) have been manufactured. A description of the TBS-1A batches are presented in Table 2.1.P.5.4-1.

Table 2.1.P.5.4-1: Description of TBS-1 A Batches

| Formulation | 4%TBS-1A | 4% TBS-1A (alternative) | 8% TBS-1 A |
|---|---|---|---|
| Batch no. | IMP 11001 | IMP 11002 | IMP 11003 |
| Batch size | 1,500 g | 1,000 g | 1,500 g |
| Date of manufacture | March 2011 | March 2011 | March 2011 |
| Equipment | Laboratory scale | Laboratory scale | Laboratory scale |
| Filling quantity per syringe | 148 µl | 148 µl | 148 µl |

Release data on the bulk gel is presented in Table 2.1.P.5.4-2.

Table 2.1.P.5.4-2: Batch Analysis - Batches 11001, 11002, 11003

| Test Parameter | Acceptance Criteria | 11001 | 11002 | 11003 |
|---|---|---|---|---|
| Appearance | Clear, slightly yellowish gel | Complies | Complies | Complies |
| Identification A | Retention time and spectrum corresponds to standard | Complies | Complies | Complies |
| Identification B | UV spectrum matches reference spectrum | Complies | Complies | Complies |
| Assay | 95.0 - 105.0% | 99.0% | 98.3% | 99.8% |
| Related Compounds | TBS-1 RC4 ≤ 0.2%<br>TBS-1 RC5 ≤ 0.5%<br>Each individual unknown impurity ≤ 0,2%<br>Total impurities ≤ 1.0% | BRT<br>0.24%<br>BRT<br>0.2 | BRT<br>0.24%<br>BRT<br>0.2 | BRT<br>0.24%<br>BRT<br>0.2 |
| Viscosity | Report result | 1100 | 2260 | 1540 |
| BRT - below reporting threshold 0.1 % | | | | |

*Stability (TBS-1A, Gel)*

The applicant commits to perform a 6 month stability study on the bulk TBS-1A at real time and accelerated conditions. The bulk gel will be stored at Trimel BioPharma in glass bottles. The stability study protocol is presented in Table 2.1.P.8.1-1 and the test parameters and the acceptance criteria for the bulk stability program are presented in Table 2.1.P.8.1-2.

Table 2.1.P.8.1-1: Stability Study Protocol for 4% and 8% TBS-1A Bulk

| Storage Condition | Storage Time Interval (Months) | | | | | | |
|---|---|---|---|---|---|---|---|
| | 0 | 1 | 2 | 3 | 6 | 9 | 12 |
| Initial | W | | | | | | |
| 25°C/60%RH | | x | x | x | x | X | x |
| 40°C/75%RH | | x | x | x | x | | |
| W = tests are performed or initial release data may be used, & identity<br>X = Appearance, viscosity, related compounds, assay and microbial limits | | | | | | | |

Table 2.1.P.8.1-2: Stability Study Test Parameters of TBS-1A Bulk Gel and Corresponding Acceptance Criteria

| Test Parameter | Method/Reference | Acceptance Criteria |
|---|---|---|
| Appearance | Visually | Slightly yellowish gel |
| Identification A | STM.TBS1.001 | Retention time and spectrum corresponds to standard |
| Identification B | STM.TBS1.001 | UV spectrum matches reference spectrum |
| Assay | STM.TBS1.001 | 95.0-105,0% |
| Related Compounds | STM.TBS 1.002 | TBS-1 RC4 ≤ 0.2%<br>TBS-1 RC5 ≤ 0.5%<br>Each individual unknown impurity ≤ 0.2%<br>Total impurities ≤ 1.0% |

(continued)

| Test Parameter | Method/Reference | Acceptance Criteria |
|---|---|---|
| Viscosity | Rotational viscosimeter Ph. Eur. 2.2.10/ USP<911> | Report results |

*Stability Data [TBS-1A, Gel]*

The applicant commits to provide stability data as it becomes available.

*NON-CLINICAL PHARMACOLOGY, PHARMACOKINETICS AND TOXICOLOGY*

Pilot scale product of high dose testosterone intranasal gel (TBS-1), batches 100304 and EI 014, are used in the toxicology studies per Table 2.2.1-1.

Table 2.2.1-1: Summary of Test Materials used in Toxicity Studies

| Type of Study | Species (strain) Number of Animals | Dose (mg/kg) [duration] | Lot Number |
|---|---|---|---|
| HET-CAM test | Hen eggs; 4 eggs/group | 0.3 ml of TBS-1 or 0.3 ml of Mygliol* [30 sec] | 100304 |
| Single Dose Local Tolerance | Rat (Sprague Dawley); 3 males | 0.1 ml of TBS-1 (right nostril) or 0.1 ml of Mygliol* (left nostril) [single dose] | 100304 |
| | Rabbit (New Zealand White); 3 males | 0.1 ml of TBS-1 (right nostril) or 0.1 ml of Mygliol* (left nostril) [single dose] | 100304 |
| Repeat Dose Local Tolerance | Rat (Sprague Dawley); 3 males | 0.1 ml of TBS-1 (right nostril) or 0.1 ml of Mygliol* (left nostril) [14 days/daily] | 100304 |
| | Rabbit (New Zealand White); 3 males | 0.1 ml of TBS-1 (right nostril) or 0.1 ml of Mygliol* (left nostril) [14 days/daily] | 100304 |
| Repeat Dose Toxicity Study | Rabbit (New Zealand White) 5 males/group; 5 groups | 0.093 mg/kg<br>0.280 mg/kg<br>0.933 mg/kg<br>Placebo gel<br>Control<br>[90 days, twice daily] | EI 014 |

The impurities androstenedione, epitestosterone and Δ-6-testosterone in the finished product are analysed by HPLC, as well as the unknown impurities. Impurity Δ4-Androstenediol (Androst-4-ene-3β,17β-diol, Ph. Eur. Impurity D) is determined by GC/MS. Table 2.2.1-2 presents the impurities found in the test material used in the toxicology studies. The impurity profile was not determined in Batch 100304.

Table 2.2.1-2: Impurity Profile of Batch EI 014

| Impurities | EI 014 |
|---|---|
| Androstenedione ≤ 0.1 | < 0.05 |
| Epitestosterone ≤ 0.5 | 0.208 |
| Δ-6-testosterone ≤ 0.2 | < 0.05 |
| Δ-4-androstenediol ≤ 0.2 | < 0.05 |
| Single impurities ≤ 0.1 | <0.05 |
| Total impurities ≤ 1.0 | < 0.6 |

*Integrated Assessment of the Data Package*

For this section reference is made to the Investigator's Brochure, Version 5, August 2010.

*List of Studies Conducted and References*

The following non-clinical studies were performed by the sponsor. Details hereto and to studies published by other parties are provided in the Investigator's Brochure, Version 5, August 2010.

| Study or Report Number | Authors | Title of Report |
|---|---|---|
| 22712040417 | Confarma S.A.R.L. | Three-month toxicity study in male rabbits |
| 208040401 | Confarma S.A.R.L. | Local tolerance after a single-dose administration in rats and rabbits |
| 208040402 | Confarma S.A.R.L. | Local tolerance after a repeated-dose administration in rats and rabbits |
| 208040403 | Confarma S.A.R.L. | HET-CAM test |

Per a literature review of testosterone, numerous pharmacology, pharmacokinetics and toxicology studies have been performed on testosterone and summarized in the Investigator's Brochure, Version 1, August 2010.

*GLP Statement and Bioanalytical Methods*

All toxicology studies performed at Conforma are conducted in accordance to good laboratory practice. GLP statements) can be found in the Appendix. Bioanalytical methods to quantify testosterone, DHT and estradiol were validated.

*References*

Study of HET-CAM of "Nasobol" (study report 208040403), 2004
Local tolerance, single application of "Nasobol" (study report 208040401) plus histopathology report (analysis number 208040401), 2004
Local tolerance, multiple application of "Nasobol" during 2 weeks (study report 208040402) plus histopathology report (analysis number 208040402), 2004
Repeat-dose toxicity study of "Nasobol" (study number 227120417), 2005

*Clinical Pharmacokinetics*

*Open Label, Randomized, Balanced, Three Treatments, Parallel Design, Pharmacokinetic Study of Intranasal TBS-1 Administration to Hypogonadal Men Study (Phase II, Protocol ID number: TBS-1-2010-01)*

Study TBS-1-2010-01 examined the efficacy and tolerability of 4.0% and 4.5% TBS-1 in hypogonadal men. In this study, higher concentrations of TBS-1 in reduced volumes for equivalent doses to those studied in Nasobol-01-2009 are evaluated. The highest b.i.d. dose is similar to the highest dose in the Nasobol-01-2009 study, 27.0 mg and 28.0 mg respectively, but in a smaller volume. In addition, this study evaluated t.i.d. dosing as described below.

The doses and dosing regimens in study TBS-1-2010-01 are described below:

- Treatment A: 10.0 mg TBS-1 (4.0%) t.i.d. at 2100,0700 and 1300 hours; total daily dose 30.0 mg
- Treatment B: 13.5 mg TBS-1 (4.5%) b.i.d. at 2100 and 0700 hours; total daily dose 27.0 mg
- Treatment C: 11.25 mg TBS-1 (4.5%) t.i.d. at 2100, 0700 and 1300 hours; total daily dose 33.75 mg

The mean serum testosterone pharmacokinetic profile results are summarized in Table 2.3.1.1-1.

Table 2.3.1.1-1: Treatment Groups and Mean Serum Testosterone PK Parameters

| Treatment | Daily Dose, mg/Day | N | Mean AUC 0-$\tau$ h*ng/dL | $C_{avg}$ ng/dL | $C_{max}$ ng/dL | $C_{min}$ ng/dL | $C_{avg}$ Below RR | $C_{avg}$ Within RR | $C_{avg}$ Above RR |
|---|---|---|---|---|---|---|---|---|---|
| A | 30 | 8 | 9920 ± 3300 | 413 ± 138 | 830 ± 188 | 239 ± 78 | 1 (12.5%) | 7 (87.5%) | 0 (0%) |
| B | 27 | 7 | 10058 ± 3493 | 419 ± 146 | 1050 ± 463 | 228 + 97 | 0 (0%) | 7 (100%) | 0(0%) |
| c | 33.75 | 7 | 9505 ± 2650 | 396 ± 110 | 883 ± 346 | 222 ± 57 | 1(14.3%) | 6(85.7%) | 0(0%) |

The results from all the treatment groups in study TBS-1-2010-01 met the criteria for efficacy global average total T concentration ($C_{avg}$) in the normal range, a 24 hour $C_{avg}$ value ≥ 300ng/dL and ≤ 1050 ng/dL.

*Efficacy and tolerability of Nasobol, an intranasal testosterone product for testosterone replacement therapy in hypogonadal men (Phase II, Protocol ID number: Nasobol-01-2009)*

Study Nasobol-01-2009 examines the efficacy and tolerability of TBS-1 (3.2%), in hypogonadal men. Efficacy is determined by the testosterone pharmacokinetic profile. It is a 4-period cross over design in which all subjects receive each of the following doses of TBS-1 and an active control for 7 days:

- 8.0 mg TBS-1 (3.2%) b.i.d. at 0700 and 2100 hours; total daily dose 16.0 mg
- 11.0 mg TBS-1 (3.2%) b.i.d. at 0700 and 2100 hours; total daily dose 22.0 mg
- 14.0 mg TBS-1 (3.2%) b.i.d. at 0700 and 2100 hours; total daily dose 28.0 mg
- 5.0 mg Androderm® Patch at 2100 hours

In order to achieve the three different strengths of TBS-1, 3.2% TBS-1 gel is filled as 123.9 mg per nostril for the 8mg dose, 170.1 mg per nostril for the 11mg dose and 217 mg per nostril for the 14 mg dose. In this study, 52% of the subjects recieving 14.0 mg TBS-1 b.i.d. acheive a $C_{avg}$ testosterone serum value within the reference range. The $C_{avg}$ values following administration of 11.0 mg b.i.d. and 8.0 mg b.i.d. are within the reference range in 36.5% and 49.1% of the subjects respectively. The 14.0 mg and 11.0 mg doses successfully meet the testosterone global $C_{avg}$ > 300 ng/dL.

*Multiple-doses, one period, three arms, parallel-group, open, randomised dose finding study conducted for TBS-1 gel for nasal application administered to hypogonadal men for 14 consecutive days (Phase II, protocol ID number: TST-DF-02-MAT105)*

The pharmacokinetic profile of testosterone (and DHT) is determined following intranasal administration of TBS-1 in three different dose schedules and to find out the optimum schedule for initial treatment. The study is designed as an open-label, 3-arms parallel group, multiple-dose pharmacokinetic study on 21 adult hypogonadal men.

The patients are treated according to the following dosing scheme:

| | |
|---|---|
| Schedule A: | 7.6 mg testosterone b.i.d. (8:00 h, 14:00 h) |
| Schedule B: | 7.6 mg testosterone b.i.d. (8:00 h, 20:00 h) |
| Schedule C: | 7.6 mg testosterone t.i.d. (8:00 h, 14:00 h, 20:00 h) |

The trough concentration rose rapidly during the first two days from the initial low (almost castrate) testosterone levels to reach a new steady-state between 200 and 400 ng/dl. The mean of average steady-state concentrations remain within the physiological range in all 3 treatment groups, but only in Group C (t.i.d.) the 95% CI was also entirely within the physiological range. In all 3 groups, $C_{max}$ of individual patients (only one patient in each group) sometimes slightly exceeded the upper limit of the normal range, but this was short-lasting.

These results indicate that a b.i.d. regimen and an increased testosterone dose per administration are preferred to maintain the serum testosterone concentration over the full 24 h-period above the lower limit of the physiological range.

*2.3.1.4 24-h pharmacokinetics of testosterone after nasal administration of single doses of 7.6 mg, 15.2 mg and 22.8 mg of testosterone in hypogonadal men (Phase II, protocol ID number: TST-PKP-01-MAT/04)*

The pharmacokinetic profile of testosterone (and DHT) was determined following intranasal administered of TBS-1 in 8 hypogonadal men. Each subject received TBS-1 at three different doses: 7,6, 15.2 and 22.8 mg of testosterone with a 7 day washout period in-between doses. The highest dose is investigated for safety reasons to determine whether supraphysiological concentrations of testosterone would be reached following this dose.

Testosterone is well absorbed after intranasal administration of different doses of TBS-1, The maximum serum concentration is reached approximately 1 to 2 hours after administration (which is significantly shorter than those periods known from transdermal administration, i.e. gel and patches) indicating a rapid absorption from the nasal cavity. Testosterone is cleared from serum with a half-life of approximately 10 hours. The concentration of DHT remains low over the observation period and the half-life ranged from 20-23 hours. Fig. 21 presents the mean concentration-time curves of testosterone and DHT after administration of the three different doses of TBS-1.

*Human Exposure*

Testosterone is indicated as a hormone replacement therapy for the treatment of hypogonadism in men. The currently available options for administration of testosterone are oral, buccal, injectable, implantable and transdermal. According to the HMA Homepage and different authority databases, the following testosterone-containing medicinal products arc systemic therapy are currently approved in the EU:

- Testopatch® (approved in 9 EU countries via MRP), Andropatch® (approved in the UK, 2002)
- Transdermal gels, e.g. Androgel®, Testim® , Testogel® (approved in 15 EU countries via MRP)
- Injectables, e.g. Nebido® (approved in 17 EU countries via MRP), Reandron® (approved in 8 EU countries via MRP with different product names).
- Capsules, buccal tablets e.g. Andriol® (approved in DE 1979) Striant® (approved in approx 17 EU countries via MRP).

To date, over 100 men have been exposed TBS-1. No Serious Adverse Events are reported. None of the subjects are discontinued from the TBS-1 investigational medicinal product because of an AE. The reported adverse events are classified as mild or moderate in severity. Adverse events from each of the trials are summarized below.

*Study title: Open Label, Randomized, Balanced, Three Treatments, Parallel Design, Pharmacokinetic Study of Intranasal TBS-1 Administration to Hypogonadal Men Study (Phase II, Protocol ID number: TBS-1-2010-01)*

Twenty-two (22) hypogonadal men were exposed to TBS-1. All three dose levels are well tolerated by subjects. There are no deaths in the study and none of the subjects experienced any SAEs. Eight (8) adverse events are encountered in the present study. Two adverse events are classified as possibly related and six (6) as not related to the study drug. All events are of mild to moderate severity. None of the subjects are discontinued from the treatment because of an AE. The pharmacokinetic profile of DHT and Estradiol shows appropriate increases following TBS-1 administrations. The increases in serum DHT and Estradiol all remain well within the reference ranges for serum DHT and Estradiol respectively, and returned to basal levels after discontinuation of treatment. Physical and nasal examination, vital signs and clinical laboratory evaluation results do not reveal any additional clinically significant findings related to study treatment.

*Study title: Efficacy and tolerability of Nasobol, an intranasal testosterone product for testosterone replacement therapy in hypogonadal men (Phase II, protocol ID number: Nasobol-01-2009)*

In this study (Nasobol-01-2009), fifty seven (57) hypogonadal men are exposed to testosterone intranasal gel. There are no deaths in the study, no serious AEs or discontinuations due to AEs in this study. The majority of reported AEs are mild in intensity. Most AEs are considered unrelated to study drug. A total of 56 AEs are reported; 46 are considered mild, 22 of which are related to the study drugs. Ten (10) AEs are considered moderate, only 2 of which are related to study treatments.

*Study title: Multiple-doses, one period, three arms, parallel-group, open, randomised dose finding study conducted for TBS-1gel for nasal application administered to hypogonadal men for 14 consecutive days (Phase II, protocol ID number: TST-DF-02-MAT/05)*

Twenty-one (21) hypogonadal men are exposed to TBS-1. There are no deaths in the study and none of the

subjects experienced any SAEs. Thirty-six (36) adverse events are encountered in the present study. All adverse events are classified as unlikely or not related to the study drug and were of mild to moderate severity. None of the subjects are discontinued from the treatment because of an AE. The pharmacokinetic profile of DHT show that the average steady-state concentration of DHT do not exceed the upper limit of the physiological range (85 ng/dl), indicating no safety concern due to increases in DHT levels.

*Study title: 24-h pharmacokinetics of testosterone after nasal administration of single doses of 7.6 mg, 15.2 mg and 22.8 mg of testosterone in hypogonadal men (Phase II, protocol ID number: TST-PKP-01-MAT/04)*

Eight (8) hypogondal men are exposed to single doses of TBS-1. There are no deaths in the study and none of the subjects experience any SAEs. Two adverse events in 1 patient (fever and nausea), not related to TBS-1 occurred (patient is excluded from the study before first administration). None of the AEs are considered study drug-related.

**Example 8**

**Determination Of In Vitro Release Rate Of Testosterone From Testosterone Gel (0.15%, 0.6%, 4.0%, 4.5% W/W) Using Modified Franz Cell With Uplc Quantitative Method 1.0 PURPOSE**

This analytical method will be used for determination of *in vitro* release rate of Testosterone from Testosterone Gel (0.15%, 0.6%, 4.0%, 4.5% w/w) as well as for comparison between products using release rate. Rate comparison study may be performed by following the procedure from Appendix I.

**2.0 SAFETY PRECAUTIONS**

Related MSDS should be read. Proper personal protection should be worn and adequate ventilation should be maintained when handling the materials. Dispose all used materials as per relevant laboratory procedures.

**3.0 MATERIALS AND EQUIPMENT**

**3.1 Equipment**

FDC-6 Transdermal Diffusion Cell Drive Console, Logan Instruments Corp.

**UPLC System with TUV or PDA detector and data acquisition system**

UPLC Column, Acquity BEH C18, 1.7 μm, 50 mm x 2.1 mm column with *VanGuard* Pre-*Column* BEH C18, 1.7 μm, 5 mm x 2.1 mm
Thermostat, VTC 200, FDC Heater
FDC-6 Magnetic Stirrer
Analytical balance, capable of accurately weighing 0.1 mg
Filter, 0.45 μm, 90 mm Nylon Membrane Filter
Durapore HVLP, 0.45 μm pore size, 25mm in diameter, HVLP02500, Millipore
Class A volumetric glassware

Micropipette

Other standard laboratory miscellaneous glassware and equipments

**3.2 Materials**

Testosterone reference standard, USP
Ethanol Anhydrous, HPLC grade or equivalent
Acetonitrile (ACN)
Purified water

## 4.0 PROCEDURAL HIGHLIGHTS

### 4.1 Franz Diffusion Cell System

Thickness of the Ring: 3.2mm
Orifice Diameter of the Teflon Ring: 15mm
Orifice Diameter of the Franz Cell: 15mm
Surface Area: 1.7671 cm2

Diffusion Medium: Ethanol: Water = 50 : 50 (refer to Section 4.4 for the preparation)

Temperature (°C): 37°C±0.5°C
Stirring speed: 600 rpm
Pre-Soaking of Membrane: ≥30min
Medium volume: 12mL
Aliquot Volume: 0.5mL, with medium replacement
Number of Aliquots Withdrawn: 6
Sampling Time (minutes): 60, 120, 180, 240, 300 and 360

### 4.2 Chromatographic Conditions

Instrument: ACQUITY UPLC system with TUV or PDA detector
UPLC Column: Acquity UPLC BEH C18, 1.7 μm, 50 mm x 2.1 mm column with *VanGuard* Pre-*Column* BEH C18,1.7 μm, 5 mm x 2.1 mm

Column Temperature: 30°C±5°C
Mobile phase A: 50%
Mobile phase B: 50%
Detector Wavelength: UV at 254 nm
Injection volume: 2 μL
Flow rate: 0.3 mL/min
Run time: 3 minutes
Strong Wash: 80% ACN
Weak Wash: 50% ACN
Seal Wash: 50% ACN

### 4.3 Preparation of Solution

#### 4.3.1 Mobile Phases:

Mobile phase A: 100% ACN
Mobile phase B: Water

#### 4.3.2 Strong Wash Solution (80% ACN)

To prepare 1000mL of strong wash solution, mix 800mL ACN and 200mL H2O in an appropriate container. Adjust preparation volume proportionately as necessary.

#### 4.3.3 Weak Wash Solution (50% ACN)

To prepare 1000mL of weak wash solution, mix 500mL ACN and 500mL H2O in an appropriate container. Adjust preparation volume proportionately as necessary

#### 4.3.4 Seal Wash Solution (50% ACN)

To prepare 1000mL of seal wash solution, mix 500mL ACN and 500mL H2O in an appropriate container. Adjust preparation volume proportionately as necessary.

#### 4.4 Preparation of Diffusion Medium

**(Ethanol: Water = 50 : 50)**

To prepare 1000 mL of diffusion medium, transfer 500 mL of ethanol anhydrous into a suitable container add 500 mL of purified water and mix well. Filter through 0.45 μm nylon membrane filter and sonicate. This preparation can be scaled as needed.

#### 4.5 Preparation of Diluent

Use diffusion medium as diluent.

#### 4.6 Preparation of Standard Solutions

#### 4.6.1 Preparation of Testosterone Standard Stock Solution 1

Accurately weigh approximately 20mg of Testosterone reference standard into a 100mL volumetric flask. Add approximately 70mL of diluent. Sonicate 5 min to dissolve. Dilute to volume with diluent, and mix well. This Stock 1 solution has a concentration of approximately 200μg/mL Testosterone.

#### 4.6.2 Preparation of Testosterone Standard Stock Solution 2

Refer to section 4.6.1

#### 4.6.3 Preparation of Testosterone Working Standard Solutions

Testosterone sample will be quantified against a multiple point linearity standards. Six calibration standards will be prepared from Testosterone Standard Stock Solution 1 in concentrations outlined in Table 1.

Transfer specified volume of Testosterone standard solutions into each specified volumetric flask and make to the volume with diluent. Mix well.

***Note:*** **Prepare proportionately larger volumes as necessary. The working standard solution is stable for 10 days at ambient temperature and refrigeration (~4 C).**

| Table 1. Preparation of Working Standard Solutions STD ID | Volume of Standard Stock Solution 1 (200.0μg/mL) Stan | Final Volume (mL) | Approximate Concentration (μg/mL) | Percentage of Max. Testing Concentration* (652.3 μg/mL) (%) |
|---|---|---|---|---|
| STD-1 | 4 mL of STD-2 | 25 | 1.0 | 0.15 |
| STD-2 | 5 mL of STD-3 | 20 | 6.25 | 0.96 |
| STD-3 | 10 mL of STD-4 | 20 | 25 | 3.83 |
| STD-4 | 10 mL of STD-5 | 20 | 50 | 7.67 |
| STD-5 | 10 mL of Std. Stock 1 | 20 | 100 | 15.3 |
| STD-6 | Std. Stock 1 | N/A | 200 | 30.6 |
| *Maximum testing concentration is 652.3 μg/mL (9.0% gel formulation), as obtained in the method development study (Reference LNB-10-056 page 68). | | | | |

#### 4.6.4 Preparation of Testosterone Check Standard Solutions

Three levels of check standard solutions will be used to monitor the accuracy and precision within the run. The Check Standard Solutions will be prepared from Standard Stock Solution 2 in concentrations outlined in Table 2.

Transfer specified volume of Testosterone standard solution into each specified volumetric flask and make to the volume with diluent. Mix well.

| Table 2. Preparation of Check Standard Solutions | | | | |
|---|---|---|---|---|
| CSTD-1 | 3mL of CSTD-2 | 50 | 3.0 | N/A |
| CSTD-2 | 5 mL of Std. Stock 2 | 20 | 50.0 | Conc. as STD 4 |
| CSTD-3 | Std. Stock 2 | N/A | 200.0 | Conc. as STD 6 |

### 4.7 Preparation of Sample Solution

#### *4.7.1 Membrane Preparation*

Soak the selected membrane in diffusion Medium for at least 30 minutes, check for defects and discard any membrane with defects.

#### *4.7.2 In-vitro* Release Franz Cell Method

Transfer diffusion medium into the Franz cell, carefully remove any bubbles, put previously soaked membrane on the top of the Franz cell, use Kim wipes to remove excess medium on the membrane, put the ring on top of the membrane. Add gel sample to the middle of the ring, using a spatula to smooth the sample surface until it is same height as the ring, Put on glass and ring cover, and then clamp it.

Check the medium level and top it to the sampling port mark position if necessary. Withdraw 0.5 mL of sample at 60, 120, 180, 240,300, and 360 minutes time point, and replace medium to the mark position.

Sample Dilution

For the 0.15% and 0.6% w/w gel formulations inject sample solutions from Franz cell with no dilution. For the 4.0 % and 4.5% w/w gel formulations dilute samples prior to injections. Transfer 0.2 ml of the sample solution into the UPLC vial using a micropipette; add 1 ml of diluent and mix.

***Note:* The sample solution is stable for 2 days at ambient temperature and refrigeration (~4C).**

### 4.8 Injection Procedure

Set up injection sequence as follows:

Make at least one injection of Diluent (Diffusion Medium)
Make six consecutive injections of working standard STD-4.
Make one injection of each Testosterone working standard solution from STD-1 to STD-6.
Make injections of a set of check standards (CSTD-1 to CSTD-3) at the beginning, middle and end of the sample sequence (3 sets for each run, total 9 injections). Evenly spread the testing samples between the check standards.

### 4.9 System Suitability

#### 4.9.1 Diluent (Diffusion Medium)

Diluent is to be injected at least once at the beginning of the sequence to ensure the system is clean and stable. There should be no significant interference peak at the retention time of Testosterone from the diluent.

#### 4.9.2 Injection Reproducibility

The Testosterone working standard solution STD-4 is to be consecutively injected six times. Calculate % RSD of the peak area of Testosterone from the six replicate injections from:

$$\%RSD = \frac{100}{\overline{Astd}} \times \sqrt{\frac{\sum_{i=1}^{n}(Ai - \overline{Astd})^2}{n-1}}$$

Where A*i* is the individual values expressed as peak area, *stdA* is the average of individual peak area values and *n* is the number of injections which is 6. Calculate %RSD of the retention time of Testosterone peak from the six replicate injections of STD-4.

**4.9.3 Tailing Factor**

USP tailing factor for Testosterone will be calculated and will be included in the reported. Calculate the USP Tailing Factor (*T*) from,

$$T = \frac{W_{0.05}}{2f}$$

Where *W0.05* is the peak width at 5 % of the peak height from the baseline and *f* is the distance from the peak maximum to the leading edge of the peak measured at a point 5 % of the peak height from the baseline.

**4.9.5 Retention Time (RT)**

Average retention time (RT) of Testosterone from the six consecutive injections of STD-4 will be included in the report.

**4.9.6 Theoretical Plate Number (N)**

Theoretical plate number per column for ABC will be calculated as per USP and EP and will be included in the report.

Calculate the USP/EP theoretical plate number per column (*N*) from,

$$N = 5.54 \times \left(\frac{t}{W_{h/2}}\right)^2$$

Where *t* is the retention time of the peak and *Wh/2* is the peak width at half peak height.

**Acceptance Criteria**

1. The %RSD of peak area and retention time for 6 replicate injections of the working standard solution STD-4 should be ≤2.0%.
2. Tailing factor for Testosterone peak in the 6 replicate injections of the working standard solution STD-4 should be ≤2.
3. Theoretical plate number (*N*) of Testosterone peak in the 6 replicate injections of the working standard solution STD-4 should be NLT 1,000.

### 5.2 Calibration Curve and Acceptance Criteria

Plot a calibration curve with all STDs injected (total of 6 points). A weighted (1/x) function for linear regression analyses should be applied to meet following requirements. Using linear regression, determine the line of the best fit, $y=mx+b$, where $x$ is the concentration and $y$ is the response. Calculate the correlation coefficient (r), slope and y-intercept.
Calculate the response factor (area to concentration ratio) of each standard injection and the RSD of the response factors. The overall RSD (n=6) of the response factors should be NMT 10.0%.
The correlation coefficient (r) of the curve should be NLT 0.98.
Compare the y-intercept to the peak area of the STD-1. The y-intercept should not be greater than 20% of the STD-1 peak response.

### 5.3 Check Standards and Acceptance Criteria

The accuracy of the analysis is demonstrated by the check standards recovery.
Calculate the check standards concentration by the calibration curve and compare the concentrations to the theoretical concentration.
The % recovery of CSTD-1 should be within 90.0% to 110.0%
The %recovery of CSTD-2 and CSTD-3 should be within 98.0% to 102.0%.
Two of the nine check standards failing to meet above criteria is acceptable providing the two failed check standards are not at the same concentration level.

### 6.0 CALCULATING AND REPORTING RESULTS

**6.1 Calculation of Testosterone Concentration at Each Time Point** Calculate the concentration of Testosterone in samples from diffusion steps.
A calibration curve as equation 1 is generated by Empower 2 software by plotting standard concentrations (in μg/mL) versus corresponding standard peak area,

$$Y = AX + B \text{ (Equation 1)}$$

This equation is to be used to calculate the Testosterone concentration in the samples at each time point, in μg/mL.
*Note: Use sample dilution factor 6 where it is applicable.*

***Calculation of Accumulated Testosterone Released***

Calculate the accumulated amount of Testosterone released from the Testosterone gel at each sample time point, in μg, using equation 2,

$$A_{\text{testosterone}} = Cn \times V_1 + \left(\sum_{i=1}^{n-1} Ci\right) \times V_2 \qquad \text{(Equation 2)}$$

Where,

*Cn* = Testosterone concentration in sample solution withdrawn at *n* time point, at which time point the Testosterone concentration is being calculated
C*i* = Testosterone concentration in sample solutions withdrawn from the first time point to the time point right before the time point *n*, at which time point the Accumulated %Released is being calculated
V1 = Volume of sample solution in the vessel, 12 mL
V2 = Volume of sample solution withdrawn at each time point, 0.5 mL

### 6.3 Calculation of Testosterone Released Per Unit Surface Area

Calculate the Release Per Unit Surface Area (Q), in μg/cm2, follow equation

$$Q = \text{Release/Area} = \frac{Atestosterone}{SA} \times 100 \qquad \text{(Equation 3)}$$

Where,

$A_{Testosterone}$ = Accumulated amount of Testosterone released from Testosterone gel at certain sample time point, in μg

$$\text{SA} = \text{Membrane surface area, } \pi \cdot r^2 = 3.14159 \times \left(\frac{1.5}{2}\right)^2 = 1.7671\,\text{cm}^2$$

**6.4 Calculation of Released Rate**

Drug diffusion from the vehicle obeys Fick's law. Equation 4 is a simplified solution of Fick's law of diffusion as derived by Higuchi:

$$Q = \sqrt{2D \cdot A \cdot C_{sm} \cdot t} = \sqrt{2D \cdot A \cdot C_{sm}} \times \sqrt{t} = Slope \times \sqrt{t} \qquad \text{(Equation 4)}$$

Where,

Q = amount of drug released per unit area (μg/cm2)
t = elapsed time in minutes

Thus, a plot of the amount released (Q) vs. $\sqrt{t}$ should be linear with a slope of $\sqrt{2D \cdot A \cdot C_{sm}}$. The slope of $\sqrt{2D \cdot A \cdot C_{sm}}$ is reported as Release Rate.

**7.0 TYPICAL CHROMATOGRAMS**

**See Figures 36-38.**

**7.3 A Typical Chromatogram of Testosterone Sample Solution**

Appendix to Example 8

In Vitro Release Rate Comparison Testing

The release rates of the reference formulation (pre changed lot) and the post change formulation to be compared should be determined on the same day, under the same conditions. The release rate comparison may be carried out as a two-stage test. To ensure unbiased comparison sensitivity, sample positions within the bank of Franz cells should be randomized or pre-assigned in a mixed arrangement to ensure unbiased comparison. An example of the cell assignment is T (test formulation) in cells A1, A3, A5, B2, B4, B6, and R (reference formulation,) in cells A2, A4, A6, B1, B3, B5.

First Stage

Release rates (slopes) from six cells of test formulation (T) and six cells of the reference formulation (R) are obtained. A 90% Confidence Interval (CI) for the ratio *(T/R)* of median release rates is calculated.

The comparison will be performed with following calculations:

Step 1: Generate a table with seven rows and seven columns. List the reference slopes (R) across the first row and test slopes down the first column of the table. Calculate the individual *T/R* ratios (36) between each test slope and each reference slope and enter the corresponding values in the table.
Step 2: Rank these 36 *T/R* ratios from lowest to highest.
Step 3: The eighth and twenty-ninth ordered ratios represents lower and upper limits of the 90% CI for the ratios of

median release rates.
*Note: If the data for a single cell is missing for one of the lots, there would be* 5 × *6=30 individual T/R ratios, and the limits of 90% CI would be sixth and twenty-fifth order individual T/R ratios. If the data is missing for more than one cell, the correct computation should be determined in consultation with the supervisor.*

Evaluation criteria:

Test and reference formulations are considered to be "same" if the 90% CI falls within the limits of 75% - 133.33%, If the test is not passed at the first stage, proceed with second stage.

Second Stage:

Two additional *in vitro* runs of twelve cells (six cells per formulation/lot) are to be performed, yielding 12 additional slopes for each lot, or 18 in all (including the first stage 6 results). A 90% Confidence Interval (CI) for the ratio *(T/R)* of median release rates is calculated using all 18 slopes. All 324 (18 × 18) individual ratios are obtained and are ranked from the lowest to the highest. The 110th and 215th ordered ratios represent lower and upper limits of the 90% CI for the ratios of median release rates.

Evaluation Criteria:

Test and reference formulations are considered to be "same" if the 90% CI falls within the limits of 75% - 133.33%.

**Example 9**

**TBS1A Report for 4% and 8% Bulk Gel**

***Objective:***

To follow up on IMP- Clinical batch manufacture. Main points concern process flow and bulk appearance on stability.

- Process flow improvement
- Viscosity of bulk Gel
- Stability (re-crystallization)
- Evaluation of alternate materials sources and grades
- In Vivo results, formulation changes to impact onset of release
- Testing of trials using Franz Cell, trial selection

List of Raw-materials identified for use in trials:

| Material name | Grade | Spec # | Source | Comments |
|---|---|---|---|---|
| | | | | |
| Castor Oil | (Crystal 0) | RM004A | Cas-Chem | |
| Castor Oil | (Virgin) | RM004B | - | |
| Labrafil | M1944CS | RM002A | Gattfosse | |
| DMI | ----------- | RM009A | Croda | |
| Transcutol P | ----------- | RM008A | Gattfosse | |
| Plasdone | K17 | RM011A | ISP | |
| Plasdone | S630 | RM013A | ISP | |
| Plasdone | K29-32 | Sample | ISP | |
| Plasdone | K90 | Sample | ISP | |
| HPC | Klucel HF | RM014A | Hercules | |

(continued)

| Material name | Grade | Spec # | Source | Comments |
|---|---|---|---|---|
| HPC | Nisso H | Sample | Nisso | |
| HPC | Nisso M | Sample | Nisso | |
| HPC | Nisso L | Sample | Nisso | |
| Cab-O-Sil | M-5P | RM003A | Cabot | |
| Aerosil | 200 | RM003B | Evonik | |
| Purified water | ----------- | ----------- | Trimel | |
| Testosterone | micronized | RM001A | Proquina | |
| Oleic Acid | Super-refined | sample | Croda | |
| Testosterone | Not micronized | RM | Proquina | |
| | | | | |

***Equipment used:***

In addition to the Silverson High Shear mixer, used only during the manufacture of the TBS1A IMP Clinical batches, included also a propeller type mixing unit for the trials on several pre-mix operations. The only application for the High shear action is for dispersion of the active in the Co-Solvents.

For more uniform mixing and control of temperature, recommend a jacketed container with wiping blades to remove material from inner bowl wall (especially critical for uniform bulk temperature during heating as well as cooling cycles.

***Background info on IMP bath manufacture***

Observation during the IMP Clinical batch manufacture included high viscosity during preparing the pre-mixture of the DMI/Transcutol co-solvent mix consisting of PVP K17/S640, Klucel HF and Testosterone micronized. Mixture resulting in a sticky mass when added to the Castor oil using the high shear mixer set up. With the same high shear mixer set up for the addition of the Cab-O-Sil (referenced in future to SiO2) could not obtain a vortex to incorporate the material and required additional manual mixing during addition stage, hence the recommendation for propeller type mixing unit). Even though the material was viscous during that addition stage, on further mixing the viscosity of the final Bulk Gel dropped to approximately 1,500 - 2,000 cps. Mixing time and speed had to be controlled not to overshoot targeted gel temperature (no cooling system).

***Outline of trials:***

The initial trials (Placebo) concentrated on changing the order of addition to identify impact on viscosity. Previous process included the addition of the SiO2 at the final stage (see comments above), changed to dispersion of the SiO2 into the Castor oil prior to addition of the alternate active mixture. The resulting viscosity of the Castor Oil/SiO2 mixture, used various percentages, increased with the addition of a small percentage of Arlasolve (DMI).

Next step was to duplicate these results using the active mixture (Co-solvents/PVP/HPC/active) and added that mixture to the premix of Castor oil and SiO2. This however resulted in a low viscosity solution, indicating an impact of the active mixture on formation of a viscous gel.

Since the co-solvent mix without additional materials resulted in an increase of viscosity, the quantities of solvent were split into 2 parts, adding part of the solvent mix only to the Oil mixture and remaining solvent mix used to disperse the PVP,HPC and active. The active mixture with the reduced co-solvent ended up more viscose, plus similar low viscosity when added to the castor Oil premix. Additional trials included the prep of active in only DMI (no PVP) and obtained good viscosity. HPC was prepared separately in the Transcutol P, creating problems of stringing when added to the mixture (similar to IMP observations). Addition of SiO2 at a level of 0.1 - 0.3% resolved the problem.

The above process to dissolve active in the Co-solvents is sufficient and doesn't require PVP to increase solubility for the 4% formulation, however not sufficient co-solvents in the formulation to achieve solubility for the 8% strength. Trials on the 8% included an alternate successful approach for preparing the active dispersion containing PVP by including SiO2 into that mixture. As demonstrated on evaluation trials evaluating impact of SiO2 added to the DMI

as well as Transcutol P, resulted in good viscosity forming with DMI, however not with Transcutol. Active dispersion therefore id prepared by dissolving the PVP in DMI only, followed by addition of the active at 55 C (50-60C) and portion of available SiO2.

Please note that this process was only developed during the trial work on the 8%, hence it can be scaled down to the 4% strength if PVP indicate additional functionality (Franz Cell test).

Comments related to addition of purified water (noted in Table xxx) indicate increase in viscosity with trials containing HPC, no viscosity increase in trials using only PVP. These trials were only included for information to study water uptake and impact on viscosity after application into the nasal cavity.

Critical step during HPC set up is to provide at least 24 hours of solvating to obtain a clear solution.

As outlined in the trial objectives, formulation ratios were implemented using also alternate grades and sources of materials and are identified in the formulation table.

To identify the impact of the process change (such as reaction of viscosity increase adding the co-solvents), performed trials to study impact if related to DMI or Transcutol P. Trials were initiated to disperse SiO2 (at the same ratio as used for Castor Oil mixture) in DMI only as well as in Transcutol P only. The Mixture with the DMI resulted in a viscous mixture while Transcutol P mixture was very fluid.

Similar trials were initiated to use the co-solvents individually to study solubility of the Polymers as well as active for potential reduction in Transcutol P. No noticeable difference in solubility using the mixture or individual solvents at the 4% strength. However, if PVP and HPC are prepared only in DMI, observed separation of the two materials when stored overnight (not apparent when mixed in the co-solvent mixture).

To eliminate the stickiness of the dispersion when adding the active/polymer mixture, removed the HPC from the formulation and using PVP only (individual grades K17-K29/32-K90, no mixtures). This resulted in various degrees of viscosity related to the grade used.

Material also included the use of Labrafil M 1944 CS and are outlined in batch description and selected for testing in Franz Cell.

***Comments:***

The various trials are outlined below for 4 % strength as well as 8%.

Trial lots of both strength have been selected for testing on the Franz Cell. Selected lots are identified.

All trials will be monitored for physical evidence of re-crystallization and change in appearance (separation), tested for change in viscosity. Viscosity values of the trials will be documented and updated

Pending Franz Cell result evaluation, optimization of formulation and process can be implemented. This is critical to identify since the trial outline did not include impact on viscosity related to all process parameters (need to include analytical testing and stability data).

Observations during viscosity test using the Brookfield Viscometer Model DV-II+, with Spindle #6, at 50 rpm for 30 seconds, did actually show an increase in viscosity values over the test time in samples prepared with higher viscosity grade HPC. This can be attributed to the stickiness of the Gel causing agglomeration to the spindle shaft and disk creating a drag (not a true viscosity value of the results reported). The bulk Gel of several trials is not thixotropic. Also tested on some trials viscosity at 37 C.

Tested several trials using the new Haupt method with spindle 4 at 6 rpm.

The various attached tables show the trial numbers for active Gels, pre-mixes and Placebos

***Discussion and Considerations for follow up trials with both strength***

Even though 'viscosity improvement' was not the primary target to initiate trials, it was certainly a designed effort to study the cause for low viscosity considering the high percentage of SiO2 present in the formulation. A cross check against SiO2 alternate source comparison did not indicate major differences, nor did various ratios of Co-Solvents, limited adjustment since a certain percentage required to dissolve the Testosterone. Changes in grades of PVP indicated impact on viscosity when used in the active dispersion, however not when added to the rest of the mixture. Changes in grades of HPC (used alternate source of fine material) showed impact on the final Gel, however the higher the Molecular weight of the HPC, impact of stickiness and stringing in the final Gel. Testing viscosity after several weeks did show a separation in the Gel of viscose settlement on the bottom of the container.

With indication of SiO2 retaining Testosterone, adding more to increase viscosity was not an option, aim was to reduce the % used. especially for the TBS1 A 4 % strength which indicated a much higher percentage of T retained compared to the 8% TBS1A. Target was to at least obtain the same ratio of SiO2 to T of the 8% strength for the 4 % strength (hence aimed for scale down to 3 %). With the trials completed and showing impact on viscosity related to process and formulation changes, a reduction in SiO2 for the definitely possible for the 4 % strength that would also include the use of PVP in the formulation by taking advantage of the process change on the 8% strength.

The above is only based on viscosity; however impact on the changes in formulation to slow down initial absorption rate in vivo can only be evaluated from the data obtained on the trials used for the analytical test using the Franz Cell. These results will be reviewed and evaluated with potential recommendations for further trials to either duplicate earlier trials or based on DOE.

The attached Tables for viscosity show the date of manufacture and latest test results (to help with trial selection on Franz Cell). In the Comment column original data will be reference or referenced in the Trial process description. Further alternate material source evaluation is recommended once a primary formulation and process for each strength has been established for direct comparison.

## Formulation/composition of TBS1A - 4%

### Table 1A (See the formulations in the Examples above and including Example 10)

| Trial number | Active % | Castor oil % | Labrafil % | PVP grade % | DMI % | TranscutolP % | HPC Nisso % | SiO2 % C=Cabosil A=Aerosil200 |
|---|---|---|---|---|---|---|---|---|
| RD11037 | 4 | 52 | 000000 | K17 = 3<br>S630 = 2 | 25 | 10 | 0000000 | C = 4 |
| RD11038 | 4 | 57 | 000000 | K17 = 3<br>S630 = 2 | 20 | 10 | 0000000 | C = 4 |
| RD11039 | 4 | 29 | 29 | K17 = 3<br>S630 = 2 | 20 | 10 | 0000000 | C = 3 |
| RD11040 | 4 | 57 | 0000000 | 0000000 | 25 | 10<br>6 + 4 | 00000000 | C = 4 |
| RD11041 | 4 | 53 | 0000000 | K17 = 3<br>S630 = 2 | 25 | 10<br>6 + 4 | 0000000 | C = 3 |
| RD11042 | 4 | 29 | 29 | 00000000 | 25 | 10<br>6 + 4 (split) | 000000 | C = 3 |
| RD11050 | 4 | 66.7 | 000000 | K17 = 3 | 24<br>20 + 4 | 0000000 | N-H = 0.3 | A = 2 |
| RD11050A | 4 | 66.7 | 000000 | K17= 3 | 24<br>20 + 4 | 0000000 | N-H = 0.3 | 1 % additional to final 11050 |
| RD11051 | 4 | 66.7 | 000000 | K30 = 3 | 24<br>20 + 4 | 0000000 | N-M = 0.3 | A = 2 |

| | | | | | | | | | |
|---|---|---|---|---|---|---|---|---|---|
| RD11051A | 4 | 66.7 | 000000 | K30 = 3 | 24<br>20 + 4 | 0000000 | N-M = 0.3 | 1 % additional to final 11051 |
| RD11053 | 4 | 61.7 | 000000 | K17 = 3 | 22<br>16 +6 | 6<br>4+2 | N-H = 0.3 | A = 3 |
| RD11054 | 4 | 61.4 | 000000 | K30 = 3 | 23<br>16 +7 | 5<br>4+1 | N-M = 0.6 | A = 3 |
| RD11055 | 4 | 62.0 | 000000 | K90 = 3 | 23<br>16 +7 | 5<br>4 + 1 | 0000000 | C = 3 |
| RD11056 | 4 | 62.0 | 000000 | K90 = 3 | 28<br>20 + 8 | 00000 | 0000000 | C = 3 |
| RD11059 | 4 | 75.0 | 000000 | K30 = 2.5 | 14<br>10 + 4 | 2 | 0000000 | C = 2.5 |
| RD11060 | 4 | 71.5 | 000000 | K30 = 2.0 | 18<br>9 + 9 | 1 | 00000000 | C= 3.5 |
| RD11061 | 4 | 71.0 | 2 | K17 = 2 | 16 | 2 | 0000000 | C=3 |
| RD11062 | 4 | 62.35 | 0000000 | K17=1.5<br>K30=1.0 | 22<br>6+16 | 6<br>2+4 | N-H = 0.15 | A=3 |
| RD11063 | 4 | 70.5 | 00000oo | K17=1.5<br>K30=1.5 | 18<br>6 + 12 | 00000000 | N-H = 0.2 | A=4 |
| RD11064 | Transfer from RD11062 | Add 0.3% H2O | Increase in viscosity | | | | Formula includes HPC | |
| RD11065 | Transfer from RD11063 | Add 0.3% H2O | Increase in viscosity | | | | Formula includes HPC | |
| RD11066 | Transfer from RD11041 | Add 0.3% H2O | No increase in viscosity | | | | N0 HPC | |
| RD11070 | Transfer from RD11037 | Add 0.3% H2O | No increase in viscosity | | | | N0 HPC | |
| RD11071 | Transfer from RD11042 | Add 0.3% H2O | No increase in viscosity | | | | N0 HPC | |
| RD11072 | Transfer from RD11040 | Add 0.3% H2O | No increase in viscosity | | | | N0 HPC | |
| RD11073 | 4 | 70.5 | 000000 | 0000000 | 16<br>10 +6 (3) | 6 (3) | N-M=0.5 (0.25) | A=3 |
| RD11074 | Transfer from RD11073 | Add 0.3% H2O | | | | | Transfer from RD11040 | Add 0.3% H2O |
| RD11075 (base) | 4 | 68.0 | 000000 | K30 =1.0 | 16<br>6 +10 | 0000000 | See HPC pre-mixes | A=3 |
| RD11076 | Base of RD11075 | --------- | --------- | ---------- | ---------- | ---------- | Addition RD11067 | ---------- |
| RD11077 | Base of RD11075 | --------- | --------- | ---------- | ---------- | ---------- | Addition RD11068 | ---------- |
| RD11078 | Base of RD11075 | --------- | --------- | ---------- | ---------- | ---------- | Addition RD11069 | ---------- |
| RD11079 | Transfer from RD11076 | Add 0.3% H2O | --------- | ---------- | ---------- | ---------- | Formula includes HPC | ---------- |

| | | | | | | | | |
|---|---|---|---|---|---|---|---|---|
| RD11080 | Transfer from RD11077 | Add 0.3% H2O | ---------- | ----------- | ----------- | ------------ | Formula includes HPC | ------------- |
| RD11081 | Transfer from RD11078 | Add 0.3% H2O | ---------- | ----------- | ----------- | ------------ | Formula includes HPC | ------------- |
| RD11082 | 4 | 81.0 | 000000 | 0000000 | 10 See RD11073 (3 | See RD11073 (3 | See RD11073 (0.25) | 00000000 |
| RD11085 | 4 | 70.7 | 000000 | 0000000 | 16 10+6 | 6 | N-L =0.2 N-M =0.3 | A= 2.8 |
| RD11086 | 4 Add 0.3% H2O | 70.7 | 000000 | 0000000 | 16 10+6 | 6 | N-L =0.2 N-M =0.3 | A= 2.8 |

**Lot # RD11037**

Process duplication of IMP batch (4%) without HPC. K17 and S630 dissolved in DMI/Transcutol mixture followed by addition of the active. Clear solution. Castor oil preheated and added the above active mixture. Clear solution observed. Followed with the addition of the Cabosil with low shear. Viscosity at time of manufacture 500 cps, followed with test after 48 hours resulted in 620 cps.

Lower viscosity primarily due to missing HPC (note that IMP 4% had approx 1,500 cps)

**Lot # RD11038**

Change in order of addition using the same formulation with a reduction of DMI/Transcutol and adjusted with castor oil. Cabosil was mixed into the Castor oil obtaining a clear viscous solution. The active mixture was prepared as per RD11037. Viscosity of the Castor oil/Cabosil mixture changed to 1180 cps (expected higher viscosity based on addition of Co Solvents during the Placebo trials). Potential impact of PVP and active to solvent mixture.

**Lot # RD11039**

Duplicated performance based on Placebo mixture also containing Labrafil in castor oil plus Cabosil (for IP). Same reaction of reduced viscosity when adding the active mixture.

**Lot# RD11040**

Duplicated Placebo process adding to the Castor oil/Cabosil mixture a portion of the DMI/Transcutol P co-solvent mixture. Viscosity of the oil mixture increased. Prepared the active mixture with the remaining co-solvents without the PVP and added to the oil mixture. Final viscosity of the bulk Gel was 10,400 cps. Potential for F/C.

**Lot # RD11041**

Process was repeated as per RD11040 including the PVP K17 and S630 with the active mixture and viscosity was reduced to 500 cps (increased to 1,500 cps after 3 weeks). Clear indication of PVP impact on lowering viscosity using K17 and S630.

**Lot# RD11042**

Repeat of trial with Castor oil/ Labrafil addition as per RD11037, and reduced Cabosil, with active co solvent mixture but no PVP. Viscosity of 1,750 cps

The following trials were designed to identify impact of changing to higher PVP grades as well as alternate source of HPC (2 grades). Pre mixture were made as outlined in table 3 concentrating on mixtures without Labrafil, using Castor oil native and Aerosil 200.

**Lot # RD11050**

Dispersion (pre-mix I) of Castor Oil and Aerosil 200 was prepared and viscosity increased by adding part of the DMI (4%). The preparation of the active mixture use the pre-mix of RD11047A (PVP K17-3%) in DMI only, added 0.3% of HPC Nisso H followed by addition of active. Active mixture was added to the Pre-mix I

**Lot # RD11050A**

Same basic formulation as RD11050 with change of adding to a portion additional 1 % of Aerosil 200

**Lot # RD11051**

Dispersion (pre-mix I) of Castor Oil and Aerosil 200 was prepared and viscosity increased by adding part of the DMI (4%). The preparation of the active mixture use the pre-mix of RD11047B (PVP K30-3%) in DMI only, added 0.3% of HPC Nisso M followed by addition of active. Active mixture was added to the Pre-mix I

**Lot # RD11051A**

Same basic formulation as RD11051 with change of adding to a portion additional 1 % of Aerosil 200

**Lot # RD11053**

Dispersion (pre-mix I) of Castor Oil and Aerosil 200 was prepared and viscosity increased by adding part of the DMI and Transcutol P. The preparation of the active mixture use the pre-mix of RD11048A (PVP K17-3%), added 0.3% of HPC Nisso H followed by addition of active. Active mixture was added to the Pre-mix I

**Lot # RD11054**

Dispersion (pre-mix I) of Castor Oil and Aerosil 200 was prepared and viscosity increased by adding part of the DMI and Transcutol P. The preparation of the active mixture use the pre-mix of RD11048B (PVP K30-3%), added 0.3% of HPC Nisso H followed by addition of active. Active mixture was added to the Pre-mix I

**Lot # RD11055**

Dispersion (pre-mix I) of Castor Oil and Aerosil 200 was prepared and viscosity increased by adding part of the DMI and Transcutol P. The preparation of the active mixture use the pre-mix of RD11048C (PVP K90-3%). No HPC added .Active mixture was added to the Pre-mix I

**Lot # RD11056**

Dispersion (pre-mix I) of Castor Oil and Aerosil 200 was prepared and viscosity increased by adding part of the DMI. The preparation of the active mixture use the pre-mix of RD11047C (PVP K90-3%). No HPC added Active mixture was added to the Pre-mix I

**Lot # RD11059**

Prepared mixture of Castor Oil and Cabosil (2.5%). Active was dissolved in DMI and Transcutol P. Resulted in milky appearance. Adding that mix to the Castor Oil pre-mix, mixture did not clear up. Prepared the PVP (K30) solution with DMI, added to the mix, no change in appearance however reduced viscosity.
Note, no change in evaluation adding a mixture of 0.1 % HPC to appearance, slight increase in viscosity. Trial not reported under trial a lot number.

**Lot # RD11060**

Prepared the Castor Oil adding 3.5% Cabosil, followed by addition of a mixture of DMI/ Transcutol P for thickening. The active dispersion was prepared in a PVP (K30) with DMI as co-solvent. (no HPC)

**Lot # RD11061**

Prepared the Castor Oil adding 3% Cabosil, followed by addition of Labrafil (2%) for thickening. The active dispersion was prepared in a DMI mixture containing PVP K17 (2%). Mix resulted in low viscosity, however could be considered for F/C test.

**Lot # RD11062**

Castor Oil native mixed with Aerosil 200 (3%) and added a mixture of DMI/Transcutol P (6+2) for thickening. A PVP mixture of K17 and K30 was dissolved in DMI/Transcutol P and followed with HPC H and solvate for 4 days. Mixture was reheated prior to addition of active. Castor Oil premix was heated prior to adding the active dispersion. Recommended for F/C

**Lot # RD11063**

Castor Oil native mixed with Aerosil 200 (4%) and added the DMI (6%) resulting in a high viscose mix. A mixture of PVP K17 and L29/32 was dissolved in DMI, plus HPC Nisso H (0.2). On overnight setup, noticed a separation, required remixing. Active was added to the high viscosity Castor Oil premix. To be followed up with modification to composition

Potential for F/C or to use RD11065

**Lot # RD11064**

Addition of 0.3% to portion of lot RD11062

**Lot # RD11065**

Addition of 0.3% to portion of lot RD11063

**Lot # RD11066**

Addition of 0.3% to portion of lot RD11041

**Lot # RD11070**

Addition of 0.3% to portion of lot RD11037

**Lot # RD11071**

Addition of 0.3% to portion of lot RD11042

**Lot # RD11072**

Addition of 0.3% to portion of lot RD11040

**Lot # RD11073**

Prepared Castor Oil / Aerosil 200 pre-mixture. Dissolve in DMI (6%) without PVP, the Testosterone and add to the Castor oil pre-mix. Obtained a viscosity of 6,300 cps. In a mixture of Transcutol P and DMI disperse the HPC M (only used 0.25% of prep) and add to main mix. Proposed for F/C

**Lot # RD11074**

Addition of 0.3% to portion of lot RD11072

**Lot # RD11075**

Prepared a stock mixture to complete 3x500 g trials consisting of Castor-Oil (68%) Aerosil 200 (3%) DMI (6%).

To this mix was added PVP K29-32 (1 %) in DMI (10) and active. Bulk split into 3 parts to be completed for 3 trials containing different mixtures and grades of HPC Nisso in Transcutol (ref lots RD11067/68/69)

**Lot # RD11076**

Used bulk from RD11075 and added HPC mix RD11067 (Transcutol P with Nisso H (0.15%)

**Lot # RD11077**

Used bulk from **RD11075** and added HPC mix RD11068 (Transcutol P with Nisso H (0.2%)

**Lot # RD11078**

Used bulk from **RD11075** and added HPC mix RD11069 (Transcutol P with Nisso H (0.1) and M (0.1)

**Lot # RD11079**

Addition of 0.3% to portion of lot RD11076

**Lot #RD11080**

Addition of 0.3% to portion of lot RD11077

**Lot #RD11081**

Addition of 0.3% to portion of lot RD11078

**Lot #RD11082**

Trial attempt to prepare a batch without the use of SiO2 failed

**Lot #RD11085**

Prepared Castor-Oil pre-mix adding 2.5% Aerosil 200 followed with a mix of DMI (10) and Testosterone. Obtained viscosity of 3,100 cps. Followed with the addition of HPC Nisso L (0.2%) and Nisso M (0.3%) mixed in DMI and Transcutol plus 0.3% Aerosil 200 to reduce stickiness. Material was added without any stringing to the main mixture and obtained a viscosity of 4,800 cps at day of manufacture and 4,900 cps 3 weeks later. Proposed for F/C

**Lot #RD11086**

Addition of 0.3% to portion of lot RD11085

**Table 2 TBS1A 4% strength**

Viscosity values using spindle 6, 20 rpm, Repeat test ref to Franz Cell: F/C

| Lot number | Trial Manuf date | Test date and values | Comments |
|---|---|---|---|
| RD11037 | Jul 15/11 | Oct 04/11 | Clear solution, previous results in July |

| | | | |
|---|---|---|---|
| | | 940 cps | 620 cps and follow up test 9/15/11 was 900 cps |
| RD11038 | Jul 15/11 | Oct 04/11 1,800 cps | Clear solution, original test 1,180 cps , follow up 09/15/11 1,660 cps |
| RD11039 | Jul 20/11 | Oct 04/11 1,380 cps | Clear solution, previous results in July 980cps and follow up test 9/15/11 was 1,300 cps |
| RD11040 | Jul 20/11 | Oct 04/11 11,040 cps | Clear Gel, previous results in July 10,400 cps and follow up test 9/15/11 was 10,140 cps |
| RD11041 | Jul 21/11 | Oct 04/11 1,420 cps | Clear solution, previous results in July 500 cps and follow up test 9/15/11 was 1,500 cps |
| RD11042 | Jul 21/11 | Oct 04/11 1,430 cps | Clear solution, test 9/15/11 was 1,720 cps |
| RD11050 | Aug.09/11 | Oct 04/11 Test not valid | Original comment sticky mixture, 09/15/11 results 2,460 Do not use trial lot for F/C Poor mixture, HPC settled to bottom as a slug |
| RD11050A | Aug.09/11 | Oct 04/11 Test not valid | Original comment sticky mixture, results 09/15/11 3,000 cps (increased during test from 2,400) Do not use trial lot for F/C Poor mixture, HPC settled to bottom as a slug |
| RD11051 | Aug.09/11 | Oct 04/11 2,100 ▲ cps | Clear , results 09/15/11 1,940 cps Note: viscosity values increase during 30 sec test |
| RD11051A | Aug.09/11 | Oct 04/11 2,540 ▲ cps | Clear , results 09/15/11 2,560 cps Note: viscosity values increase during 30 sec test |
| RD11053 | Aug.10/11 | Oct 04/11 4,500 ▲ cps | Clear but sticky with air bubbles , results 09/15/11 4,060 cps Note: viscosity values increase during 30 sec test |
| RD11054 | Aug.10/11 | Oct 04/11 14,000 ▲ cps | 09/15/11 test HPC globules, 15,000 cps Do not use trial lot for F/C , Note: viscosity values increase during 30 sec test Build up of HPC on spindle |
| RD11055 | Aug.10/11 | Oct 04/11 | 09/15/11, EEEEEE Do not use trial lot for F/C Note, error message indicates above |

| | | | |
|---|---|---|---|
| | | EEEEEE | 20,000 tester limit at that setting |
| RD11056 | Aug.10/11 | Oct 04/11<br>EEEEEE | 09/15/11, EEEEEE<br>Do not use trial lot for F/C<br>Note, error message indicates above 20,000 tester limit at that setting |
| RD11059 | Aug.22/11 | Oct 04/11<br>Test not valid | Do not use trial lot for F/C<br>Separation of HPC (?)Build up of HPC on spindle |
| RD11060 | Aug.23/11 | Oct 05/11<br>3,540 cps | Uniform texture |
| RD11061 | Aug.23/11 | Oct 05/11<br>960 cps | Uniform texture |
| RD11062 | Aug.24/11 | Oct 05/11<br>3,200 cps | Original viscosity 2,400 cps |
| RD11063 | Aug.24/11 | Oct 05/11<br>3,460 cps | Original viscosity 1,600 cps |
| RD11064 | Aug.31/11 | Oct 05/11<br>6,440 cps | Original viscosity 5,800 cps<br>Clear, thick, |
| RD11065 | Aug.31/11 | Oct 05/11<br>12,500 cps | Added .3% H2O to RD11063 09/31/11 resulted in 9,100 cps<br>Air bubbles |
| RD11066 | Aug.31/11 | Oct 05/11<br>2,600 cps | Added .3% H2O to RD11041 09/31/11 resulted in 1,500 cps<br>Clear , thick |
| RD11070 | Aug.31/11 | Oct 05/11<br>1,540 cps | Added .3% H2O to RD110370 9/31/11 resulted in 720 cps<br>Liquid and clear |
| RD11071 | Aug.31/11 | Oct 05/11<br>1,820 cps | Added .3% H2O to RD11042 9/31/11 resulted in 1,760 cps<br>Liquid and clear |
| RD11072 | Aug.31/11 | Oct 05/11<br>7,920 cps | Added .3% H2O to RD11040 resulted in 7,920 cps<br>Clear and thick, no change in viscosity |
| RD11073 | Sep.07/11 | Oct 05/11<br>9,980 cps | Started off in Sept with viscosity of 5,500 cps |
| RD11074 | Sep.07/11 | Oct 05/11<br>10,100 | Added .3% H2O to RD11073 increases viscosity to 7,200 cps. |

| | | | |
|---|---|---|---|
| | | cps | |
| RD11076 | Sep.06/11 | Oct 05/11 1,700 cps | Clear, however noticed separation in bulk |
| RD11077 | Sep.06/11 | Oct 05/11 1,600 cps | Clear |
| RD11078 | Sep.06/11 | Oct 05/11 2,700 cps | Clear and fluid |
| RD11079 | Sep.06/11 | Oct 05/11 3,500 cps | Added 0.3% H2O to RD11076 Clear, fluid |
| RD11080 | Sep.06/11 | Oct 05/11 3,900 cps | Added 0.3% H2O to RD11077 Clear, fluid |
| RD11081 | Sep.06/11 | Oct 05/11 2,600 cps | Added 0.3% H2O to RD11078 Clear, fluid |
| RD11085 | Sep.14/11 | Oct 05/11 4,900 cps | Original test 4,800 cps<br>Thick and clear |
| RD11086 | Sep.20/11 | Oct 05/11 5,180 cps | Addition of 0.3% H2O to RD11085 = 5,200 cps original<br>Thick gel and clear |

## TBS1A 8 % Formulation/composition

## Table 3

| *Trial number* | *Active micronized %* | *Castor oil %* | *Labrafil %* | *PVP grade %* | *DMI %* | *TranscutolP %* | *HPC Nisso %* | *SiO2 % C=Cabosil A=Aerosil200* |
|---|---|---|---|---|---|---|---|---|
| | | | | | | | | |
| RD11087 | 8 | 55.9 | 0000000 | 0000000 | 27 20 + 7 | 6 | N-L = 0.2 N-M = 0.3 | A = 2.6 |
| RD11088 | 8 | same | 0000000 | 0000000 | same | same | same | Same plus (0.3% H2O) |
| RD11089 | 8 | 46.5 | 0000000 | K17 = 3 S630 = 2 | 25 | 10 | N- M = 0.5 | C = 5 |
| RD11089A | 8 | same | 0000000 | same | same | same | same | Same plus (0.3% H2O) |
| RD11090 | 8 | 39.0 | 0000000 | K17 = 5.0 | 32 | 12 | N-H = 0.3 N-M = 0.2 | C = 3.5 |
| RD11100 | 8 | same | 0000000 | same | same | same | same | Added C = 2% for total of 5.5 |
| RD11101 | 8 | 46.1 | 0000000 | K17 = 5.0 | 25 | 10 | N-L = 0.4 N-M = 0.4 | C = 5.1 |
| RD11102 | 8 | 46.1 | 0000000 | K17 = 5.0 | 25 | 10 | N-L = 0.4 N-M = 0.4 | C = 5.1 plus Addition of 1% for total of 6.1 |
| RD11103 | 8 | 46.1 | 0000000 | K17 = 5.0 | 25 | 10 | N-L = 0.4 N-M = 0.4 | C = 5.1 plus addition of 0.3% water |
| RD11104 | 8 | 42.2 | 4.0 | K17 = 5.0 | 25 | 10 | N-L = 0.4 N-M = 0.4 | A = 5.0 |
| RD11105 | 8 | same | same | same | same | same | same | A = 5.0 addition of 0.5% total 5.5% |

**Process outline for active trials:**

**Lot # RD11087**

Trial was initiated without PVP to identify impact on T solubility. The active dispersion in % DMI used did not provide a clear solution and did not clear up when adding to the Castor Oil/SiO2 mix. Even the co-solvents present in the HPC mixture did not provide a clear bulk Gel. To the HPV mixture 0.1 % SiO2 was added to reduce stringing and stickiness.

Viscosity at 4,400

This trial however will be selected for the Franz Cell test to identify diffusion rate eliminating PVP.

**Lot # RD11088**

0.3% water was added to a portion of Lot RD11087 to identify impact on viscosity. As observed on 4% trials, increase in viscosity is not evident on the bulk mixed with SiO2 in the HPC. This trial not considered for F/C.

**Lot # RD11089**

This trial used the same quantitative formulation as the IMP Clinical 8%, however using an alternate source of HPC (original HPC source Klucel HF). Also made minor process changes, dissolved PVP in DMI only and added active. HPC was prepared in Transcutol and added to main bulk separately.

Obtained a clear solution when adding the active co-solvent mixture into the Castor-oil and no significant stringing with the addition of the HPC after addition of SiO2.

Viscosity of Gel on day of manufacture was 1,800 cps, when retested after 24 hours, 3,700 and after 48 hours up to 4,300. The re-test on October 3 (see table) recorded 4,500 cps.

This trial was selected for F/C test

**Lot#RD11089A**

0.3% water was added to a portion of Lot RD11089 to identify impact on viscosity.

Viscosity change over time similar to above trial, day of manufacture 2,700 cps, when retested after 24 hours, 3,920 and after 48 hours up to 4,600. The re-test on October 3 (see table) recorded 5,040 cps.

Selected for study on impact of water

**Lot # RD11090**

Used higher percentage of DMI and Transcutol to be split for various pre-mixes, similar with SiO2 to be added HPC. Made a pre-mix of Castor oil and SiO2, however due to the lower ratio between the 2 excipients, the mixture became quite thick and further thickened up when adding part of the DMI.

Did finish off the trial, ended up at low viscosity, day of manufacture 900 cps, test Oct 03 -1,260 cps. Lower level of SiO2 was considered for study impact, however considering the processing issue (see RD11100)
not suitable for F/C test

**Lot# RD11100**

Using a portion of above trial RD11090, added an additional 2% SiO2 (for total of 5.5%) to study impact on Viscosity. Increased to 1,900 cps on day of manufacture and retest October 03 (see table) resulted in a value of 3.060

**Lot # RD11101**

To potentially reduce the impact of PVP, required to dissolve the active, during the addition to the Castor oil/SiO2 mixture, added 2% of SiO2 to the DMI-PVP-Testosterone mix, obtaining a viscous mix. After addition of that mixture to a dispersion of Castor oil containing 1 % SiO2, maintained a viscous mixture at the temperature of 50% (would thicken up further on cooling). Further increase in viscosity with the addition of the HPC mix and final amount of SiO2.

Viscosity after cooling Gel to 21 C was 3,800 cps. (note that re-testing over time will be required, batch manufactured Oct 03)
This trial selected for F/C

**Lot#RD11102**

With the target for a 5,000 cps viscosity for the TBS1A project, the above RD11101 was so far the best candidate to evaluate impact of further addition of SiO2, hence to a portion of that lot additional 1 % SiO2 was added. The rational for 6% was to obtain the same ratio of active to SiO2 as the targeted level of 3% SiO2 for the 4% strength.

Viscosity increase to 8,000 cps , this lot was selected for F/C study to identify impact of viscosity on rate of diffusion compared to RD11101 of same composition with exception of 1 % addition in SiO2, may need to consider on assay obtained.

**Lot#RD11103**

Addition of water for impact on viscosity, not considered for follow up testing (see viscosity table for results, increase to RD11101 from 3,800 to 4,500 cps)

**Lot#RD11104**

Included this trial to evaluate addition of Labrafil. Labrafil was added to the Castor Oil mixed with SiO2 at 1 %. As observed previously, addition of Labrafil to the Castor oil containing SiO2 increases viscosity. All other mixture prepared and added as per trial RD11101, with addition of 2% SiO2 to complete mixture. This mixture contains a larger percentage of air bubbles, common on formulations containing Labrafil.
Viscosity obtained of 3,300 cps, will be followed up and tested at various time points. Selected for F/C testing.

**Lot#RD11105**

Added to RD11104 an additional 0.5% SiO2 (% adjusted to avoid high increase observed on RD11102)

Increase from 3,300 to 4,100 cps
Not selected for F/C test
Note: Placebo trials are drawn up to identify impact on viscosity using the 2 different sources for Castor Oil and SiO2. These trials will also answer potential questions related to TBS1 and TBS2.

**TABLE 4 TBS1A 8 % strength**

Viscosity values using spindle #6, 20 rpm, Franz Cell = F/C

| Lot number | Trial Manuf date | Test date and values | Comments |
|---|---|---|---|
| RD11087 | Sept 20/11 | Oct 03/11 4,400 cps | No PVP, solution not clear, 2.6% SiO2<br>Selected for Franz Cell |
| RD11088 | Sept 20/11 | Oct 03/11 4,040 cps | Added 0.3% H2Oto RD11087 |
| RD11089 | Sept 25/11 | Oct 03/11 4,500 cps | Based on original IMP, change in HPC source and minor process step changes<br>Selected for Franz Cell |
| RD11089A | Sept 25/11 | Oct 03/11 5,040 cps | As RD11089 plus 0.3% H2O<br>Selected for Franz Cell |
| RD11090 | Sept 26/11 | Oct 03/11 1,260 cps | 3.5% SiO2<br>Potential for F/C |
| RD11091 | Sept 26/11 | Oct 03/11 | Added 0.3% H2O to RD11090 |

| | | | |
|---|---|---|---|
| RD11100 | Sept 26/11 | Oct 03/11 3,060 cps | Added to RD11090 to reach 5% SiO2 content |
| RD11101 | Oct 03/11 | Oct 04/11 3,800 cps | 5% SiO2 Selected for Franz Cell |
| RD11102 | Oct 04/11 | Oct 04/11 8,000 cps | 6% SiO2 Selected for Franz Cell |
| RD11103 | Oct 04/11 | Oct 04/11 4,500 cps | 0.3% with 5% SiO2 |
| RD11104 | Oct 04/11 | Oct 05/11 3,300 cps | Includes 4% Labrafil, same comp for polymers as RD11101 (air-bubbles) Selected or Franz Cell |
| RD11105 | Oct 05/11 | Oct 05/11 4,100 cps | Added additional 0.5% of SiO2 to RD11104 |
| | | | |

<u>**Pre-mix RD Trials (used for addition in active trials)**</u>

## Table 5

| Trial #/observation test | Evaluation | Composition | Results/comments | Used in RD trial # |
|---|---|---|---|---|
| EV001A (pg 41) | Dissolving HPC Nisso grade **M** | DMI – 100 g<br>Transcutol P 50 g<br>Nisso HPC **M** - 2.5 g | Low viscosity grade<br>Stored for hydration 72 hrs<br>Suitable viscosity for further additions | Not transferred for use to RD trials |
| EV001B (pg 41) | Dissolving HPC Nisso grade **H** | DMI – 100 g<br>Transcutol P 50 g<br>Nisso HPC **H** - 2.5 g | high viscosity grade<br>Stored for hydration 72 hrs<br>Viscosity too high | Not transferred for use to RD trials |
| EV002A (pg 41) | Dispersing Cabosil in DMI (purpose to study impact on viscosity in final Gel) | DMI - 125 g<br>Cabosil 10 g<br>Ratio related to Castor oil/Cabosil | Obtained clear and viscous dispersion | Not transferred for use to RD trials |
| EV002B (pg 41) | Dispersing Cabosil in Transcutol P (purpose to study impact on viscosity in final Gel) | Transcutol P 250 g<br>Cabosil 20 g<br>Ratio related to Castor oil/Cabosil | Obtained no increase viscosity. Solution milky in appearance | Not transferred for use to RD trials |
| RD11047 A | Addition of PVP K17 in DMI only. | DMI- 100 g<br>PVP K17 15 g<br>Ratio represents 3% of PVP based on final Bulk Gel formula | Suitable for additional mixing with HPC H and active. Note: used higher viscosity HPC grade with lower viscosity PVP grade | Used in RD trial for addition of HPC-H and active (see RD1150 and RD1150A) |
| RD11047B | Addition of PVP | DMI- | Suitable for | Used in RD trial |

| | | | | |
|---|---|---|---|---|
| | K29/32 in DMI only. | 100 g<br>PVP K29/32 15 g<br>Ratio represents 3% of PVP based on final Bulk Gel formula | additional mixing with HPC M and active. Note: used lower viscosity HPC grade with higher viscosity PVP grade | for addition of HPC-M and active (see RD1151 and RD1151A) |
| RD11047C | Addition of PVP K90 in DMI only. | DMI-<br>100 g<br>PVP K90<br>15 g<br>Ratio represents 3% of PVP based on final Bulk Gel formula | Not suitable to add any grade HPC, however suitable to add the active portion. | Used in RD trial without HPC addition RD11056 |
| RD11048 A | Addition of PVP K17 in DMI and Transcutol P | DMI-<br>80 g<br>Transcutol P 20 g<br>PVP K17<br>15 g<br>Ratio represents 3% of PVP based on final Bulk Gel formula | Suitable for additional mixing with HPC H and active. Note: used higher viscosity HPC grade with lower viscosity PVP grade | Used in RD trial for addition of HPC-H and active (see RD11053 |
| RD11048B | Addition of PVP K29/32 in DMI and Transcutol P. | DMI-<br>80 g<br>Transcutol P 20 g<br>PVP K29/32<br>15 g<br>Ratio represents 3% of PVP based on final Bulk Gel formula | Suitable for additional mixing with HPC M and active. Note: used lower viscosity HPC grade with higher viscosity PVP grade | Used in RD trial for addition of HPC-M and active (see RD11054 |
| RD11048C | Addition of PVP K90 in DMI and Transcutol P | DMI-<br>100 g<br>PVP K90<br>15 g<br>Ratio represents 3% of PVP based on final Bulk Gel formula | Not suitable to add any grade HPC, however suitable to add the active portion. | Used in RD trial without HPC addition RD11055 |
| RD11067 | Prep of HPC in Transcutol P only | TP= 40g<br>N-H = 0.75g | | Used in RD11076 |
| RD11068 | Prep of HPC in Transcutol P only | TP= 40g<br>N-H = 1.0 g | | Used in RD11077 |
| RD11069 | Prep of HPC in Transcutol P only | TP= 40g<br>N-H = 0.5 g<br>N-M=0.5 g | | Used in RD11078 |
| RD11075 | Prep of base solution used RD11076/RD11077/ RD11078<br>Details in Table 2 | Castor oil /<br>Aerosil200/<br>DMI/<br>PVP K30<br>Testosterone | | |

Placebo TBS1 A trials

## Table 6

| Trial lot # | Evaluation | Composition | Results/comments |
|---|---|---|---|
| RD11032 | Evaluate change in viscosity using Labrafil versus Castor Oil Cr 0 | Labrafil M 1944 CS - 500g<br>Cab-O-Sil ---- 40 g | Viscosity 10,460 cps |
| RD11033 | Evaluate change viscosity adding Cabosil first in Castor Oil Cr 0 | Castor Oil ----- 500 g<br>Cab-O-Sil --- 40 g<br>Note: ratio used in IMP | Viscosity 14 460 cps |
| RD11034 | Impact on adding DMI and Transcutol to mixture RD11032 | RD11032- 270 g<br>DMI- 125 g<br>Transcutol P 50 g | Viscosity reduced to 8,740 |
| RD11035 | Impact on adding DMI and Transcutol to mixture RD11033 | Impact on adding DMI and Transcutol to mixture RD11032 | Viscosity reduced to 3,600 |
| RD11036A | Mixture of Castor Oil and Labrafil, adding Cabosil followed by DMI/Transcutol P | Castor oil ...... 125 g<br>Labrafil ........ 125 g<br>Cabosil ....... 20 g<br>DMI ..... 125 g<br>Transcutol P 50 g | High viscosity out of range |
| RD11036B | Mixture of Castor Oil and Labrafil followed by DMI/Transcutol P, add Cabosil last | Castor oil 0 ...... 125 g<br>Labrafil ........ 125 g<br>Cabosil ....... 20 g<br>DMI ..... 125 g<br>Transcutol P 50 g | Viscosity 7,680 cps |
| RD11043 | Castor oil and Cab0sil, followed by mixture of DMI/Transcutol P and HPC **H** | Castor oil 0 ...... 285 g<br>Cabosil ....... 20 g<br>DMI .... 100 g<br>Transcutol P 50 g<br>HPC **H** .............. 2.5 g | |
| RD11043 | Castor oil and Cab0sil, followed by mixture of DMI/Transcutol P and HPC **M and PVP K17** | Castor oil 0 ...... 285 g<br>Cabosil ....... 20 g<br>DMI .... 100 g<br>Transcutol P 50 g<br>HPC **M** .............. 2.5 g<br>PVP K15 ....... 15 g | |
| RD11057P | TBS-2 Placebo for Analytical Lab Method | -------------------------- | ----------------------------- |
| RD11058P A-B-C-D-E-F | Castor oil an Cabosil Mix followed by addition of Labrafil | A to D represents % Labrafil of 2-4% with change in viscosity | RD11058P = 2740 cps<br>Part A 2% = 11,400<br>Part B 3% = 14,000 |

| | | | |
|---|---|---|---|
| | | E impact of adding Oleic acid<br>F impact of adding DMI to RD11058-A | Part C 3.5% = 14,440<br>Part D 4% = 14,900<br>Part E with Oleic = 1,520<br>Part F - 10% DMI to part A = 13,500 cps<br>(incr. from 11,400) |
| RD11083P | Purpose of trial to decrease stringing and stickiness of HPC mixture when adding to base mix of castor oil/Aerosil and DMI | HPC mix prep of DMI/TranscutolP solvents plus Nisso HPC L and M<br>Solvated for 48 hours followed by addition of SiO2 | Viscosity of base prior to addition of HPC mixture was 5,300 cps, after addition of HPC mixture (no stringing |
| RD11084P | Used part of RD1108P to add 0.3% H2O to evaluate impact on viscosity | | |

## Example 10

### Franz Cell Studies - Testosterone Rates of Diffusion

Generally'speaking, soak the membrane for 30 minutes in the diffusion solution. After put the membrane on the Franz Cell. Put the ring and the donor chamber on the membrane and clamp it. Add approx. one gram of gel (TBS 1 A 4% or 8%). Check the level of diffusion solution in Franz Cells. It's supposed to be on the mark. Put "parafilm" on the sampling port to avoid evaporation. Withdraw 0.3mL of sample at 60, 120, 180, 240, 300 and 360 minutes using syringe. Add diffusion solution to make up to the mark of Franz Cells. Each sample should be collected in insert.

A typical Fanz cell used in accordance with this Example 9 and the invention is depicted in Fig. 12. The materials include:

Diffusion solution: Ethanol/Water 50:50
Membrane: Millipore 0.45μm.
Temperature: 37° ± 0.5 °C.
Stirring speed: 600 rpm.
Medium volume: 20mL.
Surface area: 1.7671 $cm^2$
Number of Franz Cells: 6.
Sampling time (minutes): 60, 120, 180, 240, 300 and 360.
Aliquot volume: 0.3mL.
Insert: 0.4mL.

The TBS1A formulations are as follows and as reported in the Examples above and herein. The rate of diffusion results of testosterone through the Franz cell mebrane, normalized for each gel concentrations being tested, measured as slope/mgT%, are reported below in the Franz Cell Table.

**4% TBS1A Trial formulations used in Franz Cell**

**Trial Lot # RD11063** Batch size 500 g

| Raw Materials/grade | % | Process | comments |
|---|---|---|---|
| | | | 24 hr Franz Cell |
| Testosterone micronized | 4.0 | 12% DMI to disperse PVP and active | |

| | | | |
|---|---|---|---|
| Castor Oil (**V** – O) | 70.8 | 4% SiO2 in Castor oil plus 6% of DMI | Steps: |
| PVP **K17** | 1.5 | | A: add all SiO2 to Castor Oil |
| PVP **K30** | 1.5 | | Followed by DMI portion |
| PVP **K90** | 0.0 | | B: to the DMI add PVP, follow |
| Co PVP **S630** | 0.0 | | With HPC and hold 24 hrs |
| DMI | 18.0 | | C: add active |
| Transcutol P | 0.0 | | D: add to mix A) |
| HPC Nisso **L** | 0.0 | | |
| HPC Nisso **M** | 0.0 | | Temp range NMT 60C |
| HPC Nisso **H** | 0.2 | | Homogenize active mixture |
| SiO2 (Cabosil –**Aerosil 200)** | 4.0 | | Viscosity 3,650 cps 10/05/11) |

**Trial Lot # RD11085** Batch size 500 g

| Raw Materials/grade | % | Process | comments |
|---|---|---|---|
| | | | 24 hrs Franz Cell |
| Testosterone micronized | 4.0 | 10% DMI used to dissolve active | |
| Castor Oil (V – **O**) | 70.7 | 2.5% of SiO2 mixed into Castor Oil | Steps: |
| PVP **K17** | 0.0 | ----------- | A: Active /DMI mixture added |
| PVP **K30** | 0.0 | ----------- | to Castor Oil/SiO2 mix |
| PVP **K90** | 0.0 | ----------- | B: add SiO2 to HPC after 24 h |
| Co PVP **S630** | 0.0 | ------------ | |
| DMI | 16.0 | 6% DMI used for HPC dispersion | C: add HPC mixture to main |
| Transcutol P | 6.0 | Used to disperse HPC and solvate for 24 hrs | bulk |
| HPC Nisso **L** | 0.2 | 0.3% of SiO2 mixed into HPC mixture | |
| HPC Nisso **M** | 0.3 | | Temp range NMT 60C |
| HPC Nisso **H** | 0.0 | | Homogenize active mixture |
| SiO2 (Cabosil –**Aerosil 200** ) | 2.8 | | Viscosity 4,900 cps (10/05/11) |

**Trial Lot # RD11038** Batch size 500 g

| Raw Materials/grade | % | Process | comments |
|---|---|---|---|
| | | | 6 hr Franz Cell |
| Testosterone micronized | 4.0 | Add to PVP mixture | |
| Castor Oil (V – **O**) | 57.0 | All Cabosil into Castor Oil | A: add to the Castor Oil /SiO2 |
| PVP **K17** | 3.0 | | Mix the PVP active mixture |
| PVP **K30** | 0.0 | | |
| PVP **K90** | 0.0 | | |
| Co PVP **S630** | 2.0 | | |
| DMI | 20.0 | All DMI and Transcutol P to disperse PVP | |
| Transcutol P | 10.0 | | |
| HPC Nisso **L** | 0.0 | | |
| HPC Nisso **M** | 0.0 | | Homogenize active mixture |
| HPC Nisso **H** | 0.0 | | |
| SiO2 (**Cabosil** –Aerosil 200) | 4.0 | | Viscosity 1,800 cps |

***Trial Lot # RD11039*** *Batch size 500 g*

| Raw Materials/grade | % | Process | comments |
|---|---|---|---|
| | | | 6 hr Franz Cell |
| Testosterone micronized | 4.0 | | |
| Castor Oil (V – **O**) | 29.0 | Mix Castor oil+Labrafil+Cabosil | |
| PVP **K17** | 3.0 | | |

| | | | |
|---|---|---|---|
| PVP **K30** | 0.0 | | |
| PVP **K90** | 0.0 | | |
| Co PVP **S630** | 2.0 | PVP into DMI+Tr-P followed by active | |
| DMI | 20.0 | | |
| Transcutol P | 10.0 | | |
| Labrafil | 29.0 | | |
| HPC Nisso **M** | 0.0 | | |
| HPC Nisso **H** | 0.0 | | |
| SiO2 (**Cabosil** –Aerosil 200) | 3.0 | | Viscosity 1,380 |

**Trial Lot # RD11040** Batch size 500 g

| Raw Materials/grade | % | Process | comments |
|---|---|---|---|
| | | | 6 hr Franz Cell |
| Testosterone micronized | 4.0 | Mix in 12% DMI and 6% Tr-P | |
| Castor Oil (V – **O**) | 57.0 | Combine Castor oil + SiO2+ 13% DMI+4% TrP | |
| PVP **K17** | 0.0 | | |
| PVP **K30** | 0.0 | | |
| PVP **K90** | 0.0 | | |
| Co PVP **S630** | 0.0 | | |
| DMI | 25.0 | | |
| Transcutol P | 10.0 | | |
| HPC Nisso **L** | 0.0 | | |
| HPC Nisso **M** | 0.0 | | |
| HPC Nisso **H** | 0.0 | | |
| SiO2 (**Cabosil** –Aerosil 200) | 4.0 | | Viscosity 11,040 |

***Trial Lot # RD11042*** Batch size 500 g

| Raw Materials/grade | % | Process | comments |
|---|---|---|---|
| | | | 6 hr Franz Cell |
| Testosterone micronized | 4.0 | Active dissolve in 13% DMI+ 4% Tr-P | |
| Castor Oil (V – **O**) | 29.0 | Castor oil+Labrafil+ SiO2 +12 % DMI+6% Tr-P | |
| PVP **K17** | 0.0 | | |
| PVP **K30** | 0.0 | | |
| PVP **K90** | 0.0 | | |
| Co PVP **S630** | 0.0 | | |
| DMI | 25.0 | | |
| Transcutol P | 10.0 | | |
| Labrafil | 29.0 | | |
| HPC Nisso **M** | 0.0 | | |

| | | | |
|---|---|---|---|
| HPC Nisso **H** | 0.0 | | |
| SiO2 (**Cabosil** –Aerosil 200) | 3.0 | | Viscosity 1,430 cps |

**Trial Lot # RD11051** Batch size 500 g

| **Raw Materials/grade** | **%** | **Process** | **comments** |
|---|---|---|---|
| | | | 6 hr Franz Cell |
| Testosterone micronized | 4.0 | 20% DMI+PVP+N-M +0.2% iO2 | |
| Castor Oil (V – O) | 66.7 | Castor Oil + SiO2 1.8 %+4% DMI | |
| PVP **K17** | 0.0 | | Easier addition of HPC adding |
| PVP **K30** | 3.0 | | Small % of SiO2 |
| PVP **K90** | 0.0 | | |
| Co PVP **S630** | 0.0 | | |
| DMI | 24.0 | | |
| Transcutol P | 0.0 | | |
| HPC Nisso **L** | 0.0 | | |
| HPC Nisso **M** | 0.3 | | |
| HPC Nisso **H** | 0.0 | | |
| SiO2 (Cabosil –Aerosil 200) | 2.0 | | Viscosity 2,100 cps |

**Trial Lot # RD11055** Batch size 500 g

| **Raw Materials/grade** | **%** | **Process** | **comments** |
|---|---|---|---|
| | | | 6 hr Franz Cell |
| Testosterone micronized | 4.0 | DMI 16% +Transc 4% +pvp+active | |
| Castor Oil (V – **O**) | 62.0 | Castor Oil + SiO2 3 %+7% DMI+Trans 1% | |
| PVP **K17** | 0.0 | | |
| PVP **K30** | 0.0 | | |
| PVP **K90** | 3.0 | | |
| Co PVP **S630** | 0.0 | | |
| DMI | 23.0 | | |
| Transcutol P | 5.0 | | |
| HPC Nisso **L** | 0.0 | | |
| HPC Nisso **M** | 0.0 | | |
| HPC Nisso **H** | 0.0 | | |
| SiO2 (**Cabosil** –Aerosil 200) | 3.0 | | Exceeded test range |

**Trial Lot # RD11078** Batch size 500 g

| **Raw Materials/grade** | **%** | **Process** | **comments** |
|---|---|---|---|
| | | | 6 hr Franz Cell |
| Testosterone micronized | 4.0 | | |
| Castor Oil (V – **O**) | 68.0 | Castor oil +SiO2 -3% + 6% DMI | To be corrected to 67.8% |
| PVP **K17** | | | for repeat (base) |

| | | | |
|---|---|---|---|
| | 0.0 | | |
| PVP **K30** | 1.0 | DMI 10% + pvp + active | Base prep RD11075 |
| PVP **K90** | 0.0 | | |
| Co PVP **S630** | 0.0 | | |
| DMI | 16.0 | | |
| Transcutol P | 8.0 | Transc P + both HPC | Prep on RD11069 |
| HPC Nisso **L** | 0.0 | | |
| HPC Nisso **M** | 0.1 | | Requires adjustment of |
| HPC Nisso **H** | 0.1 | | Castor oil by 0.2 % |
| SiO2 (Cabosil –**Aerosil 200**) | 3.0 | | Viscosity 2,700 cps |

## Trial Lot # RD11054

Batch size 500 g

| Raw Materials/grade | % | Process | comments |
|---|---|---|---|
| | | | 6 hr Franz Cell |
| Testosterone micronized | 4.0 | | |
| Castor Oil (V – O) | 61.4 | Castor Oil +SiO2 3% + DMI 7% + Transc 1% | |
| PVP **K17** | 0.0 | | |
| PVP **K30** | 3.0 | DMI 16%+Trans 4%+pvp+HPC +active | |
| PVP **K90** | 0.0 | | |
| Co PVP **S630** | 0.0 | | |
| DMI | 23.0 | | |
| Transcutol P | 5.0 | | |
| HPC Nisso **L** | 0.0 | | |
| HPC Nisso **M** | 0.6 | | |
| HPC Nisso **H** | 0.0 | | |
| SiO2 (Cabosil –**Aerosil 200**) | 3.0 | | Viscosity 14,000 cps |

## Trial Lot # RD11061

Batch size 500 g

| Raw Materials/grade | % | Process | comments |
|---|---|---|---|
| | | | 6 hr Franz Cell |
| Testosterone micronized | 4.0 | | |
| Castor Oil (V – O) | 71.0 | Castor oil + SiO2 + Labrafil | |
| PVP **K17** | 2.0 | DMI 16% + Transc 2% + PVP+ active | |
| PVP **K30** | 0.0 | | |
| PVP **K90** | 0.0 | | |
| Co PVP **S630** | 0.0 | | |
| DMI | 16.0 | | |
| Transcutol P | 2.0 | | |
| Labrafil | 2.0 | | |
| HPC Nisso **M** | 0.0 | | |
| HPC Nisso **H** | 0.0 | | |
| SiO2 (**Cabosil** –Aerosil 200) | 3.0 | | Viscosity 960 cps |

## Franz Cell Table – Slope/mgT%

| Lot nr/composition%/Franz | Testosterone | Castor Oil | PVP K17 | PVP K30 | PVP K90 | CoPVP 630 | Labrafil | DMI | Transcutol | HPC L | HPC M | HPC H | HPC XHF | SiO2 | water | Intercept | slope | Intercept/mgT% | Slope/mgT% | Povidone T | HPC Total | control |
|---|---|---|---|---|---|---|---|---|---|---|---|---|---|---|---|---|---|---|---|---|---|---|
| Reference | 4 | 88 | 0 | 0 | 0 | 0 | 4 | 0 | 0 | 0 | 0 | 0 | 0 | 4 | 0 | -241.78 | 132.62 | -60.45 | 33.16 | 0.00 | 0 | 100 |
| R viscous | 4 | 87.7 | 0 | 0 | 0 | 0 | 4 | 0 | 0 | 0 | 0 | 0 | 0 | 4 | 0.3 | -389.81 | 135.27 | -97.45 | 33.82 | 0.00 | 0 | 100 |
| TBS1a IMP11001 4% | 4 | 50.5 | 3 | 0 | 0 | 2 | 0 | 25 | 10 | 0 | 0 | 0 | 0.5 | 5 | 0 | -1371.8 | 300.02 | -342.95 | 75.01 | 5.00 | 0.5 | 100 |
| TBS1a IMP11002 4% | 4 | 65.5 | 3 | 0 | 0 | 2 | 0 | 15 | 5 | 0 | 0 | 0 | 0.5 | 5 | 0 | -991.01 | 220.68 | -247.75 | 55.17 | 5.00 | 0.5 | 100 |
| TBS1a IMP11003 8% | 8 | 46.5 | 3 | 0 | 0 | 2 | 0 | 25 | 10 | 0 | 0 | 0 | 0.5 | 5 | 0 | -2673.9 | 613.24 | -334.24 | 76.66 | 5.00 | 0.5 | 100 |
| RD11089 | 8 | 46.5 | 3 | 0 | 0 | 2 | 0 | 25 | 10 | 0 | 0.5 | 0 | 0 | 5 | 0 | -1999.2 | 489.46 | -249.90 | 61.18 | 5.00 | 0.5 | 100 |
| RD11089A | 8 | 46.5 | 3 | 0 | 0 | 2 | 0 | 25 | 10 | 0 | 0.5 | 0 | 0 | 5 | 0.3 | -1454.6 | 425.39 | -181.83 | 53.17 | 5.00 | 0.5 | 100.3 |
| RD11087 | 8 | 55.9 | 0 | 0 | 0 | 0 | 0 | 27 | 6 | 0.2 | 0.3 | 0 | 0 | 2.6 | 0 | -2810.2 | 636.05 | -351.28 | 79.51 | 0.00 | 0.5 | 100 |
| RD11101 | 8 | 46.1 | 5 | 0 | 0 | 0 | 0 | 25 | 10 | 0.4 | 0.4 | 0 | 0 | 5.1 | 0 | -2085.1 | 525.63 | -260.64 | 65.70 | 5.00 | 0.8 | 100 |
| RD11102 | 8 | 46.1 | 5 | 0 | 0 | 0 | 0 | 25 | 10 | 0.4 | 0.4 | 0 | 0 | 6.1 | 0 | -2069.9 | 499.5 | -258.74 | 62.44 | 5.00 | 0.8 | 101 |
| RD11104 | 8 | 42.2 | 5 | 0 | 0 | 0 | 4 | 25 | 10 | 0.4 | 0.4 | 0 | 0 | 5 | 0 | -3397.2 | 755.62 | -424.65 | 94.45 | 5.00 | 0.8 | 100 |
| RD11038 | 4 | 57 | 3 | 0 | 0 | 2 | 0 | 20 | 10 | 0 | 0 | 0 | 0 | 4 | 0 | -1265.3 | 271.06 | -316.33 | 67.77 | 5.00 | 0 | 100 |
| RD11039 | 4 | 29 | 3 | 0 | 0 | 2 | 29 | 20 | 10 | 0 | 0 | 0 | 0 | 3 | 0 | -3084.4 | 508.88 | -771.10 | 127.22 | 5.00 | 0 | 100 |
| RD11040 | 4 | 57 | 0 | 0 | 0 | 0 | 0 | 25 | 10 | 0 | 0 | 0 | 0 | 4 | 0 | -312.27 | 389.27 | -78.07 | 97.32 | 0.00 | 0 | 100 |
| RD11042 | 4 | 29 | 0 | 0 | 0 | 0 | 29 | 25 | 10 | 0 | 0 | 0 | 0 | 3 | 0 | -1687.5 | 366.34 | -421.88 | 91.59 | 0.00 | 0 | 100 |
| RD11051 | 4 | 66.7 | 0 | 3 | 0 | 0 | 0 | 24 | 0 | 0 | 0.3 | 0 | 0 | 2 | 0 | -1614.1 | 313.35 | -403.53 | 78.34 | 3.00 | 0.3 | 100 |
| RD11053 | 4 | 61.7 | 3 | 0 | 0 | 0 | 0 | 22 | 6 | 0 | 0 | 0.3 | 0 | 3 | 0 | -1187.7 | 261.82 | -296.93 | 65.46 | 3.00 | 0.3 | 100 |
| RD11054 | 4 | 61.4 | 0 | 3 | 0 | 0 | 0 | 23 | 5 | 0 | 0.6 | 0 | 0 | 3 | 0 | -1214.3 | 244.7 | -303.58 | 61.18 | 3.00 | 0.6 | 100 |
| RD11055 | 4 | 62 | 0 | 0 | 3 | 0 | 0 | 23 | 5 | 0 | 0 | 0 | 0 | 3 | 0 | -1428.1 | 307.28 | -357.03 | 76.82 | 3.00 | 0 | 100 |
| RD11061 | 4 | 71 | 2 | 0 | 0 | 0 | 2 | 16 | 2 | 0 | 0 | 0 | 0 | 3 | 0 | -2327.6 | 397.43 | -581.90 | 99.36 | 2.00 | 0 | 100 |
| RD11063 | 4 | 70.8 | 1.5 | 1.5 | 0 | 0 | 0 | 18 | 0 | 0 | 0 | 0.2 | 0 | 4 | 0 | -924.39 | 187.46 | -231.10 | 46.87 | 3.00 | 0.2 | 100 |
| RD11078 | 4 | 68 | 0 | 1 | 0 | 0 | 0 | 16 | 8 | 0 | 0.1 | 0.1 | 0 | 3 | 0 | -1309.9 | 269.37 | -327.48 | 67.34 | 1.00 | 0.2 | 100.2 |
| RD11085 | 4 | 70.7 | 0 | 0 | 0 | 0 | 0 | 16 | 6 | 0.2 | 0.3 | 0 | 0 | 2.8 | 0 | -1550 | 272.4 | -387.50 | 68.10 | 0.00 | 0.5 | 100 |

The TBS-1A Gel In Vitro Release Rate Validation concerning Release Rate Study Summary for TBS-1A Gel 4.0% and TBS-1A Gel 4.5% are presented in Exhibits A and B submitted herewith.

These summaries summarize the release rate experiment data for exemplary TBS-1A Gels. There are four Nasobol Gels (0.15%, 0.6%, 4.0% and 4.5%) for the method validation. The purpose of the Day1 and Day2 test are to determine the specificity and intraday/interday precision of the slope(release rate), Day3 and Day4 are to evaluate the slope sensitivity to the sample strength variation

See Exhibit A (4.0%) and Exhibit B (4.5%) submitted herewith, both of which are incorporated herein by reference in their entireties.

The complete disclosures of the patents, patent documents, and publications cited herein are incorporated by reference in their entireties as if each were individually incorporated. In case of conflict, the present specification, including definitions, shall control. The foregoing description illustrates only certain embodiments of the present invention. The present invention therefore is not limited to the foregoing examples and illustrative embodiments and such are provided as examples only and are not intended to limit the scope of the present invention. Thus, various modifications and alterations to the present invention will become apparent to those skilled in the art without departing from the scope and spirit of this invention. That is, persons skilled in the art will appreciate and understand that modifications and variations are, or will be, possible to utilize and carry out the teachings of the present invention described herein. Accordingly, all suitable modifications, variations and equivalents may be resorted to, and such modifications, variations and equivalents are intended to fall within the scope of the present invention as described and within the scope of the claims.

In Vitro Release Rate Method Validation For Nasobol Gel 4.0%

**NASOBOL GEL IN ViTRO RELEASE RATE VALIDATION UPDATE**

**Release Rate Study Summary**

**Part 3: Nasobol Gel 4.0%**

**Purpose**

This summary summarized all release rate experiment data for Nasobol Gels There are four Nasobol Gels (0.15%, 0.6%, 4.0% and 4.5%) for the method validation.

The purpose of the Day1 and Day2 test are to determine the specificity and intraday/interday precision of the slope(release rate), Day3 and Day4 are to evaluate the slope sensitivity to the sample strength variation.

**Exhibit A**

In Vitro Release Rate Method Validation For Nasobol Gel 4.0%

Nasobol Gel 4.0% Release Rate

| Actual Amount of Active Released (μg/$cm^2$) versus $Time^{0.5}$ | | | | | | | | | |
|---|---|---|---|---|---|---|---|---|---|
| Time (minutes) | $Time^{0.5}$ | Day1 Mean(n=6) 4.0% Testosterone Gel | Day2 Mean(n=6) 4.0% Testosterone Gel | Day3 Mean(n=4) 2.0% Testosterone Gel | Day3 Mean(n=4) 4.0% Testosterone Gel | Day3 Mean(n=4) 8.0% Testosterone Gel | Day4 Mean(n=4) 2.0% Testosterone Gel | Day4 Mean(n=4) 4.0% Testosterone Gel | Day4 Mean(n=4) 8.0% Testosterone Gel |
| | | | | | | | | | 1518.098 |
| 120.00 | 10.95 | 1063.383 | 1051.045 | 526.164 | 1086.343 | 2099.274 | 498.872 | 998.688 | 2196.381 |
| 180.00 | 13.42 | 1388.71 | 1325.531 | 669.171 | 1331.988 | 2840.811 | 629.72 | 1288.523 | 2661.035 |
| 240.00 | 15.49 | 1846.814 | 1574.32 | 780.773 | 1565.054 | 3131.278 | 752.381 | 1503.981 | 3099.653 |
| 300.00 | 17.32 | 1866.587 | 1797.506 | 891.225 | 1774.893 | 3523.548 | 873.102 | 1781.223 | 3609.813 |
| 360.00 | 18.97 | 2066.757 | 1987.891 | 983.135 | 1950.68 | 3907.337 | 971.374 | 1902.587 | 3831.99 |
| Slope | n/a | 123.93 | 116.38 | 57.69 | 112.98 | 227.00 | 58.12 | 112.97 | 210.03 |
| $R^2$ | n/a | 0.9995 | 0.9976 | 0.9998 | 0.9989 | 0.9995 | 0.9981 | 0.9952 | 0.9967 |

In Vitro Release Rate Method Validation For Nasobol Gel 4.0%

4% Day1 Release Rate

Testosterone 4% Gel

| Amount Released (μg/mL) Calculation by Linear Regression Curve | | | | | | | | |
|---|---|---|---|---|---|---|---|---|
| | **Cell A#1** | **Cell A#2** | **Cell A#3** | **Cell A#4** | **Cell#5** | **Cell #6** | **Mean 1-6** | **%RSD 1-6** |
| **Time** | | | | | | | | |
| 60.00 | 100.615 | 100.156 | 101.259 | 104.065 | 96.829 | 102.531 | 100.909 | 2.4 |
| 120.00 | 151.286 | 149.754 | 151.980 | 154.908 | 150.156 | 158.241 | 152.388 | 1.7 |
| 180.00 | 193.494 | 190.313 | 193.585 | 197.317 | 188.895 | 198.299 | 193.651 | 1.9 |
| 240.00 | 222.232 | 221.742 | 222.845 | 228.588 | 218.647 | 229.252 | 223.884 | 1.9 |
| 300.00 | 248.200 | 246.211 | 245.509 | 247.784 | 240.822 | 252.989 | 246.919 | 1.6 |
| 360.00 | 263.755 | 264.893 | 209.185 | 269.287 | 256.350 | 273.175 | 266.108 | 2.2 |
| **Actual Amount of Active Released (μg/cm$^2$) versus Time$^{0.5}$** | | | | | | | | |
| **Amount Released (μg/cm$^2$)** | | | | | | | | |
| | **Cell A#1** | **Cell A#2** | **CellA#3** | **Cell A#4** | **Cell A#5** | **CellA#6** | **Mean 1-6** | **%RSD 1-6** |
| Time$^{0.5}$ | | | | | | | | |
| 7.75 | 683.255 | 680.138 | 687.628 | 706.683 | 657.545 | 696.266 | 685.253 | 2.4 |
| 10.95 | 1055.820 | 1045.287 | 1060.715 | 1081.392 | 1047.075 | 1090.010 | 1063.383 | 1.7 |
| 13.42 | 1385.252 | 1363.087 | 1386.248 | 1413.214 | 1352.630 | 1419.826 | 1386.709 | 1.9 |
| 15.49 | 1635.154 | 1630.364 | 1639.722 | 1681.399 | 1608.117 | 1686.130 | 1646.814 | 1.9 |
| 17.32 | 1874.378 | 1859.269 | 1856.682 | 1876.434 | 1820.569 | 1912.189 | 1866.587 | 1.6 |
| 18.97 | 2050.237 | 2055.800 | 2086.927 | 2092.567 | 1994.157 | 2120.851 | 2066.756 | 2.1 |
| Slope | 123.44 | 123.83 | 124.46 | 123.99 | 120.04 | 127.49 | 123.87 | 1.9 |
| $R^2$ | 0.9994 | 0.9995 | 0.9995 | 0.9993 | 0.9995 | 0.9998 | 0.9995 | 0.0 |

In Vitro Release Rate Method Validation For Nasobol Gel 4.0%

System Suitability for 4% Gel Day1

1: Medium (Diluent)

| | |
|---|---|
| Result | There are no interference peaks at the RT of Testosterone from diluent injection |
| Criteria | There should be no significant interference peaks at the RT of Tesosterone from diluent |
| Comment | Pass |

2: Injection Reproducibility RT, Tailing Factor and Theoretical Plate Number from Six Replicate Injections of STD-4

| Result | RT | %RSD of RT | % RSD of Peak area | Tailing factor(T) | Theoretical Plate (N) |
|---|---|---|---|---|---|
| | 1.14 | 0 | 0.5 | 1.5 | 5269 |
| Criteria | NMT 2.0% | | NMT 2.0% | NMT 2 | NLT 1000 |
| Comment | Pass | | | | |

3: Calibration Curve Y=16016.225515x-601.467936

| STD | Conc. (μg/ml) | Peak area | RF | RSD(%) of RF (n=6) | Coefficient (R) | Slope | Y-intercept | 20% of STD 1 Area |
|---|---|---|---|---|---|---|---|---|
| STD-1 | 1.007454 | 15678.350061 | 15562.3 | 1.3 | 1.0000 | 16016.225515 | -601.467936 | 3136 |
| STD-2 | 6.296588 | 99672.085270 | 15829.5 | | | | | |
| STD-3 | 25.186350 | 398070.161828 | 15805.0 | | | | | |
| STD-4 | 50.372700 | 810465.147148 | 16089.4 | | | | | |
| STD-5 | 100.745400 | 1622229.048515 | 16102.3 | | | | | |
| STD-6 | 201.490800 | 3218113.506026 | 15971.5 | | | | | |
| Mean | N/A | N/A | 15893 | | | | | |
| Criteria | 1): RSD of RF NMT 10.0%, 2): Coefficient( R) NLT 0.98 | | | | | | | |
| | 3): The y-intercept should be NMT 20% of the STD-1 peak response | | | | | | | |
| Comment | Pass | | | | | | | |

4: Check STD Rec. (%)

| | Beginning | Middle | End | Criteria | |
|---|---|---|---|---|---|
| CSTD-1 | 101.8 | 101.0 | 101.0 | 90.0-110.0% | Two of the nine CSTDs failing to meet the criteria is acceptable |
| CSTD-2 | 100.5 | 99.8 | 99.1 | 98.0-102.0% | providing the two failed CSTDs are not at the same concentration |
| CSTD-3 | 101.4 | 102.6 | 101.3 | 98.0-102.0% | level |
| Comment | Pass | | | | |

In Vitro Release Rate Method Validation For Nasobol Gel 4.0%

4% Day2 Release Rate

Testosterone 4% Gel

| Concentration of Active (μg/mL) versus Time | | | | | | | | |
|---|---|---|---|---|---|---|---|---|
| | Amount Released (μg/mL) Calculation by Linear Regression Curve | | | | | | | |
| | **Cell A#2** | **Cell A#4** | **Cell A#6** | **Cell B#1** | **Cell B#3** | **Cell B#5** | **Mean 1-6** | **%RSD 1-6** |
| **Time** | | | | | | | | |
| 60.00 | 98.828 | 102.754 | 103.493 | 100.481 | 97.477 | 101.409 | 100.409 | 2.3 |
| 120.00 | 147.568 | 153.454 | 156.309 | 150.502 | 143.917 | 151.716 | 150.578 | 2.9 |
| 180.00 | 181.258 | 190.061 | 184.198 | 185.232 | 181.807 | 185.787 | 184.724 | 1.7 |
| 240.00 | 216.954 | 213.139 | 220.725 | 218.645 | 212.788 | 199.829 | 213.663 | 3.5 |
| 300.00 | 238.102 | 241.620 | 251.876 | 240.200 | 235.770 | 218.192 | 237.627 | 4.6 |
| 360.00 | 257.241 | 260.849 | 264.068 | 253.383 | 252.537 | 245.490 | 255.761 | 2.7 |

(continued)

| Actual Amount of Active Released (μg/cm²) versus $Time^{0.5}$ | | | | | | | | |
|---|---|---|---|---|---|---|---|---|
| | Amount Released (μg/cm²) | | | | | | | |
| | Cell A#2 | Cell A#4 | CellA#6 | Cell B#1 | Cell B#3 | Cell B#5 | Mean 1-6 | %RSD 1-6 |
| $Time^{0.5}$ | | | | | | | | |
| 7.75 | 671.120 | 697.781 | 702.799 | 682.345 | 661.946 | 688.647 | 684.106 | 2.3 |
| 10.95 | 1030.066 | 1071.148 | 1090.744 | 1050.458 | 1004.891 | 1058.965 | 1051.045 | 2.9 |
| 13.42 | 1300.602 | 1363.158 | 1324.360 | 1328.887 | 1302.915 | 1333.262 | 1325.531 | 1.7 |
| 15.49 | 1594.293 | 1573.653 | 1624.526 | 1607.519 | 1564.743 | 1481.186 | 1574.320 | 3.2 |
| 17.32 | 1799.291 | 1827.369 | 1898.520 | 1816.411 | 1781.017 | 1662.427 | 1797.506 | 4.3 |
| 18.97 | 1996.631 | 2033.106 | 2052.581 | 1973.898 | 1961.589 | 1909.539 | 1987.891 | 2.6 |
| Slope | 119.08 | 118.14 | 122.09 | 116.96 | 117.37 | 104.35 | 116.33 | 5.3 |
| $R^2$ | 0.9988 | 0.9989 | 0.9959 | 0.9989 | 0.9993 | 0.9939 | 0.9976 | 0.2 |

In Vitro Release Rate Method Validation For Nasobol Gel 4.0%

System Suitability for Nasobol Gel 4% Day2

1: Medium (Diluent)

| | |
|---|---|
| Result | There are no interference peaks at the RT of Testosterone from diluent injection |
| Criteria | There should be no significant interference peaks at the RT of Tesosterone from diluent |
| Comment | Pass |

2: Injection Reproducibility RT, Tailing Factor and Theoretical Plate Number from Six Replicate Injections of STD-4

| Result | RT | %RSD of RT | % RSD of Peak area | Tailing factor(T) | Theoretical Plate (N) |
|---|---|---|---|---|---|
| | 1.14 | 0.1 | 0.3 | 1.5 | 5094 |
| Criteria | NMT 2.0% | | NMT 2.0% | NMT 2 | NLT 1000 |
| Comment | Pass | | | | |

3: Calibration Curve Y=16191.343821x-559.963706

| STD | Conc. (μg/ml) | Peak area | RF | RSD(%) of RF (n=6) | Coefficient (R) | Slope | Y-intercept | 20% of STD-1 Area |
|---|---|---|---|---|---|---|---|---|
| STD-1 | 1.003470 | 15871.570115 | 15816.7 | 0.9 | 1.0000 | 16191.343821 | -559.9637 | 3174 |
| STD-2 | 6.271688 | 100682.070782 | 16053.4 | | | | | |
| STD-3 | 25.086750 | 402654.168260 | 16050.5 | | | | | |
| STD-4 | 50.173500 | 807908.024269 | 16102.3 | | | | | |
| STD-5 | 100.347000 | 1629574.944607 | 16239.4 | | | | | |
| STD-6 | 200.694000 | 3260566.943235 | 16196.6 | | | | | |
| Mean | N/A | N/A | 16076.5 | | | | | |
| Criteria | 1): RSD of RF NMT 10.0%, 2): Coefficient( R) NLT 0.98 | | | | | | | |
| | 3): The y-intercept should be NMT 20% of the STD-1 peak response | | | | | | | |
| Comment | Pass | | | | | | | |

4: Check STD Rec. (%)

| | Beginning | Middle | End | Criteria | |
|---|---|---|---|---|---|
| CSTD-1 | 99.9 | 100.5 | 99.9 | 90.0-110.0% | Two of the nine CSTDs failing to meet the criteria is acceptable providing the two failed CSTDs are not at the same concentration level |
| CSTD-2 | 99.1 | 99.6 | 99.6 | 98.0.0-102.0% | |
| CSTD-3 | 99.5 | 99.1 | 99.8 | 98.0.0-102.0% | |
| Comment | Pass | | | | |

In Vitro Release Rate Method Validation For Nasobol Gel 4.0%

4% Day3 Release Rate

Testosterone Gel 2.0%

| Amount Released (μg/mL) Calculation by Linear Regression Curve | | | | | | |
|---|---|---|---|---|---|---|
| | **Cell A#2** | **Cell A#6** | **Cell B#3** | **Cell B#4** | **Mean 1-4** | **%RSD 1-4** |
| **Time** | | | | | | |
| 60.00 | 48.687 | 51.463 | 48.815 | 48.993 | 49.490 | 2.7 |
| 120.00 | 73.695 | 78.583 | 73.975 | 75.427 | 75.420 | 3.0 |
| 180.00 | 90.962 | 97.059 | 91.673 | 93.652 | 93.337 | 2.9 |
| 240.00 | 104.753 | 111.751 | 104.737 | 107.587 | 107.207 | 3.1 |
| 300.00 | 114.343 | 121.973 | 115.610 | 118.793 | 117.680 | 2.9 |
| 360.00 | 122.380 | 131.256 | 123.836 | 127.772 | 126.311 | 3.2 |
| **Actual Amount of Active Released (μg/cm$^2$) versus Time$^{0.5}$** | | | | | | |
| | **Amount Released (μg/cm$^2$)** | | | | | |
| | **Cell A#2** | **Cell A#6** | **Cell B#3** | **Cell B#4** | **Mean 1-4** | **%RSD 1-4** |
| **Time$^{0.5}$** | | | | | | |
| 7.75 | 330.623 | 349.474 | 331.492 | 332.701 | 336.073 | 2.7 |
| 10.95 | 514.223 | 548.202 | 516.071 | 526.071 | 526.164 | 3.0 |
| 13.42 | 652.332 | 695.903 | 657.275 | 671.175 | 669.171 | 2.9 |
| 15.49 | 771.721 | 823.136 | 771.929 | 792.304 | 789.773 | 3.1 |
| 17.32 | 866.484 | 924.172 | 875.400 | 898.843 | 891.225 | 2.9 |
| 18.97 | 953.415 | 1021.723 | 963.973 | 993.430 | 983.135 | 3.1 |
| Slope | 55.61 | 59.82 | 56.40 | 58.83 | 57.66 | 3.4 |
| $R^2$ | 0.9996 | 0.9997 | 0.9999 | 0.9999 | 0.9998 | 0.0 |

In Vitro Release Rate Method Validation For Nasobol Gel 4.0%

4.0% Day3 Release Rate

Testosterone Gel 4.0%

| Amount Released (μg/mL) Calculation by Linear Regression Curve | | | | | | |
|---|---|---|---|---|---|---|
| | **Cell A#1** | **Cell A#5** | **Cell B#2** | **Cell B#6** | **Mean 1-4** | **%RSD 1-4** |
| **Time** | | | | | | |
| 60.00 | 100.960 | 101.116 | 102.665 | 95.377 | 100.030 | 3.2 |
| 120.00 | 154.364 | 154.139 | 154.940 | 147.997 | 152.860 | 2.1 |
| 180.00 | 186.594 | 184.120 | 190.461 | 181.262 | 185.609 | 2.1 |
| 240.00 | 208.156 | 214.565 | 216.224 | 209.841 | 212.197 | 1.8 |
| 300.00 | 233.787 | 235.816 | 238.251 | 229.168 | 234.256 | 1.6 |
| 360.00 | 254.064 | 253.085 | 253.819 | 240.556 | 250.381 | 2.6 |
| **Actual Amount of Active Released (μg/cm$^2$) versus Time$^{0.5}$** | | | | | | |
| | **Amount Released (μg/cm$^2$)** | | | | | |
| | **Cell A#1** | **Cell A#5** | **Cell B#2** | **Cell B#6** | **Mean 1-4** | **%RSD 1-4** |
| **Time$^{0.5}$** | | | | | | |
| 7.75 | 685.598 | 686.657 | 697.176 | 647.685 | 679.279 | 3.2 |
| 10.95 | 1076.820 | 1075.336 | 1081.214 | 1032.003 | 1066.343 | 2.2 |
| 13.42 | 1339.364 | 1322.544 | 1366.269 | 1299.774 | 1331.988 | 2.1 |
| 15.49 | 1538.584 | 1581.386 | 1595.111 | 1545.136 | 1565.054 | 1.8 |

(continued)

| Actual Amount of Active Released (μg/cm$^2$) versus Time$^{0.5}$ | | | | | | |
|---|---|---|---|---|---|---|
| | Amount Released (μg/cm$^2$) | | | | | |
| | Cell A#1 | Cell A#5 | Cell B#2 | Cell B#6 | Mean 1-4 | %RSD 1-4 |
| Time$^{0.5}$ | | | | | | |
| 17.32 | 1771.536 | 1786.408 | 1805.872 | 1735.756 | 1774.893 | 1.7 |
| 18.97 | 1975.383 | 1970.402 | 1979.004 | 1877.932 | 1950.680 | 2.5 |
| Slope | 112.89 | 114.01 | 114.24 | 110.57 | 112.93 | 1.5 |
| $R^2$ | 0.9985 | 0.9994 | 0.9996 | 0.9981 | 0.9989 | 0.1 |

In Vitro Release Rate Method Validation For Nasobol Gel 4.0%

4% Day3 Release Rate

Testosterone Gel 8.0%

| Amount Released (μg/mL) Calculation by Linear Regression Curve | | | | | | |
|---|---|---|---|---|---|---|
| | Cell A#3 | Cell A#4 | Cell B#1 | Cell B#5 | Mean 1-4 | %RSD 1-4 |
| Time | | | | | | |
| 60.00 | 194.025 | 206.537 | 196.509 | 200.268 | 199.335 | 2.7 |
| 120.00 | 291.732 | 312.939 | 296.662 | 301.987 | 300.830 | 3.0 |
| 180.00 | 366.623 | 382.831 | 355.576 | 367.135 | 368.041 | 3.0 |
| 240.00 | 420.979 | 432.753 | 416.753 | 429.097 | 424.931 | 1.7 |
| 300.00 | 462.747 | 469.037 | 459.111 | 469.069 | 464.991 | 1.1 |
| 360.00 | 500.130 | 507.265 | 493.510 | 507.861 | 502.192 | 1.2 |
| Actual Amount of Active Released (μg/cm$^2$) versus Time$^{0.5}$ | | | | | | |
| | Amount Released (μg/cm$^2$) | | | | | |
| | Cell A#3 | Cell A#4 | Cell B#1 | Cell B#5 | Mean 1-4 | %RSD 1-4 |
| Time$^{0.5}$ | | | | | | |
| 7.75 | 1317.582 | 1402.549 | 1334.451 | 1359.977 | 1353.640 | 2.7 |
| 10.95 | 2035.989 | 2183.542 | 2070.171 | 2107.395 | 2099.274 | 3.0 |
| 13.42 | 2627.103 | 2746.709 | 2554.183 | 2635.248 | 2640.811 | 3.0 |
| 15.49 | 3099.959 | 3194.041 | 3071.205 | 3159.900 | 3131.276 | 1.8 |
| 17.32 | 3502.713 | 3562.885 | 3475.838 | 3552.754 | 3523.547 | 1.2 |
| 18.97 | 3887.507 | 3955.197 | 3839.340 | 3948.905 | 3907.737 | 1.4 |
| Slope | 229.29 | 225.09 | 223.05 | 230.19 | 226.91 | 1.5 |
| $R^2$ | 0.9999 | 0.9991 | 0.9994 | 0.9997 | 0.9995 | 0.0 |

In Vitro Release Rate Method Validation For Nasobol Gel 4.0%

System Suitability for Nasobol Gel 4.0% Day3

1: Medium (Diluent)

| | |
|---|---|
| Result | There are no interference peaks at the RT of Testosterone from diluent injection |
| Criteria | There should be no significant interference peaks at the RT of Tesosterone from diluent |
| Comment | Pass |

2: Injection Reproducibility RT, Tailing Factor and Theoretical Plate Number from Six Replicate Injections of STD-4

| Result | RT | %RSD of RT | % RSD of Peak area | Tailing factor(T) | Theoretical Plate (N) |
|---|---|---|---|---|---|
| | 1.14 | 0.1 | 0.1 | 1.5 | 4972 |
| Criteria | NMT 2.0% | | NMT 2.0% | NMT 2 | NLT 1000 |
| Comment | Pass | | | | |

3: Calibration Curve Y=16400.350881x-586.919769

| STD | Conc. (μg/ml) | Peak area | RF | RSD(%) of RF (n=6) | Coefficient (R) | Slope | Y-intercept | 20% of STD-1 Area |
|---|---|---|---|---|---|---|---|---|
| STD-1 | 1.003470 | 16088.445108 | 16032.8 | 0.9 | 1.0000 | 16400.350881 | -586.91977 | 3218 |
| STD-2 | 6.271688 | 101510.699703 | 16185.6 | | | | | |
| STD-3 | 25.086750 | 409080.663218 | 16306.6 | | | | | |
| STD-4 | 50.173500 | 818519.065346 | 16313.8 | | | | | |
| STD-5 | 100.347000 | 1648916.727544 | 16432.1 | | | | | |
| STD-6 | 200.694000 | 3293160.361302 | 16408.8 | | | | | |
| Mean | N/A | N/A | 16280.0 | | | | | |
| Criteria | 1): RSD of RF NMT 10.0%, 2): Coefficient( R) NLT 0.98 | | | | | | | |
| | 3): The y-intercept should be NMT 20% of the STD-1 peak response | | | | | | | |
| Comment | Pass | | | | | | | |

4: Check STD Rec. (%)

| | Beginning | Middle | End | Criteria | |
|---|---|---|---|---|---|
| CSTD-1 | 100.6 | 98.3 | 98.7 | 90.0-110.0% | Two of the nine CSTDs failing to meet the criteria is acceptable providing the two failed CSTDs are not at the same concentration level |
| CSTD-2 | 99.4 | 98.6 | 98.1 | 98.0.0-102.0% | |
| CSTD-3 | 98.8 | 98.9 | 98.1 | 98.0.0-102.0% | |
| Comment | Pass | | | | |

In Vitro Release Rate Method Validation For Nasobol Gel 4.0%

Testosterone Gel 2% Day4 Release Rate

**Concentration of Active (μg/mL) versus time**

| | Cell A#1 | Cell A#5 | Cell B#2 | Cell B#6 | Mean 1-4 | %RSD 1-4 |
|---|---|---|---|---|---|---|
| **Time** | | | | | | |
| 60.00 | 46.114 | 45.990 | 46.957 | 48.744 | 46.951 | 2.7 |
| 120.00 | 71.036 | 70.056 | 70.143 | 74.792 | 71.507 | 3.1 |
| 180.00 | 86.556 | 87.932 | 89.263 | 87.432 | 87.796 | 1.3 |
| 240.00 | 101.793 | 102.943 | 101.472 | 102.594 | 102.201 | 0.7 |
| 300.00 | 114.336 | 115.560 | 116.237 | 116.744 | 115.719 | 0.9 |

(continued)

| | Cell A#1 | Cell A#5 | Cell B#2 | Cell B#6 | Mean 1-4 | %RSD 1-4 |
|---|---|---|---|---|---|---|
| Time | | | | | | |
| 360.00 | 124.001 | 124.783 | 128.162 | 124.530 | 125.369 | 1.5 |
| Actual Amount of Active Released (μg/cm²) versus $Time^{0.5}$ | | | | | | |
| | Amount Released (μg/cm²) | | | | | |
| | Cell A#1 | Cell A#5 | Cell B#2 | Cell B#6 | Mean 1-4 | %RSD 1-4 |
| $Time^{0.5}$ | | | | | | |
| 7.75 | 313.150 | 312.308 | 318.875 | 331.010 | 318.836 | 2.7 |
| 10.95 | 495.438 | 488.748 | 489.613 | 521.689 | 498.872 | 3.1 |
| 13.42 | 620.931 | 629.963 | 639.299 | 628.68 | 629.720 | 1.2 |
| 15.49 | 748.893 | 756.779 | 747.465 | 756.387 | 752.381 | 0.6 |
| 17.32 | 862.872 | 871.586 | 876.442 | 881.506 | 873.102 | 0.9 |
| 18.97 | 960.856 | 966.916 | 990.312 | 967.412 | 971.374 | 1.3 |
| Slope | 57.62 | 58.68 | 59.48 | 56.59 | 58.09 | 2.2 |
| $R^2$ | 0.9990 | 0.9994 | 0.9971 | 0.9971 | 0.9982 | 0.1 |

In Vitro Release Rate Method Validation For Nasobol Gel 4.0%

Testosterone Gel 4% Day4 Release Rate

**Concentration of Active (μg/mL) versus time**

| | Cell A#3 | Cell A#4 | Cell B#1 | Cell B#5 | Mean 1-4 | %RSD 1-4 |
|---|---|---|---|---|---|---|
| Time | | | | | | |
| 60.00 | 97.536 | 101.702 | 92.098 | 101.777 | 98.278 | 4.7 |
| 120.00 | 139.740 | 145.279 | 136.327 | 149.357 | 142.676 | 4.1 |
| 180.00 | 174.342 | 184.915 | 173.585 | 185.982 | 179.706 | 3.7 |
| 240.00 | 202.707 | 208.453 | 197.116 | 207.509 | 203.946 | 2.6 |
| 300.00 | 240.715 | 238.723 | 227.099 | 238.562 | 236.275 | 2.6 |
| 360.00 | 252.426 | 248.202 | 226.276 | 250.303 | 244.302 | 5.0 |
| Actual Amount of Active Released (μg/cm²) versus $Time^{0.5}$ | | | | | | |
| | Amount Released (μg/cm²) | | | | | |
| | Cell A#3 | Cell A#4 | Cell B#1 | Cell B#4 | Mean 1-4 | %RSD 1-4 |
| $Time^{0.5}$ | | | | | | |
| 7.75 | 662.346 | 690.637 | 625.418 | 691.146 | 667.387 | 4.7 |
| 10.95 | 976.542 | 1015.335 | 951.827 | 1043.049 | 996.688 | 4.1 |
| 13.42 | 1251.057 | 1325.602 | 1243.412 | 1334.022 | 1288.523 | 3.7 |
| 15.49 | 1493.007 | 1537.765 | 1452.321 | 1532.831 | 1503.981 | 2.6 |
| 17.32 | 1808.467 | 1802.303 | 1711.703 | 1802.420 | 1781.224 | 2.6 |
| 18.97 | 1956.104 | 1934.220 | 1770.372 | 1949.652 | 1902.587 | 4.7 |
| Slope | 118.16 | 113.57 | 106.62 | 113.31 | 112.92 | 4.2 |
| $R^2$ | 0.9927 | 0.9973 | 0.9925 | 0.9981 | 0.9952 | 0.3 |

In Vitro Release Rate Method Validation For Nasobol Gel 4.0%

Testosterone Gel 8% Day4 Release Rate
Testosterone Gel 8%

| Amount Released (μg/mL) Calculation by Linear Regression Curve | | | | | | |
|---|---|---|---|---|---|---|
| | **Cell A#2** | Cell A#6 | Cell B#3 | Cell B#4 | Mean 1-4 | %RSD 1-4 |
| **Time** | | | | | | |
| 60.00 | 225.146 | 222.059 | 224.685 | 222.320 | 223.553 | 0.7 |
| 120.00 | 317.633 | 309.500 | 316.071 | 313.279 | 314.121 | 1.1 |
| 180.00 | 370.322 | 367.146 | 370.947 | 369.411 | 369.457 | 0.5 |
| 240.00 | 418.459 | 415.671 | 421.783 | 418.698 | 418.653 | 0.6 |
| 300.00 | 480.398 | 469.438 | 476.809 | 478.279 | 476.231 | 1.0 |
| 360.00 | 494.787 | 483.727 | 487.877 | 490.443 | 489.209 | 0.9 |
| **Actual Amount of Active Released (μg/cm$^2$) versus Time$^{0.5}$** | | | | | | |
| Amount Released (μg/cm$^2$) | | | | | | |
| | Cell A#2 | Cell A#6 | Cell B#3 | Cell B#4 | Mean 1-4 | %RSD 1-4 |
| **Time$^{0.5}$** | | | | | | |
| 7.75 | 1528.919 | 1507.955 | 1525.788 | 1509.728 | 1518.097 | 0.7 |
| 10.95 | 2220.683 | 2164.580 | 2209.945 | 2190.316 | 2196.381 | 1.1 |
| 13.42 | 2668.357 | 2643.615 | 2672.029 | 2660.139 | 2661.035 | 0.5 |
| 15.49 | 3100.027 | 3077.021 | 3122.204 | 3099.361 | 3099.654 | 0.6 |
| 17.32 | 3639.045 | 3559.756 | 3615.218 | 3622.433 | 3609.113 | 1.0 |
| 18.97 | 3872.686 | 3789.616 | 3825.291 | 3840.365 | 3831.990 | 0.9 |
| Slope | 211.76 | 207.16 | 209.08 | 211.75 | 209.94 | 1.1 |
| $R^2$ | 0.9959 | 0.9976 | 0.9968 | 0.9964 | 0.9967 | 0.1 |

In Vitro Release Rate Method Validation For Nasobol Gel 4.0%

System Suitability

**1: Medium (Diluent)**

| | |
|---|---|
| Result | There are no interference peaks at the RT of Testosterone from diluent injection |
| Criteria | There should be no significant interference peaks at the RT of Testosterone from diluent |
| Comment | Pass |

**2.Injection reproducibility RT, Tailing Factor and Theoretical Plate Number from Six Replica Injections of STD-1**

| Result | RT | %RSD of RT | % RSD of Peak area | Tailing Factor (T) | Theoretical Plate (N) |
|---|---|---|---|---|---|
| | 1.4 | 0.0 | 0.2 | 1.5 | 4952 |
| Criteria | Report | NMT 2.0% | NMT 2.0% | NMT 2 | NLT 1000 |
| Comment | | Pass | | | |

**3. Calibration Curve Y=16123.2297X - 239.5651**

| STD | Conc, μg/ml | Peak Area | RF | RSD (%) of RF (n=6) | Co-efficient (R) | Slope | Y-intercept | 20% of STD-1 area |
|---|---|---|---|---|---|---|---|---|
| STD 1 | 1.0050 | 15966.5100 | 15887.1 | 0.8 | 1.0000 | 16123.227 | -239.5651 | 3193 |
| STD 2 | 6.2810 | 100144.6504 | 15944.1 | | | | | |
| STD 3 | 25.1241 | 405793.1322 | 16151.5 | | | | | |
| STD 4 | 50.2482 | 815888.1322 | 16237.2 | | | | | |
| STD 5 | 100.4964 | 1622551.1592 | 16079.4 | | | | | |
| STD 6 | 200.9928 | 3231917.0302 | 16079.8 | | | | | |
| Mean | N/A | N/A | 16074 | | | | | |
| Criteria | 1): RSD of RF NMT 10.0% | | | | 2): Coefficient R)NLT 0.98 | | | |
| | 3): The y intercept should be NMT 20% of the STD-1 peak response | | | | | | | |
| Comment | Pass | | | | | | | |

**4: Check STD Rec.** (%)

| | Beginning | Middle | End | | Criteria |
|---|---|---|---|---|---|
| CSTD-1 | 100.6 | 100.4 | 99.2 | 90.0-110.0% | Two of the nine CSTDs failing to meet the criteria is acceptable providing the two failed CSTDs are not of the same concentration level |
| CSTD-2 | 100.2 | 100.7 | 99.9 | 98.0-102.0% | |
| CSTD-3 | 100.1 | 100.9 | 100.0 | 98.0-102.0% | |
| Comment | Pass | | | | |

In Vitro Release Rate Method Validation For Nasobol Gel 4.5%

**NASOBOL GEL IN ViTRO RELEASE RATE VALIDATION UPDATE**

**Release Rate Study Summary**

**Part 4: Nasobol Gel 4.5%**

**Purpose**

This summary summarized all release rate experiment data for Nasobol Gels There are four Nasobol Gels (0.15%, 0.6%, 4.0% and 4.5%) for the method validation.

The purpose of the Day1 and Day2 test are to determine the specificity and intraday/interday precision of the slope(release rate), Day3 and Day4 are to evaluate the slope sensitivity to the sample strength variation.

**Exhibit B**

In Vitro Release Rate Method Validation For Nasobol Gel 4.5%

Nasobol Gel 4.5% Release Rate

| Actual Amount of Active Released ($\mu g/cm^2$) versus $Time^{0.5}$ | | | | | | | | | |
|---|---|---|---|---|---|---|---|---|---|
| Time (minutes) | $Time^{0.5}$ | Day1 Mean(n=6) 4.5% Testosterone Gel | Day2 Mean(n=6) 4.5% Testosterone Gel | Day3 Mean(n=4) 2.25% Testosterone Gel | Day3 Mean(n=4) 4.5% Testosterone Gel | Day3 Mean(n=4) 9.0% Testosterone Gel | Day4 Mean(n=4) 2.25% Testosterone Gel | Day4 Mean(n=4) 4.5% Testosterone Gel | Day4 Mean(n=4) 9.0% Testosterone Gel |
| 60.00 | 7.75 | 773.048 | 754.566 | 375.945 | 756.458 | 1504.963 | 373.524 | 771.827 | 1475.93 |
| 120.00 | 10.95 | 1201.776 | 1171.379 | 581.446 | 1169.142 | 2330.18 | 574.515 | 1208.152 | 2323.68 |
| 180.00 | 13.42 | 1522.374 | 1502.618 | 745.099 | 1480.649 | 2941.464 | 738.392 | 1539.298 | 2985.825 |
| 240.00 | 15.49 | 1801.026 | 1770.356 | 873.015 | 1735.057 | 3424.621 | 877.604 | 1828.254 | 3531.463 |
| 300.00 | 17.32 | 2024.528 | 2025.728 | 987.164 | 1958.076 | 3898.337 | 1000.244 | 2075.853 | 4017.858 |
| 360.00 | 18.97 | 2254.199 | 2260.112 | 1101.569 | 2194.036 | 4346.992 | 1102.393 | 2305.255 | 4463.589 |
| Slope | n/a | 131.44 | 133.81 | 64.35 | 126.88 | 250.89 | 65.44 | 136.58 | 266.09 |
| $R^2$ | n/a | 0.9997 | 0.9996 | 0.9998 | 0.9996 | 0.9995 | 0.9997 | 0.9999 | 1.0000 |

In Vitro Release Rate Method Validation For Nasobol Gel 4.5%

4.5% Day1 Release Rate
Testosterone 4.5% Gel

**Concentration of Active ($\mu$g/mL) versus time**

| Amount Released ($\mu$g/mL) Calculation by Linear Regression Curve | | | | | | | | |
|---|---|---|---|---|---|---|---|---|
| | **Cell A#1** | **Cell A#2** | **Cell A#3** | **Cell A#4** | **Cell #5** | **Cell #6** | **Mean 1-6** | **%RSD 1-6** |
| **Time** | | | | | | | | |
| 60.00 | 111.296 | 111.319 | 113.485 | 115.937 | 112.821 | 118.159 | 113.838 | 2.4 |
| 120.00 | 169.171 | 167.895 | 170.642 | 175.455 | 172.935 | 177.272 | 172.228 | 2.1 |
| 180.00 | 212.183 | 206.080 | 211.257 | 213.547 | 211.031 | 219.031 | 212.261 | 2.0 |

(continued)

| Amount Released (μg/mL) Calculation by Linear Regression Curve | | | | | | | | |
|---|---|---|---|---|---|---|---|---|
| | Cell A#1 | Cell A#2 | Cell A#3 | Cell A#4 | Cell #5 | Cell #6 | Mean 1-6 | %RSD 1-6 |
| 240.00 | 243.968 | 240.483 | 243.556 | 243.442 | 212.788 | 249.362 | 244.452 | 1.2 |
| 300.00 | 265.616 | 264.479 | 266.919 | 269.161 | 264.241 | 272.660 | 267.179 | 1.2 |
| 360.00 | 289.376 | 284.952 | 288.553 | 292.152 | 287.059 | 297.115 | 289.868 | 1.5 |
| **Actual Amount of Active Released (μg/cm$^2$) versus Time$^{0.5}$** | | | | | | | | |
| **Amount Released (μg/cm$^2$)** | | | | | | | | |
| | Cell A#1 | Cell A#2 | CellA#3 | Cell A#4 | Cell #5 | Cell #6 | Mean 1-6 | %RSD 1-6 |
| **Time$^{0.5}$** | | | | | | | | |
| 7.75 | 755.787 | 755.944 | 770.652 | 787.303 | 766.211 | 802.393 | 773.048 | 2.4 |
| 10.95 | 1180.295 | 1171.637 | 1190.904 | 1224.282 | 1206.290 | 1237.249 | 1201.776 | 2.1 |
| 13.42 | 1520.248 | 1478.449 | 1514.995 | 1532.601 | 1513.924 | 1574.027 | 1522.374 | 2.0 |
| 15.49 | 1796.130 | 1770.383 | 1794.106 | 1812.747 | 1793.731 | 1839.058 | 1801.026 | 1.3 |
| 17.32 | 2012.167 | 2001.379 | 2021.673 | 2040.265 | 2003.855 | 2067.826 | 2024.528 | 1.3 |
| 18.97 | 2248.672 | 2215.241 | 2244.109 | 2272.551 | 2233.574 | 2311.044 | 2254.199 | 1.5 |
| Slope | 132.51 | 130.29 | 131.17 | 131.37 | 129.66 | 133.29 | 131.38 | 1.0 |
| $R^2$ | 0.9997 | 0.9998 | 0.9999 | 0.9998 | 0.9995 | 0.9997 | 0.9998 | 0.0 |

In Vitro Release Rate Method Validation For Nasobol Gel 4.5%

System Suitability for 4% Gel Day1

1: Medium (Diluent)

| | |
|---|---|
| Result | There are no interference peaks at the RT of Testosterone from diluent injection |
| Criteria | There should be no significant interference peaks at the RT of Tesosterone from diluent |
| Comment | Pass |

2: Injection Reproducibility RT, Tailing Factor and Theoretical Plate Number from Six Replicate Injections of STD-4

| | RT | %RSD of RT | % RSD of Peak area | Tailing factor(T) | Theoretical Plate (N) |
|---|---|---|---|---|---|
| Result | 1.15 | 0 | 0.3 | 1.5 | 5099 |
| Criteria | NMT 2.0% | | NMT 2.0% | NMT 2 | NLT 1000 |
| Comment | Pass | | | | |

3: Calibration Curve Y=16214.013222x-404.968835

| STD | Conc. (μg/ml) | Peak area | RF | RSD(%) of RF (n=6) | Coefficient (R) | Slope | Y-intercept | 20% of STD 1 Area |
|---|---|---|---|---|---|---|---|---|
| STD-1 | 1.005000 | 16020.780118 | 15941.1 | 0.9 | 1.0000 | 16214.013222 | -404.968835 | 3204 |
| STD-2 | 6.281000 | 100148.430437 | 15944.7 | | | | | |
| STD-3 | 25.124100 | 407824.982776 | 16232.4 | | | | | |
| STD-4 | 50.248200 | 815077.609088 | 16221.0 | | | | | |
| STD-5 | 100.496400 | 1634589.943207 | 16265.2 | | | | | |
| STD-6 | 200.992800 | 3252481.085742 | 16182.1 | | | | | |
| Mean | N/A | N/A | 16131 | | | | | |
| Criteria | 1): RSD of RF NMT 10.0%, 2): Coefficient( R) NLT 0.98<br>3): The y-intercept should be NMT 20% of the STD-1 peak response | | | | | | | |
| Comment | Pass | | | | | | | |

4: Check STD Rec. (%)

| | Beginning | Middle | End | Criteria | |
|---|---|---|---|---|---|
| CSTD-1 | 100.1 | 99.9 | 98.6 | 90.0-110.0% | Two of the nine CSTDs failing to meet the criteria is acceptable providing the two failed CSTDs are not at the same concentration level |
| CSTD-2 | 100.6 | 99.7 | 99.0 | 98.0.0-102.0% | |
| CSTD-3 | 100.5 | 100.2 | 98.7 | 98.0.0-102.0% | |
| Comment | Pass | | | | |

In Vitro Release Rate Method Validation For Nasobol Gel 4.5%

4.5% Day2 Release Rate
Testosterone 4.5% Gel

**Concentration of Active (μg/mL versus time**

| Amount Released (μg/mL) Calculation by Linear Regression Curve | | | | | | | | |
|---|---|---|---|---|---|---|---|---|
| | **Cell A#2** | **Cell A#4** | **Cell A#6** | **Cell B#1** | **Cell B#3** | **Cell B#5** | **Mean 1-6** | **%RSD 1-6** |
| **Time** | | | | | | | | |
| 60.00 | 108.443 | 114.787 | 116.970 | 110.229 | 103.462 | 112.806 | 111.116 | 4.4 |
| 120.00 | 165.786 | 171.091 | 175.955 | 165.082 | 160.313 | 168.966 | 167.866 | 3.2 |
| 180.00 | 205.096 | 212.317 | 217.530 | 208.624 | 199.765 | 214.561 | 209.649 | 3.1 |
| 240.00 | 236.056 | 241.544 | 254.045 | 236.272 | 228.447 | 245.676 | 240.340 | 3.7 |

(continued)

| Amount Released (μg/mL) Calculation by Linear Regression Curve | | | | | | | | |
|---|---|---|---|---|---|---|---|---|
| | **Cell A#2** | **Cell A#4** | **Cell A#6** | **Cell B#1** | **Cell B#3** | **Cell B#5** | **Mean 1-6** | **%RSD 1-6** |
| 300.00 | 262.962 | 267.630 | 280.500 | 266.963 | 255.981 | 273.553 | 267.932 | 3.2 |
| 360.00 | 284.142 | 294.602 | 303.353 | 289.922 | 277.465 | 298.212 | 291.283 | 3.3 |
| **Actual Amount of Active Released (μg/cm2) versus $Time^{0.5}$** | | | | | | | | |
| | | | | **Amount Released (μg/$cm^2$)** | | | | |
| | **Cell A#2** | **Cell A#4** | **CellA#6** | **Cell B#1** | **Cell B#3** | **Cell B#5** | **Mean 1-6** | **%RSD 1-6** |
| **$Time^{0.5}$** | | | | | | | | |
| 7.75 | 736.413 | 779.494 | 794.318 | 748.542 | 702.588 | 766.042 | 754.566 | 4.4 |
| 10.95 | 1156.501 | 1194.321 | 1227.970 | 1152.226 | 1117.926 | 1179.331 | 1171.379 | 3.2 |
| 13.42 | 1470.356 | 1522.689 | 1560.083 | 1494.620 | 1431.197 | 1536.765 | 1502.618 | 3.1 |
| 15.49 | 1738.631 | 1781.238 | 1869.598 | 1741.402 | 1682.493 | 1808.771 | 1770.356 | 3.7 |
| 17.32 | 1988.136 | 2026.727 | 2121.131 | 2016.671 | 1934.110 | 2067.591 | 2025.728 | 3.2 |
| 18.97 | 2206.369 | 2285.614 | 2355.688 | 2248.118 | 2152.433 | 2312.447 | 2260.111 | 3.3 |
| Slope | 130.70 | 132.72 | 139.44 | 133.37 | 128.58 | 137.72 | 131.38 | 3.1 |
| $R^2$ | 0.9999 | 0.9992 | 0.9998 | 0.9992 | 0.9998 | 0.9996 | 0.9996 | 0.0 |

In Vitro Release Rate Method Validation For Nasobol Gel 4.5%

System Suitability for Nasobol Gel 4.5% Day2

1: Medium (Diluent)

| | |
|---|---|
| Result | There are no interference peaks at the RT of Testosterone from diluent injection |
| Criteria | There should be no significant interference peaks at the RT of Tesosterone from diluent |
| Comment | Pass |

2: Injection Reproducibility RT, Tailing Factor and Theoretical Plate Number from Six Replicate Injections of STD-4

| Result | RT | %RSD of RT | % RSD of Peak area | Tailing factor(T) | Theoretical Plate (N) |
|---|---|---|---|---|---|
| | 1.16 | 0.1 | 0.4 | 1.4 | 5112 |
| Criteria | NMT 2.0% | | NMT 2.0% | NMT 2 | NLT 1000 |
| Comment | Pass | | | | |

3: Calibration Curve Y=16354.946833x-532.850889

| STD | Conc. (µg/ml) | Peak area | RF | RSD(%) of RF (n=6) | Coefficient (R) | Slope | Y-intercept | 20% of STD 1 Area |
|---|---|---|---|---|---|---|---|---|
| STD-1 | 1.005000 | 16235.695068 | 16154.9 | 0.8 | 1.0000 | 16354.946833 | -532.8509 | 3247 |
| STD-2 | 6.281000 | 101006.460064 | 16081.3 | | | | | |
| STD-3 | 25.124100 | 409182.516272 | 16286.5 | | | | | |
| STD-4 | 50.248200 | 819359.918359 | 16306.3 | | | | | |
| STD-5 | 100.496400 | 1627876.594574 | 16198.4 | | | | | |
| STD-6 | 200.992800 | 3305853.648774 | 16447.6 | | | | | |
| Mean | N/A | N/A | 16245.8 | | | | | |
| Criteria | 1): RSD of RF NMT 10.0%, 2): Coefficient( R) NLT 0.98 | | | | | | | |
| | 3): The y-intercept should be NMT 20% of the STD-1 peak response | | | | | | | |
| Comment | Pass | | | | | | | |

4: Check STD Rec. (%)

| | Beginning | Middle | End | Criteria | |
|---|---|---|---|---|---|
| CSTD-1 | 100.0 | 99.9 | 100.2 | 90.0-110.0% | Two of the nine CSTDs failing to meet the criteria is acceptable providing the two failed CSTDs are not at the same concentration level |
| CSTD-2 | 99.1 | 99.3 | 98.9 | 98.0.0-102.0% | |
| CSTD-3 | 100.7 | 99.9 | 99.5 | 98.0.0-102.0% | |
| Comment | Pass | | | | |

In Vitro Release Rate Method Validation For Nasobol Gel 4.5%

4.5% Day3 Release Rate

Testosterone Gel 2.25%

**Concentration of Active (µg/mL versus time**

| | Amount Released (µg/mL) Calculation by Linear Regression Curve | | | | | |
|---|---|---|---|---|---|---|
| | **Cell A#2** | **Cell A#6** | **Cell B#3** | **Cell B#4** | **Mean 1-4** | **%RSD 1-4** |
| **Time** | | | | | | |
| 60.00 | 54.994 | 57.714 | 54.019 | 54.717 | 55.361 | 2.9 |
| 120.00 | 82.496 | 86.872 | 80.964 | 82.932 | 83.316 | 3.0 |
| 180.00 | 102.145 | 108.350 | 101.811 | 103.469 | 103.944 | 2.9 |
| 240.00 | 116.492 | 123.255 | 116.098 | 117.953 | 118.450 | 2.8 |
| 300.00 | 127.040 | 135.982 | 128.371 | 129.901 | 130.324 | 3.0 |

(continued)

| Amount Released ($\mu$g/mL) Calculation by Linear Regression Curve | | | | | | |
|---|---|---|---|---|---|---|
| | Cell A#2 | Cell A#6 | Cell B#3 | Cell B#4 | Mean 1-4 | %RSD 1-4 |
| 360.00 | 137.604 | 148.125 | 139.169 | 142.064 | 141.741 | 3.3 |
| Actual Amount of Active Released ($\mu$g/cm$^2$) versus Time$^{0.5}$ | | | | | | |
| Amount Released ($\mu$g/cm$^2$) | | | | | | |
| | Cell A#2 | Cell A#6 | Cell B#3 | Cell B#4 | Mean 1-4 | %RSD 1-4 |
| Time$^{0.5}$ | | | | | | |
| 7.75 | 373.453 | 391.923 | 366.832 | 371.572 | 375.945 | 2.9 |
| 10.95 | 575.773 | 606.259 | 565.094 | 578.656 | 581.446 | 3.0 |
| 13.42 | 732.548 | 776.692 | 729.570 | 741.584 | 745.098 | 2.9 |
| 15.49 | 858.877 | 908.567 | 855.398 | 869.218 | 873.015 | 2.8 |
| 17.32 | 963.468 | 1029.868 | 971.591 | 983.729 | 987.164 | 3.0 |
| 18.97 | 1071.151 | 1150.804 | 1081.240 | 1103.081 | 1101.569 | 3.2 |
| Slope | 61.87 | 67.19 | 63.58 | 64.65 | 64.32 | 3.5 |
| $R^2$ | 0.9998 | 0.9998 | 0.9999 | 0.9997 | 0.9998 | 0.0 |

In Vitro Release Rate Method Validation For Nasobol Gel 4.5%

4.5% Day3 Release Rate
Testosterone Gel 4.5%

**Concentration of Active ($\mu$g/mL versus time**

| Amount Released ($\mu$g/mL) Calculation by Linear Regression Curve | | | | | | |
|---|---|---|---|---|---|---|
| | Cell A#1 | Cell A#5 | Cell B#2 | Cell B#6 | Mean 1-4 | %RSD 1-4 |
| Time | | | | | | |
| 60.00 | 114.924 | 110.831 | 111.525 | 108.299 | 111.395 | 2.5 |
| 120.00 | 170.368 | 168.304 | 167.373 | 164.053 | 167.525 | 1.6 |
| 180.00 | 208.336 | 206.437 | 208.201 | 202.691 | 206.416 | 1.3 |
| 240.00 | 239.862 | 235.028 | 233.826 | 232.401 | 235.279 | 1.4 |
| 300.00 | 261.230 | 258.500 | 258.483 | 255.056 | 258.317 | 1.0 |
| 360.00 | 287.153 | 282.227 | 279.233 | 280.592 | 282.301 | 1.2 |
| Actual Amount of Active Released ($\mu$g/cm$^2$) versus Time$^{0.5}$ | | | | | | |
| Amount Released ($\mu$g/cm$^2$) | | | | | | |
| | Cell A#1 | Cell A#5 | Cell B#2 | Cell B#6 | Mean 1-4 | %RSD 1-4 |
| Time$^{0.5}$ | | | | | | |
| 7.75 | 780.424 | 752.630 | 757.343 | 735.435 | 756.458 | 2.5 |
| 10.95 | 1189.451 | 1174.276 | 1168.150 | 1144.692 | 1169.142 | 1.6 |
| 13.42 | 1495.489 | 1480.851 | 1492.763 | 1453.493 | 1480.649 | 1.3 |
| 15.49 | 1768.524 | 1733.417 | 1725.687 | 1712.599 | 1735.057 | 1.4 |
| 17.32 | 1981.498 | 1959.312 | 1959.288 | 1932.202 | 1958.075 | 1.0 |
| 18.97 | 2231.450 | 2193.579 | 2173.335 | 2177.779 | 2194.036 | 1.2 |
| Slope | 127.93 | 126.91 | 125.41 | 127.04 | 126.82 | 0.8 |
| $R^2$ | 0.9994 | 0.9997 | 0.9996 | 0.9995 | 0.9996 | 0.0 |

In Vitro Release Rate Method Validation For Nasobol Gel 4.5%

4.5% Day3 Release Rate
Testosterone Gel 9.0%

**Concentration of Active (μg/mL versus time**

| **Amount Released (μg/mL) Calculation by Linear Regression Curve** | | | | | | |
|---|---|---|---|---|---|---|
| | Cell A#3 | **Cell A#4** | **Cell B#1** | **Cell B#5** | **Mean 1-4** | **%RSD 1-4** |
| **Time** | | | | | | |
| 60.00 | 216.929 | 227.909 | 217.968 | 223.670 | 221.619 | 2.3 |
| 120.00 | 328.565 | 344.343 | 328.394 | 334.315 | 333.904 | 2.2 |
| 180.00 | 405.724 | 411.232 | 407.217 | 415.860 | 410.008 | 1.1 |
| 240.00 | 461.177 | 470.177 | 455.528 | 469.412 | 464.074 | 1.5 |
| 300.00 | 508.616 | 517.603 | 508.752 | 523.012 | 514.496 | 1.4 |
| 360.00 | 556.120 | 561.780 | 553.943 | 564.663 | 559.127 | 0.9 |
| **Actual Amount of Active Released (μg/cm$^2$) versus Time$^{0.5}$** | | | | | | |
| **Amount Released (μg/cm$^2$)** | | | | | | |
| | **Cell A#3** | **Cell A#4** | **Cell B#1** | **Cell B#5** | **Mean 1-4** | **%RSD 1-4** |
| **Time$^{0.5}$** | | | | | | |
| 7.75 | 1473.119 | 1547.682 | 1480.174 | 1581.895 | 1504.967 | 2.3 |
| 10.95 | 2292.595 | 2402.847 | 2291.728 | 2333.549 | 2330.180 | 2.2 |
| 13.42 | 2909.533 | 2954.507 | 2919.917 | 2981.898 | 2941.464 | 1.1 |
| 15.49 | 3400.901 | 3471.148 | 3363.208 | 3463.226 | 3424.621 | 1.5 |
| 17.32 | 3853.539 | 3926.244 | 3853.533 | 3960.032 | 3898.337 | 1.4 |
| 18.97 | 4320.042 | 4372.697 | 4304.366 | 4390.861 | 4346.991 | 1.0 |
| Slope | 250.91 | 248.45 | 248.81 | 254.97 | 250.79 | 1.2 |
| $R^2$ | 0.9996 | 0.9994 | 0.9993 | 0.9998 | 0.9995 | 0.0 |

In Vitro Release Rate Method Validation For Nasobol Gel 4.5%

System Suitability for Nasobol Gel 4.5% Day3

1: Medium (Diluent)

| | |
|---|---|
| Result | There are no interference peaks at the RT of Testosterone from diluent injection |
| Criteria | There should be no significant interference peaks at the RT of Tesosterone from diluent |
| Comment | Pass |

2: Injection Reproducibility RT, Tailing Factor and Theoretical Plate Number from Six Replicate Injections of STD-4

| | | | | | |
|---|---|---|---|---|---|
| Result | RT | %RSD of RT | % RSD of Peak area | Tailing factor(T) | Theoretical Plate (N) |
| | 1.17 | 0.1 | 0.5 | 1.4 | 5248 |
| Criteria | NMT 2.0% | | NMT 2.0% | NMT 2 | NLT 1000 |
| Comment | Pass | | | | |

3: Calibration Curve Y=16446.511438x-909.542212

| STD | Conc. (µg/ml) | Peak area | RF | RSD(%) of RF (n=6) | Coefficient (R) | Slope | Y-intercept | 20% of STD-1 Area |
|---|---|---|---|---|---|---|---|---|
| STD-1 | 1.005000 | 15991.845134 | 15912.3 | 1.4 | 1.0000 | 16446.511438 | -909.542212 | 3198 |
| STD-2 | 6.281000 | 100802.069561 | 16048.7 | | | | | |
| STD-3 | 25.124100 | 410316.947514 | 16331.6 | | | | | |
| STD-4 | 50.248200 | 820151.545344 | 16322.0 | | | | | |
| STD-5 | 100.496400 | 1656889.901975 | 16487.1 | | | | | |
| STD-6 | 200.992800 | 3308276.689704 | 16459.7 | | | | | |
| Mean | N/A | N/A | 16260 | | | | | |
| Criteria | 1): RSD of RF NMT 10.0%, 2): Coefficient( R) NLT 0.98 | | | | | | | |
| | 3): The y-intercept should be NMT 20% of the STD-1 peak response | | | | | | | |
| Comment | Pass | | | | | | | |

4: Check STD Rec. (%)

| | Beginning | Middle | End | Criteria | |
|---|---|---|---|---|---|
| CSTD-1 | 101.5 | 100.5 | 100.3 | 90.0-110.0% | Two of the nine CSTDs failing to meet the criteria is acceptable providing the two failed CSTDs are not at the same concentration level |
| CSTD-2 | 99.5 | 98.3 | 97.7 | 98.0-102.0% | |
| CSTD-3 | 100.3 | 99.5 | 98.9 | 98.0-102.0% | |
| Comment | Pass | | | | |

In Vitro Release Rate Method Validation For Nasobol Gel 4.5%

4.5% Day4 Release Rate
Testosterone Gel 2.25%

**Concentration of Active (µg/mL versus Time**

| | Amount Released (µg/mL) Calculation by Linear Regression Curve | | | | | |
|---|---|---|---|---|---|---|
| | **Cell A#1** | **Cell A#5** | **Cell B#2** | **Cell B#6** | **Mean 1-4** | **%RSD 1-4** |
| **Time** | | | | | | |
| 60.00 | 55.058 | 54.174 | 55.844 | 54.942 | 55.005 | 1.2 |
| 120.00 | 82.450 | 81.172 | 83.609 | 82.010 | 82.310 | 1.2 |
| 180.00 | 103.031 | 102.307 | 103.860 | 102.854 | 103.013 | 0.6 |
| 240.00 | 119.731 | 117.894 | 120.468 | 118.790 | 119.221 | 0.9 |
| 300.00 | 132.888 | 130.313 | 134.287 | 131.764 | 132.313 | 1.3 |

(continued)

| Amount Released ($\mu$g/mL) Calculation by Linear Regression Curve | | | | | | |
|---|---|---|---|---|---|---|
| | Cell A#1 | Cell A#5 | Cell B#2 | Cell B#6 | Mean 1-4 | %RSD 1-4 |
| 360.00 | 143.479 | 138.721 | 143.770 | 141.399 | 141.842 | 1.6 |
| Actual Amount of Active Released ($\mu g/cm^2$) versus $Time^{0.5}$ | | | | | | |
| Amount Released ($\mu g/cm^2$) | | | | | | |
| | Cell A#1 | Cell A#5 | Cell B#2 | Cell B#6 | Mean 1-4 | %RSD 1-4 |
| $Time^{0.5}$ | | | | | | |
| 7.75 | 373.887 | 367.884 | 379.225 | 373.099 | 373.524 | 1.2 |
| 10.95 | 575.479 | 566.550 | 583.572 | 572.458 | 574.515 | 1.2 |
| 13.42 | 738.569 | 733.041 | 744.749 | 737.210 | 738.392 | 0.7 |
| 15.49 | 881.128 | 867.837 | 886.918 | 874.531 | 877.603 | 0.9 |
| 17.32 | 1004.352 | 985.530 | 1014.846 | 996.246 | 1000.243 | 1.2 |
| 18.97 | 1113.874 | 1079.499 | 1117.240 | 1098.958 | 1102.392 | 1.6 |
| Slope | 66.23 | 248.45 | 66.22 | 65.12 | 250.79 | 1.6 |
| $R^2$ | 0.9998 | 0.9994 | 0.9997 | 0.9998 | 0.9995 | 0.0 |

In Vitro Release Rate Method Validation For Nasobol Gel 4.5%

4.5% Day4 Release Rate
Testosterone Gel 4.5%

**Concentration of Active ($\mu$g/mL versus Time**

| Amount Released ($\mu$g/mL) Calculation by Linear Regression Curve | | | | | | |
|---|---|---|---|---|---|---|
| | Cell A#3 | Cell A#4 | Cell B#1 | Cell B#5 | Mean 1-4 | %RSD 1-4 |
| Time | | | | | | |
| 60.00 | 112.124 | 114.480 | 112.222 | 115.806 | 113.658 | 1.6 |
| 120.00 | 170.342 | 175.929 | 170.587 | 175.841 | 173.175 | 1.8 |
| 180.00 | 211.969 | 215.483 | 212.348 | 219.092 | 214.723 | 1.5 |
| 240.00 | 245.587 | 250.181 | 244.691 | 252.851 | 248.328 | 1.6 |
| 300.00 | 268.986 | 276.034 | 270.206 | 282.540 | 274.442 | 2.3 |
| 360.00 | 290.134 | 298.167 | 293.683 | 305.212 | 296.788 | 2.2 |
| Actual Amount of Active Released ($\mu g/cm^2$) versus $Time^{0.5}$ | | | | | | |
| Amount released ($\mu g/cm^2$) | | | | | | |
| | Cell A#3 | Cell A#4 | Cell B#1 | Cell B#5 | Mean 1-4 | %RSD 1-4 |
| $Time^{0.5}$ | | | | | | |
| 7.75 | 761.410 | 777.409 | 762.076 | 786.414 | 771.827 | 1.6 |
| 10.95 | 1188.482 | 1227.088 | 1190.173 | 1226.866 | 1208.152 | 1.8 |
| 13.42 | 1519.360 | 1545.470 | 1522.031 | 1570.329 | 1539.297 | 1.5 |
| 15.49 | 1807.629 | 1842.068 | 1801.749 | 1861.571 | 1828.254 | 1.6 |
| 17.32 | 2036.016 | 2088.419 | 2044.251 | 2134.726 | 2075.853 | 2.2 |
| 18.97 | 2255.737 | 2316.823 | 2270.827 | 2368.632 | 2305.255 | 2.1 |
| Slope | 133.35 | 136.81 | 134.82 | 141.11 | 136.52 | 2.5 |
| $R^2$ | 0.9999 | 0.9999 | 0.9999 | 0.9998 | 0.9999 | 0.0 |

In Vitro Release Rate Method Validation For Nasobol Gel 4.5%

4.5% Day4 Release Rate
Testosterone Gel 9.0%

Concentration of Active (μg/mL versus Time

| Amount Released (μg/mL) Calculation by Linear Regression Curve | | | | | | |
|---|---|---|---|---|---|---|
| | Cell A#2 | Cell A#6 | Cell B#3 | Cell B#4 | Mean 1-4 | %RSD 1-4 |
| **Time** | | | | | | |
| 60.00 | 215.853 | 222.792 | 211.379 | 219.348 | 217.343 | 2.2 |
| 120.00 | 329.810 | 344.136 | 321.102 | 337.453 | 333.125 | 3.0 |
| 180.00 | 411.198 | 428.858 | 404.979 | 421.971 | 416.752 | 2.6 |
| 240.00 | 474.841 | 493.977 | 462.253 | 487.875 | 479.737 | 2.9 |
| 300.00 | 522.127 | 550.136 | 512.332 | 540.875 | 531.373 | 3.2 |
| 360.00 | 565.951 | 593.682 | 555.027 | 584.821 | 574.870 | 3.1 |

| Actual Amount of Active Released (μg/cm²) versus $Time^{0.5}$ | | | | | | |
|---|---|---|---|---|---|---|
| Amount released (μg/cm²) | | | | | | |
| | Cell A#2 | Cell A#6 | Cell B#3 | Cell B#4 | Mean 1-4 | %RSD 1-4 |
| **$Time^{0.5}$** | | | | | | |
| 7.75 | 1465.812 | 1512.933 | 1435.430 | 1489.546 | 1457.930 | 2.2 |
| 10.95 | 2300.745 | 2399.993 | 2240.345 | 2353.636 | 2323.680 | 3.0 |
| 13.42 | 2946.753 | 3072.695 | 2900.791 | 3023.062 | 2985.825 | 2.6 |
| 15.49 | 3495.287 | 3636.250 | 3404.316 | 3589.998 | 3531.463 | 2.9 |
| 17.32 | 3950.753 | 4157.384 | 3875.186 | 4088.110 | 4017.858 | 3.2 |
| 18.97 | 4396.088 | 4608.756 | 4310.082 | 4539.429 | 4463.589 | 3.0 |
| Slope | 260.67 | 275.63 | 255.83 | 271.79 | 265.98 | 3.5 |
| $R^2$ | 1.0000 | 1.0000 | 0.9999 | 1.0000 | 1.0000 | 0.0 |

In Vitro Release Rate Method Validation For Nasobol Gel 4.5%

System Suitability for Nasobol Gel 4.5% Day4

1: Medium (Diluent)

| | |
|---|---|
| Result | There are no interference peaks at the RT of Testosterone from diluent injection |
| Criteria | There should be no significant interference peaks at the RT of Tesosterone from diluent |
| Comment | Pass |

2: Injection Reproducibility RT, Tailing Factor and Theoretical Plate Number from Six Replicate Injections of STD-4

| | RT | %RSD of RT | % RSD of Peak area | Tailing factor(T) | Theoretical Plate (N) |
|---|---|---|---|---|---|
| Result | 1.18 | 0.1 | 0.4 | 1.4 | 5511 |
| Criteria | NMT 2.0% | | NMT 2.0% | NMT 2 | NLT 1000 |
| Comment | Pass | | | | |

3: Calibration Curve Y=16050.748753x-354.26124

| STD | Conc. (μg/ml) | Peak area | RF | RSD(%) of RF (n=6) | Coefficient (R) | Slope | Y-intercept | 20% of STD 1 Area |
|---|---|---|---|---|---|---|---|---|
| STD-1 | 1.005000 | 15753.550011 | 15675.2 | 1.2 | 1.0000 | 16050.748753 | -354.261240 | 3151 |
| STD-2 | 6.281000 | 99637.415904 | 15863.3 | | | | | |
| STD-3 | 25.124100 | 403531.348593 | 16061.5 | | | | | |
| STD-4 | 50.248200 | 811049.854983 | 16140.9 | | | | | |
| STD-5 | 100.496400 | 1623794.951359 | 16157.7 | | | | | |
| STD-6 | 200.992800 | 3209962.318234 | 15970.5 | | | | | |
| Mean | N/A | N/A | 15978 | | | | | |
| Criteria | 1): RSD of RF NMT 10.0%, 2): Coefficient( R) NLT 0.98 | | | | | | | |
| | 3): The y-intercept should be NMT 20% of the STD-1 peak response | | | | | | | |
| Comment | Pass | | | | | | | |

4: Check STD Rec. (%)

| | Beginning | Middle | End | Criteria | |
|---|---|---|---|---|---|
| CSTD-1 | 100.4 | 100.6 | 99.6 | 90.0-110.0% | Two of the nine CSTDs failing to meet the criteria is acceptable providing the two failed CSTDs are not at the same concentration level |
| CSTD-2 | 100.3 | 100.6 | 99.2 | 98.0.0-102.0% | |
| CSTD-3 | 100.3 | 100.5 | 99.8 | 98.0.0-102.0% | |
| Comment | Pass | | | | |

## Claims

1. An intranasal testosterone gel for use in a method of treating male testosterone deficiency, wherein the intranasal testosterone gel is applied topically onto the nasal mucosal in the nasal cavity of each nostril at least once per day to achieve a constant effective testosterone blood level to treat the male testosterone deficiency.

2. The intranasal testosterone gel for use according to claim 1, wherein the intranasal testosterone gel is topically applied at least twice a day onto the nasal mucosal in the nasal cavity of each nostril.

3. The intranasal testosterone gel for use according to either of claim 1 or claim 2, wherein the intranasal testosterone gel is topically applied to the outer external wall inside the naval cavity.

4. The intranasal testosterone gel for use according to claim 3, wherein the intranasal testosterone gel is topically applied about the middle section to about the upper section of the outer external wall inside the naval cavity.

5. The intranasal testosterone gel for use according to claim 3, wherein the intranasal testosterone gel is topically applied about or under the cartilage section of the outer external wall inside the naval cavity.

6. The intranasal testosterone gel for use according to any of the preceding claims, wherein the outer skin of nose is massaged to distribute the intranasal testosterone gel throughout the nasal cavity of each nostril.

7. The intranasal testosterone gel for use according to any of the preceding claims, wherein the intranasal testosterone gel is topically applied with a device pre-filled with the intranasal testosterone gel.

8. The intranasal testosterone gel for use according to claim 7, wherein the pre-filled device includes a dispensing element for topically applying the intranasal testosterone gel at about the middle section to about the upper section of the outer external wall inside the naval cavity.

9. The intranasal testosterone gel for use according to claim 7, wherein the pre-filled device includes a dispensing element for topically applying the intranasal testosterone gel at about or under the cartilage section of the outer external wall inside the naval cavity.

10. The intranasal testosterone gel for use according to any of the preceding claims, wherein the male testosterone deficiency causes a decrease in sexual libido or sexual activity.

11. The intranasal testosterone gel for use according to any of claims 1 to 9, wherein the male testosterone deficiency causes male depression.

12. The intranasal testosterone gel for use according to any of claims 1 to 9, wherein the male testosterone deficiency is hypogonadism.

13. The intranasal testosterone gel for use according to any of claims 1 to 9, wherein the male testosterone deficiency causes a decrease in male fertility, low spermatogenesis or aspermatogenesis.

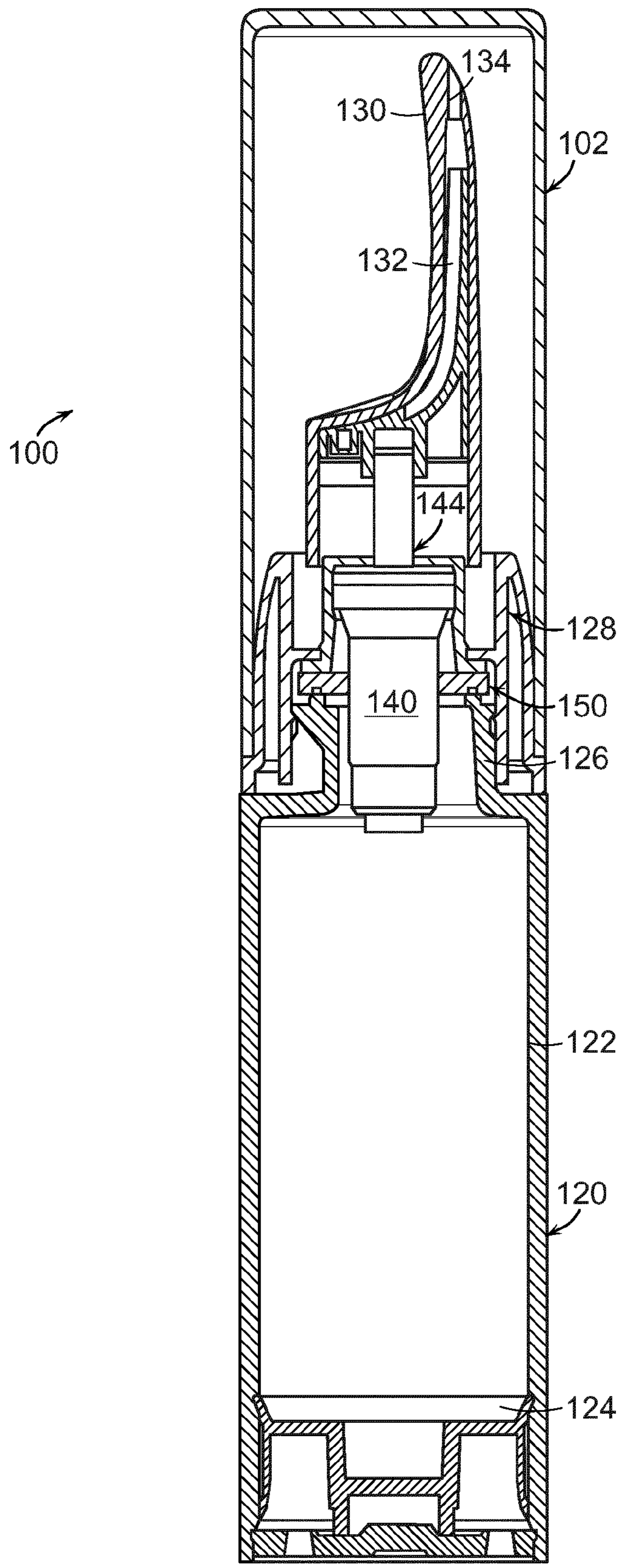


FIG. 1

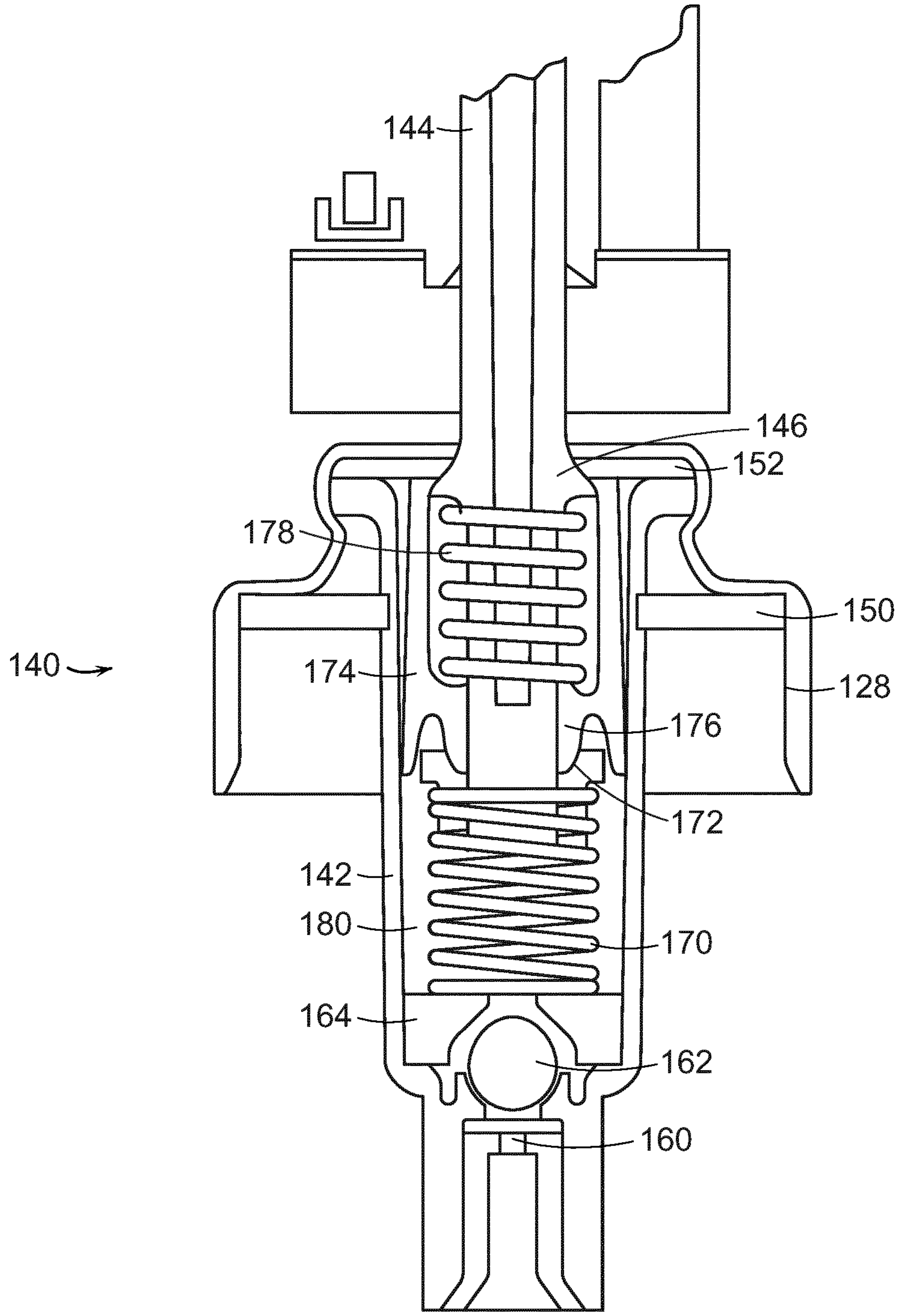


FIG. 2

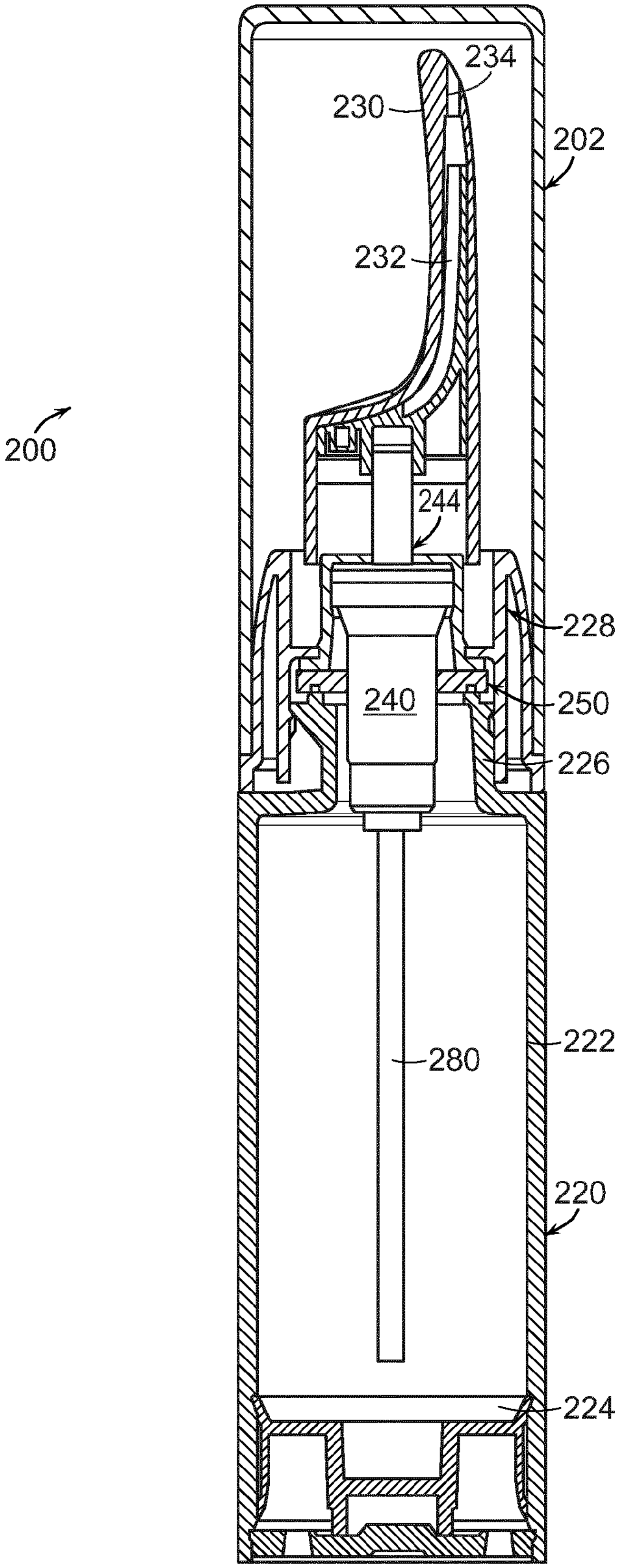


FIG. 3

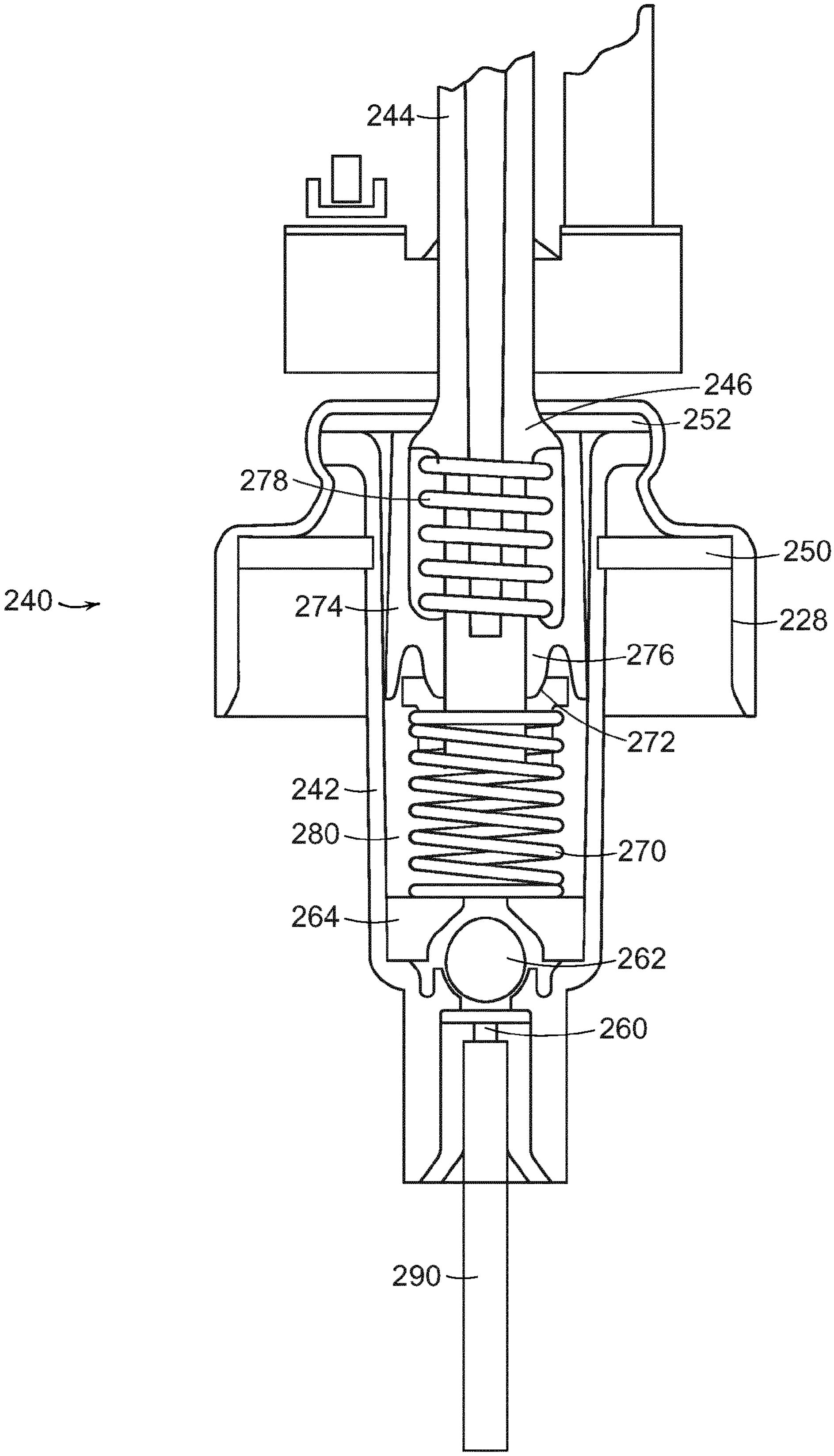
244
246
252
278
250
240
274
228
276
272
242
280
270
264
262
260
290

FIG. 4

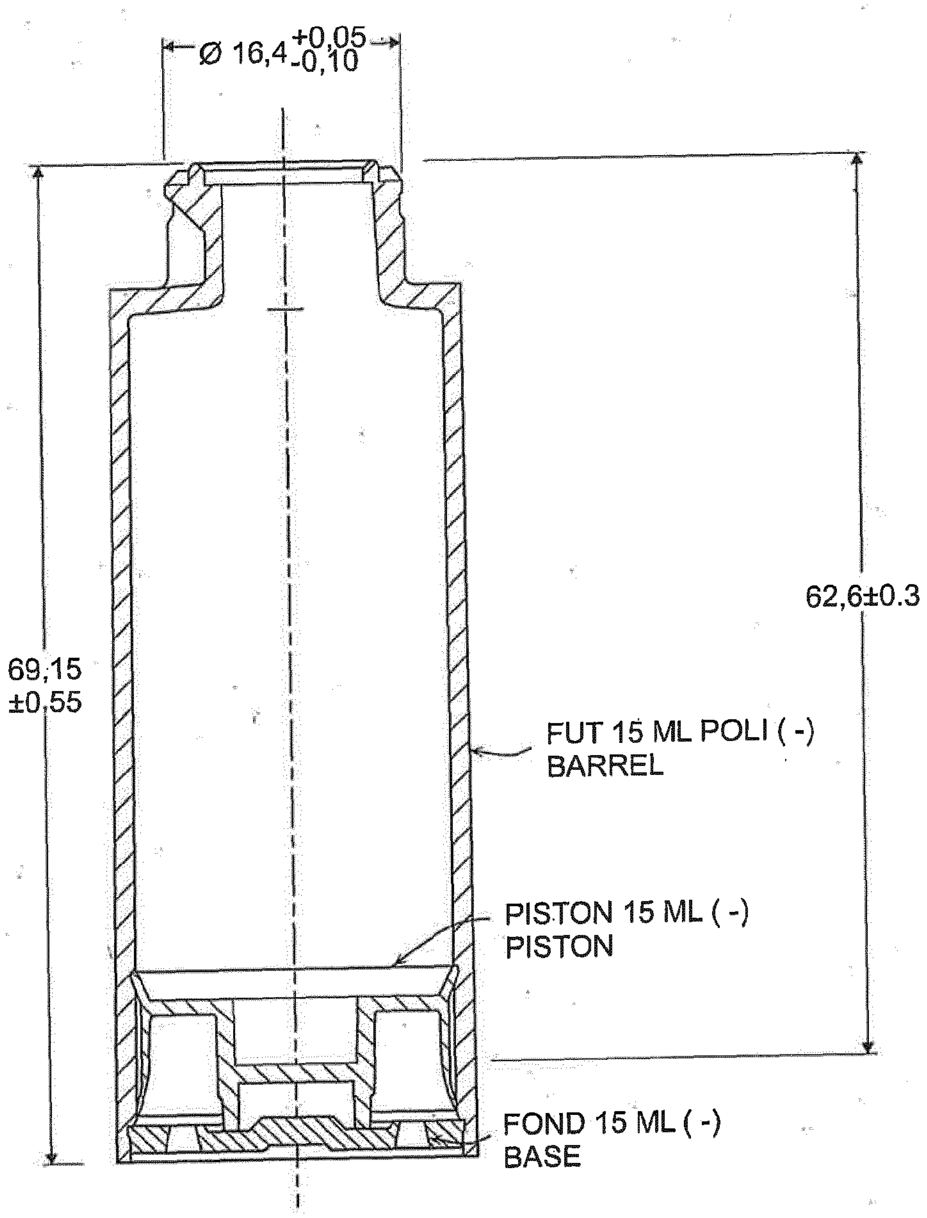
Ø 16,4 +0,05 -0,10
69,15 ±0,55
62,6±0.3
FUT 15 ML POLI ( -)
BARREL
PISTON 15 ML ( -)
PISTON
FOND 15 ML ( -)
BASE

FIG. 5

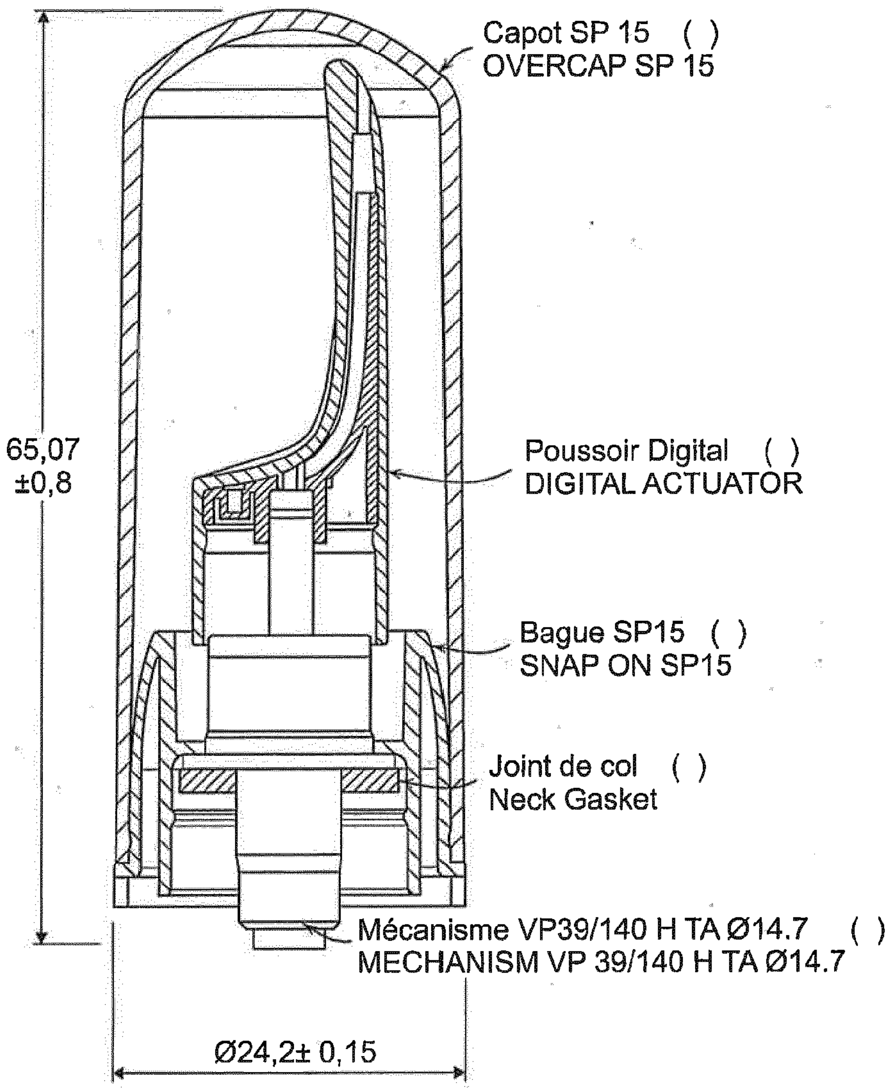
Capot SP 15 ( )
OVERCAP SP 15
65,07
±0,8
Poussoir Digital ( )
DIGITAL ACTUATOR
Bague SP15 ( )
SNAP ON SP15
Joint de col ( )
Neck Gasket
Mécanisme VP39/140 H TA Ø14.7 ( )
MECHANISM VP 39/140 H TA Ø14.7
Ø24,2± 0,15

FIG. 6

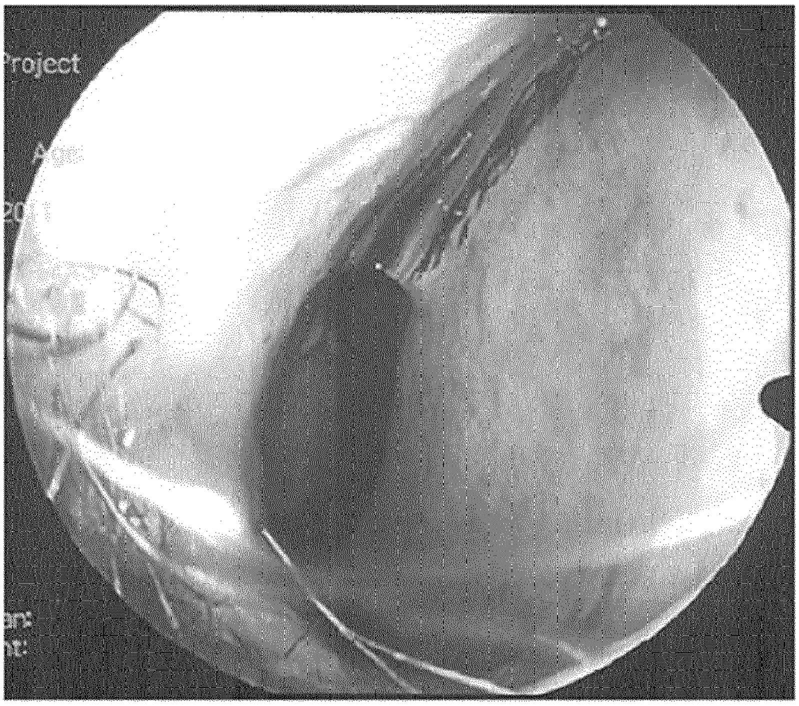


FIG. 7A

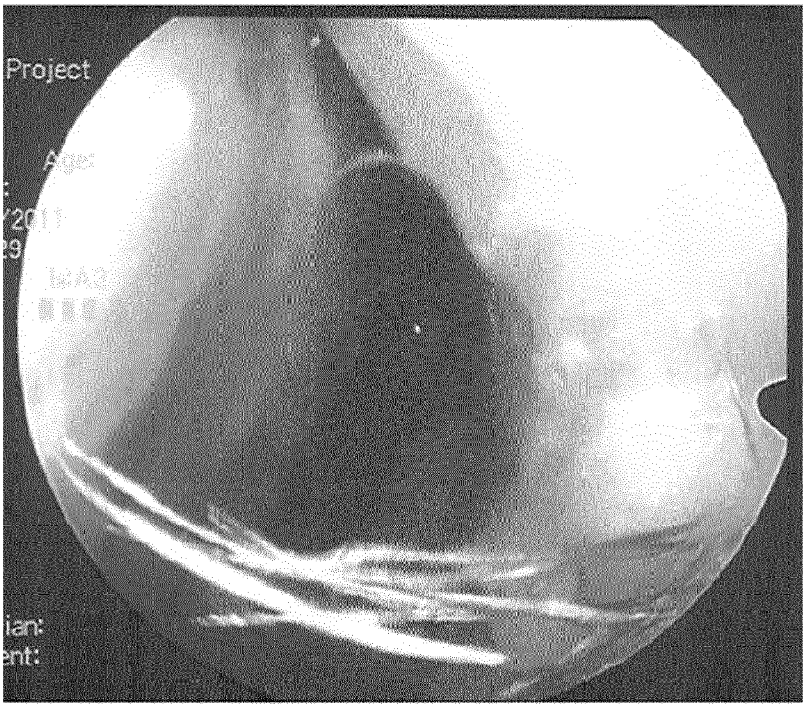


FIG. 7B

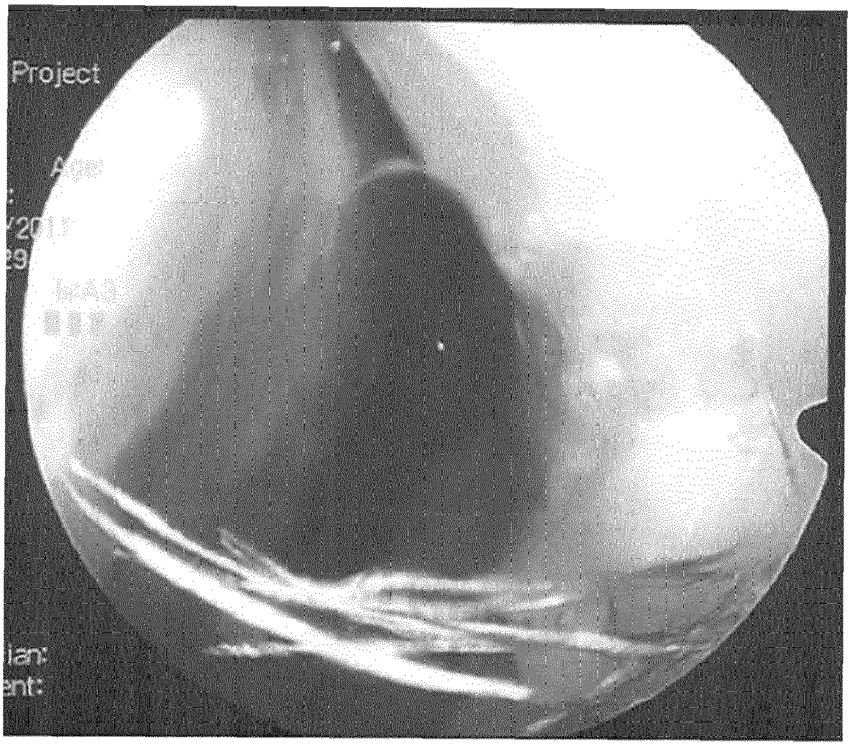


FIG. 8A

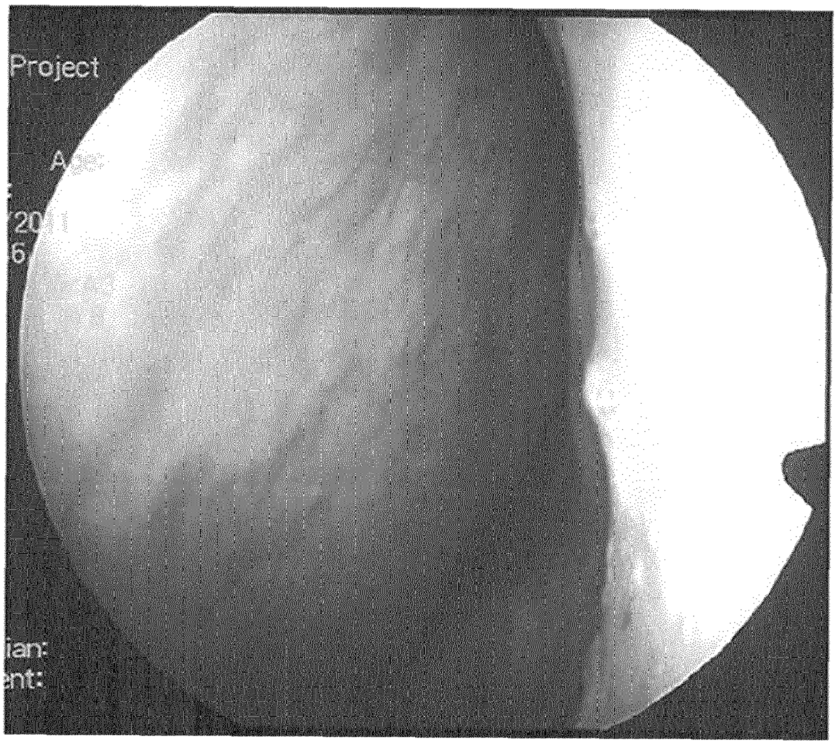


FIG. 8B

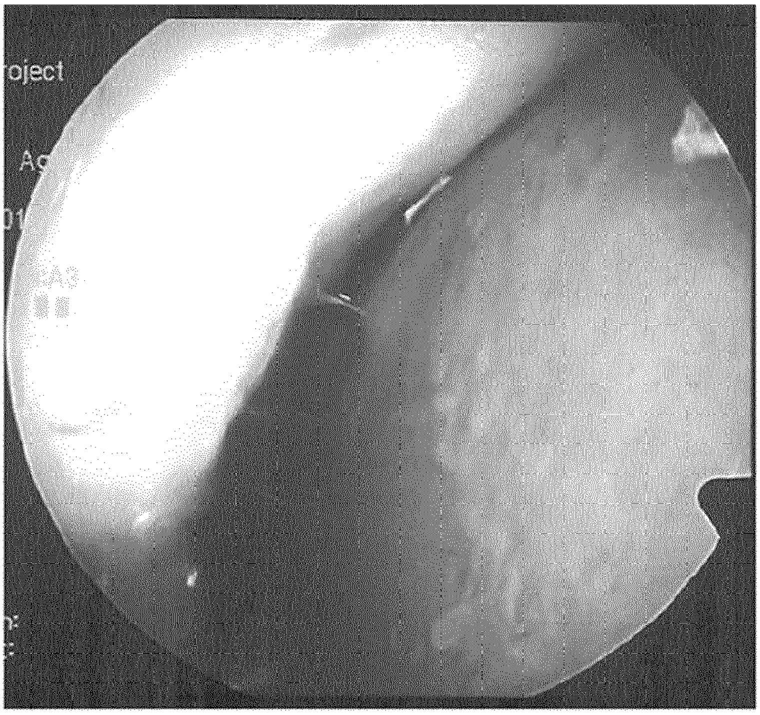

FIG. 9A

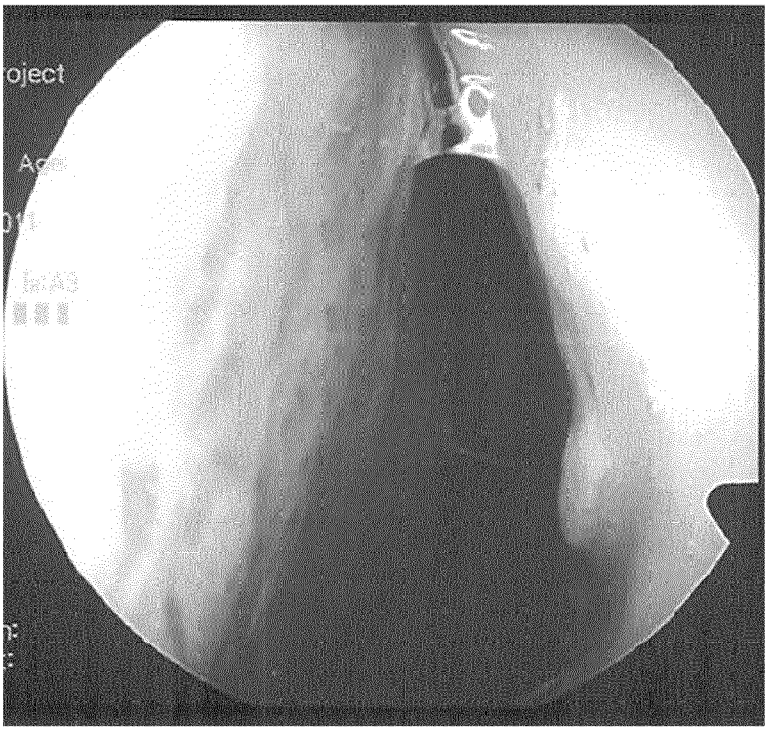

FIG. 9B

**STEP 1**

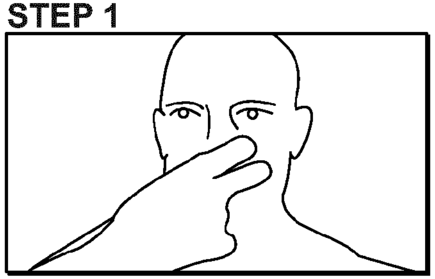

Blow your nose to clear your nostrils.

**STEP 2**

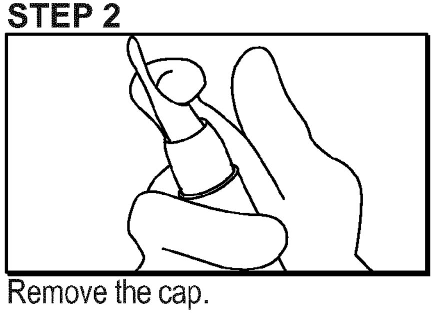

Remove the cap.

**STEP 3**

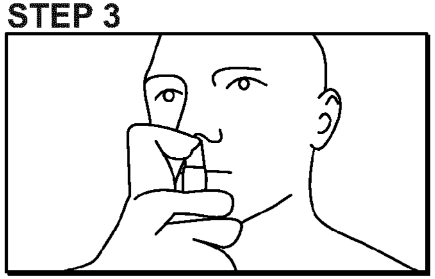

Place your finger on the pump of the actuator and in front of a mirror, advance the tip of the actuator into your left nostril until the finger on the pump reaches the base of the nose.

**STEP 4**

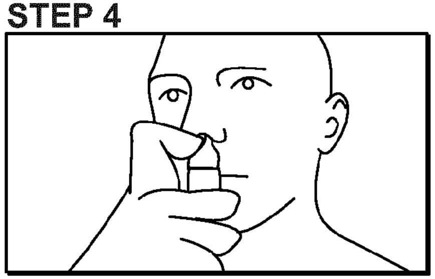

Aim the tip of the actuator to the inner corner of your left eye. The opening on the tip of the actuator must face the nasal mucosa.

**STEP 5**

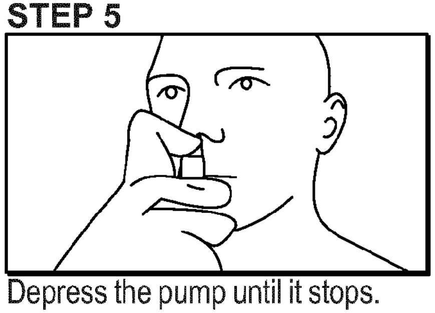

Depress the pump until it stops.

**STEP 6**

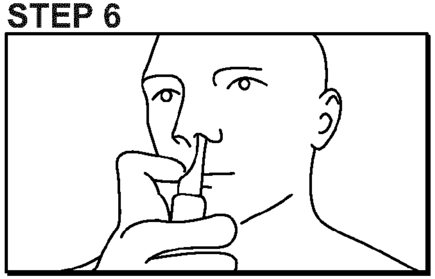

Slowly remove the actuator from your nose.

**STEP 7**

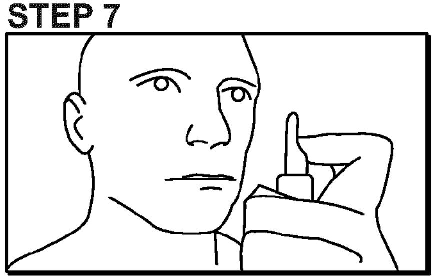

Repeat the process for the right nostril.

**STEP 8**

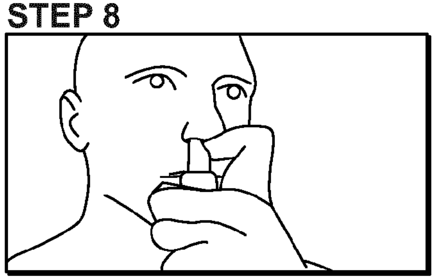

Place your finger on the pump of the actuator and, in front of a mirror, advance the tip of the actuator into your right nostril until the finger on the pump reaches the base of the nose.

**STEP 9**

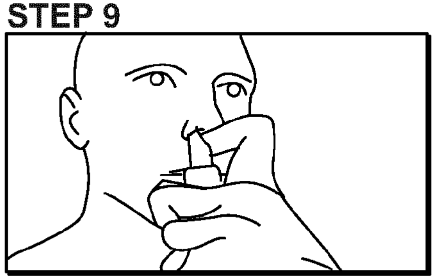

Aim the tip of the actuator to the inner corner of your right eye. The opening on the tip of the actuator must face the nasal mucosa.

FIG. 10A

**STEP 10**

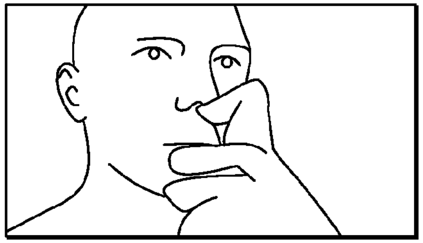

Depress the pump until it stops. Slowly remove the actuator from your nose.

**STEP 11**

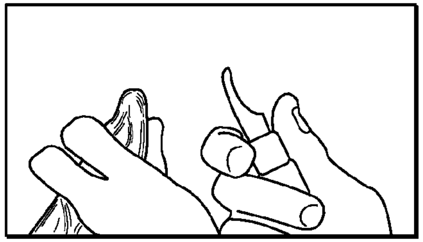

Wipe away any gel that remains on the tip of the actuator using a clean dry swab.

**STEP 12**

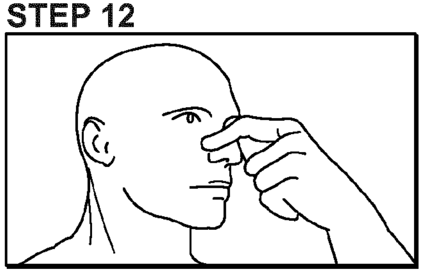

Press the nostrils near the bridge of the nose lightly together and massage for one second. **Do not blow your nose or sniff for one hour after administration of the medication.**

FIG. 10B

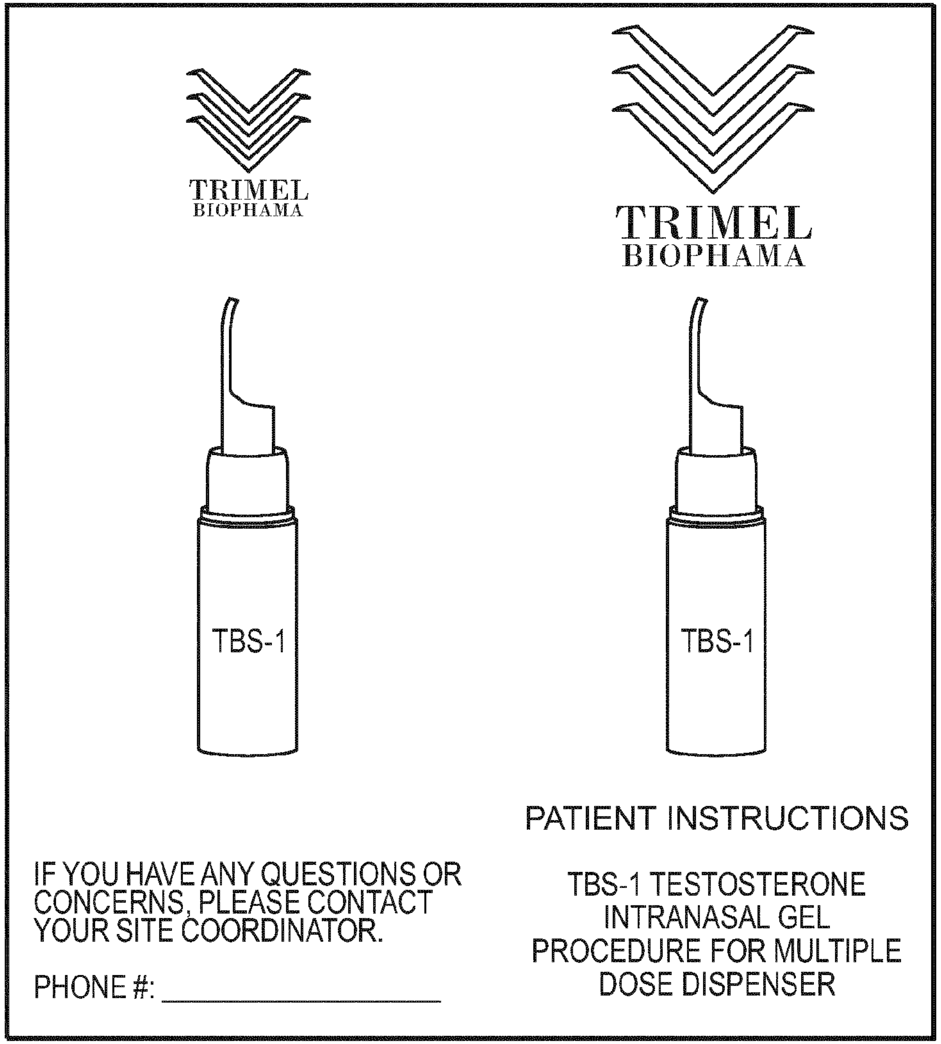


FIG. 11

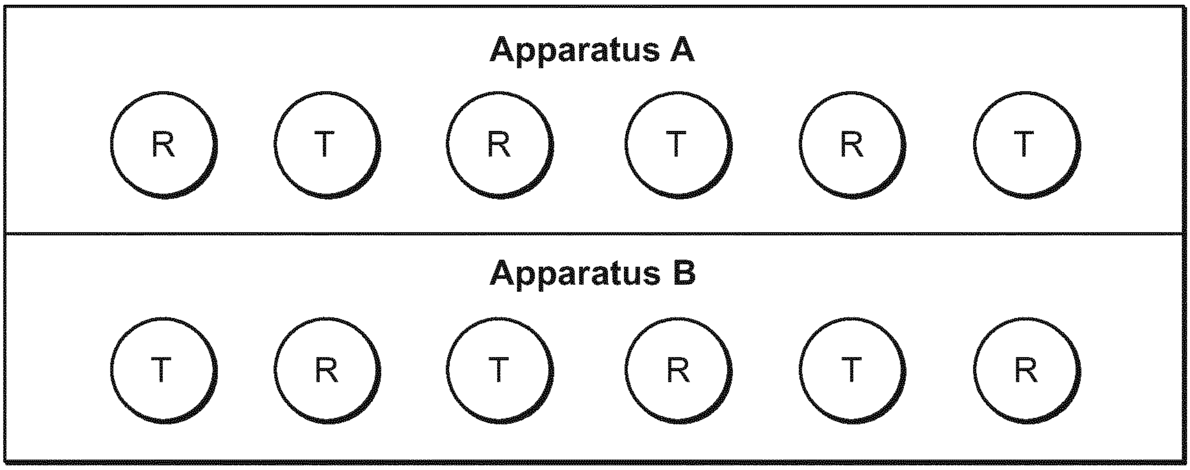
Apparatus A
R
T
R
T
R
T
Apparatus B
T
R
T
R
T
R

FIG. 12

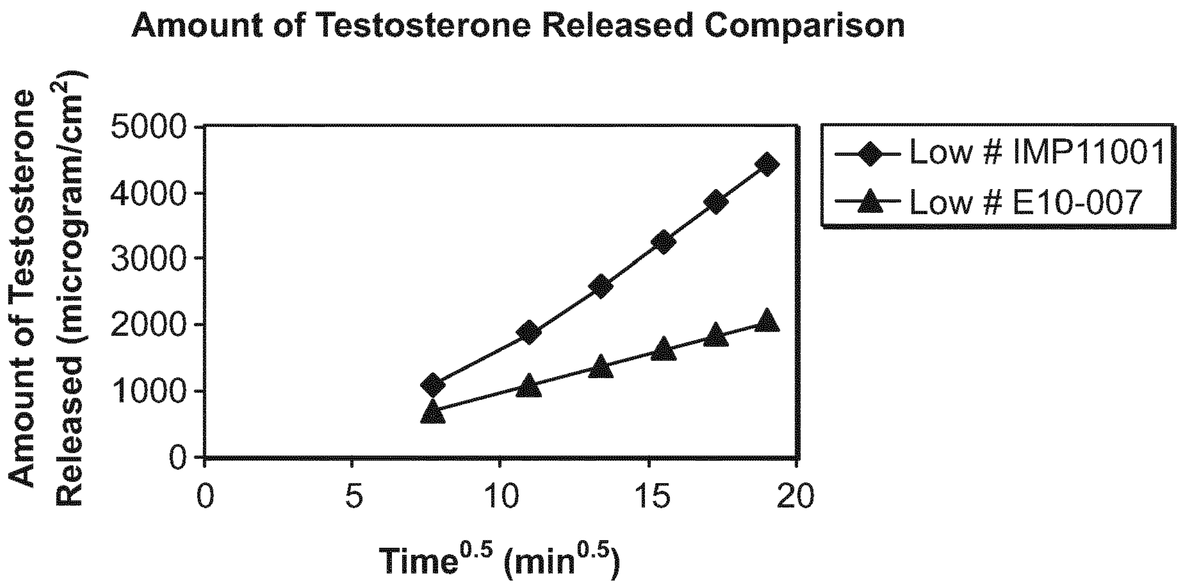
Amount of Testosterone Released Comparison
Amount of Testosterone Released (microgram/cm2)
5000
4000
3000
2000
1000
0
0
5
10
15
20
Time0.5 (min0.5)
Low # IMP11001
Low # E10-007

FIG. 13

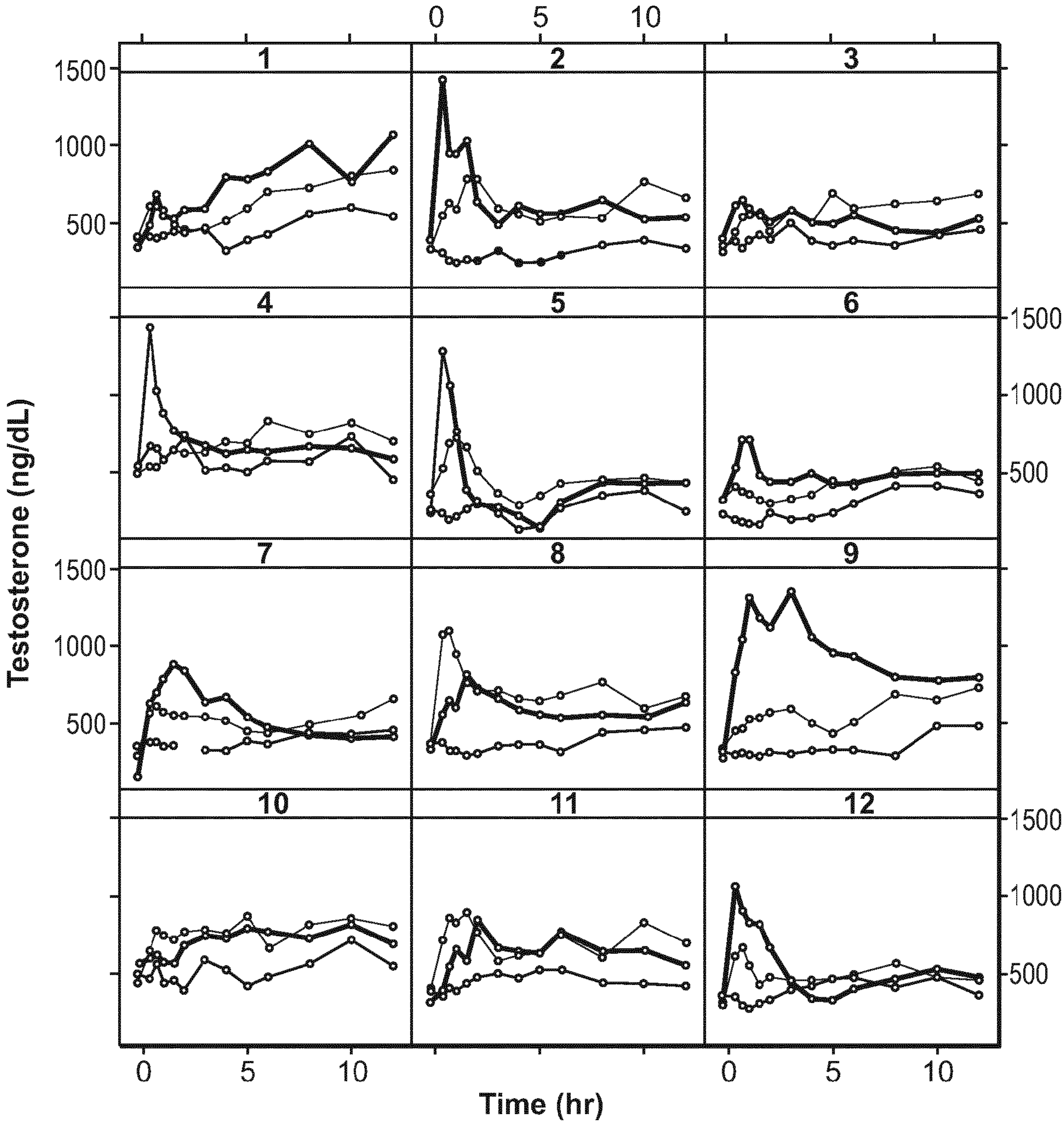
Testosterone (ng/dL)
Time (hr)
1
2
3
4
5
6
7
8
9
10
11
12
0
5
10
500
1000
1500

FIG. 14

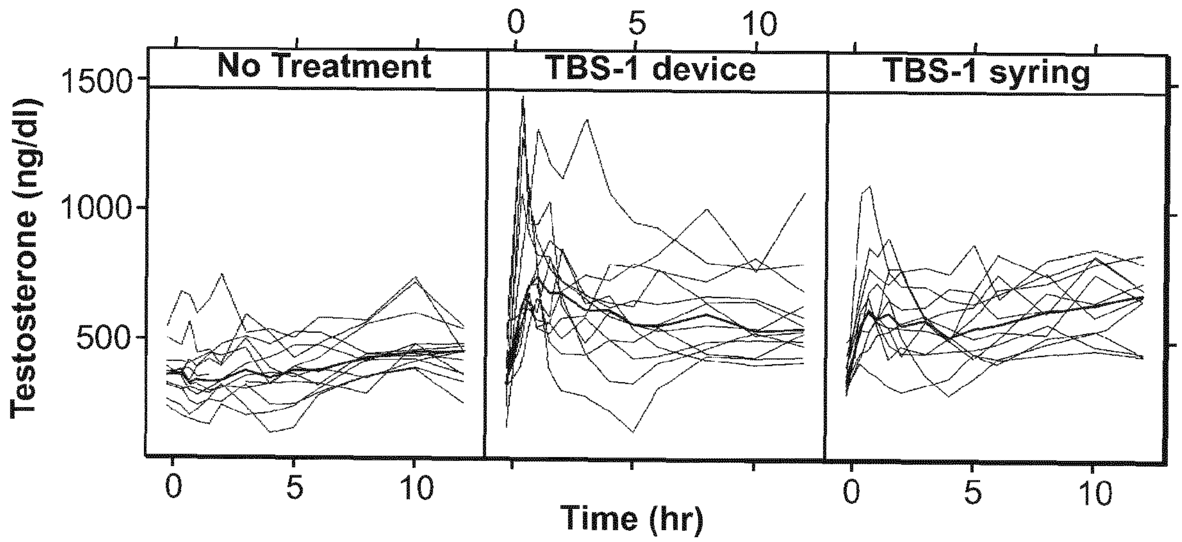
No Treatment
TBS-1 device
TBS-1 syring
Testosterone (ng/dl)
1500
1000
500
0
5
10
Time (hr)

FIG. 15

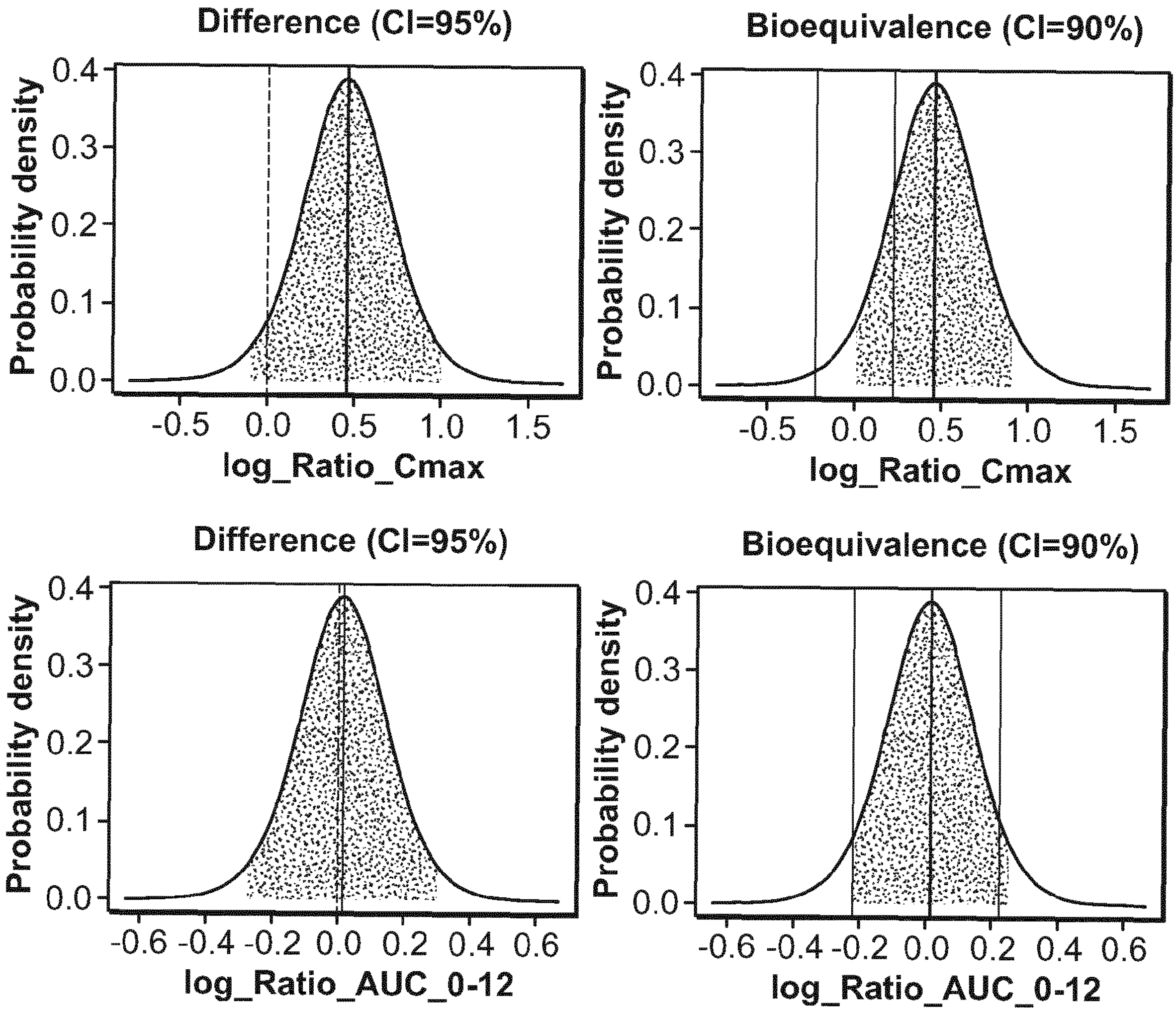
Difference (CI=95%)
Bioequivalence (CI=90%)
Probability density
0.4
0.3
0.2
0.1
0.0
-0.5 0.0 0.5 1.0 1.5
log_Ratio_Cmax
Difference (CI=95%)
Bioequivalence (CI=90%)
-0.6 -0.4 -0.2 0.0 0.2 0.4 0.6
log_Ratio_AUC_0-12

FIG. 16

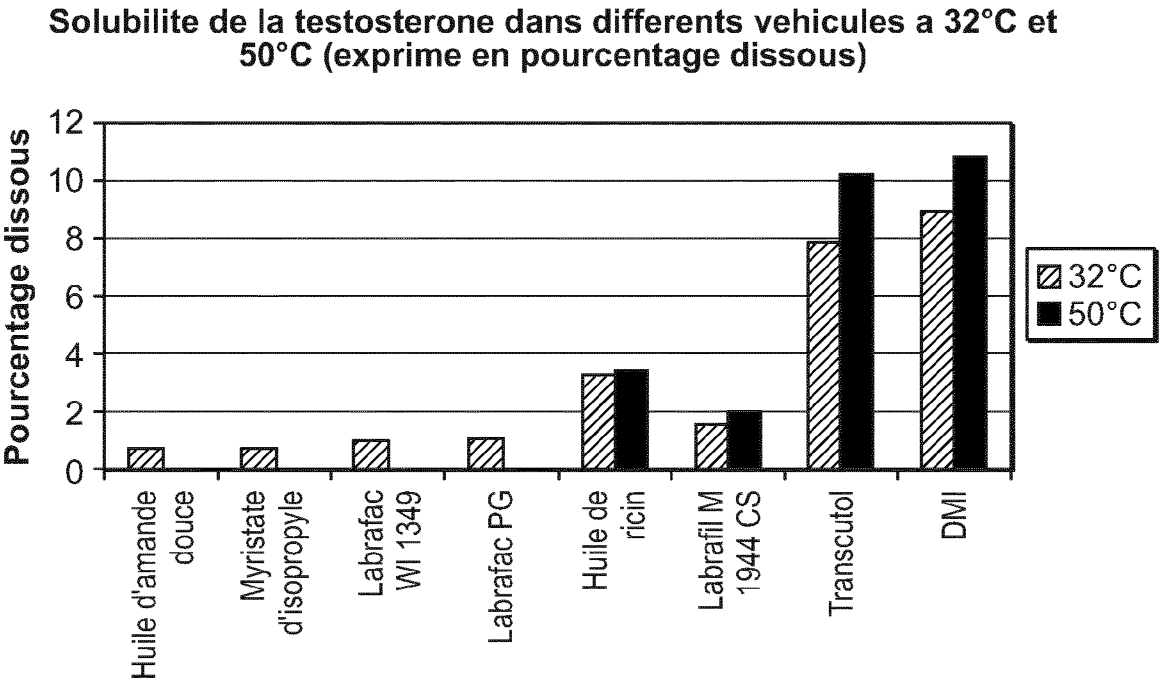
Solubilite de la testosterone dans differents vehicules a 32°C et 50°C (exprime en pourcentage dissous)
Pourcentage dissous
0
2
4
6
8
10
12
32°C
50°C
Huile d'amande douce
Myristate d'isopropyle
Labrafac WI 1349
Labrafac PG
Huile de ricin
Labrafil M 1944 CS
Transcutol
DMI

FIG. 17

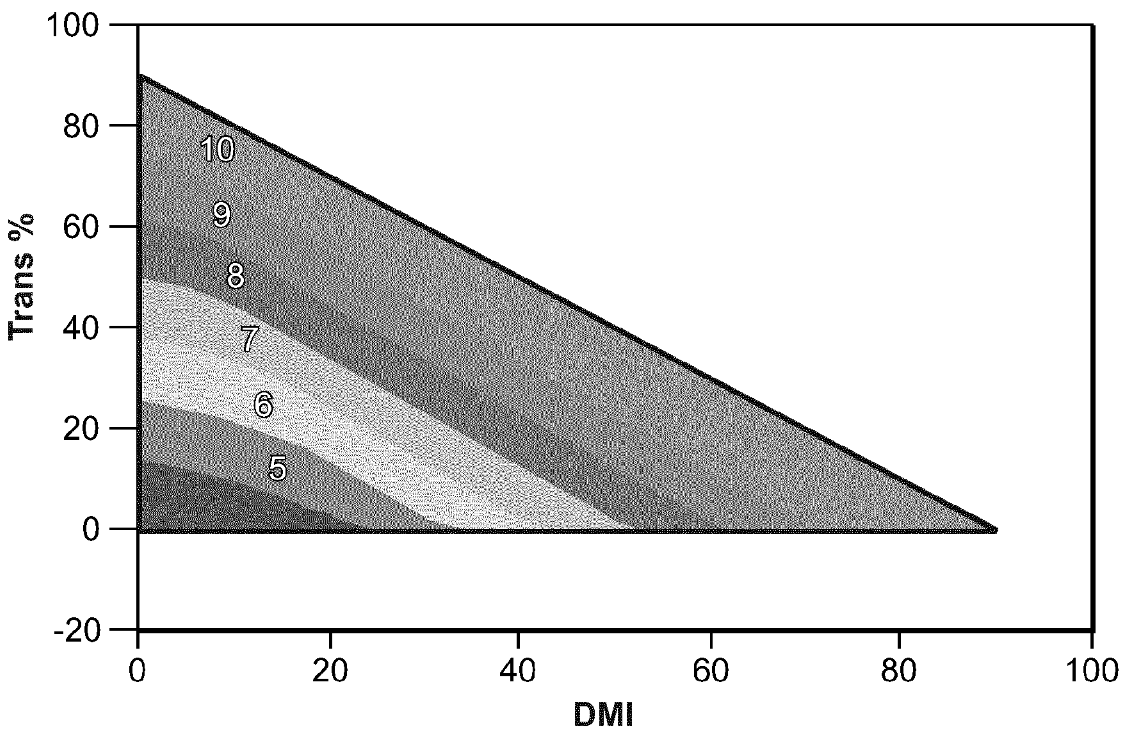
Trans %
100
80
60
40
20
0
-20
10
9
8
7
6
5
0
20
40
60
80
100
DMI

FIG. 18

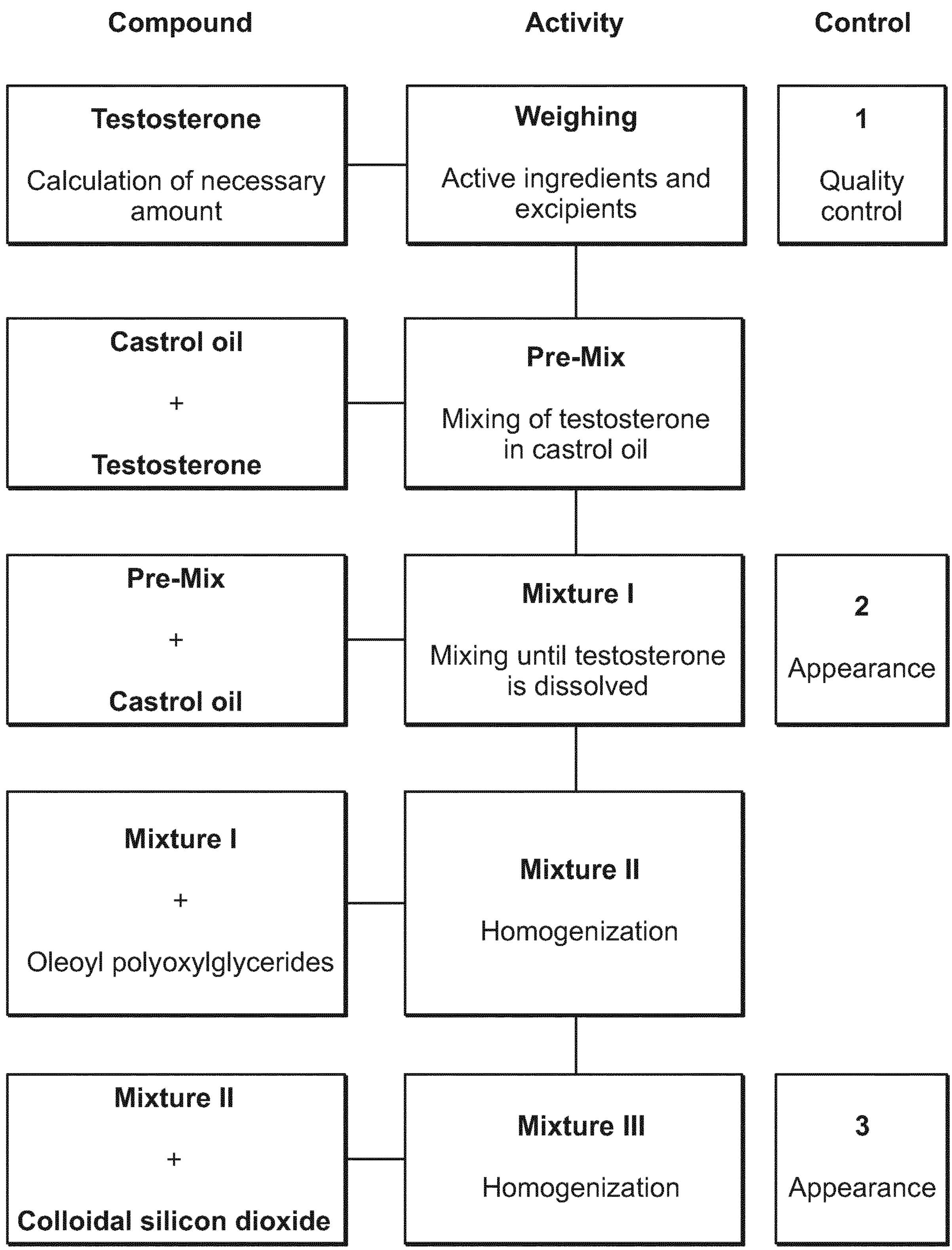
Compound
Activity
Control
Testosterone
Calculation of necessary amount
Weighing
Active ingredients and excipients
1
Quality control
Castrol oil
+
Testosterone
Pre-Mix
Mixing of testosterone in castrol oil
Pre-Mix
+
Castrol oil
Mixture I
Mixing until testosterone is dissolved
2
Appearance
Mixture I
+
Oleoyl polyoxylglycerides
Mixture II
Homogenization
Mixture II
+
Colloidal silicon dioxide
Mixture III
Homogenization
3
Appearance

FIG. 19

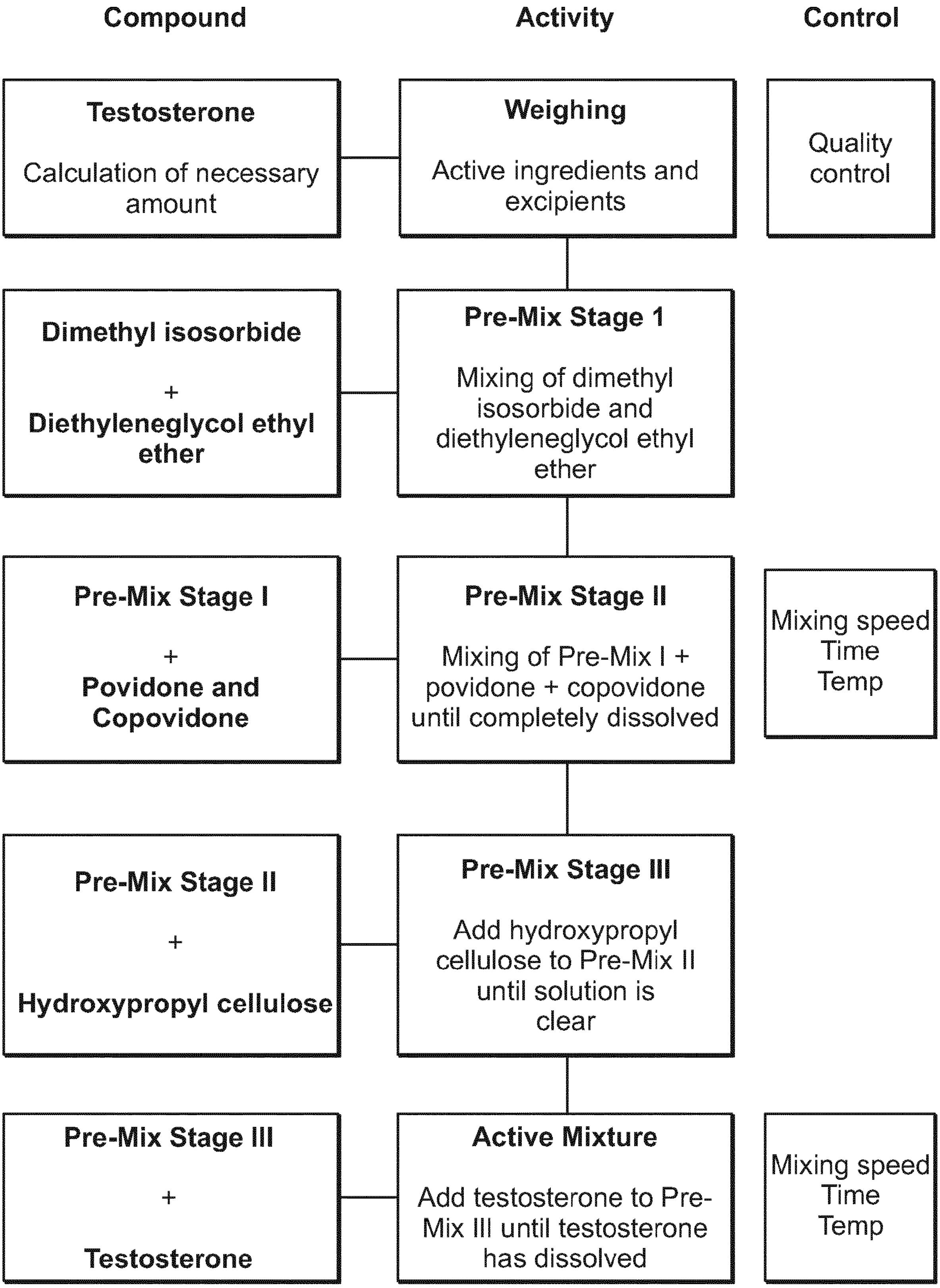
Compound
Activity
Control
Testosterone
Calculation of necessary amount
Weighing
Active ingredients and excipients
Quality control
Dimethyl isosorbide
+
Diethyleneglycol ethyl ether
Pre-Mix Stage 1
Mixing of dimethyl isosorbide and diethyleneglycol ethyl ether
Pre-Mix Stage I
+
Povidone and Copovidone
Pre-Mix Stage II
Mixing of Pre-Mix I + povidone + copovidone until completely dissolved
Mixing speed
Time
Temp
Pre-Mix Stage II
+
Hydroxypropyl cellulose
Pre-Mix Stage III
Add hydroxypropyl cellulose to Pre-Mix II until solution is clear
Pre-Mix Stage III
+
Testosterone
Active Mixture
Add testosterone to Pre-Mix III until testosterone has dissolved
Mixing speed
Time
Temp

FIG. 20A

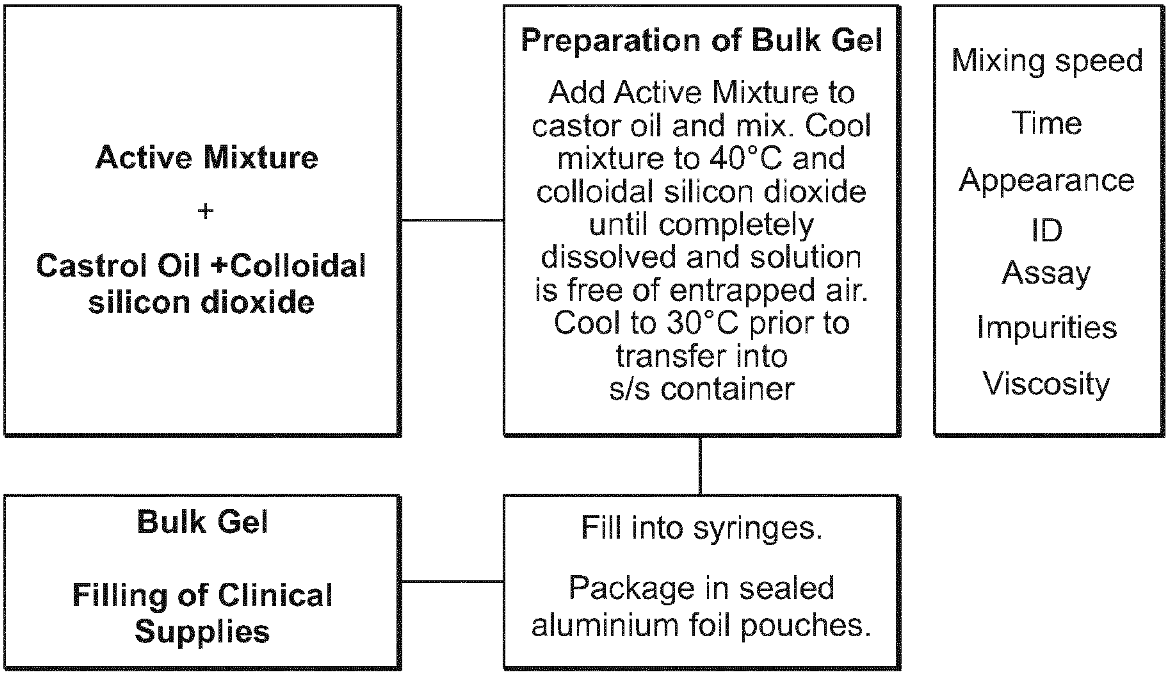


FIG. 20B

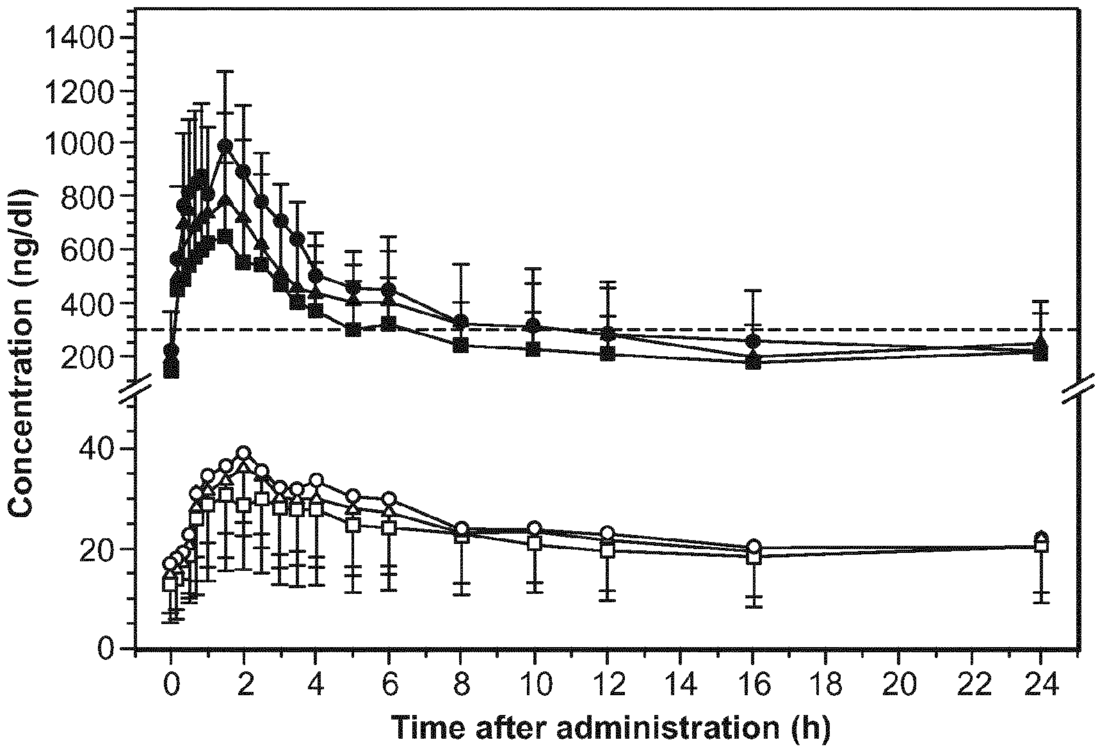


FIG. 21

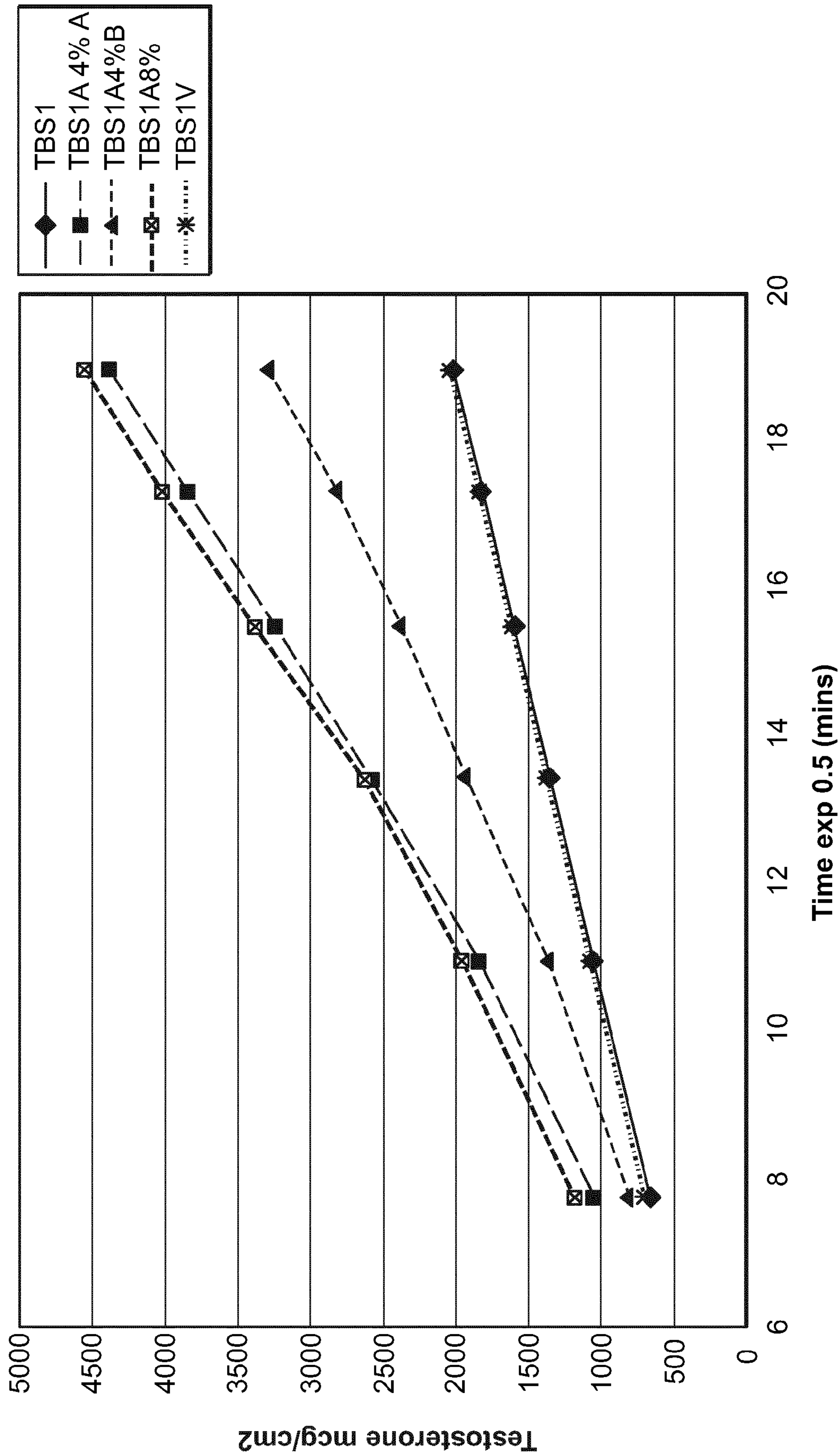
TBS1
TBS1A 4% A
TBS1A4%B
TBS1A8%
TBS1V
Testosterone mcg/cm2
0
500
1000
1500
2000
2500
3000
3500
4000
4500
5000
6
8
10
12
14
16
18
20
Time exp 0.5 (mins)

FIG. 22

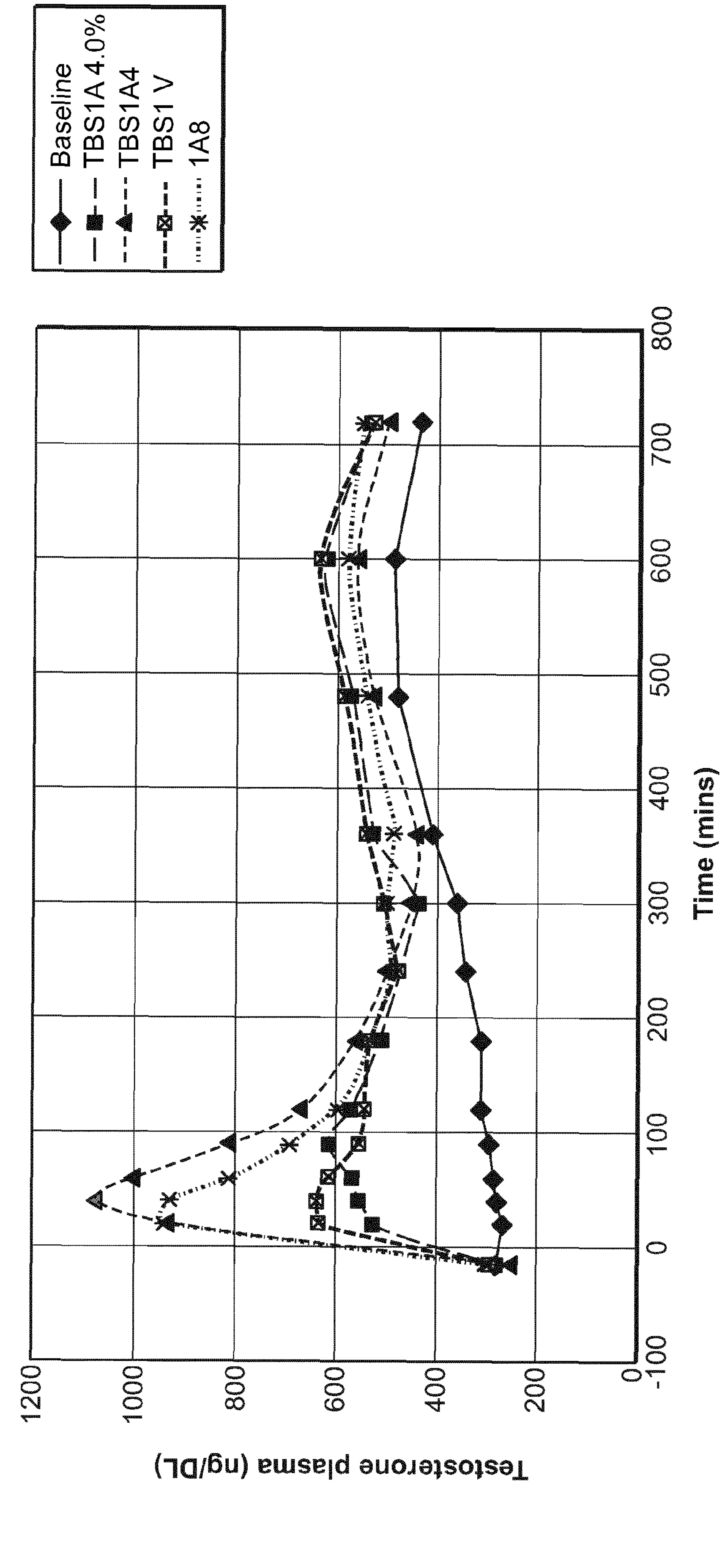
Baseline
TBS1A 4.0%
TBS1A4
TBS1 V
1A8
Testosterone plasma (ng/DL)
1200
1000
800
600
400
200
0
-100
0
100
200
300
400
500
600
700
800
Time (mins)

FIG. 23

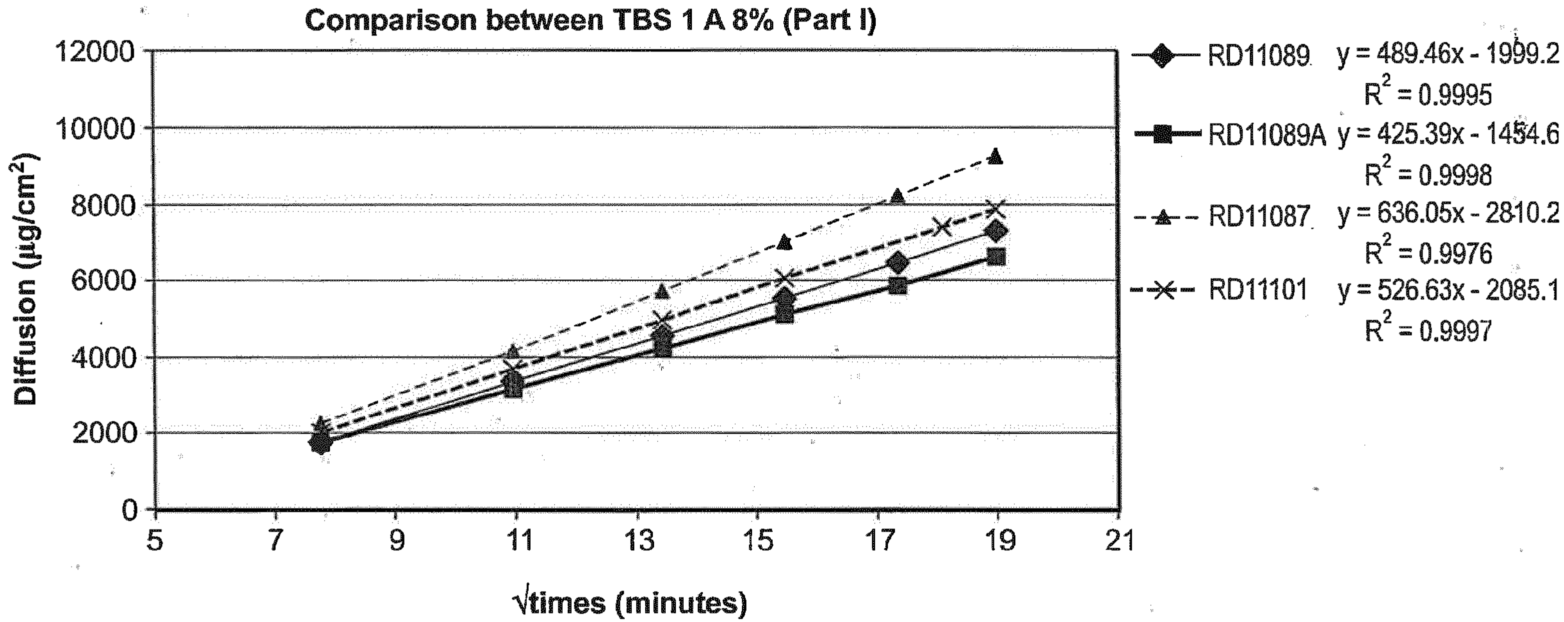


| Diffusion (µg/cm²) | RD11089 | RD11089A | RD11087 | RD11101 |
|---|---|---|---|---|
| 1 hour | 1853 | 1813 | 2294 | 2020 |
| 2 hours | 3308 | 3229 | 4014 | 3636 |
| 3 hours | 4525 | 4279 | 5588 | 4971 |
| 4 hours | 5580 | 5141 | 6998 | 6127 |
| 5 hours | 6488 | 5892 | 8252 | 7407 |
| 6 hours | 7317 | 6609 | 9356 | 7913 |

FIG. 24

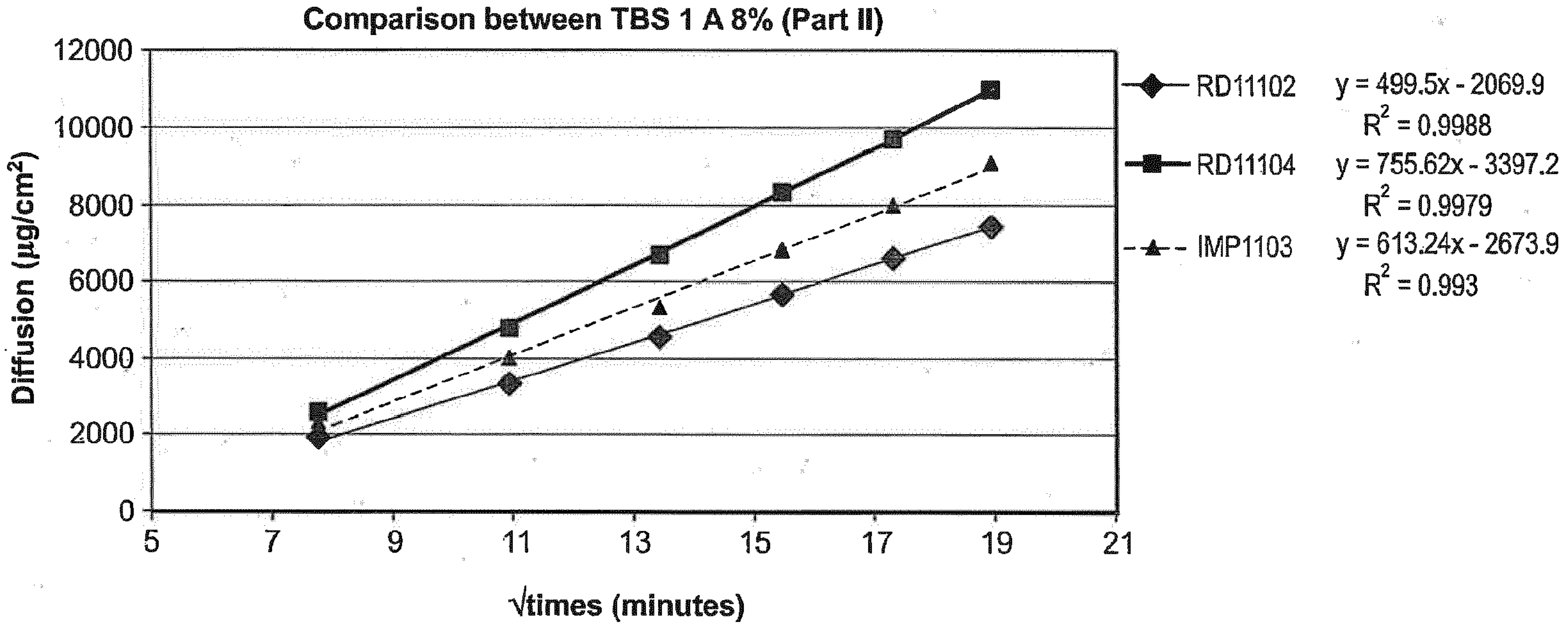


| | RD11102 | RD11104 | IMP1103 |
|---|---|---|---|
| Diffusion (µg/cm²) | | | |
| 1 hour | 1894 | 2647 | 2342 |
| 2 hours | 3333 | 4702 | 3898 |
| 3 hours | 4545 | 6589 | 5237 |
| 4 hours | 5662 | 8299 | 6768 |
| 5 hours | 6584 | 9796 | 8046 |
| 6 hours | 7471 | 10980 | 9116 |

Note: RD11101 and RD11102 were run up to 24 hours.

FIG. 25

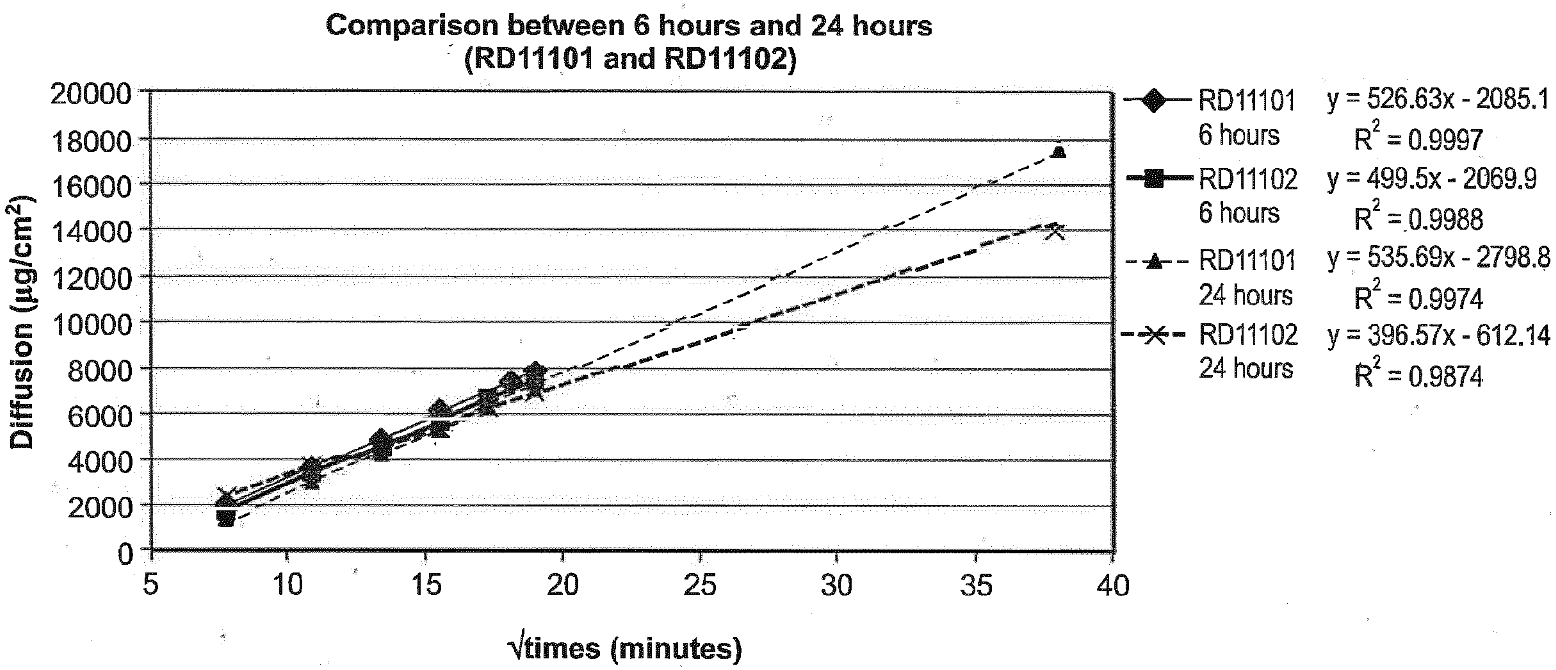


| | RD11101 6 hours | RD11102 6 hours | RD11101 24 hours | RD11102 24 hours |
|---|---|---|---|---|
| Diffusion (µg/cm²) | | | | |
| 1 hour | 2020 | 1894 | 1812 | 1957.7 |
| 2 hours | 3636 | 3333 | 3154 | 3427.49 |
| 3 hours | 4971 | 4545 | 4294 | 4626.42 |
| 4 hours | 6127 | 5662 | 5324 | 5705.54 |
| 5 hours | 7407 | 6584 | 6253 | 6682.36 |
| 6 hours | 7913 | 7471 | 7085 | 7581.86 |
| 24 hours | | | 17759 | 14031.69 |

FIG. 26

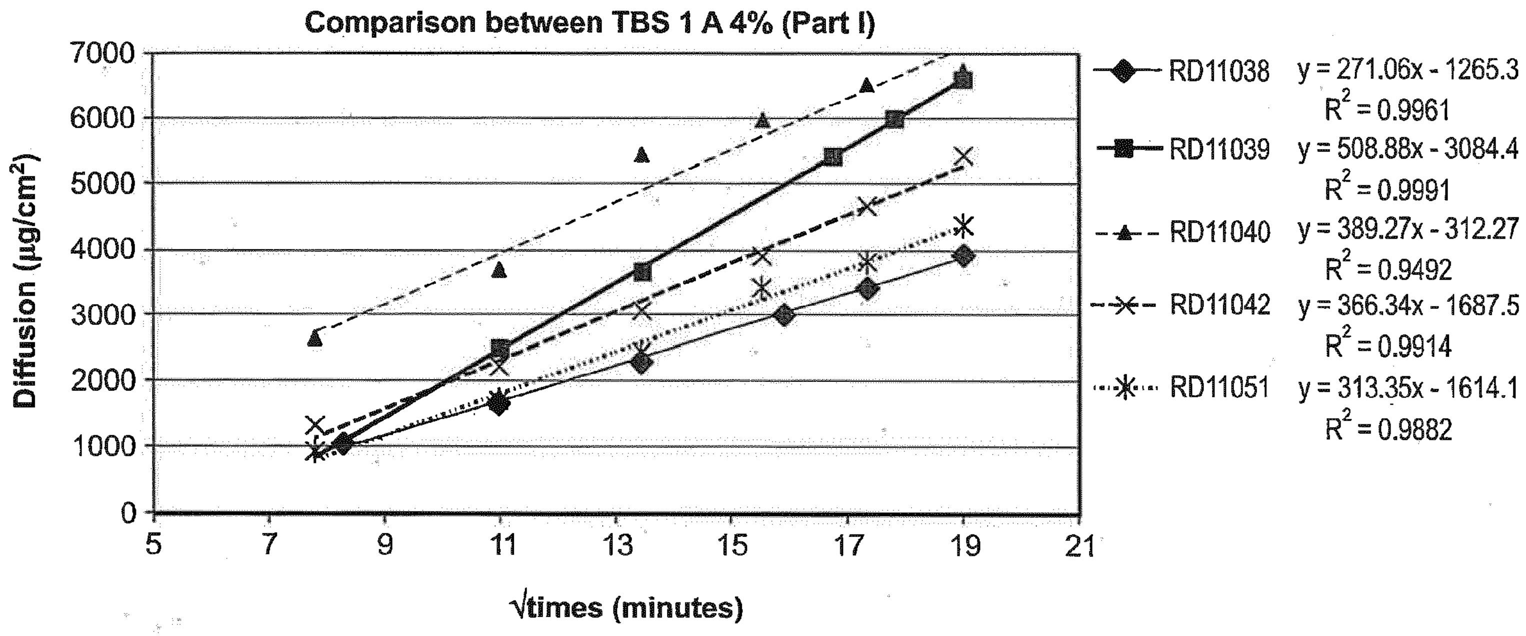


| Diffusion (µg/cm²) | RD11038 | RD11039 | RD11040 | RD11042 | RD11051 |
|---|---|---|---|---|---|
| 1 hour | 1053 | | 2539.93 | 1331.36 | 940.02 |
| 2 hours | 1668 | 2539.93 | 3676.36 | 2210.28 | 1700.72 |
| 3 hours | 2281 | 3663.66 | 5434.28 | 3084.42 | 2407.13 |
| 4 hours | 3002 | 5434.09 | 5977.58 | 3897.68 | 3421.73 |
| 5 hours | 3433 | 5977.58 | 6574.52 | 4662.27 | 3758.81 |
| 6 hours | 3952 | 6574.52 | 6583.47 | 5425.09 | 4376.96 |

FIG. 27

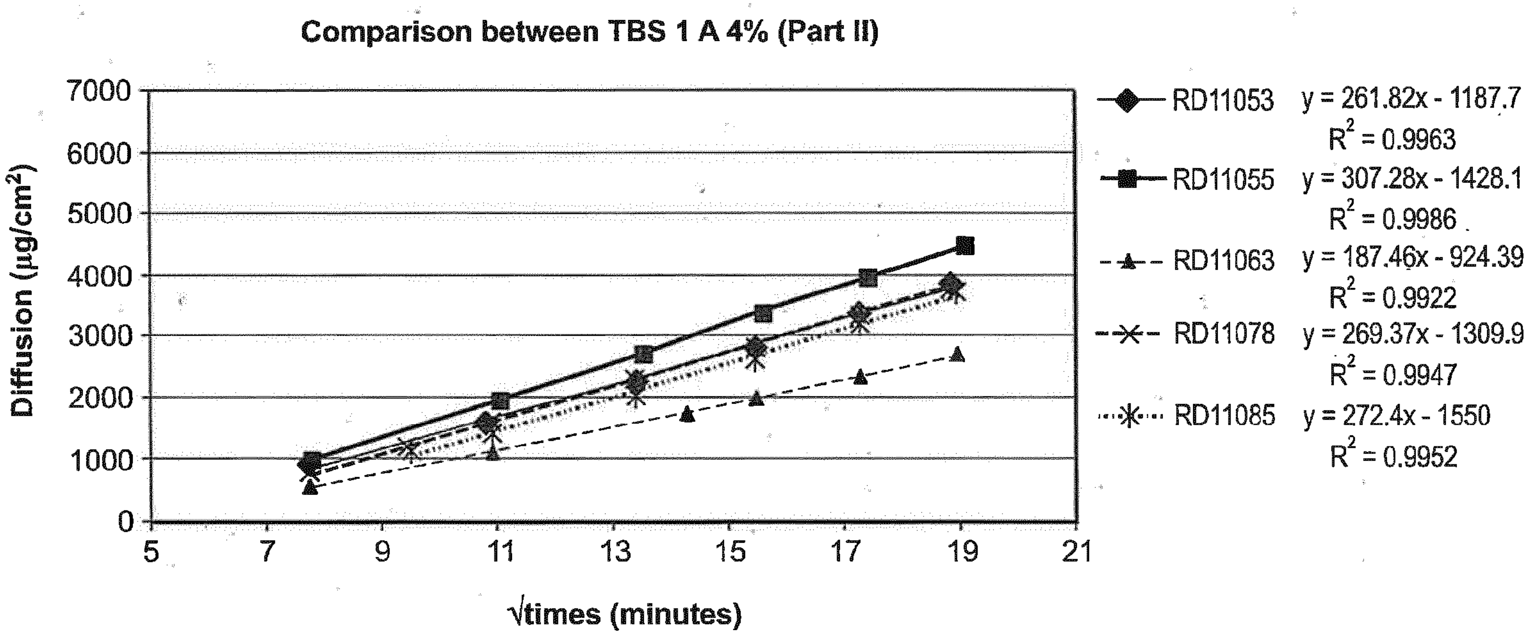


| | RD11053 | RD11055 | RD11063 | RD11078 | RD11085 |
|---|---|---|---|---|---|
| Diffusion (µg/cm$^2$) | | | | | |
| 1 hour | 926.97 | 1018.43 | 607.92 | 912.21 | 1130.37 |
| 2 hours | 1587.49 | 1874.39 | 1075.25 | 1582.63 | 1421.98 |
| 3 hours | 2251.5 | 2656.54 | 1686.95 | 2224.12 | 2021.15 |
| 4 hours | 2836.99 | 3323.18 | 1930.65 | 2835.75 | 2606.84 |
| 5 hours | 3350.39 | 3902.94 | 2334.99 | 3355.76 | 3160.31 |
| 6 hours | 3829.88 | 4436.66 | 2712.56 | 3954.08 | 3698.21 |

FIG. 28

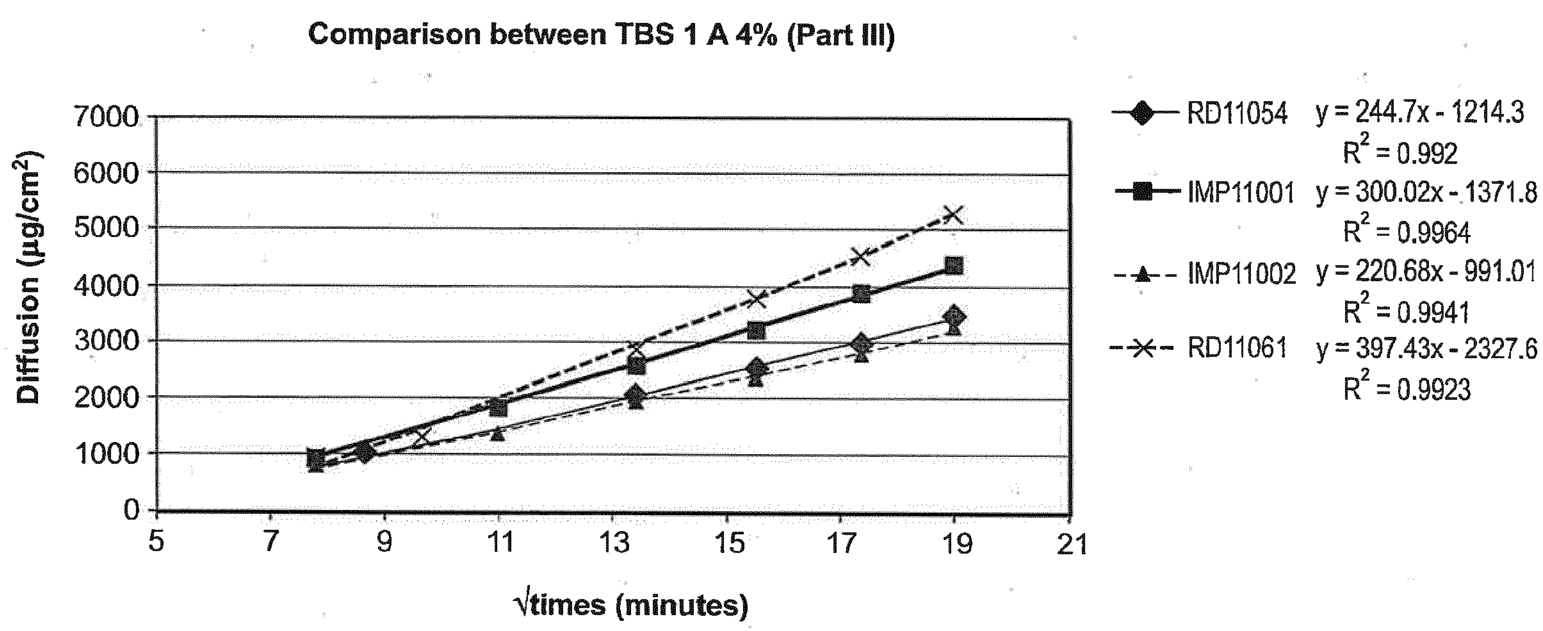


| | RD11054 | RD11061 | IMP11001 | IMP11002 |
|---|---|---|---|---|
| Diffusion (µg/cm$^2$) | | | | |
| 1 hour | 1006.97 | 975.48 | 1054.424 | 807.4 |
| 2 hours | 1415.17 | 1344.55 | 1838.037 | 1360.268 |
| 3 hours | 2059.74 | 2853.74 | 2574.543 | 1922.042 |
| 4 hours | 2512.42 | 3760.88 | 3242.818 | 2378.231 |
| 5 hours | 2990.82 | 4593.06 | 3841.022 | 2816.161 |
| 6 hours | 3543.18 | 5330.24 | 4389.895 | 3285.301 |

FIG. 29

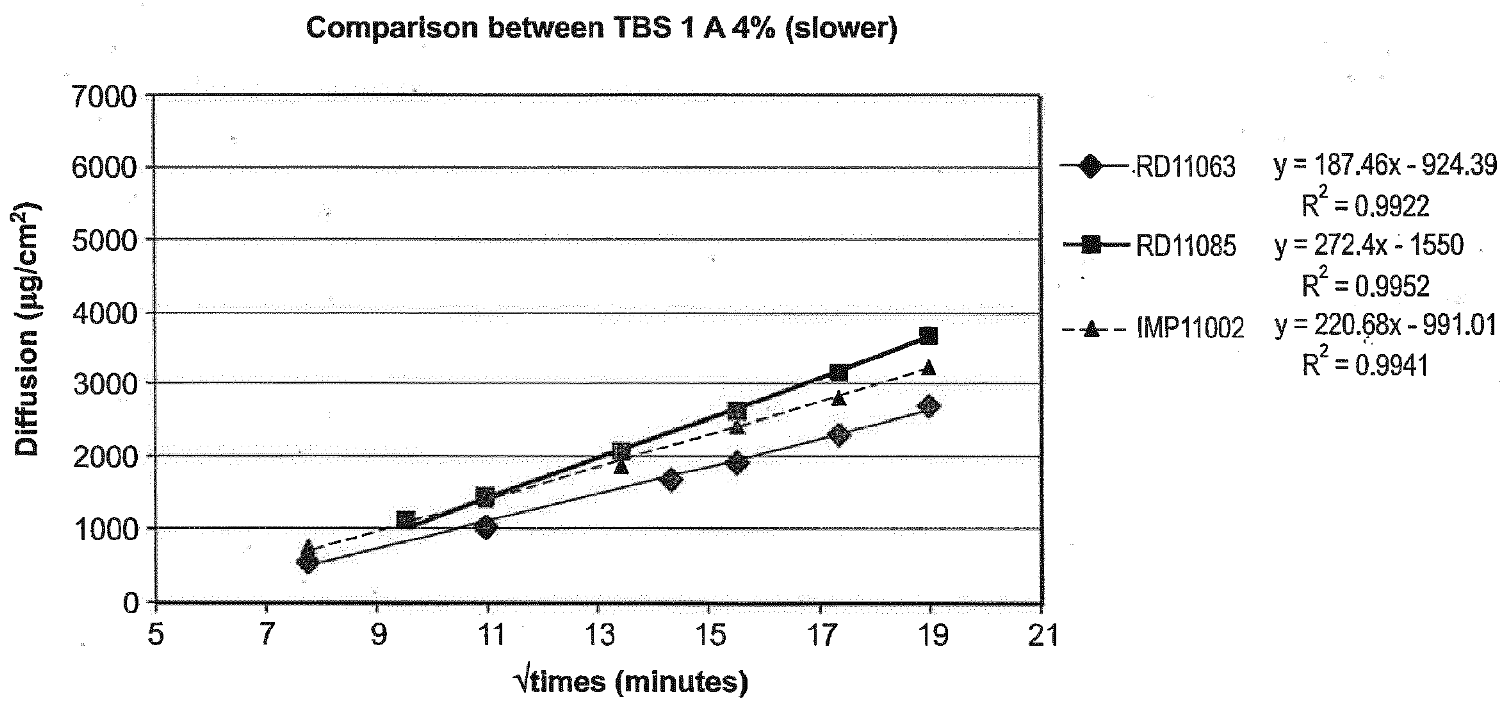


Note: RD11063 and RD11085 were run up to 24 hours.

FIG. 30

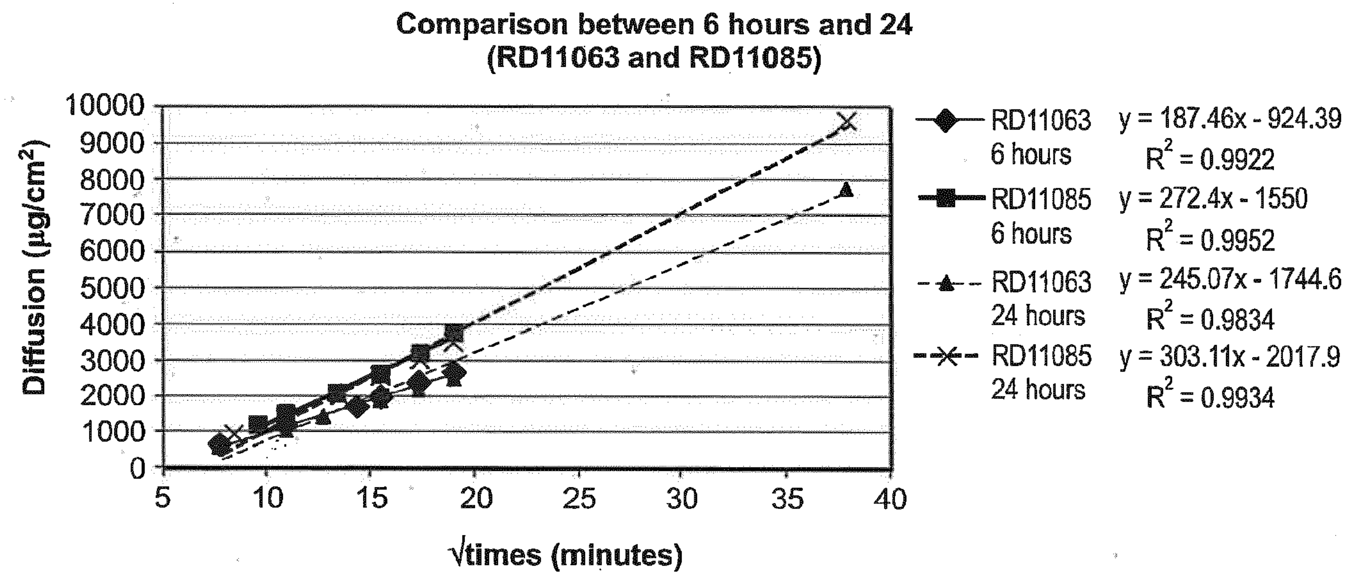


| Diffusion (µg/cm²) | RD11063 6 hours | RD11085 6 hours | RD11063 24 hours | RD11085 24 hours |
|---|---|---|---|---|
| 1 hour | 607.92 | 1130.37 | 600.49 | 913.01 |
| 2 hours | 1075.25 | 1421.98 | 1053.99 | 1407.08 |
| 3 hours | 1686.95 | 2021.15 | 1449.92 | 1972.8 |
| 4 hours | 1930.65 | 2606.84 | 1813.75 | 2514.87 |
| 5 hours | 2334.99 | 3160.31 | 2185.79 | 3017.65 |
| 6 hours | 2712.56 | 3698.21 | 2528.07 | 3488.02 |
| 24 hours | | | 7828.4 | 9680.91 |

Note: we wanted to see if there was a difference if we add 400 mg of gel.

FIG. 31

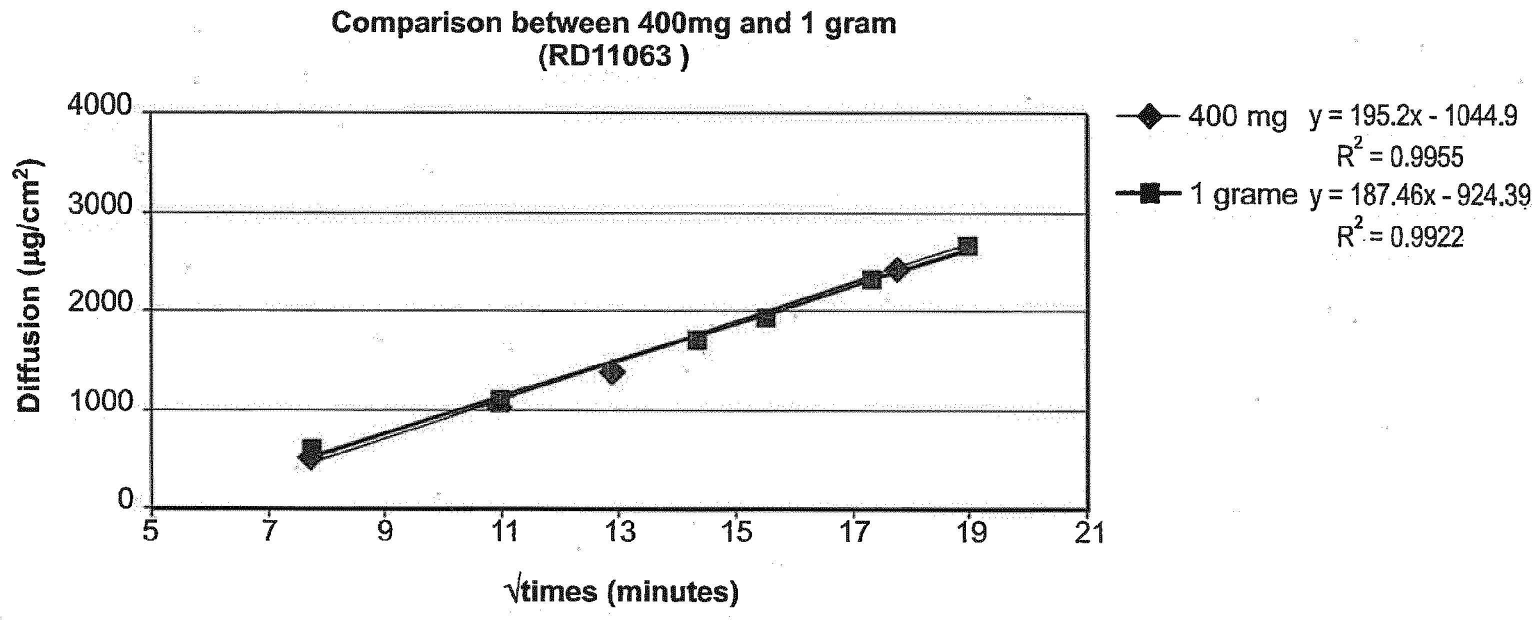


400 mg

| √Times (minutes) | Diffusion (µg/cm²) |
|---|---|
| 7.75 | 542.24 |
| 10.95 | 1042.73 |
| 12.88 | 1395.43 |
| 17.75 | 2432.99 |
| 18.97 | 2694.41 |

1 gr

| √Times (minutes) | Diffusion (µg/cm²) |
|---|---|
| 7.74 | 607.92 |
| 10.95 | 1075.25 |
| 14.32 | 1686.95 |
| 15.49 | 1930.65 |
| 17.32 | 2334.99 |
| 18.97 | 2712.56 |

FIG. 32

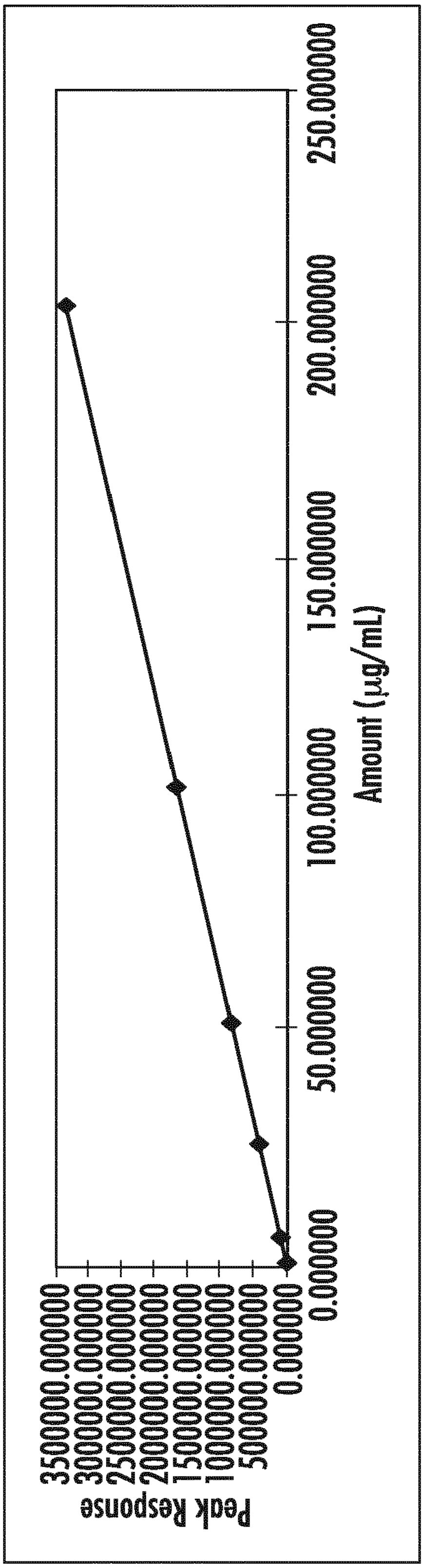
Peak Response
3500000.000000
3000000.000000
2500000.000000
2000000.000000
1500000.000000
1000000.000000
500000.000000
0.000000
0.000000
50.000000
100.000000
150.000000
200.000000
250.000000
Amount (µg/mL)

FIG. 33

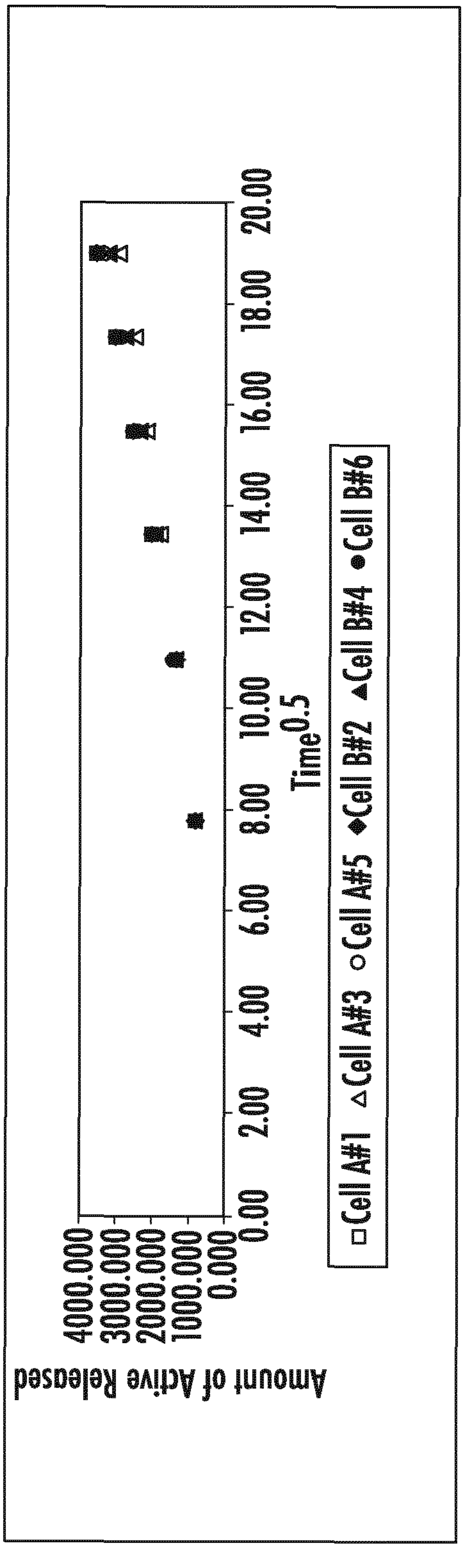
Amount of Active Released
4000.000
3000.000
2000.000
1000.000
0.000
0.00
2.00
4.00
6.00
8.00
10.00
12.00
14.00
16.00
18.00
20.00
Time0.5
□Cell A#1 △Cell A#3 ○Cell A#5 ◆Cell B#2 ▲Cell B#4 ●Cell B#6

FIG. 34

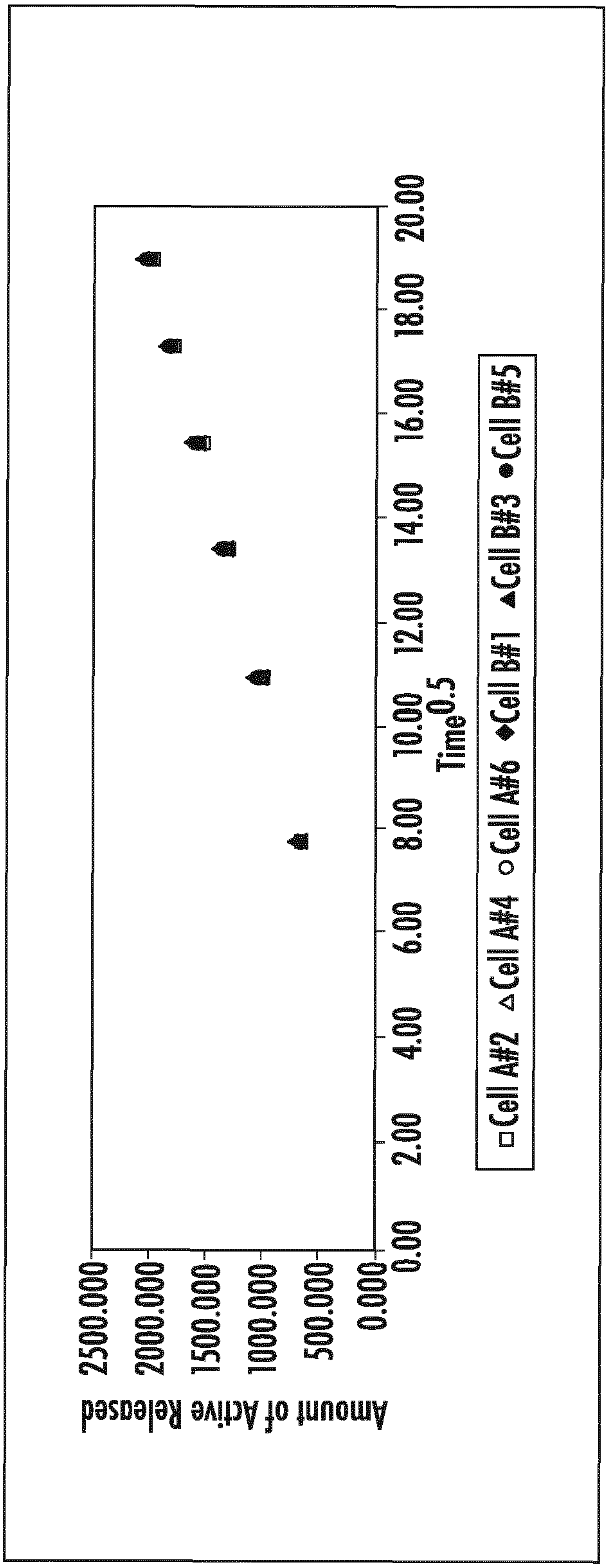
Amount of Active Released
2500.000
2000.000
1500.000
1000.000
500.000
0.000
0.00
2.00
4.00
6.00
8.00
10.00
12.00
14.00
16.00
18.00
20.00
Time0.5
Cell A#2
Cell A#4
Cell A#6
Cell B#1
Cell B#3
Cell B#5

FIG. 35

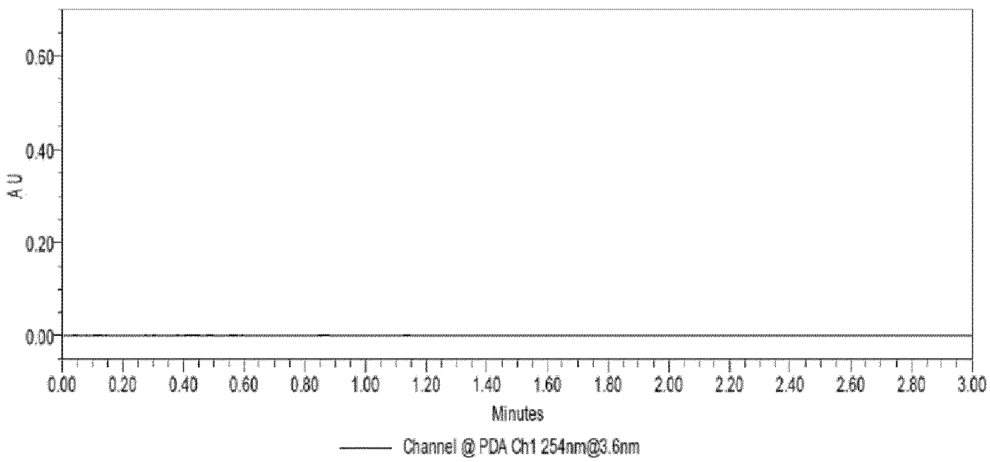
0.60
0.40
0.20
0.00
AU
0.00 0.20 0.40 0.60 0.80 1.00 1.20 1.40 1.60 1.80 2.00 2.20 2.40 2.60 2.80 3.00
Minutes
Channel @ PDA Ch1 254nm@3.6nm

FIG. 36

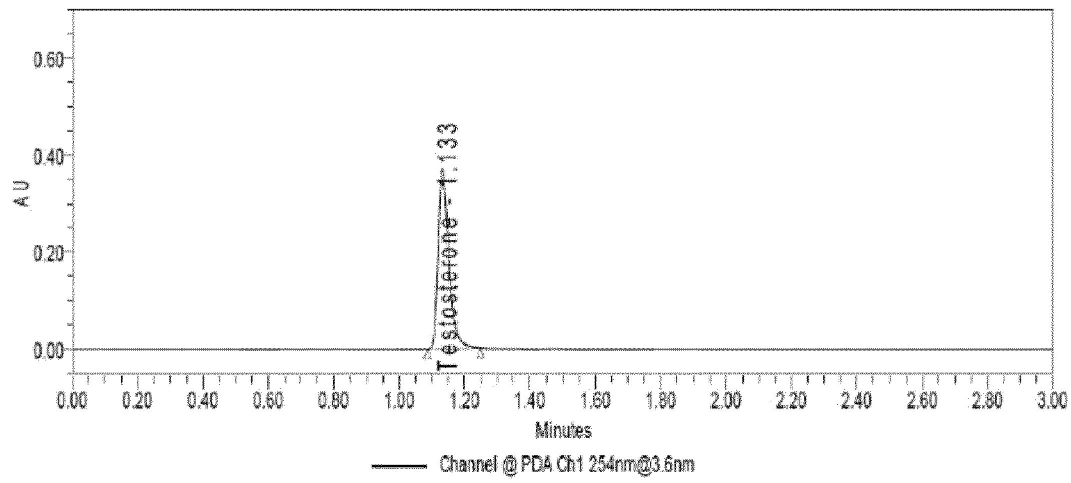
0.60
0.40
0.20
0.00
AU
Testosterone - 1.133
0.00
0.20
0.40
0.60
0.80
1.00
1.20
1.40
1.60
1.80
2.00
2.20
2.40
2.60
2.80
3.00
Minutes
Channel @ PDA Ch1 254nm@3.6nm

FIG. 37

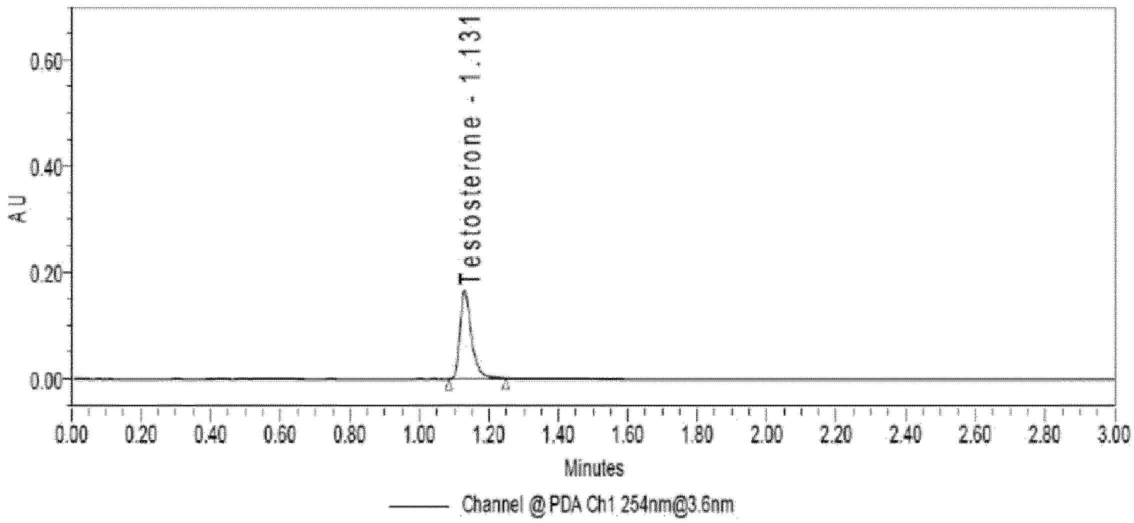
0.60
0.40
0.20
0.00
AU
Testosterone - 1.131
0.00
0.20
0.40
0.60
0.80
1.00
1.20
1.40
1.60
1.80
2.00
2.20
2.40
2.60
2.80
3.00
Minutes
Channel @ PDA Ch1 254nm@3.6nm

FIG. 38

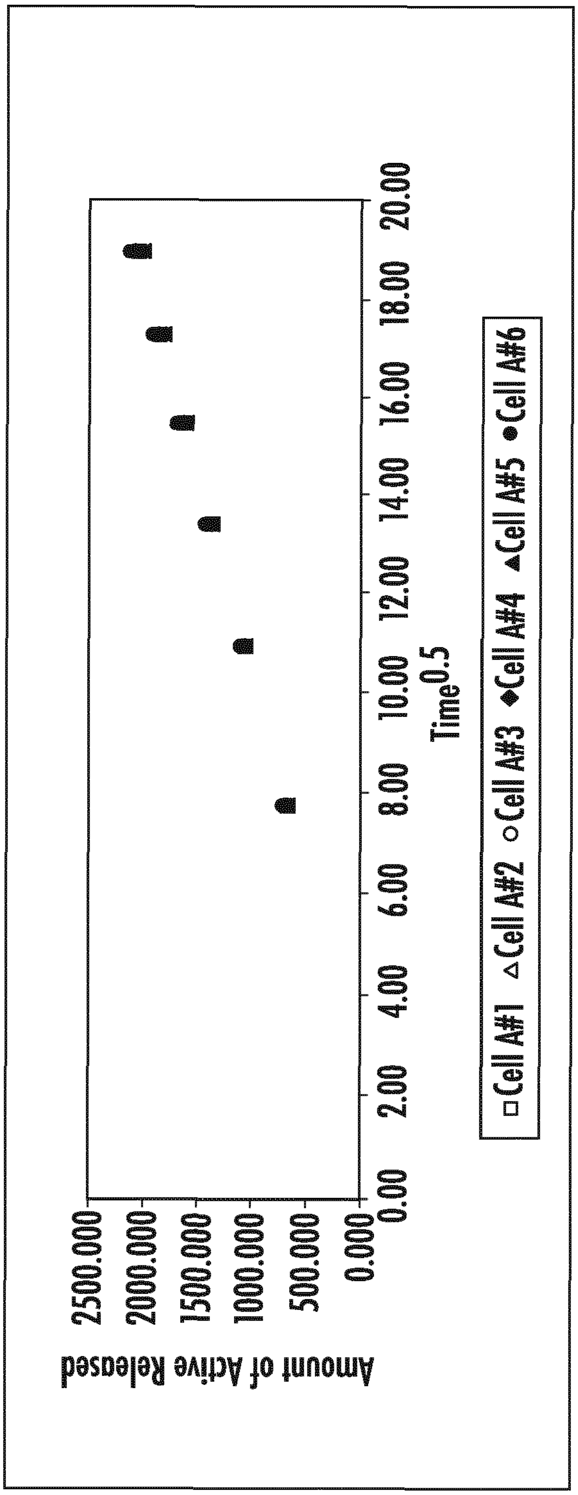
Amount of Active Released
2500.000
2000.000
1500.000
1000.000
500.000
0.000
0.00
2.00
4.00
6.00
8.00
10.00
12.00
14.00
16.00
18.00
20.00
Time0.5
□Cell A#1 △Cell A#2 ○Cell A#3 ◆Cell A#4 ▲Cell A#5 ●Cell A#6

FIG. 39

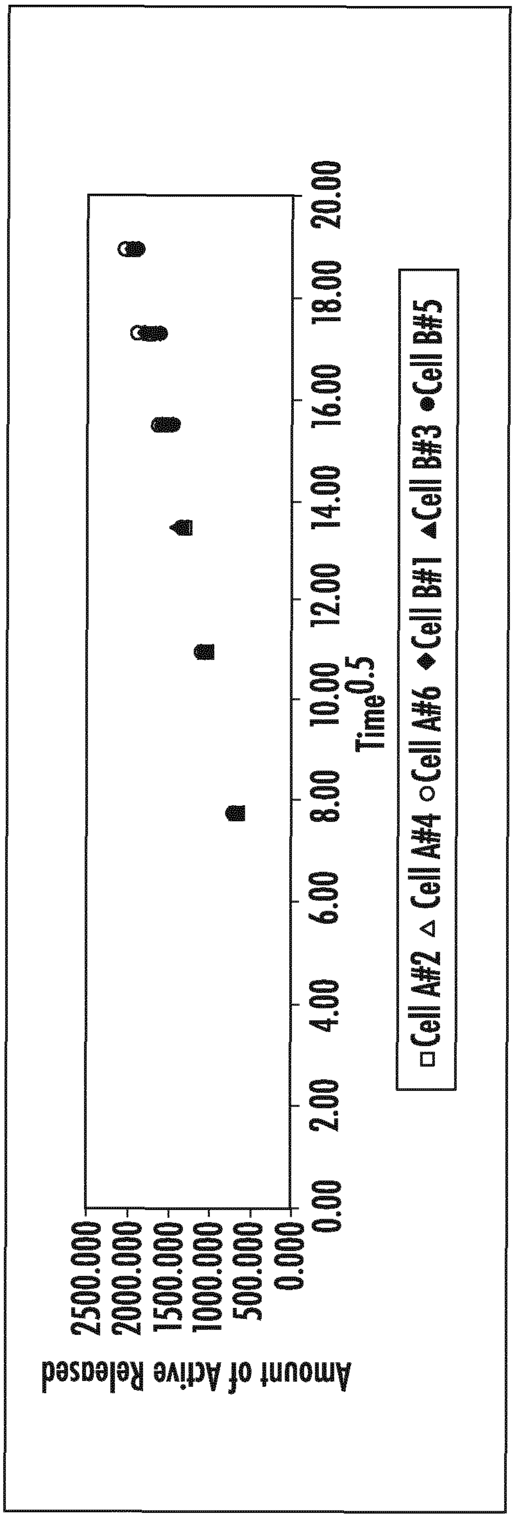
Amount of Active Released
2500.000
2000.000
1500.000
1000.000
500.000
0.000
0.00
2.00
4.00
6.00
8.00
10.00
12.00
14.00
16.00
18.00
20.00
Time^0.5
□Cell A#2 △ Cell A#4 ○Cell A#6 ◆Cell B#1 ▲Cell B#3 ●Cell B#5

FIG. 40

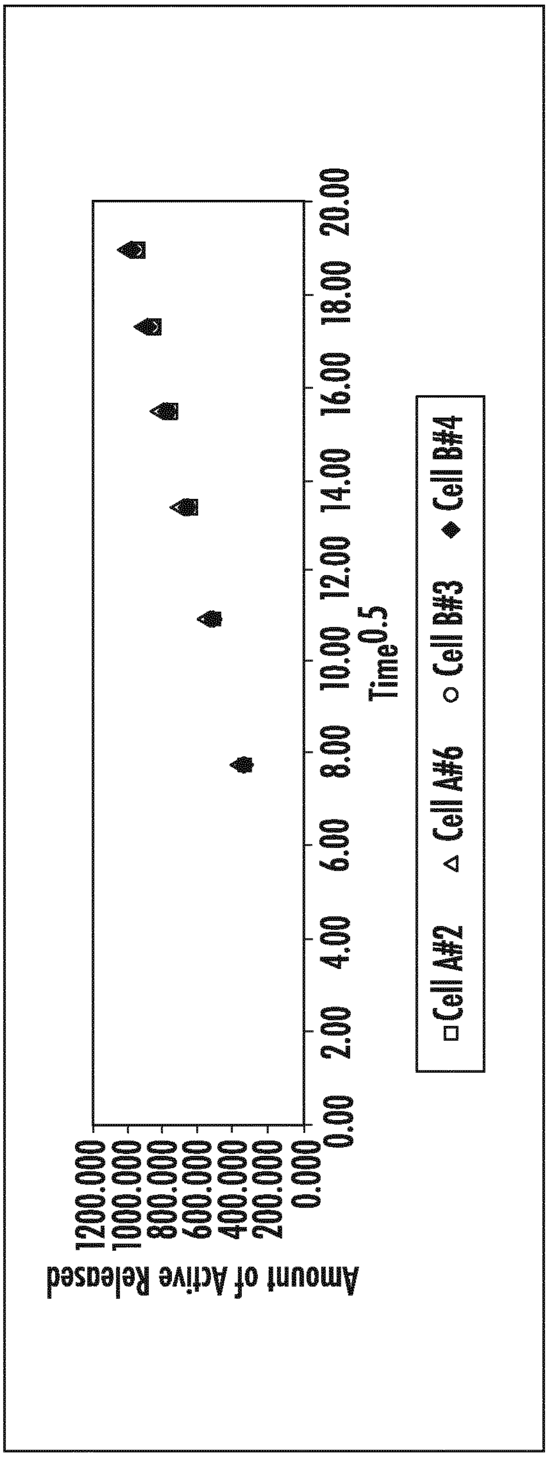
Amount of Active Released
1200.000
1000.000
800.000
600.000
400.000
200.000
0.000
0.00
2.00
4.00
6.00
8.00
10.00
12.00
14.00
16.00
18.00
20.00
Time0.5
Cell A#2
Cell A#6
Cell B#3
Cell B#4

FIG. 41

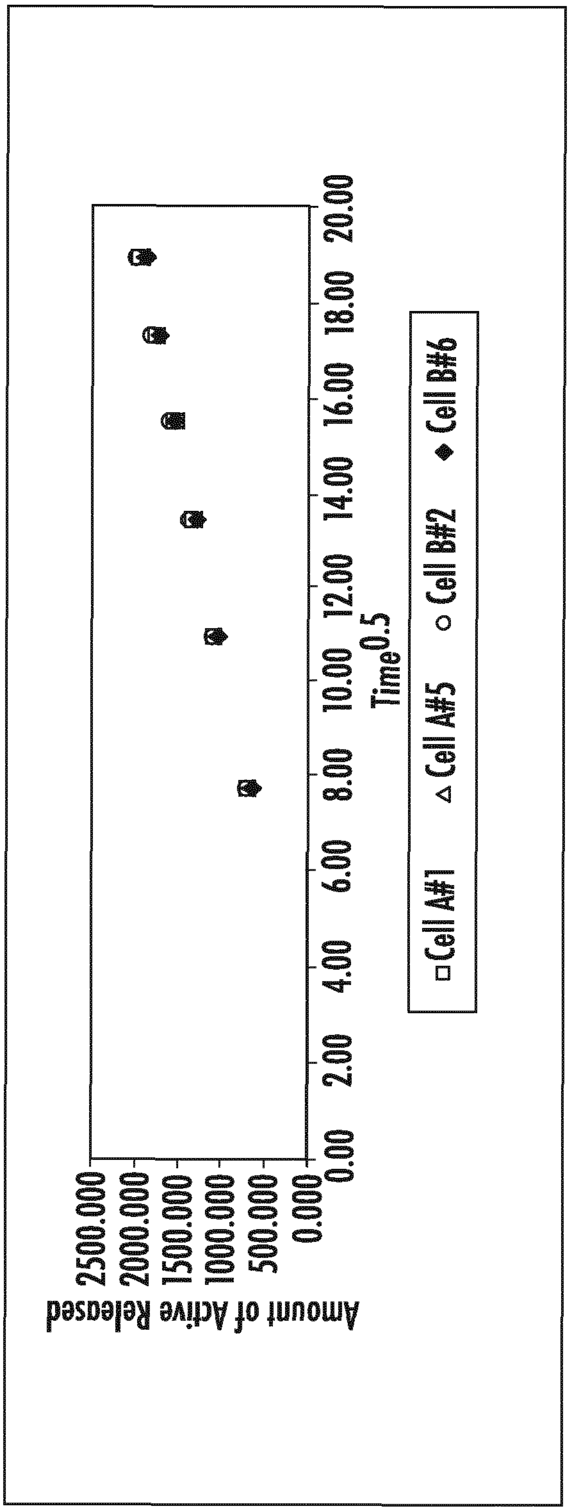
Amount of Active Released
2500.000
2000.000
1500.000
1000.000
500.000
0.000
0.00
2.00
4.00
6.00
8.00
10.00
12.00
14.00
16.00
18.00
20.00
Time0.5
Cell A#1
Cell A#5
Cell B#2
Cell B#6

FIG. 42

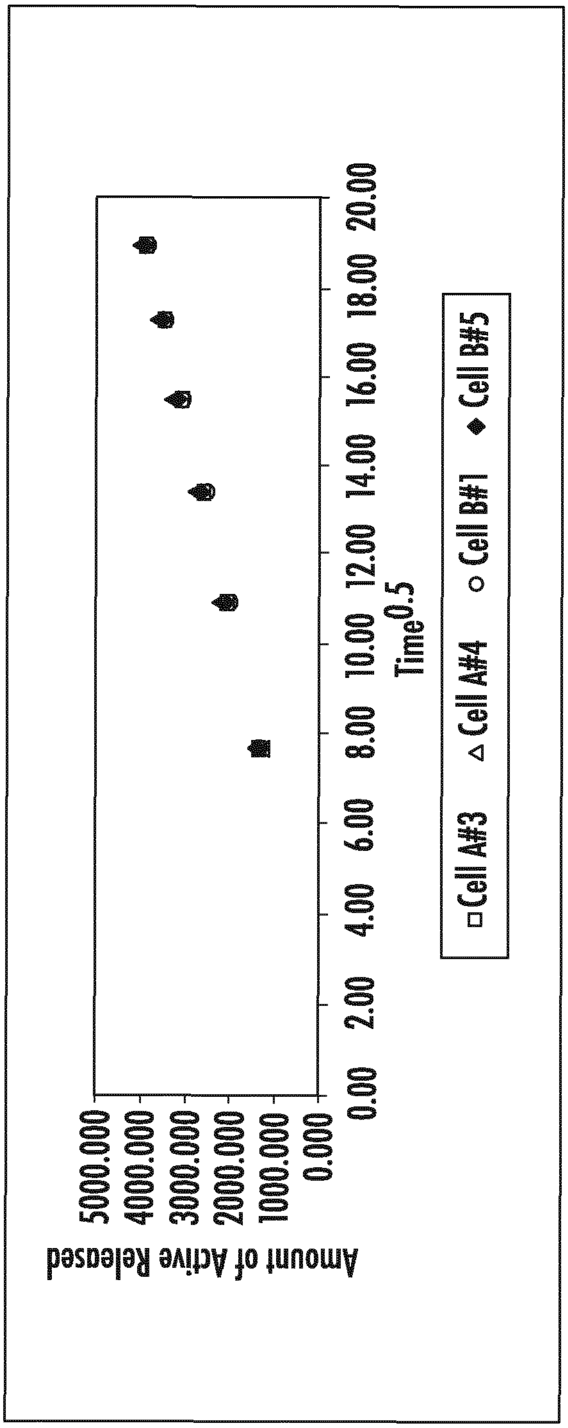
Amount of Active Released
5000.000
4000.000
3000.000
2000.000
1000.000
0.000
0.00
2.00
4.00
6.00
8.00
10.00
12.00
14.00
16.00
18.00
20.00
Time^0.5
Cell A#3
Cell A#4
Cell B#1
Cell B#5

FIG. 43

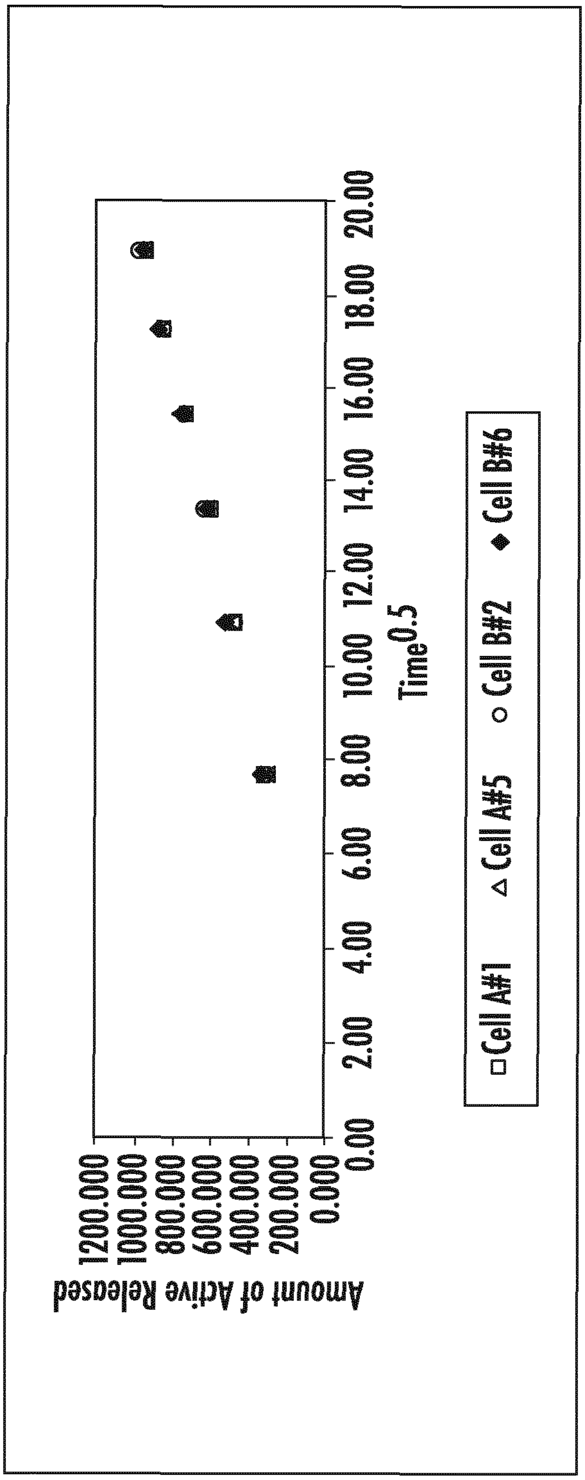
Amount of Active Released
1200.000
1000.000
800.000
600.000
400.000
200.000
0.000
0.00
2.00
4.00
6.00
8.00
10.00
12.00
14.00
16.00
18.00
20.00
Time$^{0.5}$
□ Cell A#1
△ Cell A#5
○ Cell B#2
◆ Cell B#6

FIG. 44

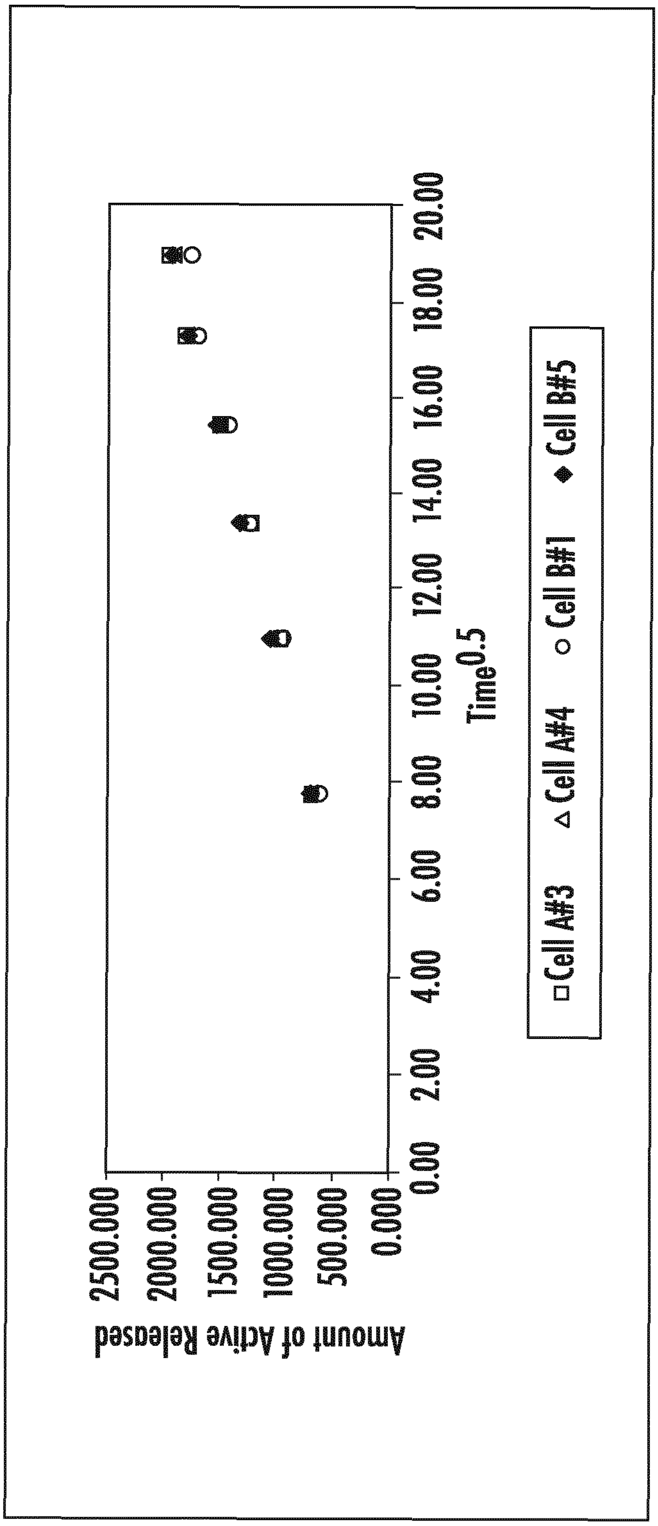
Amount of Active Released
2500.000
2000.000
1500.000
1000.000
500.000
0.000
0.00
2.00
4.00
6.00
8.00
10.00
12.00
14.00
16.00
18.00
20.00
Time0.5
Cell A#3
Cell A#4
Cell B#1
Cell B#5

FIG. 45

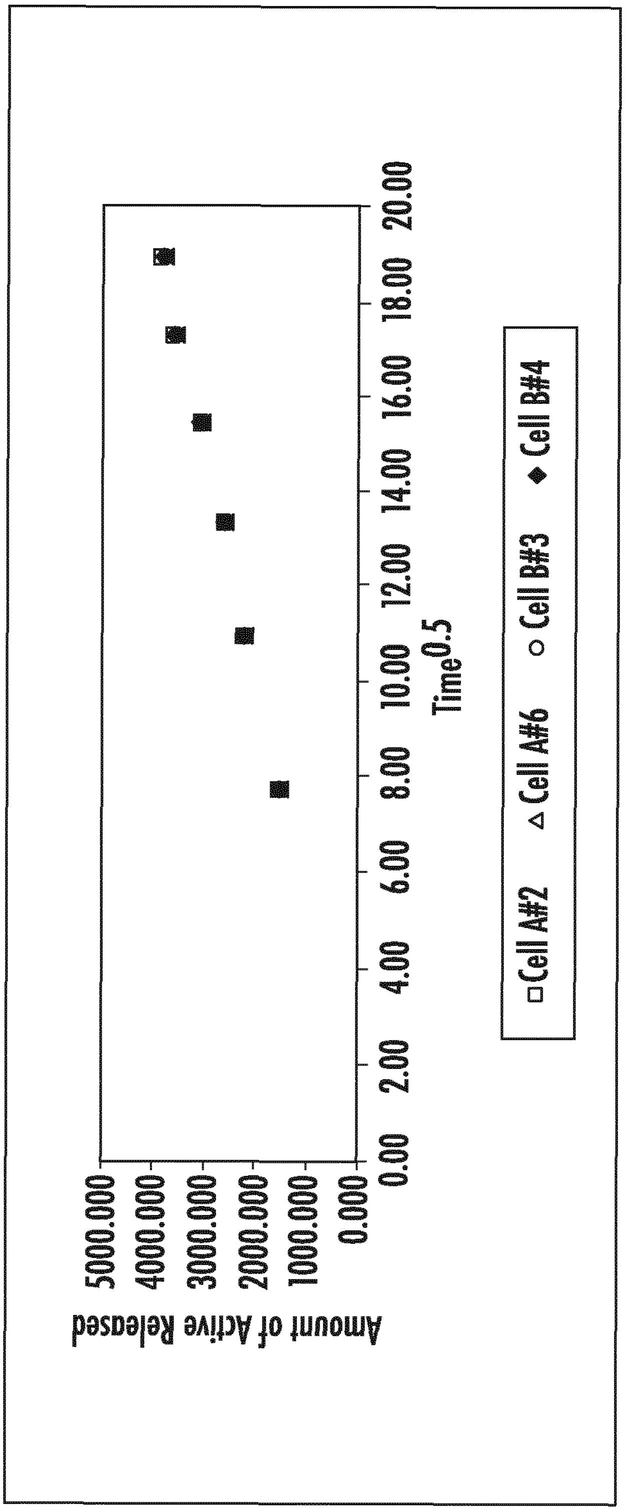
Amount of Active Released
5000.000
4000.000
3000.000
2000.000
1000.000
0.000
0.00
2.00
4.00
6.00
8.00
10.00
12.00
14.00
16.00
18.00
20.00
Time0.5
□Cell A#2
△ Cell A#6
○ Cell B#3
◆ Cell B#4

FIG. 46

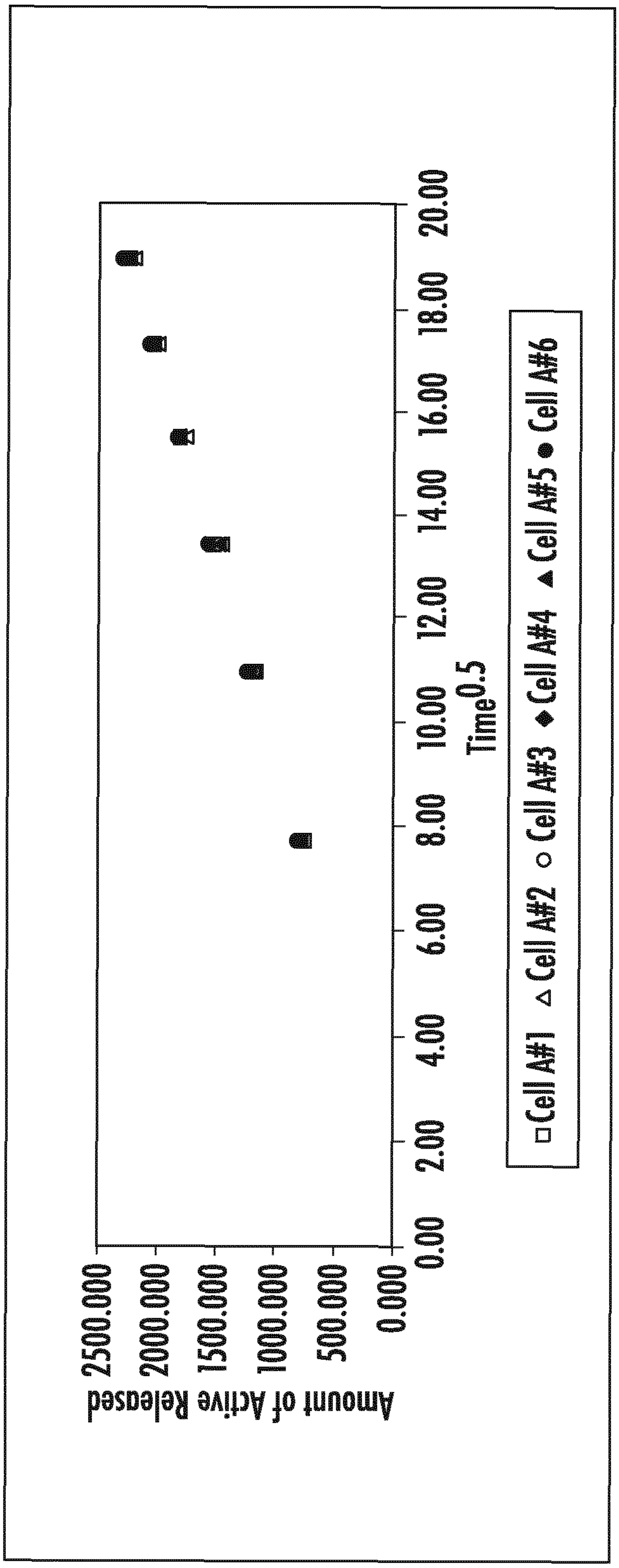
Amount of Active Released
2500.000
2000.000
1500.000
1000.000
500.000
0.000
0.00
2.00
4.00
6.00
8.00
10.00
12.00
14.00
16.00
18.00
20.00
Time0.5
Cell A#1
Cell A#2
Cell A#3
Cell A#4
Cell A#5
Cell A#6

FIG. 47

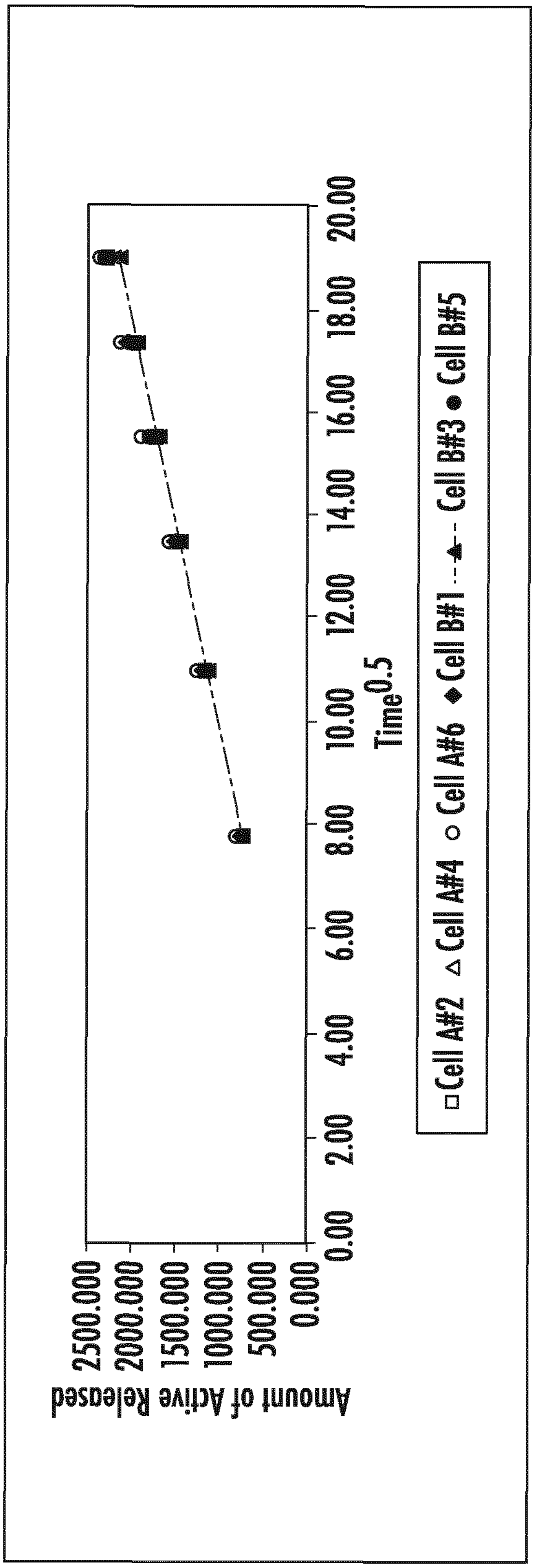
Amount of Active Released
2500.000
2000.000
1500.000
1000.000
500.000
0.000
0.00
2.00
4.00
6.00
8.00
10.00
12.00
14.00
16.00
18.00
20.00
Time0.5
Cell A#2
Cell A#4
Cell A#6
Cell B#1
Cell B#3
Cell B#5

FIG. 48

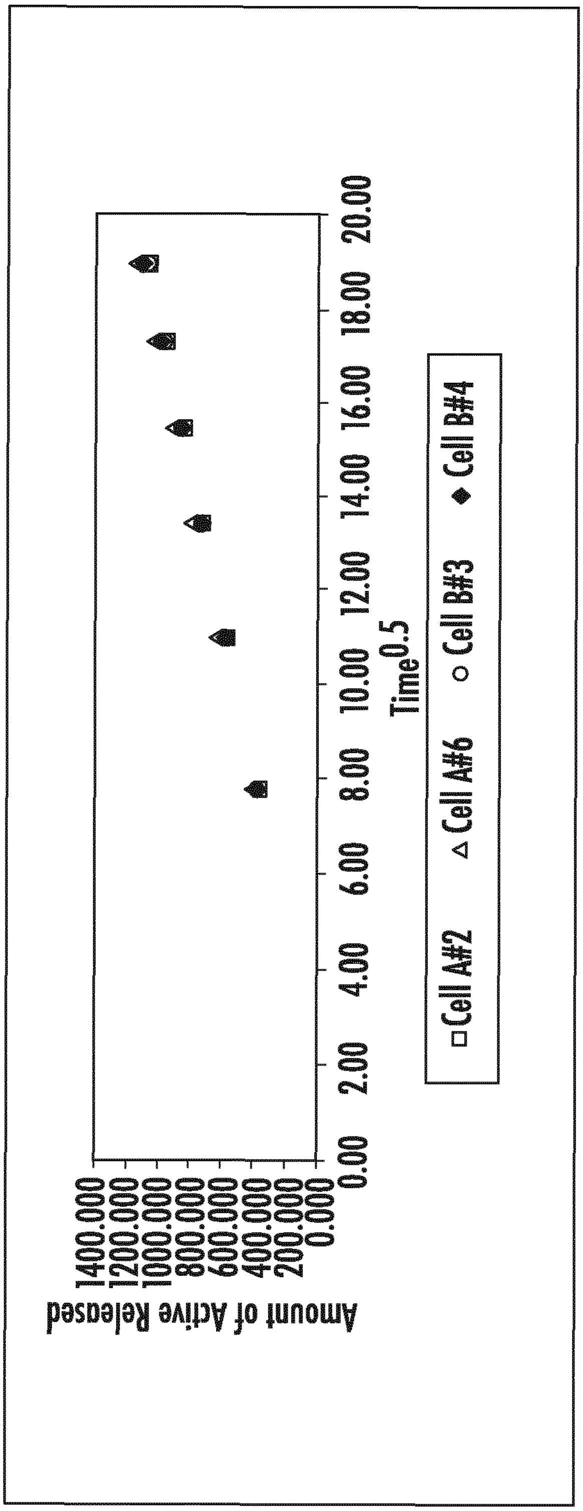
Amount of Active Released
1400.000
1200.000
1000.000
800.000
600.000
400.000
200.000
0.000
0.00
2.00
4.00
6.00
8.00
10.00
12.00
14.00
16.00
18.00
20.00
Time0.5
Cell A#2
Cell A#6
Cell B#3
Cell B#4

FIG. 49

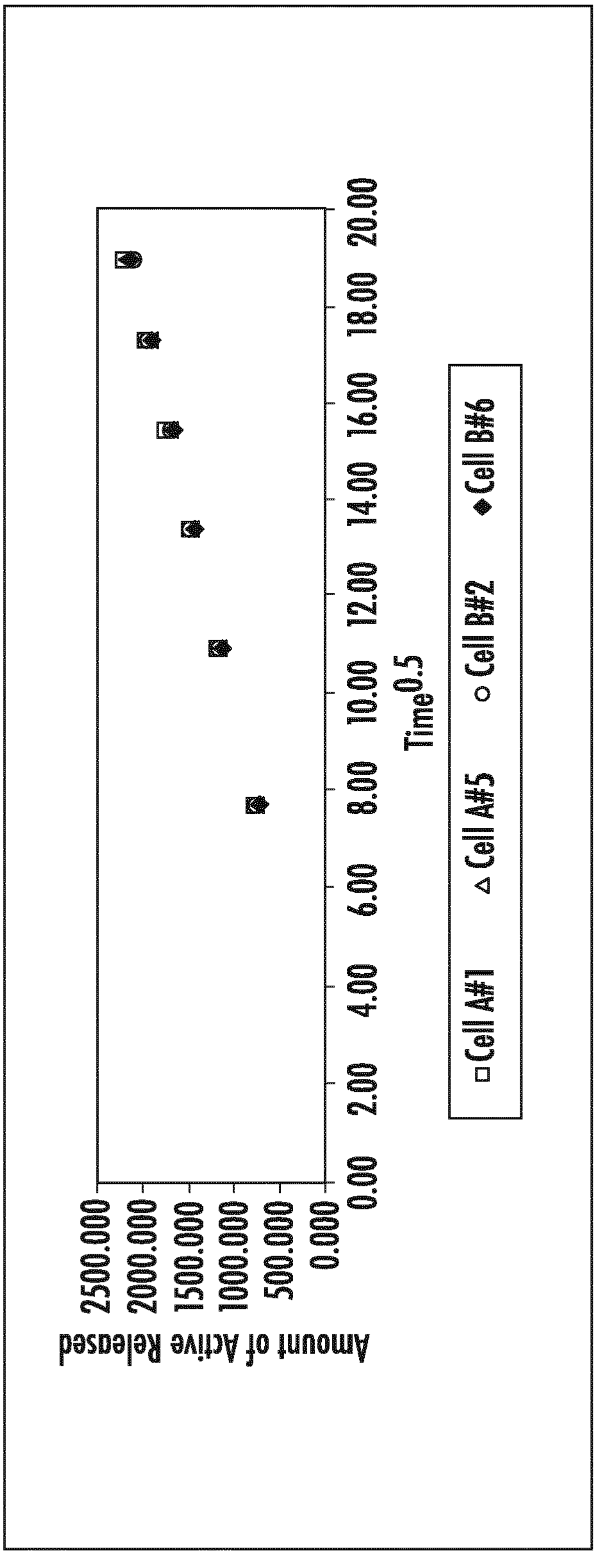

Amount of Active Released
2500.000
2000.000
1500.000
1000.000
500.000
0.000
0.00
2.00
4.00
6.00
8.00
10.00
12.00
14.00
16.00
18.00
20.00
Time0.5
□ Cell A#1
△ Cell A#5
○ Cell B#2
◆ Cell B#6


FIG. 50

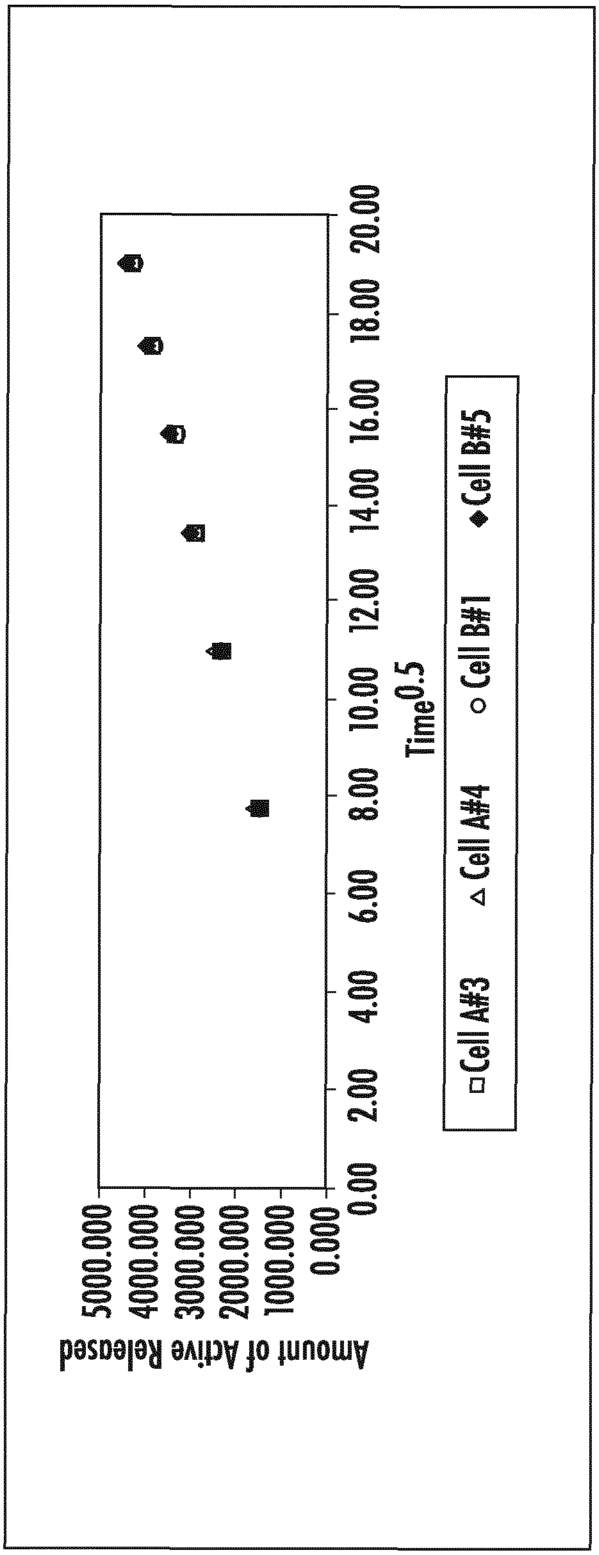

Amount of Active Released
5000.000
4000.000
3000.000
2000.000
1000.000
0.000
0.00
2.00
4.00
6.00
8.00
10.00
12.00
14.00
16.00
18.00
20.00
Time0.5
□ Cell A#3
△ Cell A#4
○ Cell B#1
◆ Cell B#5


FIG. 51

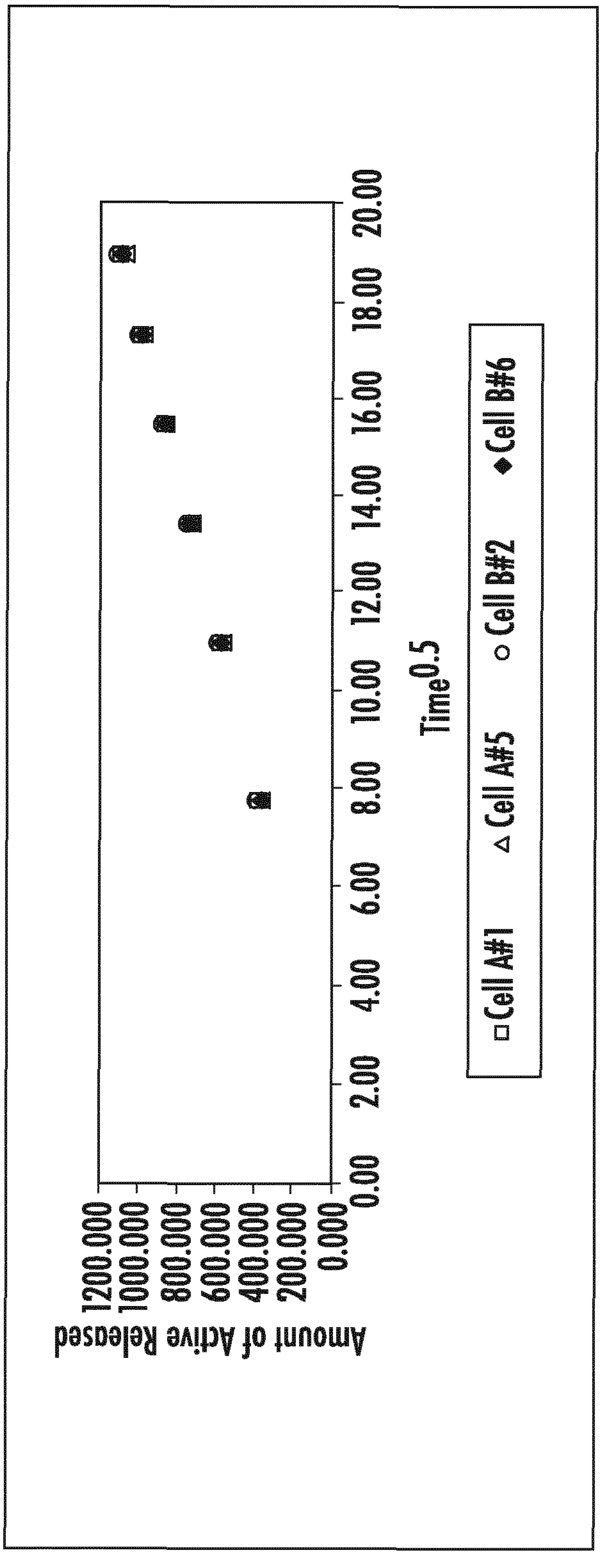
Amount of Active Released
1200.000
1000.000
800.000
600.000
400.000
200.000
0.000
0.00
2.00
4.00
6.00
8.00
10.00
12.00
14.00
16.00
18.00
20.00
Time0.5
□ Cell A#1
△ Cell A#5
○ Cell B#2
◆ Cell B#6

FIG. 52

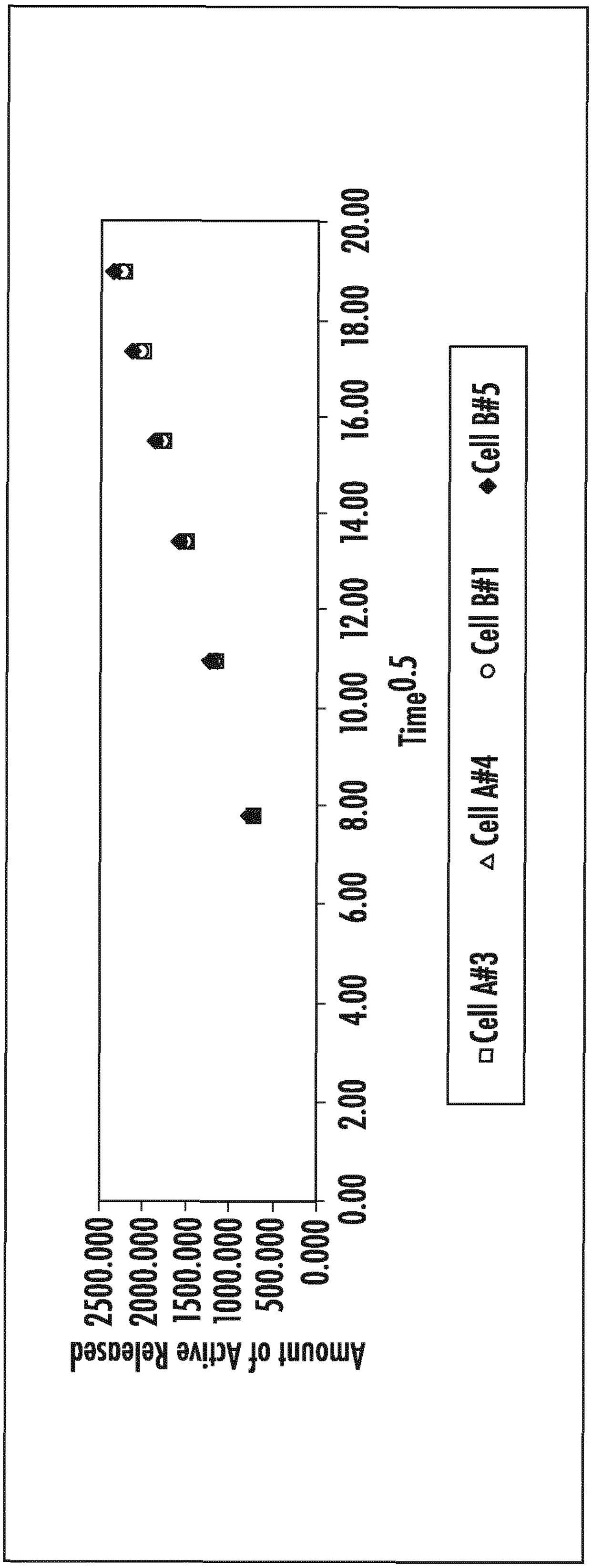
Amount of Active Released
2500.000
2000.000
1500.000
1000.000
500.000
0.000
0.00
2.00
4.00
6.00
8.00
10.00
12.00
14.00
16.00
18.00
20.00
Time0.5
Cell A#3
Cell A#4
Cell B#1
Cell B#5

FIG. 53

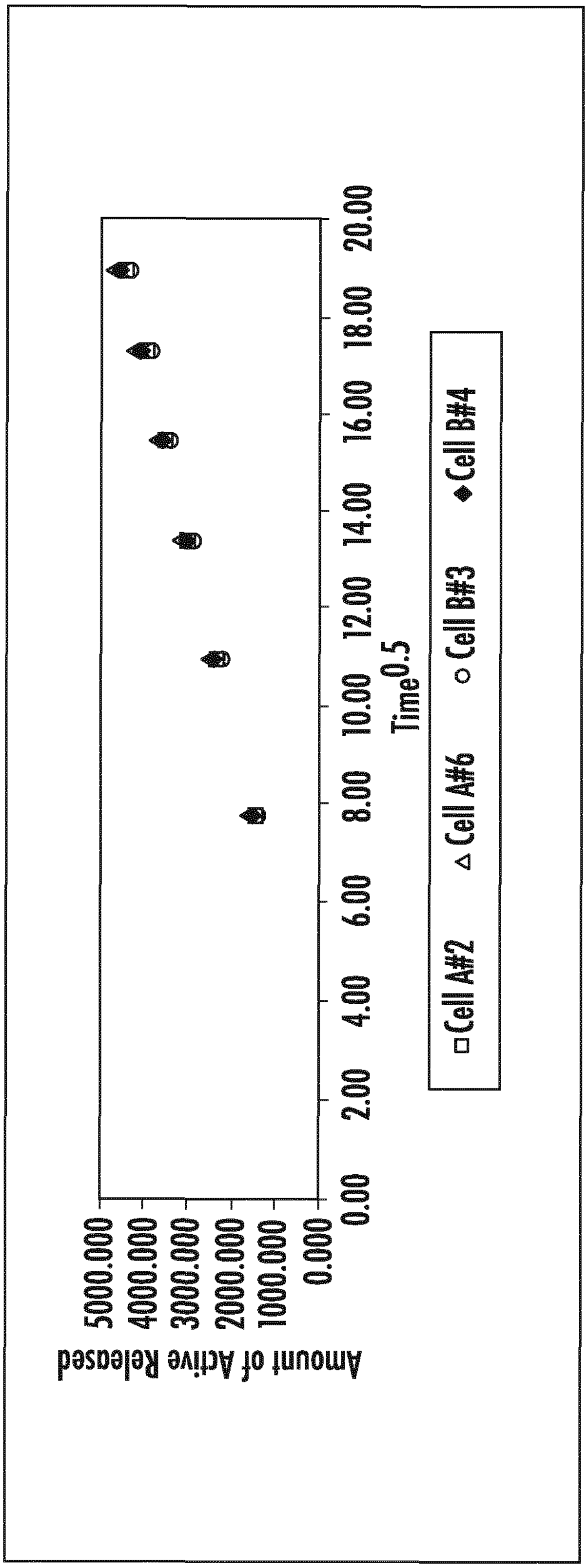
Amount of Active Released
5000.000
4000.000
3000.000
2000.000
1000.000
0.000
0.00
2.00
4.00
6.00
8.00
10.00
12.00
14.00
16.00
18.00
20.00
Time0.5
Cell A#2
Cell A#6
Cell B#3
Cell B#4

FIG. 54

Europäisches Patentamt
European Patent Office
Office européen des brevets

# EUROPEAN SEARCH REPORT

Application Number
EP 21 19 1386

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/311707 A1 (MATTERN CLAUDIA [CH]) 9 December 2010 (2010-12-09)<br>* paragraphs [0002], [0039] - [0043], [0052] - [0053] *<br>* figures 1-3 *<br>----- | 1,3,7, 10-13 | INV.<br>A61K9/00<br>A61K9/06<br>A61K31/568<br>A61K47/34<br>A61K47/44<br>A61P5/26 |
| A | EP 2 068 825 A1 (M & P PATENT AG [LI]) 17 June 2009 (2009-06-17)<br>* page 3, paragraph 17-25 *<br>----- | 1-13 | |
| X | CLAUDIA MATTERN ET AL: "Testosterone supplementation for hypogonadal men by the nasal route",<br>THE AGING MALE,<br>vol. 11, no. 4, 2008, pages 171-178,<br>XP008156424,<br>* page 1, paragraph 2 *<br>----- | 1-13 | |

TECHNICAL FIELDS SEARCHED (IPC)
A61K
A61P

1 The present search report has been drawn up for all claims

| Place of search | Date of completion of the search | Examiner |
|---|---|---|
| The Hague | 15 February 2022 | Siebum, Bastiaan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 21 19 1386

This annex lists the patent family members relating to the patent documents cited in the above-mentioned European search report. The members are as contained in the European Patent Office EDP file on
The European Patent Office is in no way liable for these particulars which are merely given for the purpose of information.

15-02-2022

| Patent document cited in search report | Publication date | Patent family member(s) | Publication date |
|---|---|---|---|
| US 2010311707 A1 | 09-12-2010 | AR 047722 A1 | 15-02-2006 |
| | | AT 319426 T | 15-03-2006 |
| | | AU 2004286781 A1 | 19-05-2005 |
| | | BR PI0416370 A | 22-05-2007 |
| | | CA 2541498 A1 | 19-05-2005 |
| | | DE 60303854 T2 | 10-08-2006 |
| | | DK 1530965 T3 | 17-07-2006 |
| | | EP 1530965 A1 | 18-05-2005 |
| | | ES 2258694 T3 | 01-09-2006 |
| | | HR P20060147 A2 | 30-11-2006 |
| | | JP 4616270 B2 | 19-01-2011 |
| | | JP 2007530446 A | 01-11-2007 |
| | | KR 20060109903 A | 23-10-2006 |
| | | MY 131483 A | 30-08-2007 |
| | | NO 338665 B1 | 26-09-2016 |
| | | PL 219338 B1 | 30-04-2015 |
| | | PT 1530965 E | 31-05-2006 |
| | | RU 2331423 C2 | 20-08-2008 |
| | | SI 1530965 T1 | 30-06-2006 |
| | | TW 200515918 A | 16-05-2005 |
| | | US 2005100564 A1 | 12-05-2005 |
| | | US 2007149454 A1 | 28-06-2007 |
| | | US 2010311707 A1 | 09-12-2010 |
| | | US 2012009250 A1 | 12-01-2012 |
| | | US 2012058176 A1 | 08-03-2012 |
| | | US 2012083480 A1 | 05-04-2012 |
| | | WO 2005044273 A1 | 19-05-2005 |
| | | ZA 200603115 B | 26-09-2007 |
| EP 2068825 A1 | 17-06-2009 | AT 496617 T | 15-02-2011 |
| | | AU 2007304471 A1 | 10-04-2008 |
| | | BR PI0717509 A2 | 17-05-2016 |
| | | CA 2664427 A1 | 10-04-2008 |
| | | CN 101600417 A | 09-12-2009 |
| | | DK 2068825 T3 | 16-05-2011 |
| | | EP 2068825 A1 | 17-06-2009 |
| | | ES 2358619 T3 | 12-05-2011 |
| | | HK 1137658 A1 | 06-08-2010 |
| | | JP 5248510 B2 | 31-07-2013 |
| | | JP 2010505778 A | 25-02-2010 |
| | | KR 20090098958 A | 18-09-2009 |
| | | NO 339802 B1 | 06-02-2017 |
| | | PL 2068825 T3 | 30-06-2011 |
| | | PT 2068825 E | 11-02-2011 |
| | | RU 2009116609 A | 10-11-2010 |
| | | US 2009227550 A1 | 10-09-2009 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO.

EP 21 19 1386

This annex lists the patent family members relating to the patent documents cited in the above-mentioned European search report. The members are as contained in the European Patent Office EDP file on
The European Patent Office is in no way liable for these particulars which are merely given for the purpose of information.

15-02-2022

| Patent document cited in search report | Publication date | Patent family member(s) | Publication date |
|---|---|---|---|
| | | US 2012009249 A1 | 12-01-2012 |
| | | US 2016089347 A1 | 31-03-2016 |
| | | US 2018200203 A1 | 19-07-2018 |
| | | WO 2008040488 A1 | 10-04-2008 |
| | | ZA 200902902 B | 28-07-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 12738595
- GB 1987000012176 A
- GB 9801147 W
- US 5578588 A
- US 5756071 A
- US 20050100564 A
- US 20070149454 A
- US 20090227550 A
- US 5576071 A


### Non-patent literature cited in the description


- **HUSSAIN et al.** Testosterone 17β-N,N-dimethylglyc-inate hydrochloride: A prodrug with a potential for nasal delivery of testosterone. *J. Pharmaceut. Sci.,* 2002, vol. 91 (3), 785-789
- **KO et al.** Emulsion formulations of testosterone for nasal administration. *J. Microencaps.,* 1998, vol. 15 (2), 197-205
- **CICINELLI et al.** Progesterone administration by nasal spray. *Fertil Steril,* 1991, vol. 56 (1), 139-141
- Nasally-administered progesterone: comparison of ointment and spray formulations. *Maturitas,* 1991, vol. 13 (4), 313-317
- Progesterone administration by nasal sprays in menopausal women: comparison between two different spray formulations. *Gynecol Endocrinol,* 1992, vol. 6 (4), 247-251
- Effects of the repetitive administration of progesterone by nasal spray in postmenopausal women. *Fertil Steril,* 1993, vol. 60 (6), 1020-1024
- Nasal spray administration of unmodified progesterone: evaluation of progesterone serum levels with three different radioimmunoassay techniques. *Maturitas,* 1994, vol. 19 (1), 43-52
- **STEEGE et al.** Bioavailability of nasally administered progesterone. *Fertil Steril,* 1986, vol. 46 (4), 727-729
- **KUMAR et al.** Pharmacokinetics of progesterone after its administration to ovariectomized rhesus monkeys by injection, infusion, or nasal spraying. *Proc. Natl. Acad. Sci. U.S.A.,* 1982, vol. 79, 4185-9
- **PROVASI et al.** Nasal delivery progesterone powder formulations comparison with oral administration. *Boll. Chim. Farm.,* 1993, vol. 132 (10), 402-404
- **HE JUNGINGER et al.** Mucoadhesive hydrogels in drug delivery. *Encyclopedia Pharm Technol,* 2002
- **SJ JACKSON ; AC PERKINS.** In vitro assessment of the mucoadhesion of cholestyramine to porcine and human gastric mucosa. *Eur J Pharm Biopharm.,* 2001, vol. 52, 121-127
- Nasobol. *Study of HET-CAM,* 2004
- *Local tolerance, single application of "Nasobol" (study report 208040401) plus histopathology report,* 2004
- *Local tolerance, multiple application of "Nasobol" during 2 weeks (study report 208040402) plus histopathology report (analysis number 208040402),* 2004
- *Repeat-dose toxicity study of "Nasobol" (study number 227120417),* 2005